# EUROPEAN PATENT APPLICATION

(11) **EP 4 653 448 A1**
(43) Date of publication of application: **26.11.2025**
(21) Application number: 24744414.4
(22) Date of filing: 19.01.2024
(51) Int. Cl.: C07H 19/20, C07H 1/00, A61K 31/7125, C12N 15/10, C12P 19/34

(54) **COMPOUND FOR RNA CAPPING AND USE THEREOF**

(30) Priority: 19.01.2023 CN 202310061987
(71) Applicant: Guangzhou Henovcom Bioscience Co. Ltd., Guangzhou, Guangdong 510700 (CN)
(72) Inventor: ZHANG, Jiancun, Guangzhou, Guangdong 510535 (CN); ZHANG, Lijun, Guangzhou, Guangdong 510535 (CN); ZHOU, Yiqian, Guangzhou, Guangdong 510535 (CN); CHEN, Jiafeng, Guangzhou, Guangdong 510535 (CN); GUO, Chen, Guangzhou, Guangdong 510535 (CN); LIU, Yanhui, Guangzhou, Guangdong 510535 (CN); ZHANG, Jufu, Guangzhou, Guangdong 510535 (CN); LI, Suyong, Guangzhou, Guangdong 510535 (CN); HE, Xiaoxi, Guangzhou, Guangdong 510535 (CN); GUO, Qi, Guangzhou, Guangdong 510535 (CN); TANG, Wanjun, Guangzhou, Guangdong 510535 (CN); CHEN, Huixuan, Guangzhou, Guangdong 510535 (CN); WU, Feng, Guangzhou, Guangdong 510535 (CN); WANG, Kun, Guangzhou, Guangdong 510535 (CN); LI, Deyao, Guangzhou, Guangdong 510535 (CN)
(74) Representative: Michalski Hüttermann & Partner Patentanwälte mbB
(86) International application number: PCT/CN2024/073369
(87) International publication number: WO 2024/153245

(57) **Abstract**

The present invention relates to the technical field of genetic engineering, and relates to two types of compounds for RNA capping and uses thereof. The compound has a structure as represented by formula (I), can be used as an initiating capped oligonucleotide primer to perform mRNA 5' end capping, and has good capping efficiency and protein expression level; and additionally, because the compound is labelled with a fluorophore, the compound can also be used for detecting and tracking dynamic changes of mRNA in cells and organisms, facilitates researches of a delivery process, pharmacokinetic performance, etc. of mRNA in the cells and organisms, and provides convenience for research of mRNA vaccines/drugs in the cells or organisms.

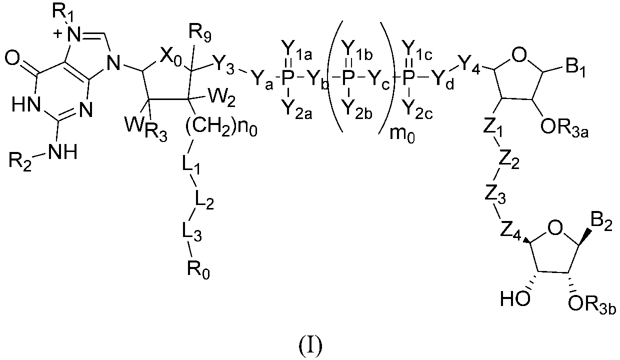

## Description

### SPECIFICATION

The present application claims the right of priority of Chinese Patent Application No. 202310061987.7, filed on January 19, 2023. The contents of the above Chinese patent applications are incorporated herein by reference in its entirety.

### TECHNICAL FIELD

The present disclosure relates to the technical field of genetic engineering, in particular to a compound for RNA capping and use thereof.

### BACKGROUND

The 5' cap structure (five-prime cap) (m7GpppN) of messenger ribonucleic acid (mRNA) was discovered in the 1970s, and its presence confers stability to the mRNA and enables efficient translation. There are usually three types of cap structures (m7G5'ppp5'Np, m7G5'ppp5'NmpNp, m7G5'ppp5'NmpNmpNp), which are referred to as type O (m7G5'ppp5'Np), type I (m7G5'ppp5'NmpNp) and type II (m7G5'ppp5'NmpNmpNp), respectively. Type O means that the ribose of the terminal nucleotide is unmethylated, type I means that the ribose of one terminal nucleotide is methylated, and type II means that the ribose of two terminal nucleotides is methylated.

In eukaryotic cells, in addition to recognizing the initiation of protein synthesis, the 5' cap structure also serves as a protective group for exonuclease cleavage from 5' to 3', that is, it is resistant to degradation by 5'-exonucleases. Additionally, during protein synthesis, the cap structure is also a unique identifier that recruits protein factors involved in pre-mRNA splicing, polyadenylation and nuclear export. It also functions as an anchor for recruiting initiation factors, facilitating ribosomes recognition and binding to mRNA, thereby ensuring proper translation initiation.

The outbreak of the pandemic has greatly advanced research on mRNA vaccines/drugs, among which the cap structure serves as an indispensable raw material. Although many studies have been conducted on cap structures, currently there are very few cap raw materials that can be widely applied. Therefore, it is necessary to provide more high-performance caps for selection, thereby promoting the faster development of mRNA vaccines/drugs without being constrained by cap structures. Hence, cap structures with high capping rate, as well as improved stability, expression levels, immunogenicity, and other properties of the capped mRNA, have tremendous application prospects and value.

In addition, in order to understand the biological processes involving exogenous RNA within cells-such as delivery, translation, migration and localization, splicing, and degradation-it is necessary to visualize the life processes of RNA molecules in cells for researchers. By integrating imaging technologies, real-time dynamic tracking of RNA molecules within cells is achieved. To achieve this objective, RNA typically requires labeling. Currently, *in vitro* transcribed mRNA is usually labeled using nucleotide triphosphates (NPTs) modified with fluorophores. However, nucleoside triphosphates modified with bulky fluorophores at spatial positions are difficult for RNA polymerase to recognize and incorporate into mRNA, leading to reduced transcription efficiency and yield. These modifications may also easily affect the stability and translation efficiency of mRNA prepared by *in vitro* transcription.

Since the cap structure of mRNA is essential for the stability and translational expression of mRNA, existing research has primarily focused on aspects such as capping rate, immunogenicity and mRNA expression levels. There has been no related research on the labeling effect of the cap. Therefore, a cap structure that retains its original function while also having a labeling effect has great application value and is necessary for further research.

### SUMMARY

Based on the importance of the 5' cap structure and the substantial unmet market demand, the present disclosure provides a compound for RNA capping. When employed for mRNA 5' capping, this compound not only exhibits good capping efficiency but also significantly enhances the performance of capped mRNA in terms of stability and expression levels. This compound exhibits favorable performance in *in vitro* conditions or in animals, outperforming positive controls.

Additionally, the present disclosure further provides a fluorophore-labeled compound for RNA capping. This compound not only maintains its original excellent performance (such as good capping rate, as well as good stability, expression levels, and other properties of the capped mRNA), but also can be utilized for detection and tracking of dynamic changes of mRNA in cells or organisms due to its fluorophore labeling. This facilitates the study of delivery processes, pharmacokinetics, and other properties of mRNA in cells or organisms, thereby providing convenience for the research of mRNA vaccines/drugs.

One objective of the present disclosure is to provide a compound having a structure represented by formula I, or a stereoisomer, pharmaceutically acceptable salt or solvate thereof: 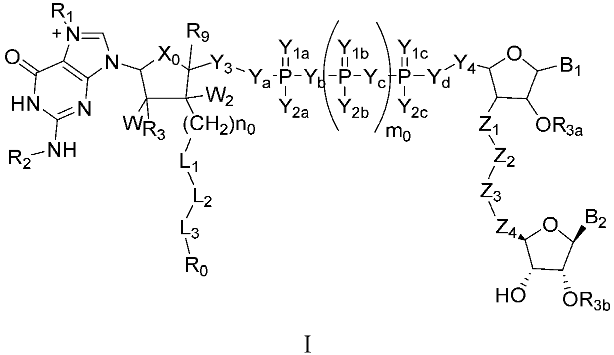 wherein
R₁ is C₁-C₆ alkyl, C₂-C₆ alkenyl, C₂-C₆ alkynyl, C₃-C₆ cycloalkyl, C₃-C₆ cycloalkenyl, benzyl, R₅-substituted C₁-C₆ alkyl, R₅-substituted C₂-C₆ alkenyl, R₅-substituted C₂-C₆ alkynyl, R₅-substituted C₃-C₆ cycloalkyl, R₅-substituted C₃-C₆ cycloalkenyl or R₅-substituted benzyl;
R₂ is H, C₁-C₆ alkyl, C₂-C₆ alkenyl, C₂-C₆ alkynyl, C₃-C₈ cycloalkyl, cycloalkenyl, benzyl, aryl, heteroaryl, R₅-substituted benzyl, R₅-substituted aryl, carbonylalkyl, carbonylalkoxy or sulfonamido;
R₃ is H, OH, C₁-C₆ alkyl, C₂-C₆ alkenyl, C₂-C₆ alkynyl, R₅-substituted C₁-C₆ alkyl, R₅-substituted C₂-C₆ alkenyl, R₅-substituted C₂-C₆ alkynyl, halogen or absent;
R₃ₐ and R_{3b} are each independently H, halogen, C₁-C₆ alkyl, C₂-C₆ alkenyl, C₂-C₆ alkynyl, R₅-substituted C₁-C₆ alkyl, R₅-substituted C₂-C₆ alkenyl or R₅-substituted C₂-C₆ alkynyl;
R₉ is H, halogen, C₁-C₃ alkyl or R₄-substituted C₁-C₃ alkyl;
R₀ is H, a fluorophore or a steroid structural group;
X₀ is O, S, NR₄, CH₂, CF₂, CHF, CCH₂ or CCF₂;
W is H, OH, OR₄, NR₄R₄, NR₄COR₄, F, Cl, N₃ or CN;
W₂ is H, OH, halogen, N₃, CN, OR₄, NR₄R₄, NR₄COR₄ or C₁-C₄ alkyl;
L₁ is O, S, NH, carbonyl or absent;
L₂ is NH, carbonyl or absent;
L₃ is -X-(CH₂)nNH-, -X-(CH₂)nNH(CH₂)mNH-, -X-(CH₂)nNHCO(CH₂)mNH-, -X-(CH₂)nCONH(CH₂)mNH-, -X-(CH₂)n(OC₂H₄)mNH-, -X-(CH₂)n(OC₂H₄)mOCH₂-, -X-(C(O)CHRaNH)n- or absent; wherein X is NH, O, 5-membered heteroaryl, 6-membered heteroaryl or absent; n and m are each independently an integer from 0 to 10; wherein Ra is a side chain group in an amino acid, and Ra at each occurrence may be the same or different (i.e., within the same substituent, when n ≥ 2, Ra may be the same or different);
Yₐ, Y_{b}, Y_{c} and Y_{d} are each independently O, S, CH₂, CCl₂, CF₂ or NH;
Y₁ₐ, Y_{1b} and Y_{1c} are each independently O or S;
Y₂ₐ, Y_{2b} and Y_{2c} are each independently OH, SH or BH₃;
Y₃ and Y₄ are each independently CH₂ or O;
Z₁ is O, OH, CH₂, S, NR₆, CO or SO₂;
Z₂ and Z₃ are each independently O, NR₆, CHR₇, CHCOOR₇, CHCONR₇R₇, S, CO, SO₂, PO(OH), PO(SH), P(O)VCO₂H or absent; Z₂ may form a ring with an oxygen atom connected to R₃ₐ;
Z₄ is O, CH₂, S, NR₆, CO, SO₂ or absent;
B₁ and B₂ are each independently a natural or modified pyrimidine nucleotide base or a natural or modified purine nucleotide base;
R₄ is H, C₁-C₈ alkyl, C₂-C₈ alkenyl or C₂-C₈ alkynyl;
R₅ is halogen, CN, SO₂, NO₂, D, N₃, C₁-C₈ alkyl, C₂-C₈ alkenyl, C₂-C₈ alkynyl, C₁-C₈ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₆ alkyl, COR₈ or SO₂R₈;
R₇ is H, halogen, C₁-C₈ alkyl, C₂-C₈ alkenyl or C₂-C₈ alkynyl;
R₈ is H, C₁-C₈ alkyl, C₂-C₈ alkenyl or C₂-C₈ alkynyl;
V is C₁-C₄ alkyl;
n₀ is an integer from 0 to 6;
m₀ is 0, 1 or 2.

In some embodiments, Ra is selected from side chain groups in common amino acid structures.

In some embodiments, the compound has a structure represented by formula II, or a stereoisomer, pharmaceutically acceptable salt or solvate of the compound having the structure represented by formula II: 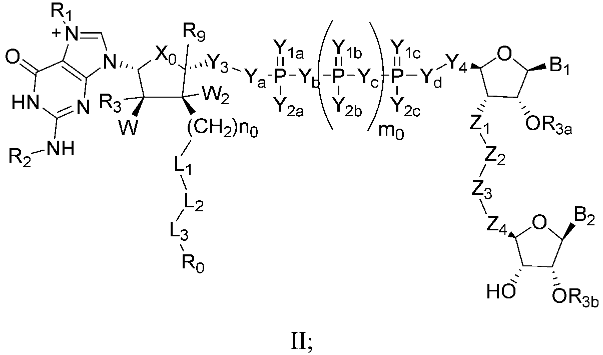 wherein R₁, R₂, R₃, R₃ₐ, R_{3b}, R₄, R₅, R₆, R₇, R₈, R₉, R₀, Ra, L₁, L₂, L₃, X, X₀, W, W₂, Yₐ, Y_{b}, Y_{c}, Y_{d}, Y₁ₐ, Y₁₆, Y_{1c}, Y₂ₐ, Y_{2b}, Y_{2c}, Y₃, Y₄, B₁, B₂, Z₁, Z₂, Z₃, Z₄, V, n₀, m₀, n, and m are as defined above, respectively.

In some embodiments, when R₀ is a fluorophore, the fluorophore is a fluorophore formed by any one of the following dyes: biotin, Cy3, Cy5, Cy7, Cy5.5, DY-776, DY-751, DY-647P1, AF647, AF555, 5-FAM, 6-FAM, 6-TET, 5-SIMA, 6-JCE, ATTO 700, ATTO 680, ATTO 655, Texas Red, DEAC, AMCA, ANT, MANT, DY-480XL, DY-485XL, ATTO 425, ATTO 390, ATTO 465, ATTO495, BDP-FL, ATTO 647N, ATTO 633, ATTO Rho14, ATTO Rho13, ATTO Rho12, ATTO Rho11, ATTO Thio12, ATTO 620, ATTO Rho101, ATTO 550, ATTO Rho6G, ATTO Rho13, ATTO 532, 5/6-TARMA, 6-ROX, ATTO 565, ATTO 590, AF594, 5/6-RHOX, AF488 or AF546.

When R₀ is a fluorophore, the fluorophore is any one of the following groups:

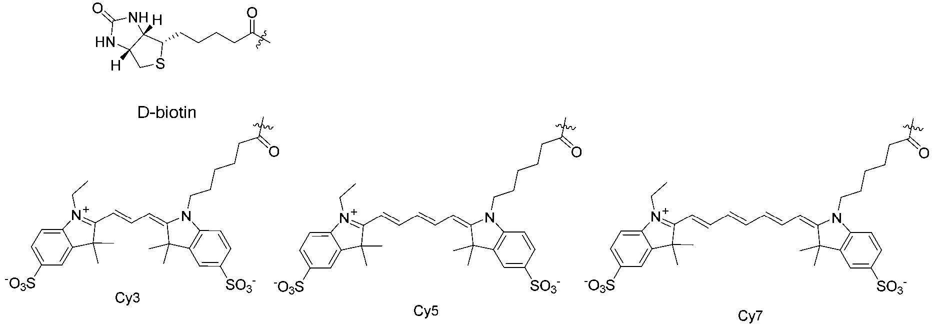

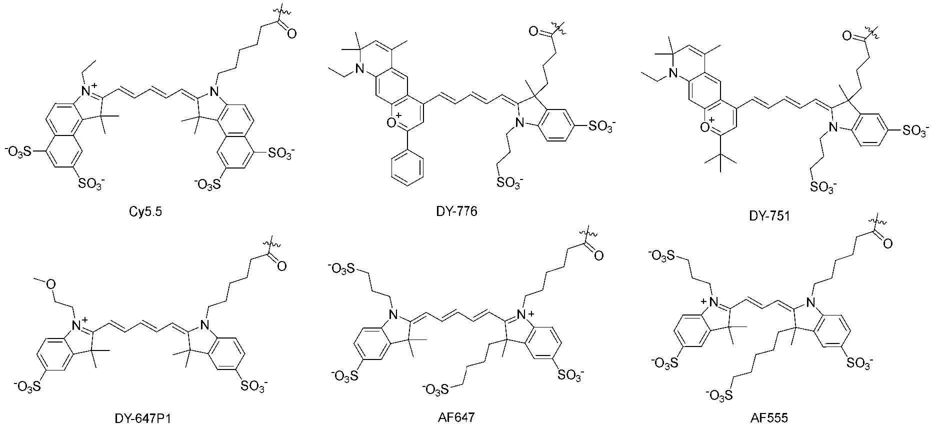

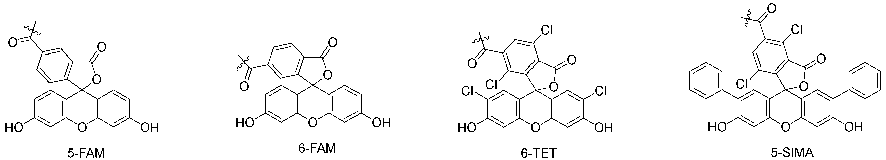

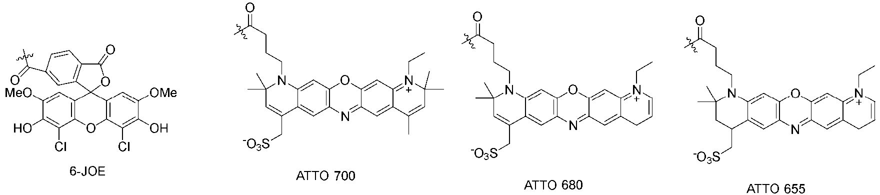

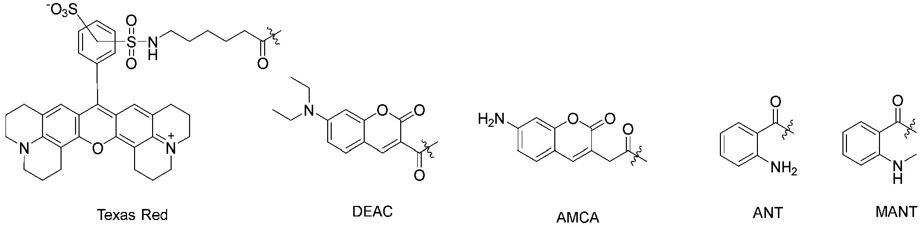

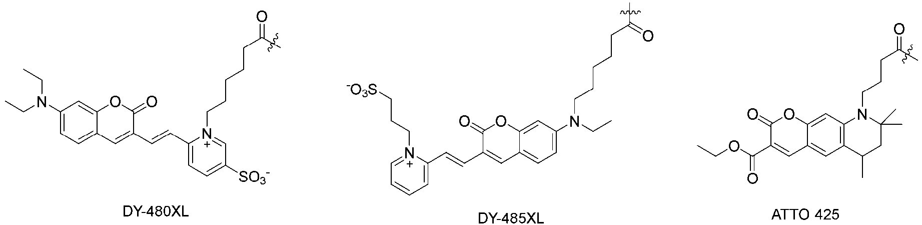

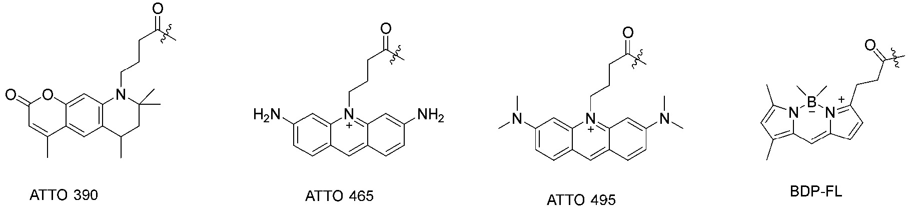

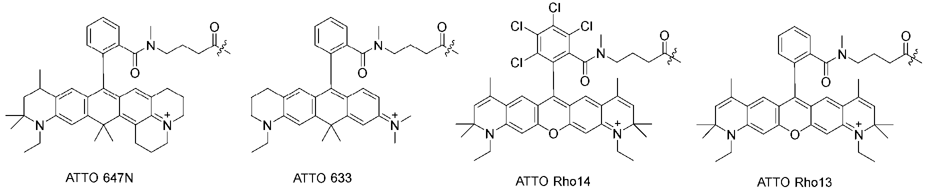

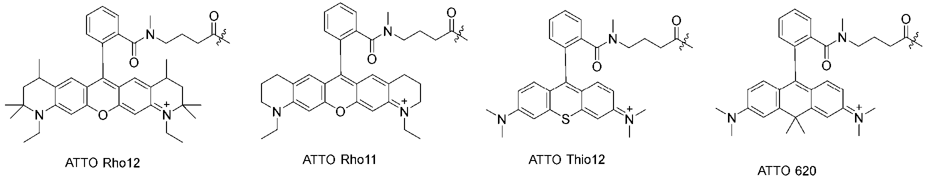

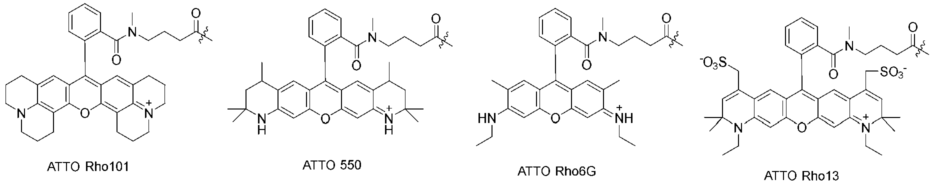

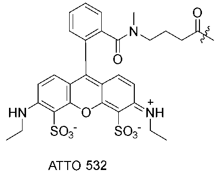

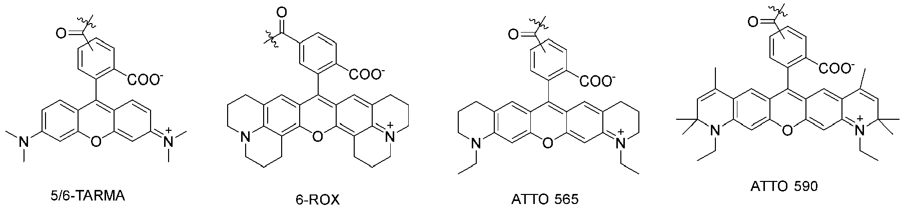

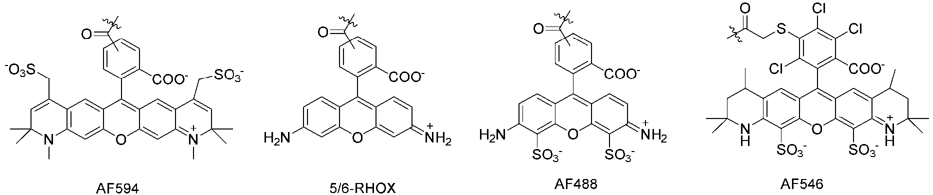

In some embodiments, when R₀ is a fluorophore, R₀ is biotin, Cy3, Cy5, Cy7, Cy5.5, AF647, AF555, 5-FAM, 6-FAM, 6-TET, 5-SIMA, 6-JCE, ATTO 700, ATTO 680, ATTO 655, ATTO 647N, ATTO 633, ATTO 620, ATTO 590, ATTO 565, ATTO 550, ATTO 532, ATTO 495, ATTO 465, ATTO 425, ATTO 390, ATTO Rho14, ATTO Rho13, ATTO Rho12, ATTO Rho11, ATTO Thio12, ATTO Rho101, ATTO Rho6G, DY-776, DY-751, DY-647P1, DY-480XL or DY-485XL.

In some embodiments, when R₀ is a fluorophore, R₀ is biotin, Cy3, Cy5, Cy7, Cy5.5, AF647, AF555, 5-FAM, 6-FAM, 6-TET, 5-SIMA, 6-JCE, Texas Red, DEAC, AMCA, ANT, MANT, BDP-FL, 5/6-TARMA, 6-ROX, AF594, 5/6-RHOX, AF488 or AF546.

In some embodiments, when R₀ is a fluorophore, R₀ is biotin, Cy3, Cy5, Cy7, Cy5.5, AF647, AF555, 5-FAM, 6-FAM, 6-TET, 5-SIMA, 6-JCE, ATTO 700, ATTO 680, ATTO 655, ATTO 647N, ATTO 633, ATTO 620, ATTO 590, ATTO 565, ATTO 550, ATTO 532, ATTO 495, DY-776, DY-751, DY-647P1, DY-480XL or DY-485XL.

In some embodiments, when R₀ is a fluorophore, R₀ is biotin, Cy3, Cy5, Cy7, Cy5.5, AF647, AF555, 5-FAM, 6-FAM, 6-TET, 5-SIMA, 6-JCE, ATTO 655, ATTO 647N, ATTO 590, ATTO 565, ATTO 550, DY-776, DY-751, DY-647P1 or DY-485XL. In some embodiments, when R₀ is a steroid structural group, the steroid structural group is a group formed by any one of the following steroid structures: cholic acid, deoxycholic acid, ursodeoxycholic acid, chenodeoxycholic acid, hyodeoxycholic acid or glycoursodeoxycholic acid.

The groups formed by cholic acid, deoxycholic acid, ursodeoxycholic acid, chenodeoxycholic acid, hyodeoxycholic acid or glycoursodeoxycholic acid are as follows:

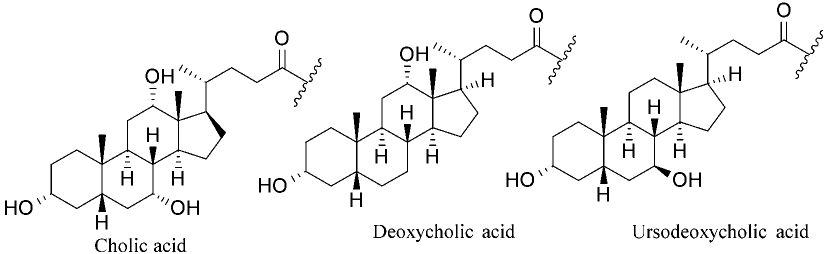

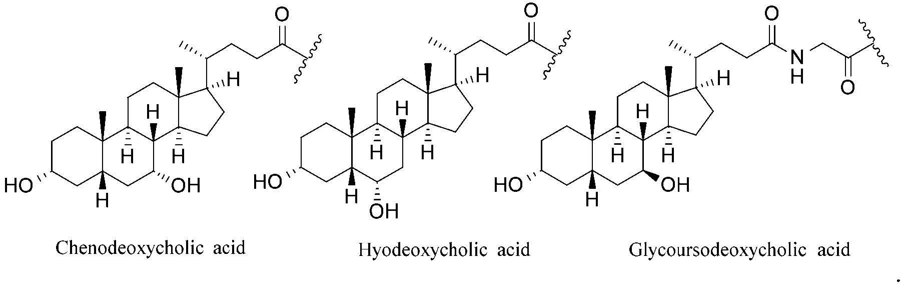

In some embodiments, L₁ and L₂ are not both NH; or L₁ and L₂ are not both carbonyl; meanwhile, L₂ and X are not both NH.

In some embodiments, when L₁ is absent, L₂ is NH, carbonyl or absent;
or, when L₁ is NH, L₂ is carbonyl or absent;
or, when L₁ is carbonyl, L₂ is NH or absent.

In some embodiments, X is NH, O, 5-membered nitrogen-containing aryl, 6-membered nitrogen-containing aryl or absent; more preferably, X is NH, O, 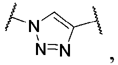 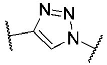 or absent.

In some embodiments, L₃ is -(CH₂)nNH-, -(CH₂)nNH(CH₂)mNH-, - (CH₂)nNHCO(CH₂)mNH-, -(CH₂)nCONH(CH₂)mNH-, -(CH₂)n(OC₂H₄)mNH-, -O-(CH₂)nNH-, -O-(CH₂)nNHCO(CH₂)mNH-, -O-(CH₂)nCONH(CH₂)mNH-, -O-(CH₂)n(OC₂H₄)mNH-, -NH-(CH₂)nNH-, -NH-(CH₂)nNHCO(CH₂)mNH-, -NH-(CH₂)nCONH(CH₂)mNH-, -NH-(CH₂)n(OC₂H₄)mNH-, 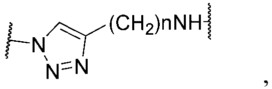 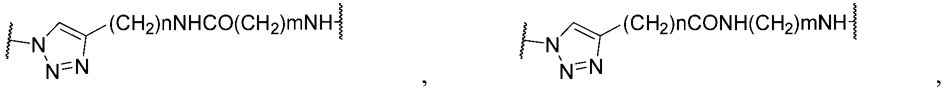 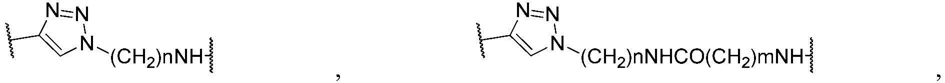 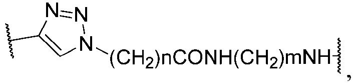 -(CH₂)n(OC₂H₄)mOCH₂-, -O-(CH₂)n(OC₂H₄)mOCH₂-, - NH-(CH₂)n(OC₂H₄)mOCH₂-, -(C(O)CHRaNH)n- or absent;
wherein Ra is H, CH₃, CH(CH₃)₂, CH₂CH(CH₃)₂, CH(CH₃)CH₂CH₃, CH₂C₆H₅, CH₂C₈NH₆, CH₂C₆H₄OH, CH₂COOH, CH₂CONH₂, (CH₂)₂COOH, (CH₂)₄NH₂, (CH₂)₂CONH₂, (CH₂)₂SCH₃, CH₂OH, CH(CH₃)OH, CH₂SH, C₃H₆, CH₂C₃H₃N₂ or (CH₂)₃NHC(NH)NH₂; Ra at each occurrence may be the same or different;
wherein n and m are each independently 0, 1, 2, 3, 4, 5, 6, 7, 8, 9 or 10.

In a specific embodiment, when n is 0, m may be 0, 1, 2, 3, 4, 5, 6, 7, 8, 9 or 10;
when n is 1, m may be 0, 1, 2, 3, 4, 5, 6, 7, 8, 9 or 10;
when n is 2, m may be 0, 1, 2, 3, 4, 5, 6, 7, 8, 9 or 10;
when n is 3, m may be 0, 1, 2, 3, 4, 5, 6, 7, 8, 9 or 10;
when n is 4, m may be 0, 1, 2, 3, 4, 5, 6, 7, 8, 9 or 10;
when n is 5, m may be 0, 1, 2, 3, 4, 5, 6, 7, 8, 9 or 10;
when n is 6, m may be 0, 1, 2, 3, 4, 5, 6, 7, 8, 9 or 10;
when n is 7, m may be 0, 1, 2, 3, 4, 5, 6, 7, 8, 9 or 10;
when n is 8, m may be 0, 1, 2, 3, 4, 5, 6, 7, 8, 9 or 10;
when n is 9, m may be 0, 1, 2, 3, 4, 5, 6, 7, 8, 9 or 10;
when n is 10, m may be 0, 1, 2, 3, 4, 5, 6, 7, 8, 9 or 10.

In some embodiments, Ra is H, CH₃, C(CH₃)₂, CH₂CH(CH₃)₂, CH(CH₃)CH₂CH₃, CH₂C₆H₅, CH₂C₆H₄OH, CH₂COOH, (CH₂)₂COOH, CH₂CONH₂, (CH₂)₂CONH₂, CH₂OH, CH(CH₃)OH, C₃H₆ or (CH₂)₃NHC(NH)NH₂; Ra at each occurrence may be the same or different.

In some embodiments, Ra is H, CH₃, CH(CH₃)₂, CH₂C₆H₅, CH₂C₆H₄OH, CH₂COOH, CH₂CONH₂, (CH₂)₂CONH₂, CH₂OH or CH(CH₃)OH; Ra at each occurrence may be the same or different.

In some embodiments, Ra is H, CH₃, CH₂C₆H₅, CH₂C₆H₄OH, CH₂CONH₂ or (CH₂)₂CONH₂; Ra at each occurrence may be the same or different.

In some embodiments, Ra is H, CH₃, CH₂C₆H₅ or (CH₂)₂CONH₂; Ra at each occurrence may be the same or different.

In some embodiments, -L₁-L₂-L₃- group is any one of the following groups: - (CH₂)n(OC₂H₄)_{M}OCH₂-, -NHCO(CH₂)n(OC₂H₄)mOCH₂-, -CONH(CH₂)n(OC₂H₄)_{M}OCH₂-, -(CH₂)nNH-, -(CH₂)nNH(CH₂)mNH-, -(CH₂)nCONH(CH₂)mNH-, - (CH₂)nNHCO(CH₂)mNH-, -NHCO(CH₂)nNHCO(CH₂)mNH-, -NHCONH(CH₂)nNH-, - NHCOO(CH₂)nNH-, -(CH₂)n(OC₂H₄)mNH-, -NHCO(CH₂)n(OC₂H₄)mNH-, - NHCONH(CH₂)n(OC₂H₄)mNH-, -CONH(CH₂)n(OC₂H₄)mNH-, 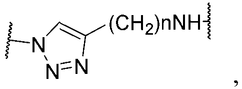 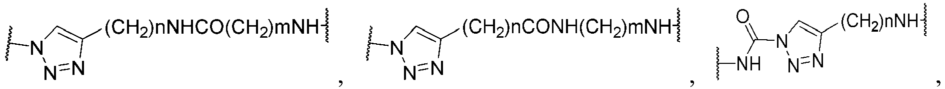 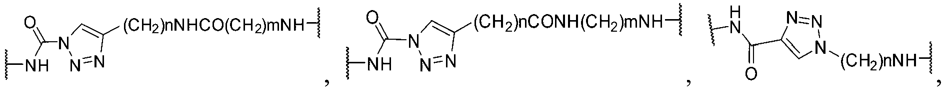 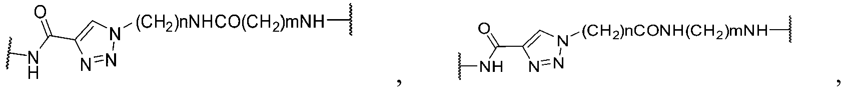 -NH-(C(O)CHRaNH)n- or absent;
wherein n and m are each independently 0, 1, 2, 3, 4, 5, 6, 7, 8, 9 or 10; and n and m at each occurrence may be the same or different.

In some embodiments, when R₀ is H, the -L₁-L₂-L₃- group is -(CH₂)n(OC₂H₄)mOCH₂-, -NHCO(CH₂)n(OC₂H₄)mOCH₂- or -CONH(CH₂)n(OC₂H₄)mOCH₂-;
wherein n and m are each independently 0, 1, 2, 3, 4, 5, 6, 7, 8, 9 or 10; and n and m at each occurrence may be the same or different; n₀ is 0, 1, 2, 3 or 4.

In some embodiments, when R₀ is a fluorophore or a steroid structural group, the -L₁-L₂-L₃- group is any one of the following groups: -(CH₂)nNH-, -(CH₂)nNH(CH₂)mNH-, - (CH₂)nCONH(CH₂)mNH-, -(CH₂)nNHCO(CH₂)mNH-, -NHCO(CH₂)nNHCO(CH₂)mNH-, - NHCONH(CH₂)nNH-, -NHCOO(CH₂)nNH-, -(CH₂)n(OC₂H₄)mNH-, - NHCO(CH₂)n(OC₂H₄)mNH-, -NHCONH(CH₂)n(OC₂H₄)mNH-, - CONH(CH₂)n(OC₂H₄)mNH-, 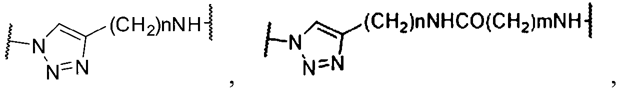 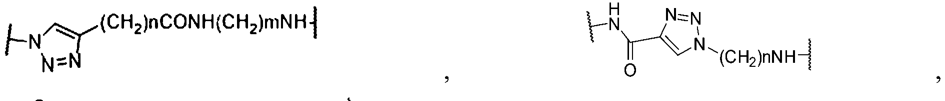 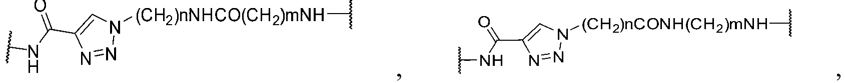 -NH-(C(O)CHRaNH)n- or absent;
wherein n and m are each independently 0, 1, 2, 3, 4, 5, 6, 7, 8, 9 or 10; and n and m at each occurrence may be the same or different; n₀ is 0, 1, 2, 3 or 4.

In a specific embodiment, when n is 0, m may be 0, 1, 2, 3, 4, 5, 6, 7, 8, 9 or 10;
when n is 1, m may be 0, 1, 2, 3, 4, 5, 6, 7, 8, 9 or 10;
when n is 2, m may be 0, 1, 2, 3, 4, 5, 6, 7, 8, 9 or 10;
when n is 3, m may be 0, 1, 2, 3, 4, 5, 6, 7, 8, 9 or 10;
when n is 4, m may be 0, 1, 2, 3, 4, 5, 6, 7, 8, 9 or 10;
when n is 5, m may be 0, 1, 2, 3, 4, 5, 6, 7, 8, 9 or 10;
when n is 6, m may be 0, 1, 2, 3, 4, 5, 6, 7, 8, 9 or 10;
when n is 7, m may be 0, 1, 2, 3, 4, 5, 6, 7, 8, 9 or 10;
when n is 8, m may be 0, 1, 2, 3, 4, 5, 6, 7, 8, 9 or 10;
when n is 9, m may be 0, 1, 2, 3, 4, 5, 6, 7, 8, 9 or 10;
when n is 10, m may be 0, 1, 2, 3, 4, 5, 6, 7, 8, 9 or 10.

In some embodiments, when R₀ is a fluorophore or a steroid structural group, -L₁-L₂-L₃- group is any one of the following groups: -(CH₂)nNH-, -(CH₂)nNH(CH₂)mNH-, - (CH₂)nCONH(CH₂)mNH-, -(CH₂)nNHCO(CH₂)mNH-, -NHCO(CH₂)nNHCO(CH₂)mNH-, - NHCONH(CH₂)nNH-, -NHCOO(CH₂)nNH-, -(CH₂)n(OC₂H₄)mNH-, - NHCO(CH₂)n(OC₂H₄)mNH-, -NHCONH(CH₂)n(OC₂H₄)mNH-, 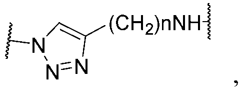 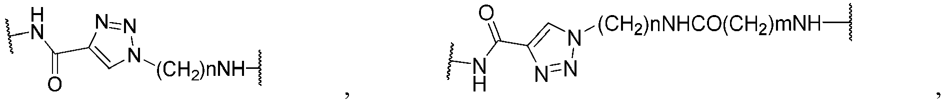 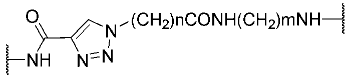 or -NH-(C(O)CHRaNH)n-.

In some embodiments, when R₀ is a fluorophore or a steroid structural group, -L₁-L₂-L₃- group is any one of the following groups: -(CH₂)nNH-, -(CH₂)nNH(CH₂)mNH-, - (CH₂)nCONH(CH₂)mNH-, -(CH₂)nNHCO(CH₂)mNH-, -NHCO(CH₂)nNHCO(CH₂)mNH-, - (CH₂)n(OC₂H₄)mNH-, -NHCO(CH₂)n(OC₂H₄)mNH-,

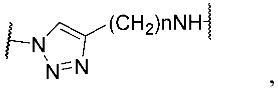

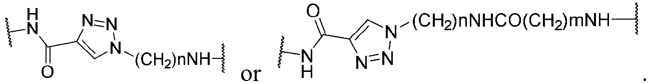

In some embodiments, when R₀ is a fluorophore or a steroid structural group, -L₁-L₂-L₃- group is any one of the following groups: -(CH₂)nNH-, -(CH₂)nCONH(CH₂)mNH-, - (CH₂)nNHCO(CH₂)mNH-, -NHCO(CH₂)n(OC₂H₄)mNH-, 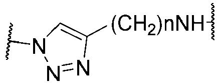 or

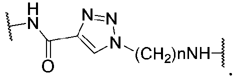

In some embodiments, when R₀ is a fluorophore or a steroid structural group, -L₁-L₂-L₃- group is any one of the following groups: -(CH₂)nNH-, -(CH₂)nNHCO(CH₂)mNH-, - NHCO(CH₂)n(OC₂H₄)mNH- or

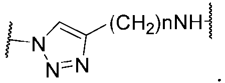

In some embodiments, R₄ is H, C₁-C₄ alkyl, C₂-C₄ alkenyl or C₂-C₄ alkynyl;
R₅ is halogen, CN, SO₂, NO₂, D, N₃, C₁-C₄ alkyl, C₂-C₄ alkenyl, C₂-C₄ alkynyl, C₁-C₄ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₄ alkyl, COR₈ or SO₂R₈;
R₇ is H, halogen, C₁-C₄ alkyl, C₂-C₄ alkenyl or C₂-C₄ alkynyl;
R₈ is H, halogen, C₁-C₄ alkyl, C₂-C alkenyl or C₂-C₅ alkynyl;
R₉ is H, halogen or C₁-C₃ alkyl.

In some embodiments, R₄ is H, methyl, ethyl, n-propyl, isopropyl, butyl, vinyl, propenyl, butenyl, ethynyl, propynyl or butynyl.

In some embodiments, R₄ is H, methyl, ethyl, n-propyl, isopropyl, vinyl, propenyl, ethynyl or propynyl.

In some embodiments, R₄ is H, methyl, ethyl, vinyl, propenyl, ethynyl or propynyl.

In some embodiments, R₄ is H, methyl, ethyl, vinyl, ethynyl or propynyl.

In some embodiments, R₅ is halogen, CN, D, N₃, C₁-C₄ alkyl, C₂-C₄ alkenyl, C₂-C₄ alkynyl, OR₇, NR₇R₇, COR₇, pyridyl, pyrimidinyl or morpholinyl.

In some embodiments, R₅ is halogen, CN, D, N₃, C₁-C₄ alkyl, C₂-C₄ alkenyl, C₂-C₄ alkynyl, OR₇, pyridyl, pyrimidinyl or morpholinyl.

In some embodiments, R₆ is H or C₁-C₄ alkyl.

In some embodiments, R₆ is H, methyl, ethyl, n-propyl, isopropyl or butyl.

In some embodiments, R₆ is H, methyl or ethyl.

In some embodiments, R₇ is H, F, Cl, methyl, ethyl, n-propyl, isopropyl, butyl, vinyl, propenyl, butenyl, ethynyl, propynyl or butynyl.

In some embodiments, R₇ is H, F, Cl, methyl, ethyl, n-propyl, isopropyl, vinyl, propenyl, ethynyl or propynyl.

In some embodiments, R₇ is H, F, Cl, methyl, ethyl, vinyl, ethynyl or propynyl.

In some embodiments, R₇ is H, F, Cl, methyl, ethyl or vinyl.

In some embodiments, R₈ is H, F, Cl, methyl, ethyl, n-propyl, isopropyl, butyl, vinyl, propenyl, butenyl, ethynyl, propynyl or butynyl.

In some embodiments, R₈ is H, fluorine, chlorine, methyl, ethyl, n-propyl, isopropyl, vinyl, propenyl, ethynyl or propynyl.

In some embodiments, R₈ is H, F, Cl, methyl, ethyl, vinyl, ethynyl or propynyl.

In some embodiments, R₈ is H, F, Cl, methyl, ethyl or vinyl.

In some embodiments, Z₁ is O, CH₂, S or NH;
Z₂ and Z₃ are each independently O, NH, CHR₇, CHCOOR₇, CHCONR₇R₇, S, CO, SO₂, PO(OH), PO(SH) or absent;
Z₄ is O, CH₂, S, NH or absent;
B₁ and B₂ are each independently a natural or modified cytosine nucleotide base, a natural or modified uracil nucleotide base, a natural or modified adenine nucleotide base or a natural or modified guanine nucleotide base.

In some more preferred embodiments, Z₂ is methylene, ethylene, CO, SO₂, PO(OH), or absent;
Z₃ is O, CH₂ or NH;
Z₄ is CH₂ or NH.

In some embodiments, Yₐ, Y_{b}, Y_{c} and Y_{d} are all O or at most one is S, CH₂, CCl₂, CF₂ or NH;
Y₁ₐ, Y_{1b} and Y_{1c} are all O or at most one is S;
Y₂ₐ, Y_{2b} and Y_{2c} are all OH or at most one is SH or BH₃;
Y₃ and Y₄ are independently CH₂.

In some embodiments, R₁ is C₁-C₄ alkyl, C₂-C₄ alkenyl, C₂-C₄ alkynyl, C₃-C₆ cycloalkyl, C₃-C₆ cycloalkenyl, benzyl, C₁-C₄ haloalkyl, C₂-C₄ haloalkenyl, C₂-C₄ haloalkynyl, C₃-C₆ halocycloalkyl, C₃-C₆ halocycloalkenyl or halobenzyl;
R₂ is H, C₁-C₄ alkyl, C₂-C₄ alkenyl, C₂-C₄ alkynyl, C₃-C₆ cycloalkyl, C₃-C₆ cycloalkenyl, C₁-C₄ haloalkyl, C₂-C₄ haloalkenyl, C₂-C₄ haloalkynyl, C₃-C₆ halocycloalkyl, C₃-C₆ halocycloalkenyl, benzyl or halobenzyl;
R₃ is H, OH, C₁-C₄ alkyl, C₂-C₄ alkenyl, C₂-C₄ alkynyl, C₁-C₄ haloalkyl, C₂-C₄ haloalkenyl, C₂-C₄ haloalkynyl, halogen or absent;
R₃ₐ and R_{3b} are each independently H, C₁-C₄ alkyl, C₂-C₄ alkenyl, C₂-C₄ alkynyl, C₁-C₄ haloalkyl, C₂-C₄ haloalkenyl or C₂-C₄ haloalkynyl.

In some embodiments, W is H, OH, OR₄, NR₄R₄, F, Cl or CN;
R₄ is H, methyl, ethyl, n-propyl, isopropyl, butyl, vinyl, propenyl, butenyl, ethynyl, propynyl or butynyl;
R₅ is halogen, CN, D, N₃, C₁-C₄ alkyl, C₂-C₄ alkenyl, C₂-C₄ alkynyl, OR₇, SR₇, NR₇R₇, COR₇, COOR₇, pyridyl, pyrimidinyl or morpholinyl;
R₇ is H, methyl, ethyl, n-propyl, isopropyl, butyl, vinyl, propenyl, butenyl, ethynyl, propynyl or butynyl.

In some more preferred embodiments, W is OH, F, Cl, methoxy or ethoxy;
R₁ is methyl, ethyl, n-propyl, isopropyl, fluoromethyl, difluoromethyl, trifluoromethyl, trifluoroethyl, trifluoropropyl or trifluoroisopropyl;
R₂ is H, methyl, ethyl, n-propyl, isopropyl, fluoromethyl, difluoromethyl, trifluoromethyl, trifluoroethyl, trifluoropropyl or trifluoroisopropyl.

In some preferred embodiments, a compound having a structure represented by formula III, or a stereoisomer, pharmaceutically acceptable salt or solvate of the compound having the structure represented by formula III: 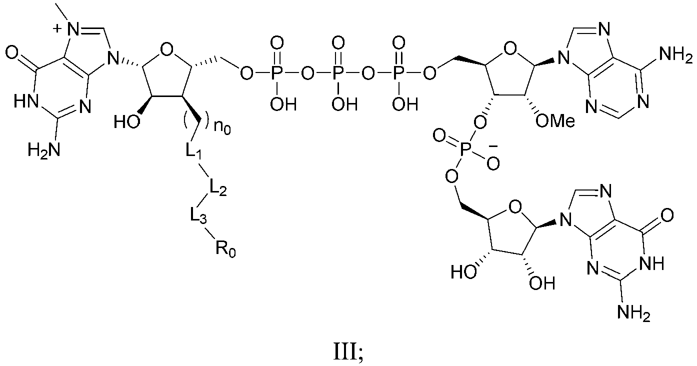
wherein L₁ is O, S, NH, carbonyl or absent;
L₂ is NH, carbonyl or absent;
L₃ is -X-(CH₂)nNH-, -X-(CH₂)nNH(CH₂)mNH-, -X-(CH₂)nNHCO(CH₂)mNH-, -X-(CH₂)nCONH(CH₂)mNH-, -X-(CH₂)n(OC₂H₄)mNH-, -X-(CH₂)n(OC₂H₄)mOCH₂-, -X-(C(O)CHRaNH)n- or absent; wherein X is NH, O, 5-membered heteroaryl, 6-membered heteroaryl or absent; n and m are each independently an integer from 0 to 10; Ra is a side chain group in an amino acid, and Ra at each occurrence may be the same or different;
R₀ is H, a fluorophore or a steroid structural group;
n₀ is an integer from 0 to 6.

In some more preferred embodiments, L₁ and L₂ are not both NH; or L₁ and L₂ are not both carbonyl; more preferably, L₂ and X are not both NH.

In some more preferred embodiments, when L₁ is absent, L₂ is NH, carbonyl or absent; or, when L₁ is NH, L₂ is carbonyl or absent; or, when L₁ is carbonyl, L₂ is NH or absent.

In some more preferred embodiments, X is NH, O, 5-membered nitrogen-containing aryl, 6-membered nitrogen-containing aryl or absent; more preferably, X is NH, O, 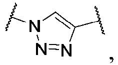 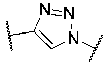 or absent.

In some more preferred embodiments, L₃ is -(CH₂)nNH-, -(CH₂)nNH(CH₂)mNH-, - (CH₂)nNHCO(CH₂)mNH-, -(CH₂)nCONH(CH₂)mNH-, -(CH₂)n(OC₂H₄)mNH-, -O-(CH₂)nNH-, -O-(CH₂)nNHCO(CH₂)mNH-, -O-(CH₂)nCONH(CH₂)mNH-, -O-(CH₂)n(OC₂H₄)mNH-, -NH-(CH₂)nNH-, -NH-(CH₂)nNHCO(CH₂)mNH-, -NH-(CH₂)nCONH(CH₂)mNH-, -NH-(CH₂)n(OC₂H₄)mNH-, 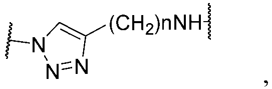 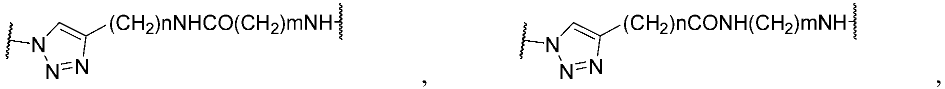 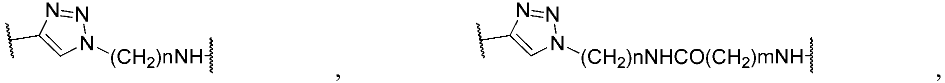 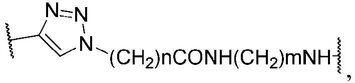 -(CH₂)n(OC₂H₄)mOCH₂-, -O-(CH₂)n(OC₂H₄)mOCH₂-, - NH-(CH₂)n(OC₂H₄)mOCH₂-, -(C(O)CHRaNH)n- or absent; wherein Ra is H, CH₃, CH(CH₃)₂, CH₂CH(CH₃)₂, CH(CH₃)CH₂CH₃, CH₂C₆H₅, CH₂C₈NH₆, CH₂C₆H₄OH, CH₂COOH, CH₂CONH₂, (CH₂)₂COOH, (CH₂)₄NH₂, (CH₂)₂CONH₂, (CH₂)₂SCH₃, CH₂OH, CH(CH₃)OH, CH₂SH, C₃H₆, CH₂C₃H₃N₂ or (CH₂)₃NHC(NH)NH₂; Ra at each occurrence may be the same or different;
wherein n and m are each independently 0, 1, 2, 3, 4, 5, 6, 7, 8, 9 or 10.

In some embodiments, Ra is H, CH₃, CH(CH₃)₂, CH₂CH(CH₃)₂, CH(CH₃)CH₂CH₃, CH₂C₆H₅, CH₂C₈NH₆, CH₂C₆H₄OH, CH₂COOH, CH₂CONH₂, (CH₂)₂COOH, (CH₂)₂CONH₂, CH₂OH, CH(CH₃)OH, C₃H₆, CH₂C₃H₃N₂ or (CH₂)₃NHC(NH)NH₂; Ra at each occurrence may be the same or different.

In some embodiments, Ra is H, CH₃, CH(CH₃)₂, CH₂CH(CH₃)₂, CH₂C₆H₅, CH₂C₈NH₆, CH₂C₆H₄OH, CH₂COOH, CH₂CONH₂, CH₂OH, CH(CH₃)OH, CH₂C₃H₃N₂ or (CH₂)₃NHC(NH)NH₂; Ra at each occurrence may be the same or different.

In some embodiments, Ra is H, CH₃, CH₂C₆H₅, CH₂C₈NH₆, CH₂C₆H₄OH, CH₂CONH₂, CH₂OH, CH₂C₃H₃N₂ or (CH₂)₃NHC(NH)NH₂; Ra at each occurrence may be the same or different.

In some more preferred embodiments, -L₁-L₂-L₃- group is any one of the following groups: -(CH₂)n(OC₂H₄)mOCH₂-, -NHCO(CH₂)n(OC₂H₄)mOCH₂-, - CONH(CH₂)n(OC₂H₄)mOCH₂-, -(CH₂)nNH-, -(CH₂)nNH(CH₂)mNH-, - (CH₂)nCONH(CH₂)mNH-, -(CH₂)nNHCO(CH₂)mNH-, -NHCO(CH₂)nNHCO(CH₂)mNH-, - NHCONH(CH₂)nNH-, -NHCOO(CH₂)nNH-, -(CH₂)n(OC₂H₄)mNH-, - NHCO(CH₂)n(OC₂H₄)mNH-, -NHCONH(CH₂)n(OC₂H₄)mNH-, - CONH(CH₂)n(OC₂H₄)mNH-, 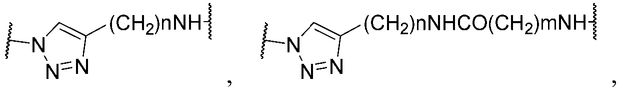 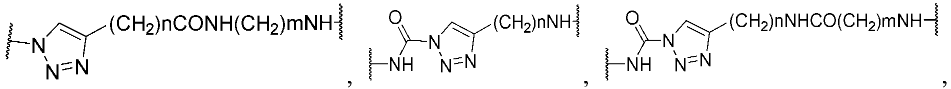 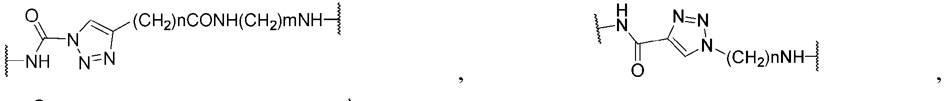 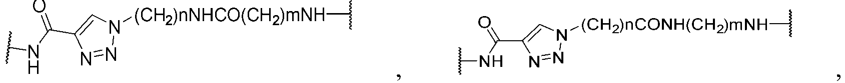 -NH-(C(O)CHRaNH)n- or absent;
wherein n and m are each independently 0, 1, 2, 3, 4, 5, 6, 7, 8, 9 or 10; and n and m at each occurrence may be the same or different.

In some more preferred embodiments, when R₀ is H, the -L₁-L₂-L₃- group is - (CH₂)n(OC₂H₄)mOCH₂-, -NHCO(CH₂)n(OC₂H₄)mOCH₂- or - CONH(CH₂)n(OC₂H₄)_{M}OCH₂-;
when n is 0, 1, 2, 3, 4, 5, 6, 7, 8, 9 or 10, m may be 1, 2, 3, 4, 5, 6, 7, 8, 9 or 10, and m at each occurrence may be the same or different;

For example, the -L₁-L₂-L₃- group may be any one of the following groups (non-exhaustive):
-OC₂H₄OCH₂-, -(OC₂H₄)₂OCH₂-, -(OC₂H₄)₃OCH₂-, -(OC₂H₄)₄OCH₂-, - (OC₂H₄)₅OCH₂-, -(OC₂H₄)₆OCH₂-, -(OC₂H₄)₇OCH₂-, -(OC₂H₄)₈OCH₂-, -(OC₂H₄)₉OCH₂-, - (OC₂H₄)₁₀OCH₂-;
-CH₂OC₂H₄OCH₂-, -CH₂(OC₂H₄)₂OCH₂-, -CH₂(OC₂H₄)₄OCH₂-, - CH₂(OC₂H₄)₅OCH₂-, -CH₂(OC₂H₄)₆OCH₂-, -CH₂(OC₂H₄)₈OCH₂-, -CH₂(OC₂H₄)₁₀OCH₂-;
-C₃H₆OC₂H₄OCH₂-, -C₃H₆(OC₂H₄)₂OCH₂-, -C₃H₆(OC₂H₄)₄OCH₂-, - C₃H₆(OC₂H₄)₈OCH₂-;
-C₄H₈(OC₂H₄)₂OCH₂-, -C₄H₈(OC₂H₄)₄OCH₂-, -C₄H₈(OC₂H₄)₅OCH₂-, - C₄H₈(OC₂H₄)₇OCH₂-;
-C₅H₁₀OC₂H₄OCH₂-, -C₅H₁₀(OC₂H₄)₂OCH₂-, -C₅H₁₀(OC₂H₄)₄OCH₂-, - C₅H₁₀(OC₂H₄)sOCH₂-, -C₅H₁₀(OC₂H₄)₆OCH₂-, -C₅H₁₀(OC₂H₄)₈OCH₂-, - C₅H₁₀(OC₂H₄)₁₀OCH₂-;
-C₆H₁₂OC₂H₄OCH₂-, -C₆H₁₂(OC₂H₄)₅OCH₂-, -C₇H₁₄(OC₂H₄)₉OCH₂-, - C₈H₁₆(OC₂H₄)₆OCH₂-;
-NHCO(OC₂H₄)₂OCH₂-, -NHCO(OC₂H₄)₃OCH₂-, -NHCO(OC₂H₄)₄OCH₂, - NHCO(OC₂H₄)₅OCH₂-, -NHCO(OC₂H₄)₆OCH₂-, -NHCO(OC₂H₄)₈OCH₂-, - NHCO(OC₂H₄)₁₀OCH₂-;
-NHCOCH₂(OC₂H₄)₂OCH₂-, -NHCOCH₂(OC₂H₄)₃OCH₂-, - NHCOCH₂(OC₂H₄)₅OCH₂-, -NHCOCH₂(OC₂H₄)₇OCH₂-, -NHCOCH₂(OC₂H₄)₉OCH₂-, - NHCOCH₂(OC₂H₄)₁₀OCH₂-;
-NHCOC₂H₄(OC₂H₄)₂OCH₂-, -NHCOC₂H₄(OC₂H₄)₃OCH₂-, - NHCOC₂H₄(OC₂H₄)₅OCH₂-, -NHCOC₂H₄(OC₂H₄)₇OCH₂-, -NHCOC₂H₄(OC₂H₄)₉OCH₂-, - NHCOC₂H₄(OC₂H₄)₁₀OCH₂-;
-NHCOC₃H₆(OC₂H₄)₂OCH₂-, -NHCOC₃H₆(OC₂H₄)₃OCH₂-, - NHCOC₃H₆(OC₂H₄)₅OCH₂-, -NHCOC₃H₄(OC₂H₄)₇OCH₂-, -NHCOC₃H₆(OC₂H₄)₉OCH₂-, - NHCOC₃H₆(OC₂H₄)₁₀OCH₂-;
-NHCOC₄H₈(OC₂H₄)₆OCH₂-, -NHCOC₄H₈(OC₂H₄)₈OCH₂-, - NHCOC₅H₁₀(OC₂H₄)₄OCH₂-, -NHCOC₅H₁ₒ(OC₂H₄)₇OCH₂-, -NHCOC₇H₁₄(OC₂H₄)₉OCH₂-, -NHCOC₈H₁₆(OC₂H₄)₁₀OCH₂-;
-CONHCH₂OC₂H₄OCH₂-, -CONHCH₂(OC₂H₄)₃OCH₂-, - CONHCH₂(OC₂H₄)₅OCH₂-, -CONHCH₂(OC₂H₄)₆OCH₂-, -CONHCH₂(OC₂H₄)₈OCH₂-, - CONHCH₂(OC₂H₄)₁₀OCH₂-;
-CONHC₂H₄OC₂H₄OCH₂-, -CONHC₂H₄(OC₂H₄)₂OCH₂-, - CONHC₂H₄(OC₂H₄)₄OCH₂-, -CONHC₂H₄(OC₂H₄)₆OCH₂-, -CONHC₂H₄(OC₂H₄)₈OCH₂-, - CONHC₂H₄(OC₂H₄)₁₀OCH₂-, -CONHC₃H₆OC₂H₄OCH₂-, -CONHC₃H₆(OC₂H₄)₂OCH₂-, - CONHC₃H₆(OC₂H₄)₄OCH₂-, -CONHC₃H₆(OC₂H₄)₆OCH₂-, -CONHC₃H₆(OC₂H₄)₈OCH₂-, - CONHC₃H₆(OC₂H₄)₁₀OCH₂-, -CONHC₄H₈0C₂H₄OCH₂-, -CONHC₄H₈(OC₂H₄)₅OCH₂-, - CONHC₄H₈(OC₂H₄)₆OCH₂-, -CONHC₄H₈(OC₂H₄)₁₀OCH₂-, -CONHC₅H₁₀OC₂H₄OCH₂-, - CONHC₇H₁₄(OC₂H₄)₃OCH₂-, -CONHC₇H₁₄(OC₂H₄)₆OCH₂-, -CONHC₈H₁₆(OC₂H₄)₈OCH₂-, -CONHC₁₀H₂₂OC₂H₄OCH₂-, -CONHC₉H₁₈(OC₂H₄)₃OCH₂-, -CONHC₁₀H₂₂(OC₂H₄)₄OCH₂-, -CONHC₉H₁₈(OC₂H₄)₆OCH₂-, -CONHC₁₀H₂₂(OC₂H₄)₈OCH₂- or - CONHC₁₀H₂₂(OC₂H₄)₉OCH₂-.

In some more preferred embodiments, when R₀ is a fluorophore or a steroid structural group, the -L₁-L₂-L₃- group is any one of the following groups: -(CH₂)nNH-, - (CH₂)nNH(CH₂)mNH-, -(CH₂)nCONH(CH₂)mNH-, -(CH₂)nNHCO(CH₂)mNH-, - NHCO(CH₂)nNHCO(CH₂)mNH-, -NHCONH(CH₂)nNH-, -NHCOO(CH₂)nNH-, - (CH₂)n(OC₂H₄)mNH-, -NHCO(CH₂)n(OC₂H₄)mNH-, -NHCONH(CH₂)n(OC₂H₄)mNH-, - CONH(CH₂)n(OC₂H₄)mNH-, 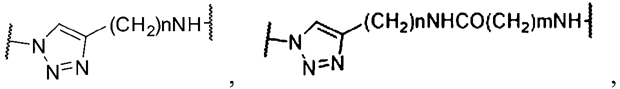 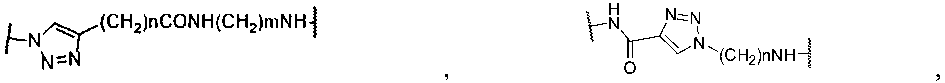 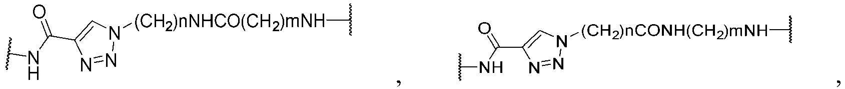 -NH-(C(O)CHRaNH)n- or absent;
wherein n and m are each independently 0, 1, 2, 3, 4, 5, 6, 7, 8, 9 or 10; and n and m at each occurrence may be the same or different.

For example, the -L₁-L₂-L₃- group may be any one of the following groups (non-exhaustive):
-NH-, -CH₂NH-, -(CH₂)₂NH-, -(CH₂)₄NH-, -(CH₂)₅NH-, -(CH₂)₆NH-;
-NH(CH₂)₂NH-, -NH(CH₂)₃NH-, -NH(CH₂)₄NH-, -NH(CH₂)₅NH-, -NH(CH₂)₈NH-;
-CH₂NH(CH₂)₂NH-, -CH₂NH(CH₂)₃NH-, -CH₂NH(CH₂)₅NH-, -CH₂NH(CH₂)₈NH-;
-(CH₂)₄NH(CH₂)₂NH-, -(CH₂)₄NH(CH₂)₃NH-, -(CH₂)₄NH(CH₂)₄NH-, - (CH₂)₄NH(CH₂)₅NH-, -(CH₂)₃NH(CH₂)₂NH-, -(CH₂)₃NH(CH₂)₅NH-, -(CH₂)₃NH(CH₂)₆NH-, -(CH₂)₄NH(CH₂)₂NH-, -(CH₂)₄NH(CH₂)₅NH-, -(CH₂)₅NH(CH₂)₃NH-, -(CH₂)₅NH(CH₂)₇NH-, -(CH₂)₆NH(CH₂)₄NH-;
-CONHCH₂NH-, -CONH(CH₂)₂NH-, -CONH(CH₂)₃NH-, -CONH(CH₂)₄NH-, - CONH(CH₂)₅NH-, -CONH(CH₂)₆NH-, -CONH(CH₂)₇NH-, -CONH(CH₂)₈NH-, - CONH(CH₂)₉NH-, -CONH(CH₂)₁₀NH-;
-CH₂CONHCH₂NH-, -CH₂CONH(CH₂)₂NH-, -CH₂CONH(CH₂)₃NH-, - CH₂CONH(CH₂)₄NH-, -CH₂CONH(CH₂)₅NH-, -CH₂CONH(CH₂)₆NH-, - CH₂CONH(CH₂)₇NH-, -CH₂CONH(CH₂)₉NH-;
-(CH₂)₂CONHCH₂NH-, -(CH₂)₂CONH(CH₂)₃NH-, -(CH₂)₂CONH(CH₂)₅NH-, - (CH₂)₂CONH(CH₂)₈NH-, -(CH₂)₃CONH(CH₂)₃NH-, -(CH₂)₃CONH(CH₂)₆NH-, - (CH₂)₄CONH(CH₂)₃NH-, -(CH₂)₄CONH(CH₂)₆NH-, -(CH₂)₅CONH(CH₂)₂NH-, - (CH₂)₅CONH(CH₂)₅NH-, -(CH₂)₆CONH(CH₂)₅NH-, -(CH₂)₇CONH(CH₂)₄NH-, - (CH₂)₈CONH(CH₂)₄NH-, -(CH₂)₈CONH(CH₂)₆NH-, -(CH₂)₈CONH(CH₂)₈NH-;
-NHCO(CH₂)₂NH-, -NHCO(CH₂)₃NH-, -NHCO(CH₂)₄NH-, -NHCO(CH₂)₅NH-, - NHCO(CH₂)₈NH-;
-CH₂NHCO(CH₂)₂NH-, -CH₂NHCO(CH₂)₃NH-, -CH₂NHCO(CH₂)₄NH-, - CH₂NHCO(CH₂)₅NH-, -CH₂NHCO(CH₂)₇NH-, -CH₂NHCO(CH₂)₉NH-, - (CH₂)₃NHCO(CH₂)₂NH-, -(CH₂)₃NHCO(CH₂)₃NH-, -(CH₂)₃NHCO(CH₂)₅NH-, - (CH₂)₃NHCO(CH₂)₇NH-, -(CH₂)₄NHCO(CH₂)₂NH-, -(CH₂)₄NHCO(CH₂)₅NH-, - (CH₂)₄NHCO(CH₂)₈NH-, -(CH₂)₅NHCO(CH₂)₄NH-, -(CH₂)₅NHCO(CH₂)₇NH-, - (CH₂)₆NHCO(CH₂)₃NH-;
-NHCOCH₂NHCOCH₂NH-, -NHCOCH₂NHCO(CH₂)₂NH-, - NHCOCH₂NHCO(CH₂)₃NH-, -NHCOCH₂NHCO(CH₂)₄NH-, -NHCOCH₂NHCO(CH₂)₆NH-, -NHCOCH₂NHCO(CH₂)₈NH-, -NHCO(CH₂)₂NHCO(CH₂)₂NH-, - NHCO(CH₂)₂NHCO(CH₂)₄NH-, -NHCO(CH₂)₂NHCO(CH₂)₆NH-, - NHCO(CH₂)₂NHCO(CH₂)₈NH-, -NHCO(CH₂)₂NHCO(CH₂)₁₀NH-, - NHCO(CH₂)₃NHCO(CH₂)₂NH-, -NHCO(CH₂)₃NHCO(CH₂)₄NH-, - NHCO(CH₂)₃NHCO(CH₂)₆NH-, -NHCO(CH₂)₄NHCO(CH₂)₂NH-, - NHCO(CH₂)₄NHCO(CH₂)₄NH-, -NHCO(CH₂)₄NHCO(CH₂)₆NH-, - NHCO(CH₂)₄NHCO(CH₂)₆NH-, -NHCO(CH₂)₅NHCO(CH₂)₂NH-, - NHCO(CH₂)₅NHCO(CH₂)₆NH-, -NHCO(CH₂)₅NHCO(CH₂)₄NH-, - NHCO(CH₂)₆NHCO(CH₂)₆NH-, -NHCO(CH₂)₈NHCO(CH₂)₄NH-, - NHCO(CH₂)₈NHCO(CH₂)₆NH-;
-NHCONHCOCH₂NH-, -NHCONHCO(CH₂)₂NH-, -NHCONHCO(CH₂)₃NH-, - NHCONHCO(CH₂)₄NH-, -NHCONHCO(CH₂)₅NH-, -NHCONHCO(CH₂)₆NH-, - NHCONHCO(CH₂)₇NH-, -NHCONHCO(CH₂)₈NH-, -NHCONHCO(CH₂)₉NH-, - NHCONHCO(CH₂)₁₀NH-;
-NHCOOCH₂NH-, -NHCOO(CH₂)₂NH-, -NHCOO(CH₂)₃NH-, -NHCOO(CH₂)₄NH-, -NHCOO(CH₂)₅NH-, -NHCOO(CH₂)₆NH-, -NHCOO(CH₂)₇NH-, -NHCOO(CH₂)₈NH-, - NHCOO(CH₂)₉NH-, -NHCOO(CH₂)₁₀NH-;
-CH₂OC₂H₄NH-, -CH₂(OC₂H₄)₂NH-, -CH₂(OC₂H₄)₃NH-, -CH₂(OC₂H₄)₄NH-, - CH₂(OC₂H₄)₅NH-, -CH₂(OC₂H₄)₆NH-, -CH₂(OC₂H₄)₇NH-, -CH₂(OC₂H₄)₈NH-, - CH₂(OC₂H₄)₉NH-, -CH₂(OC₂H₄)₁₀NH-, -(CH₂)₂OC₂H₄NH-, -(CH₂)₂(OC₂H₄)₃NH-, - (CH₂)₂(OC₂H₄)₅NH-, -(CH₂)₂(OC₂H₄)₇NH-, -(CH₂)₂(OC₂H₄)₈NH-, -(CH₂)₃(OC₂H₄)₂NH-, - (CH₂)₃(OC₂H₄)₅NH-, -(CH₂)₃(OC₂H₄)₇NH-, -(CH₂)₃(OC₂H₄)₈NH-, -(CH₂)₃(OC₂H₄)₁₀NH-, - (CH₂)₄(OC₂H₄)₂NH-, -(CH₂)₄(OC₂H₄)₅NH-, -(CH₂)₄(OC₂H₄)₆NH-, -(CH₂)₄(OC₂H₄)₈NH-, - (CH₂)₅(OC₂H₄)₂NH-, -(CH₂)₅(OC₂H₄)₅NH-, -(CH₂)₅(OC₂H₄)₇NH-, -(CH₂)₆(OC₂H₄)₄NH-, - (CH₂)₆(OC₂H₄)₆NH-, -(CH₂)₆(OC₂H₄)₈NH-, -(CH₂)₇(OC₂H₄)₄NH-, -(CH₂)₇(OC₂H₄)₇NH-, - (CH₂)₇(OC₂H₄)₁₀NH-, -(CH₂)₈(OC₂H₄)₆NH-, -(CH₂)₈(OC₂H₄)₈NH-, -(CH₂)₈(OC₂H₄)₁₀NH-,
-NHCOCH₂OC₂H₄NH-, -NHCOCH₂(OC₂H₄)₂NH-, -NHCOCH₂(OC₂H₄)₃NH-, - NHCOCH₂(OC₂H₄)₄NH-, -NHCOCH₂(OC₂H₄)₅NH-, -NHCOCH₂(OC₂H₄)₆NH-, - NHCOCH₂(OC₂H₄)₇NH-, -NHCOCH₂(OC₂H₄)₈NH-, -NHCOCH₂(OC₂H₄)₉NH-, - NHCOCH₂(OC₂H₄)₁₀NH-, -NHCO(CH₂)₂(OC₂H₄)₂NH-, -NHCO(CH₂)₂(OC₂H₄)₄NH-, - NHCO(CH₂)₂(OC₂H₄)₆NH-, -NHCO(CH₂)₂(OC₂H₄)₇NH-, -NHCO(CH₂)₂(OC₂H₄)₁₀NH-, - NHCO(CH₂)₃(OC₂H₄)₂NH-, -NHCO(CH₂)₃(OC₂H₄)₄NH-, -NHCO(CH₂)₃(OC₂H₄)₆NH-, - NHCO(CH₂)₃(OC₂H₄)₉NH-, -NHCO(CH₂)₄(OC₂H₄)₂NH-, -NHCO(CH₂)₄(OC₂H₄)₆NH-, - NHCO(CH₂)₄(OC₂H₄)₈NH-, -NHCO(CH₂)₄(OC₂H₄)₁₀NH-, -NHCO(CH₂)₅(OC₂H₄)₄NH-, - NHCO(CH₂)₅(OC₂H₄)₇NH-, -NHCO(CH₂)₆(OC₂H₄)₄NH-, -NHCO(CH₂)₆(OC₂H₄)₁₀NH-, - NHCO(CH₂)₇(OC₂H₄)₇NH-, -NHCO(CH₂)₈(OC₂H₄)₆NH-, -NHCO(CH₂)s(OC₂H₄)₁₀NH-;
-NHCONH(CH₂)₂OC₂H₄NH-, -NHCONH(CH₂)₂(OC₂H₄)₂NH-, - NHCONH(CH₂)₂(OC₂H₄)₃NH-, -NHCONH(CH₂)₂(OC₂H₄)₄NH-, - NHCONH(CH₂)₂(OC₂H₄)₅NH-, -NHCONH(CH₂)₂(OC₂H₄)₆NH-, - NHCONH(CH₂)₂(OC₂H₄)₇NH-, -NHCONH(CH₂)₂(OC₂H₄)₈NH-, - NHCONH(CH₂)₂(OC₂H₄)₉NH-,-NHCONH(CH₂)₂(OC₂H₄)₁₀NH-,
-NHCONH(CH₂)₃(OC₂H₄)₂NH-, -NHCONH(CH₂)₃(OC₂H₄)₄NH-, - NHCONH(CH₂)₃(OC₂H₄)₅NH-, -NHCONH(CH₂)₃(OC₂H₄)₇NH-, - NHCONH(CH₂)₃(OC₂H₄)₈NH-, -NHCONH(CH₂)₄(OC₂H₄)₄NH-, - NHCONH(CH₂)₄(OC₂H₄)₆NH-, -NHCONH(CH₂)₄(OC₂H₄)₄NH-, - NHCONH(CH₂)₄(OC₂H₄)₇NH-, -NHCONH(CH₂)₅(OC₂H₄)₄NH-, - NHCONH(CH₂)₅(OC₂H₄)₈NH-, -NHCONH(CH₂)₆(OC₂H₄)₃NH-, - NHCONH(CH₂)₆(OC₂H₄)₆NH-, -NHCONH(CH₂)₇(OC₂H₄)₄NH-, - NHCONH(CH₂)₈(OC₂H₄)₄NH-,-NHCONH(CH₂)₈(OC₂H₄)₁₀NH-,
-CONH(CH₂)₂OC₂H₄NH-, -CONH(CH₂)₂(OC₂H₄)₂NH-, - CONH(CH₂)₂(OC₂H₄)₃NH-, -CONH(CH₂)₂(OC₂H₄)₄NH-, -CONH(CH₂)₂(OC₂H₄)₅NH-, - CONH(CH₂)₂(OC₂H₄)₆NH-, -CONH(CH₂)₂(OC₂H₄)₇NH-, -CONH(CH₂)₂(OC₂H₄)₈NH-, - CONH(CH₂)₂(OC₂H₄)₉NH-, -CONH(CH₂)₂(OC₂H₄)₁₀NH-, -CONH(CH₂)₃(OC₂H₄)₂NH-, - CONH(CH₂)₃(OC₂H₄)₅NH-, -CONH(CH₂)₄(OC₂H₄)₇NH-, -CONH(CH₂)₄(OC₂H₄)₂NH-, - CONH(CH₂)₄(OC₂H₄)₄NH-, -CONH(CH₂)₄(OC₂H₄)₇NH-, -CONH(CH₂)₅(OC₂H₄)₄NH-, - CONH(CH₂)₅(OC₂H₄)₈NH-, -CONH(CH₂)₆(OC₂H₄)₃NH-, -CONH(CH₂)₆(OC₂H₄)₅NH-, - CONH(CH₂)₆(OC₂H₄)₈NH-, -CONH(CH₂)₇(OC₂H₄)₄NH-, -CONH(CH₂)₇(OC₂H₄)₈NH-, - CONH(CH₂)₈(OC₂H₄)₄NH-, -CONH(CH₂)₈(OC₂H₄)₈NH-, -CONH(CH₂)₈(OC₂H₄)₁₀NH-;

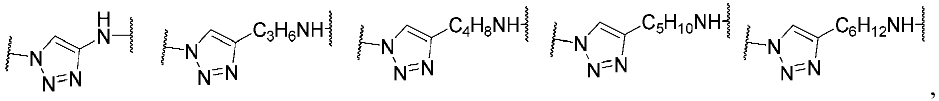

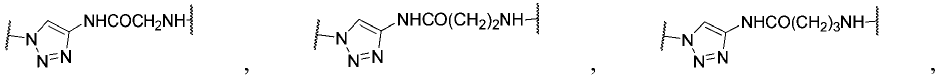

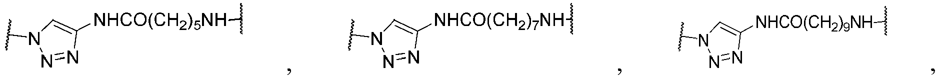

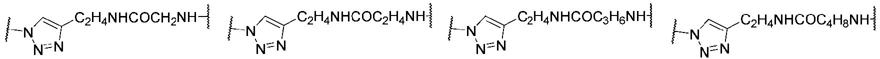

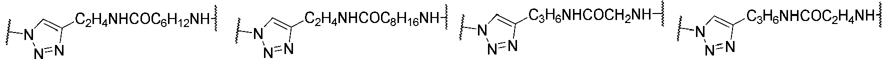

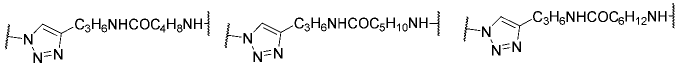

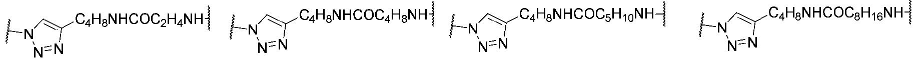

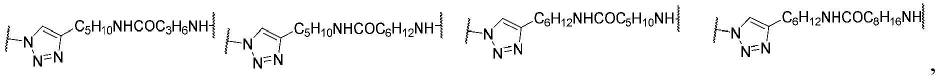

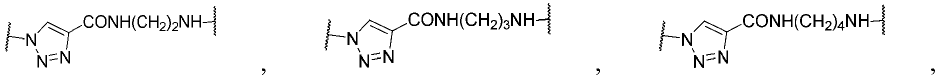

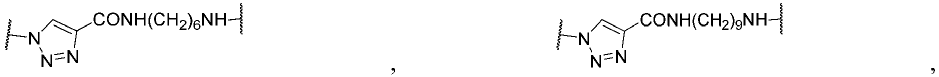

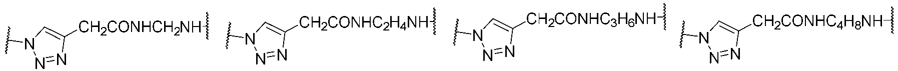

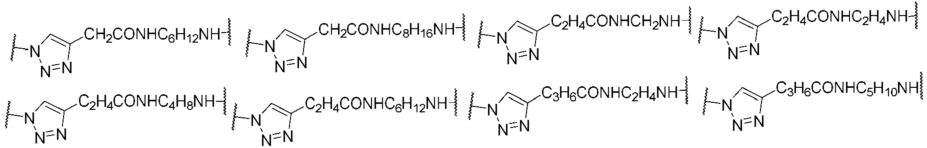

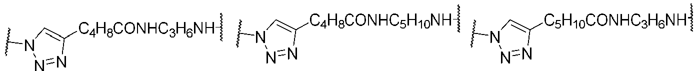

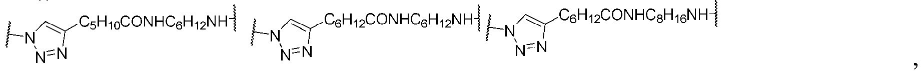

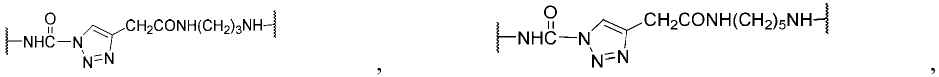

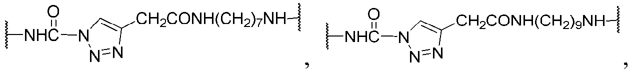

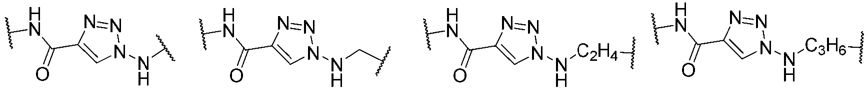

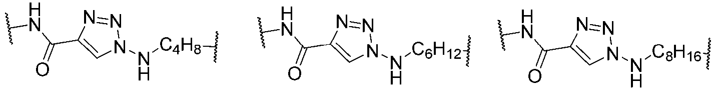

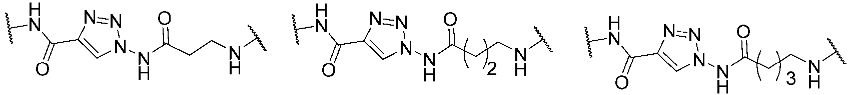

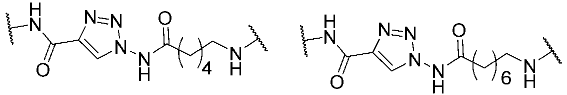

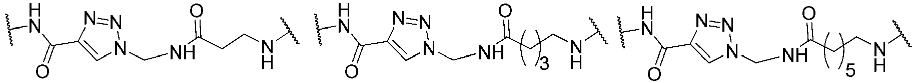

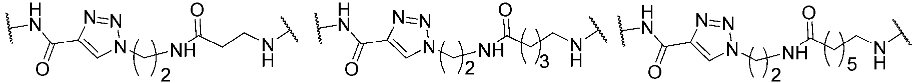

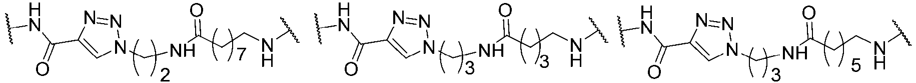

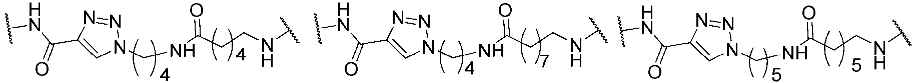

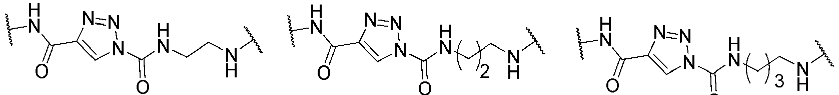

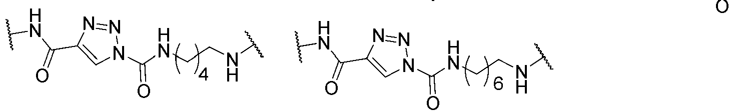

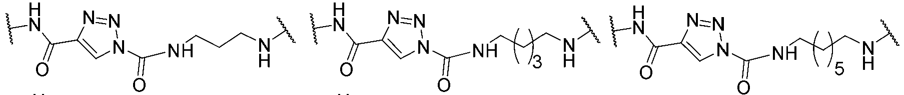

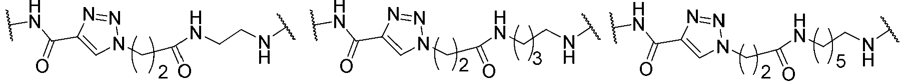

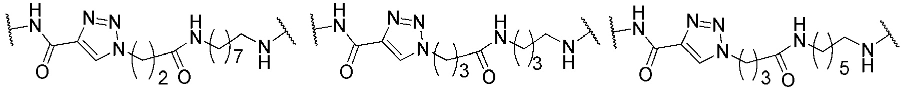

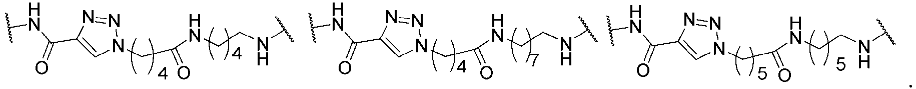

In some embodiments, the compound is represented by any one of the following structures, wherein R₀ is the aforementioned fluorophore or steroid structural group:

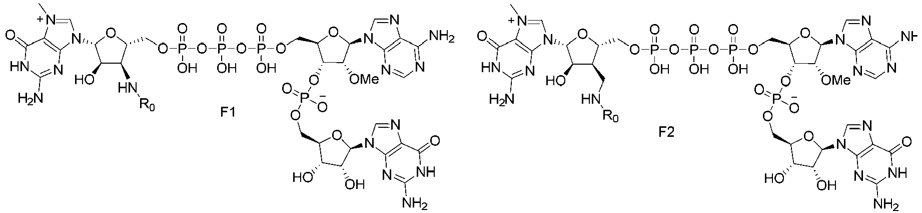

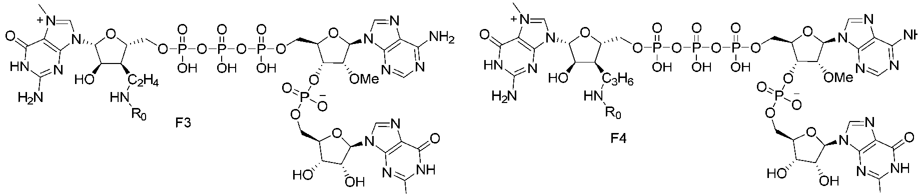

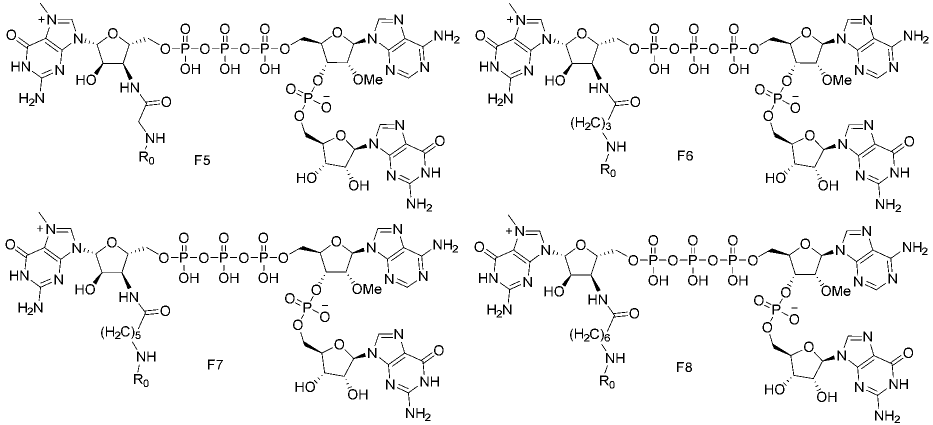

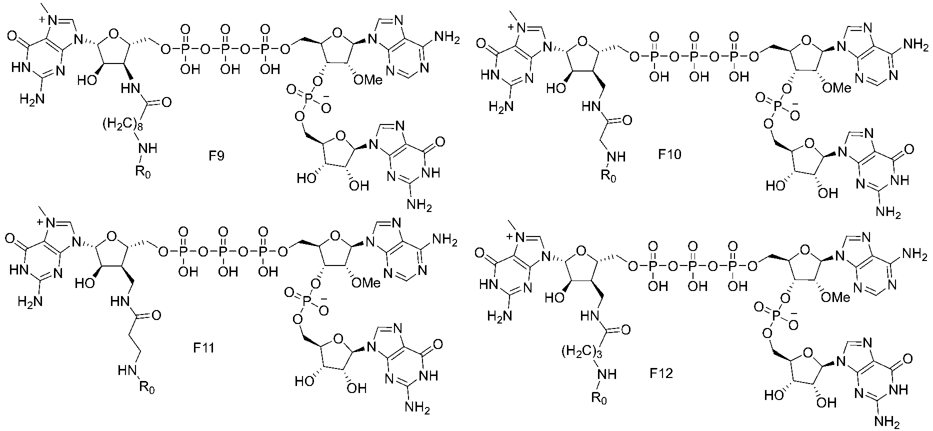

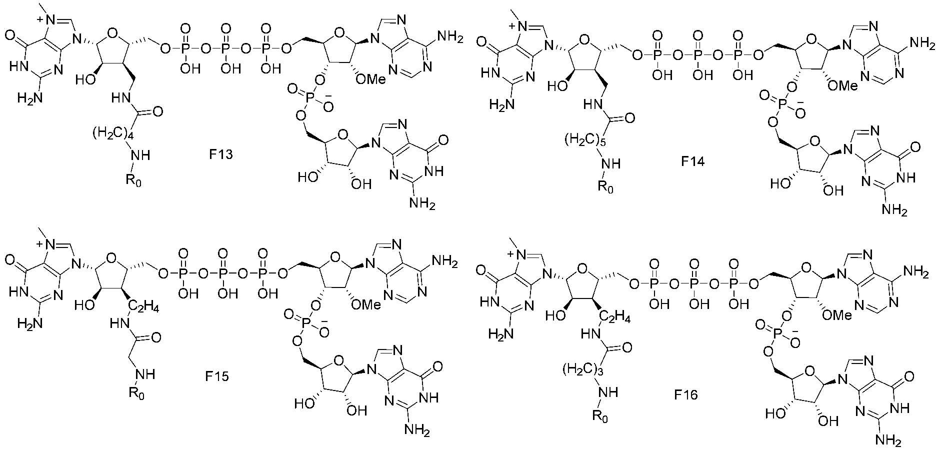

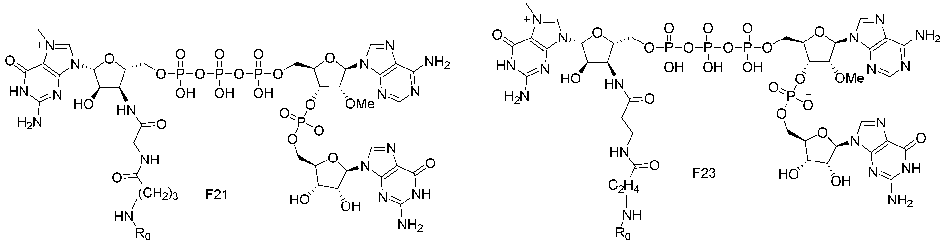

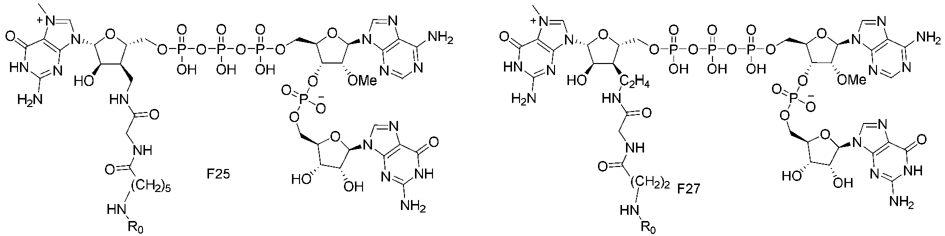

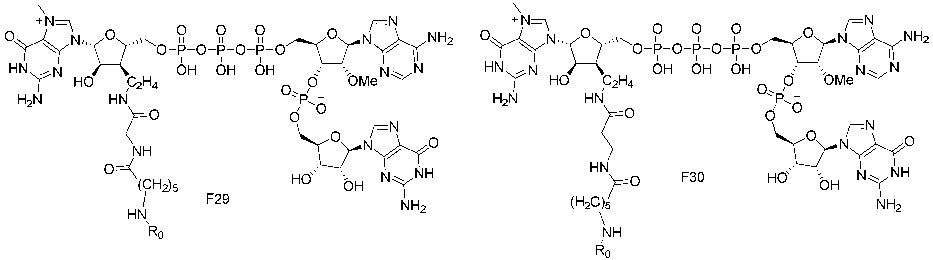

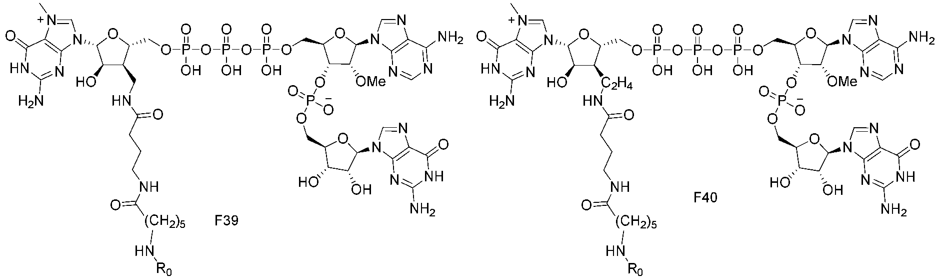

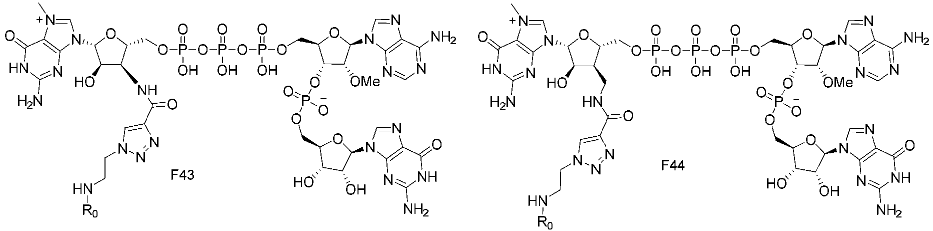

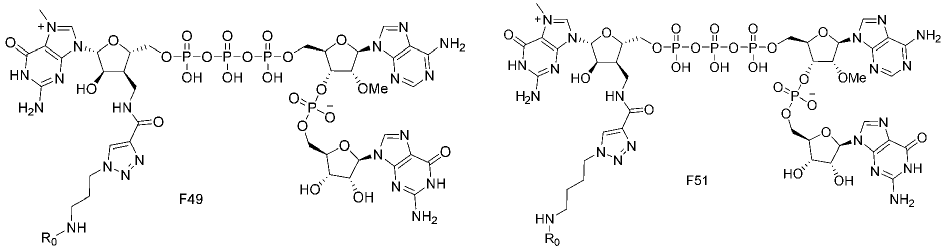

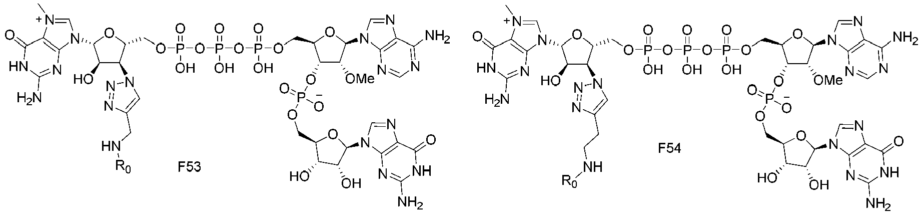

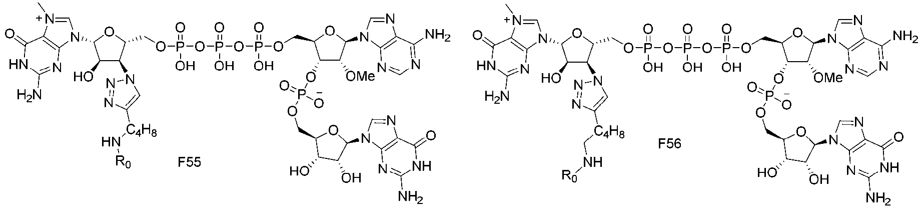

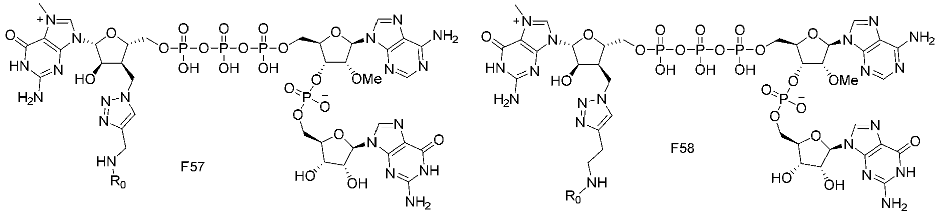

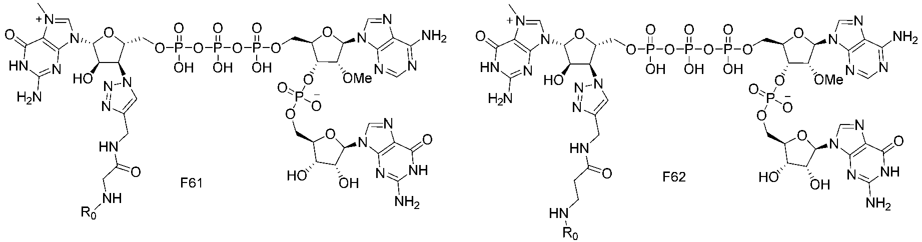

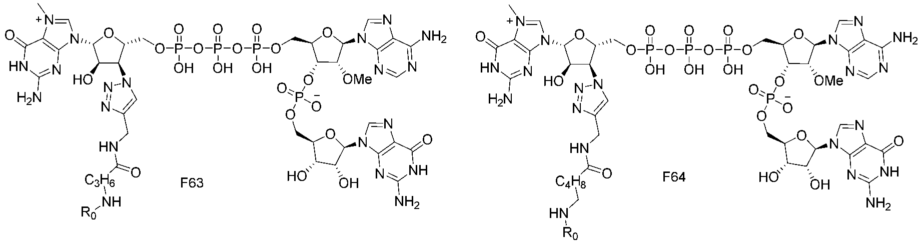

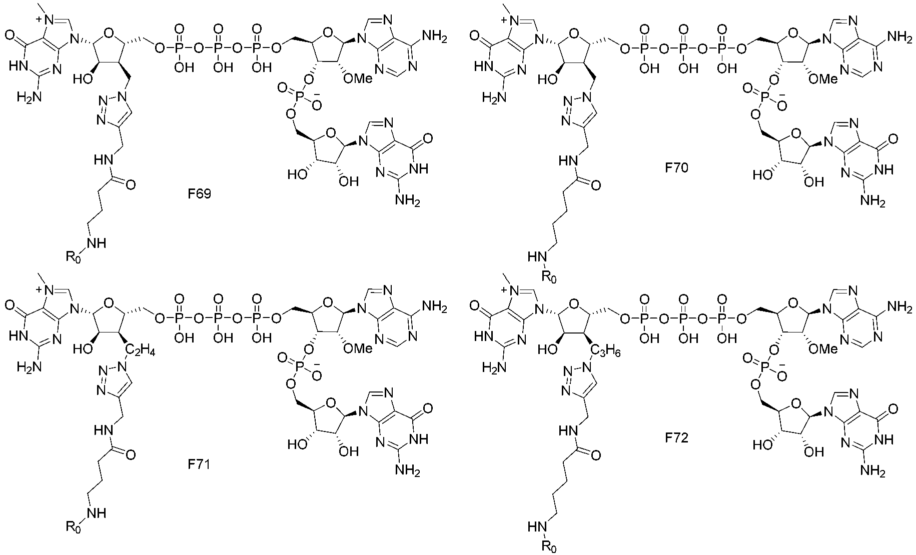

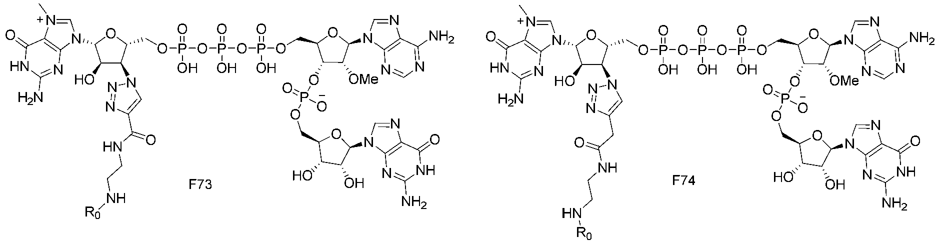

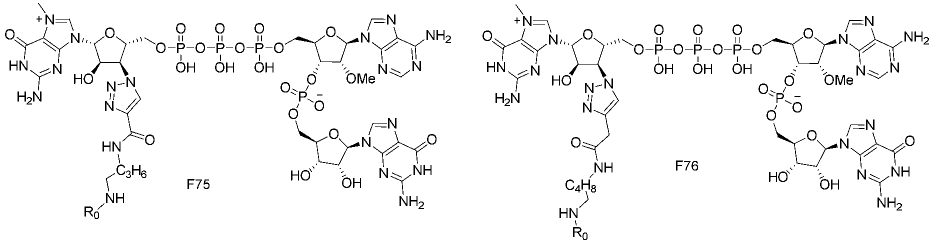

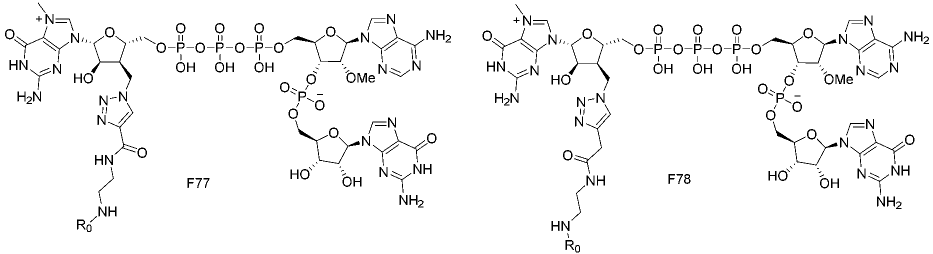

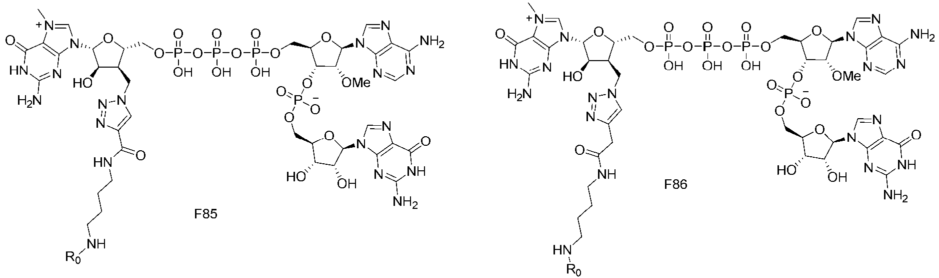

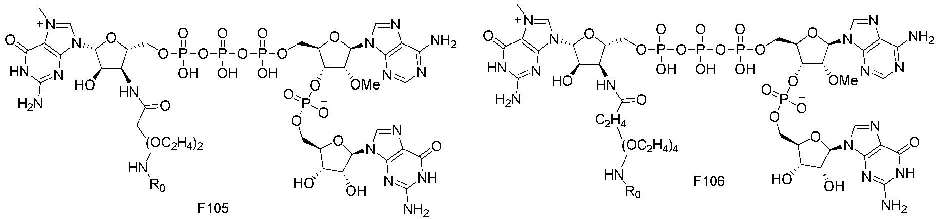

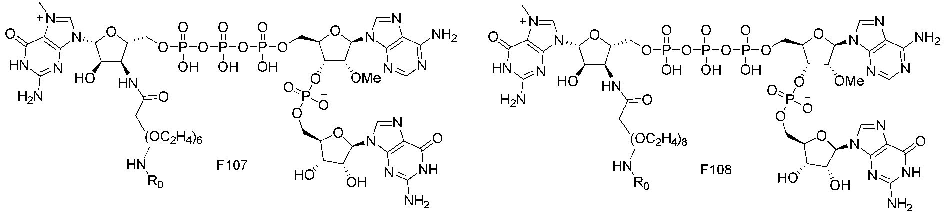

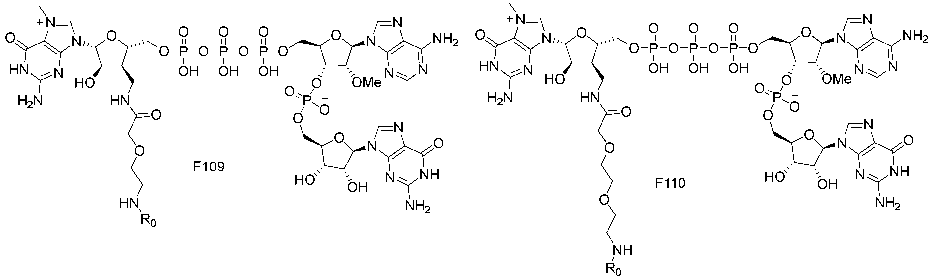

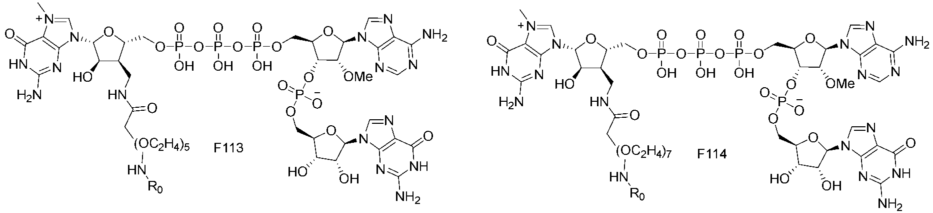

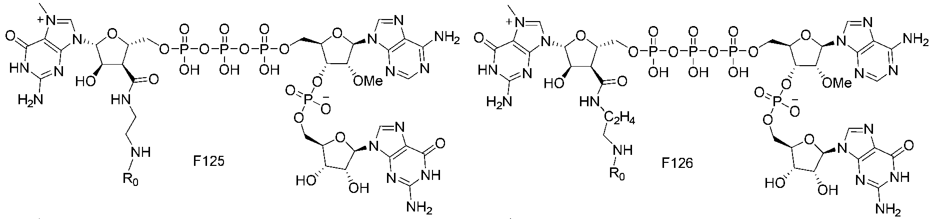

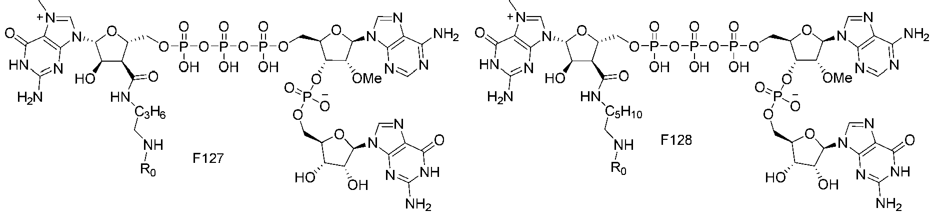

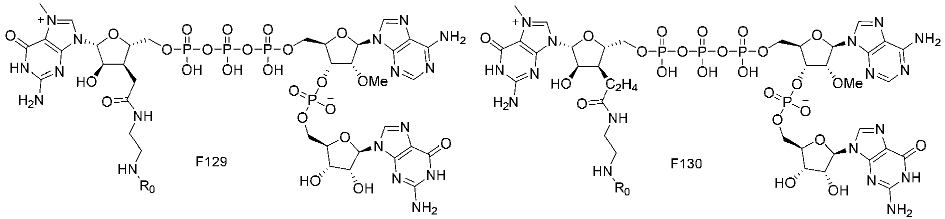

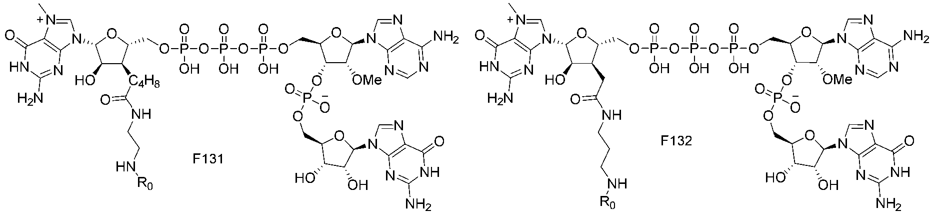

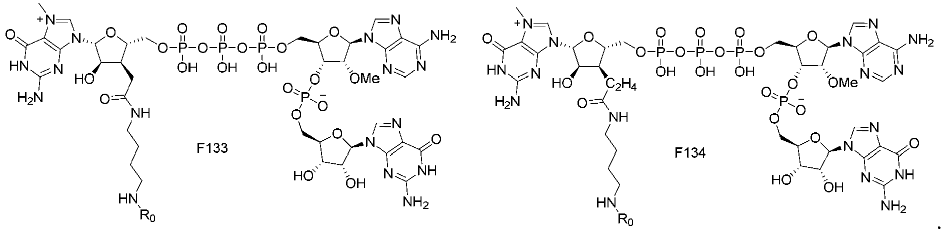

In some more preferred embodiments, R₀ is a fluorophore formed by biotin, Cy3, Cy5, Cy7, 5-FAM or 6-FAM, or is a steroid structural group formed by cholic acid, deoxycholic acid, ursodeoxycholic acid, chenodeoxycholic acid, hyodeoxycholic acid, or glycoursodeoxycholic acid.

In some more preferred embodiments, the compound is represented by any one of the following structures (non-exhaustive):

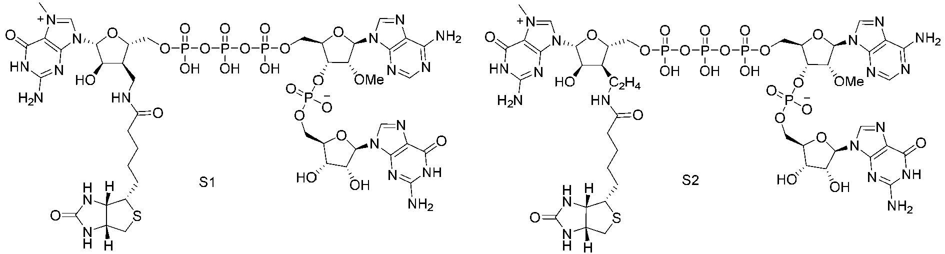

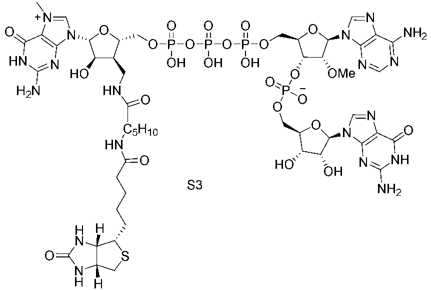

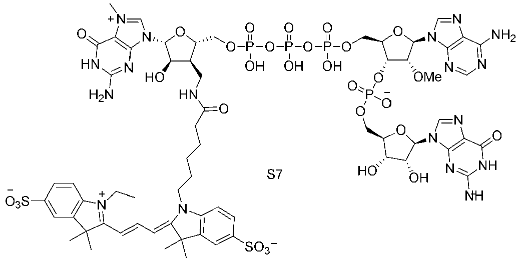

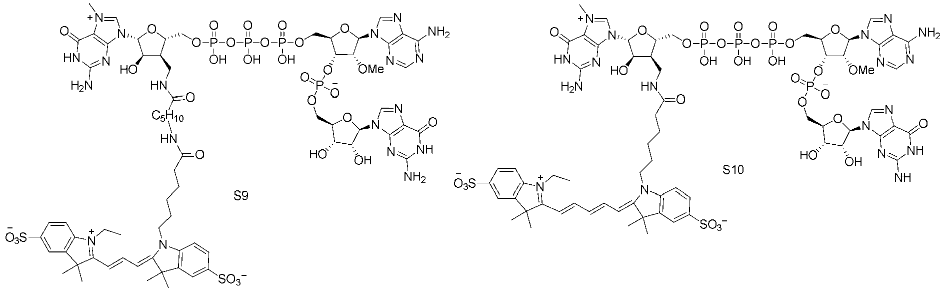

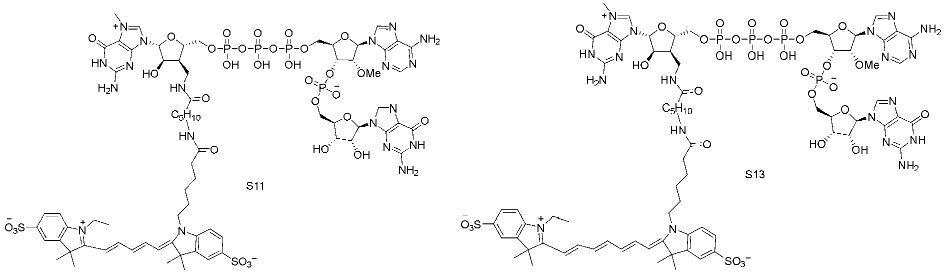

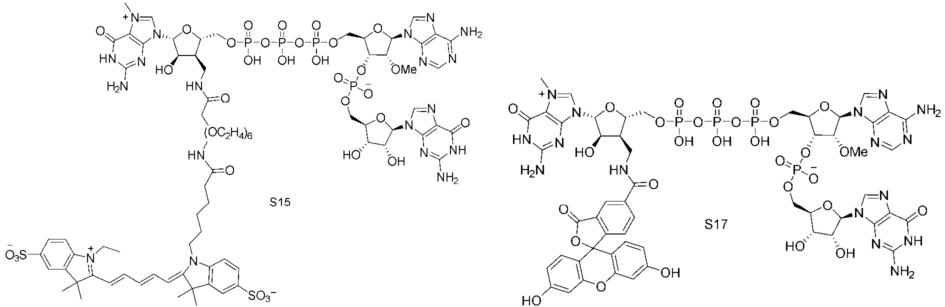

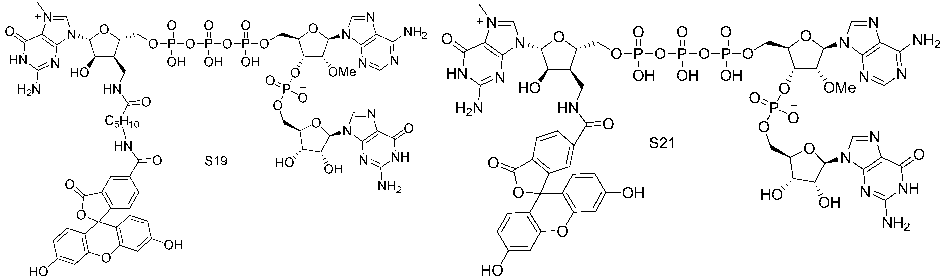

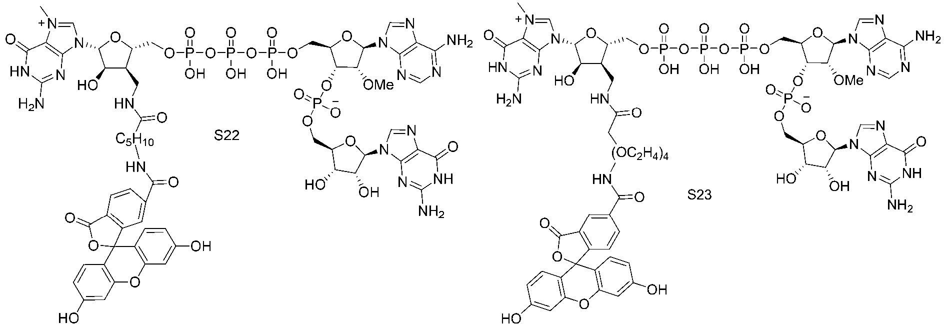

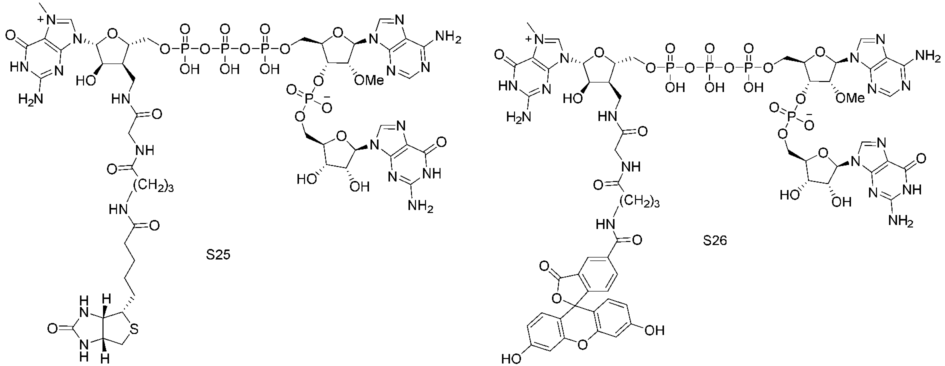

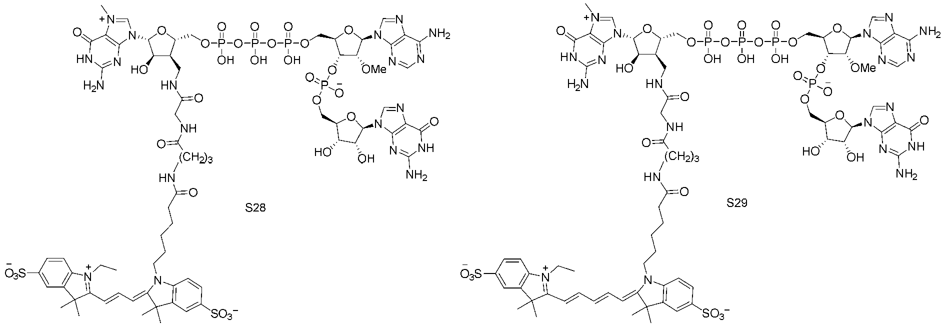

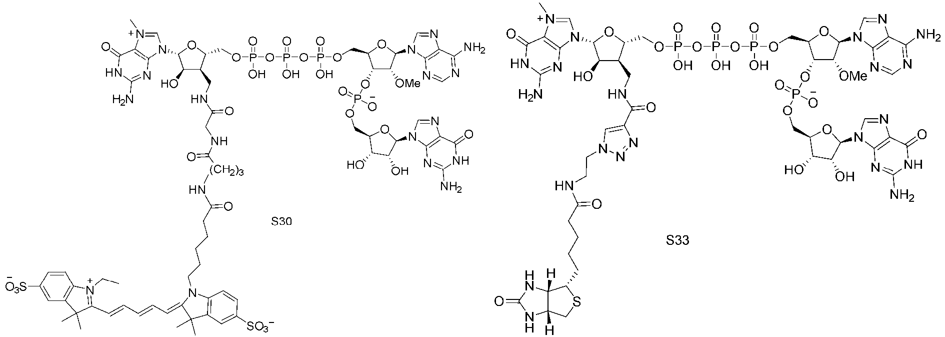

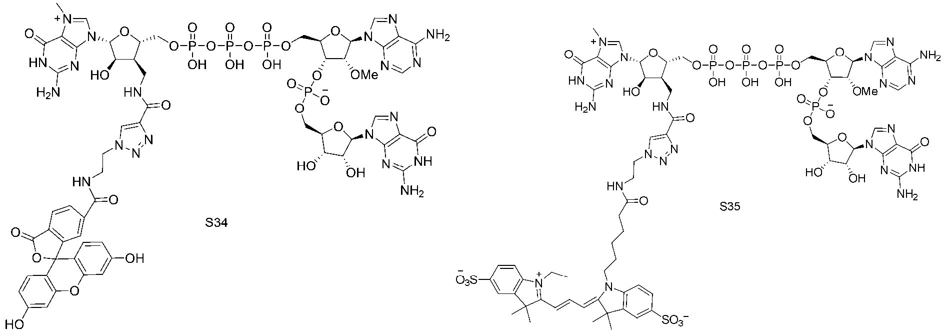

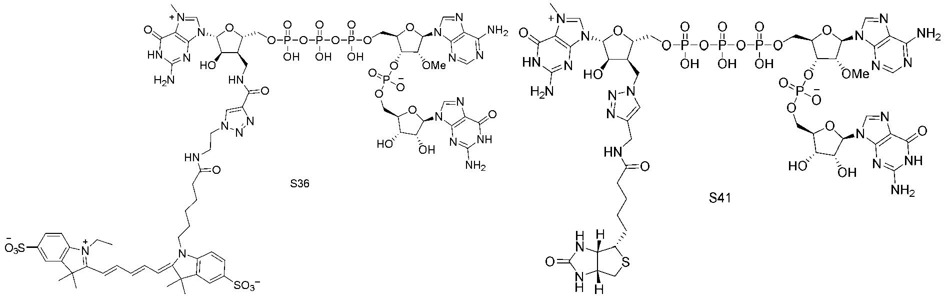

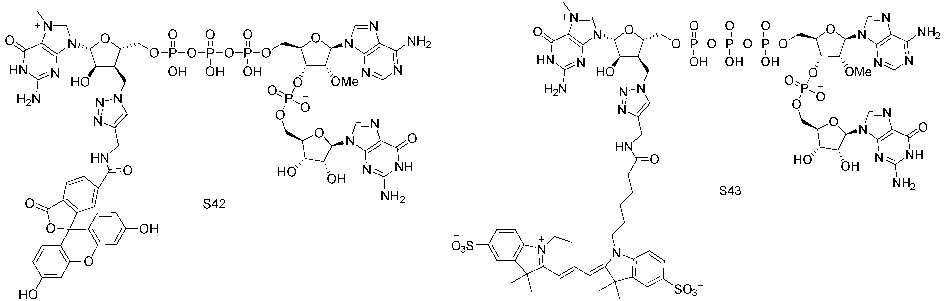

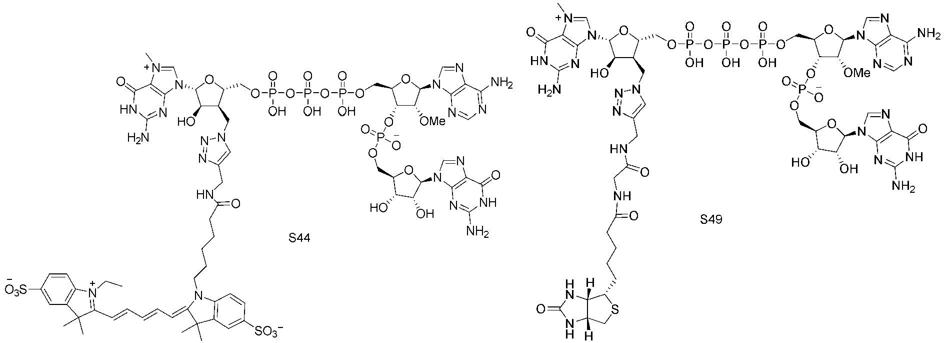

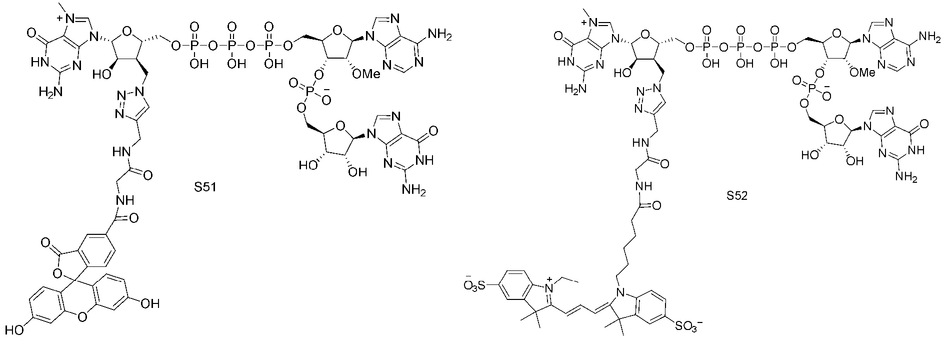

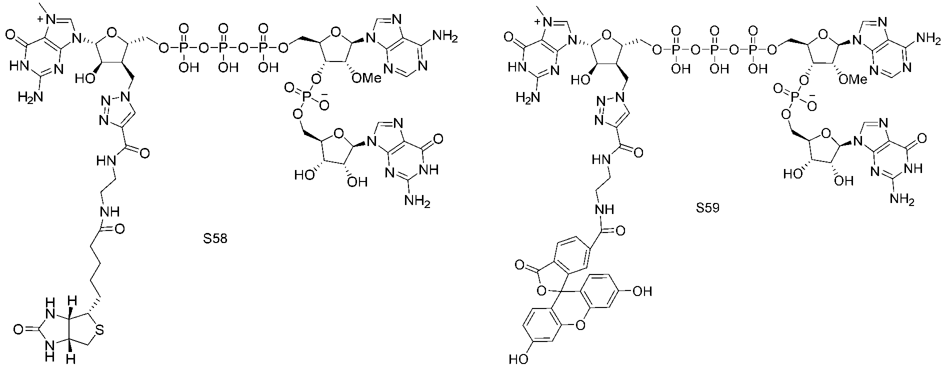

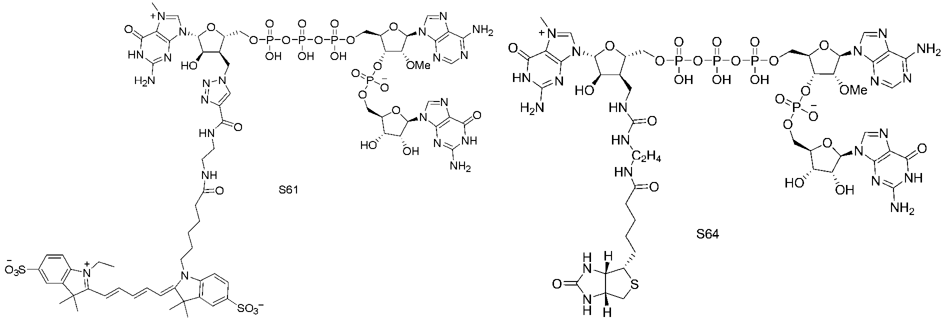

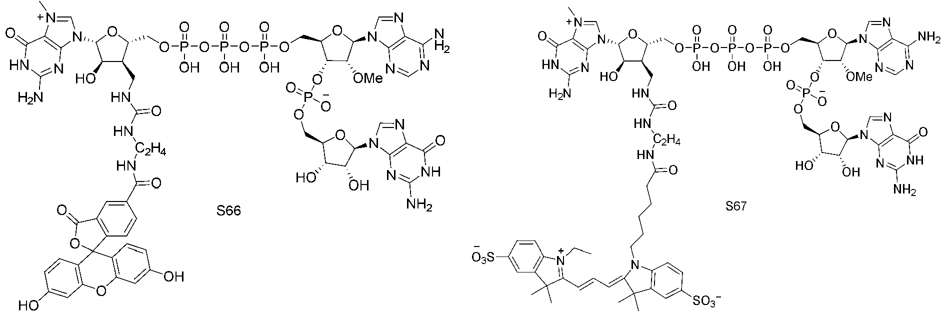

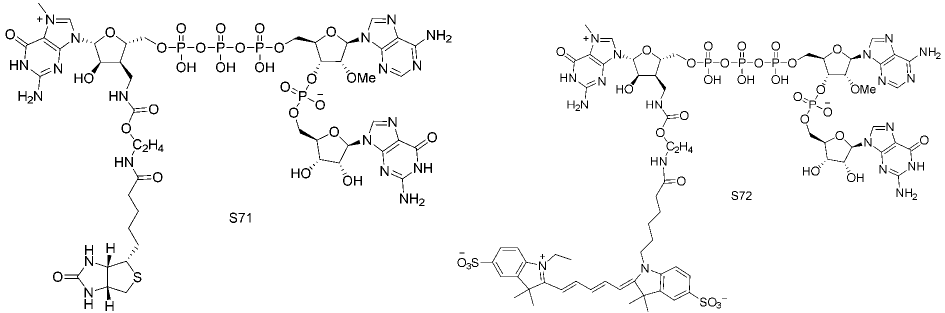

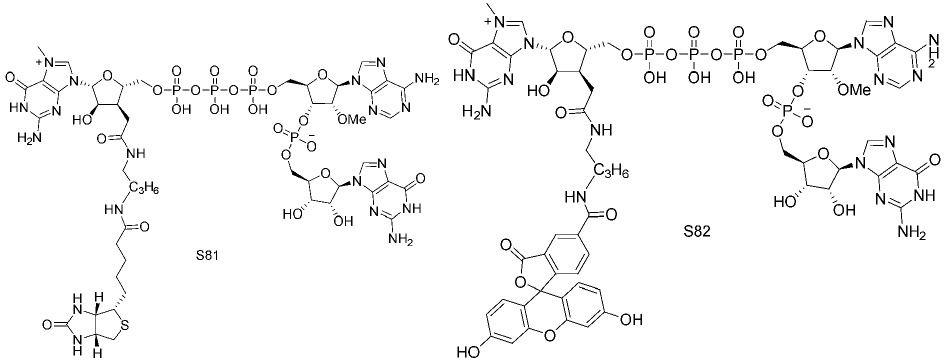

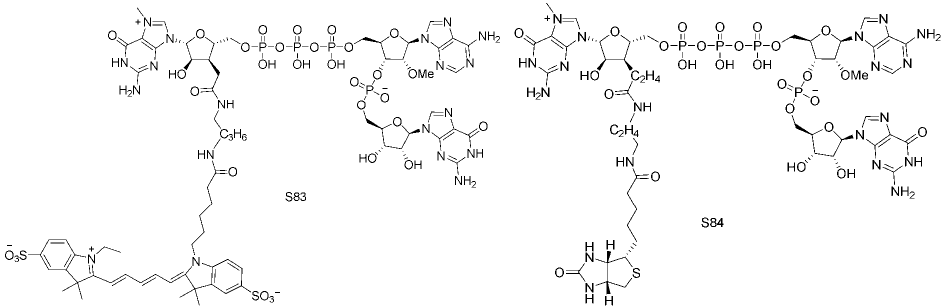

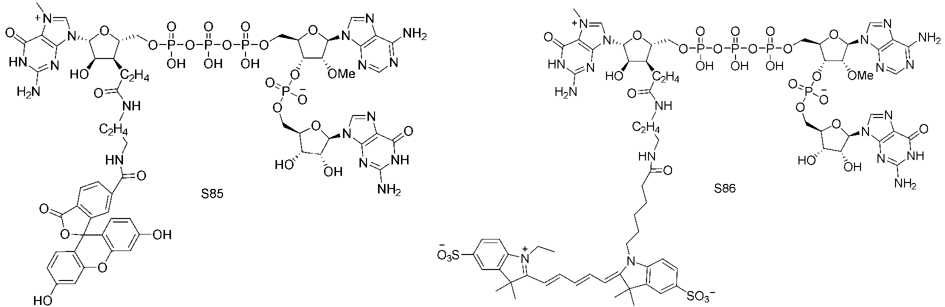

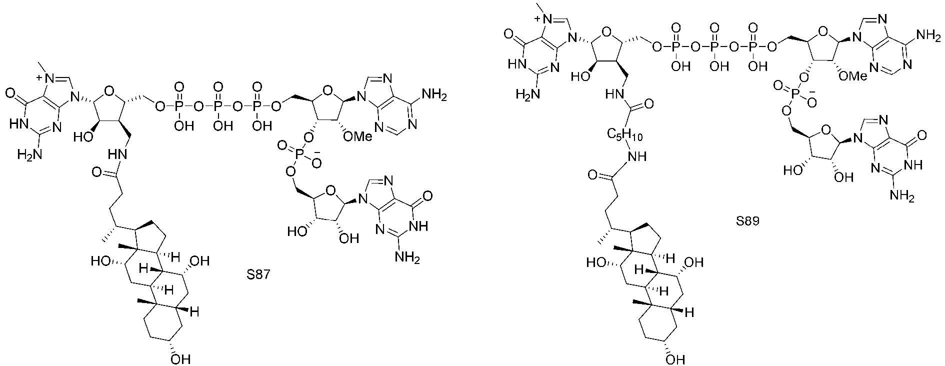

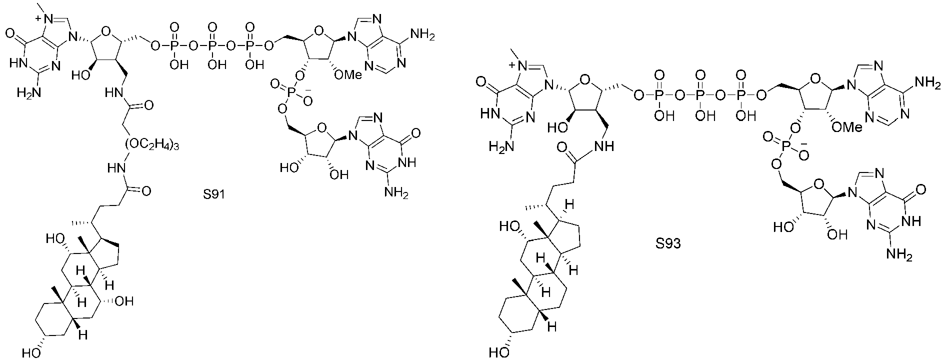

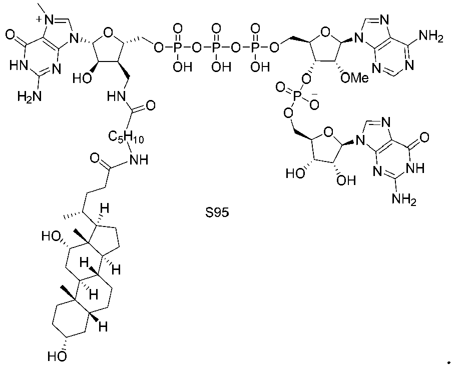

The present disclosure also protects a compound having any one of the following structures, or a stereoisomer, pharmaceutically acceptable salt or solvate thereof, wherein the compound has a structure of:

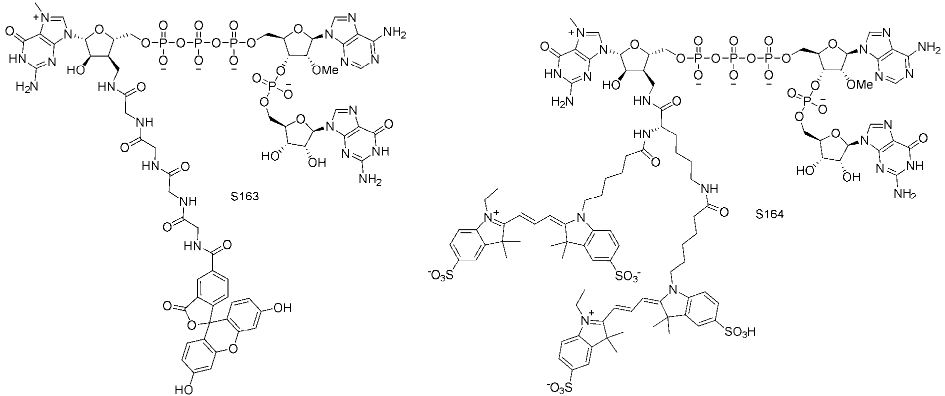

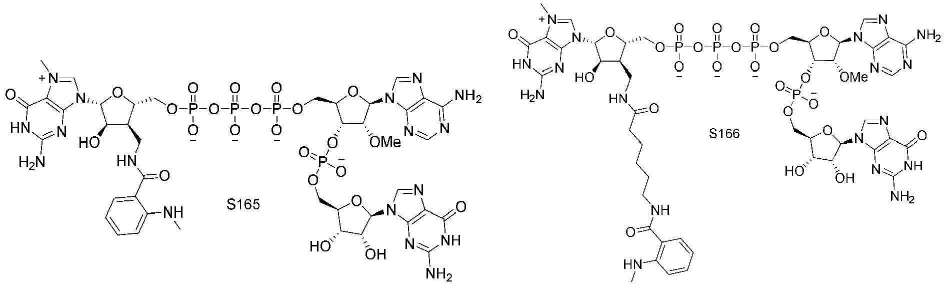

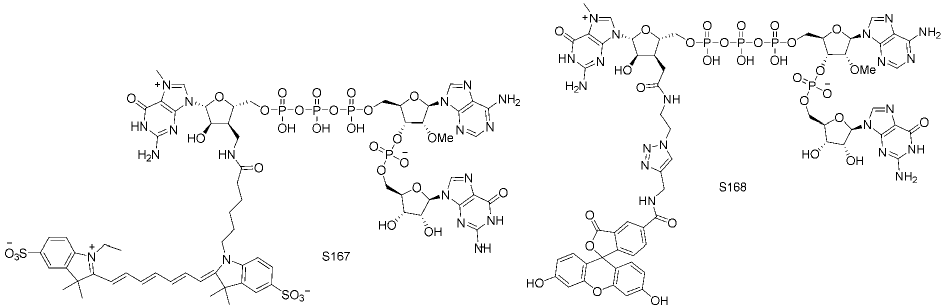

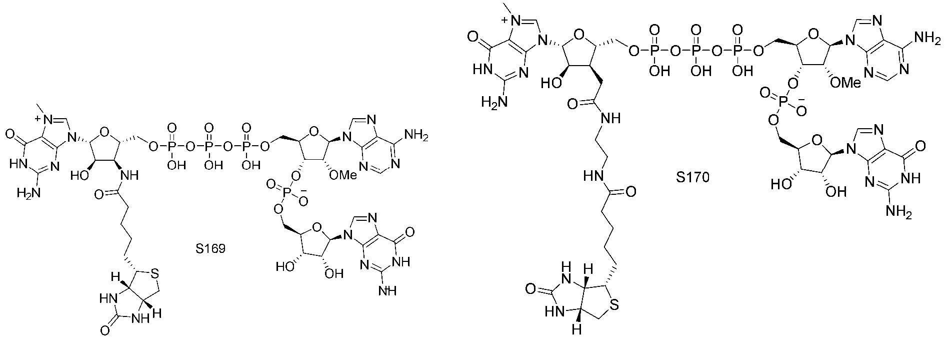

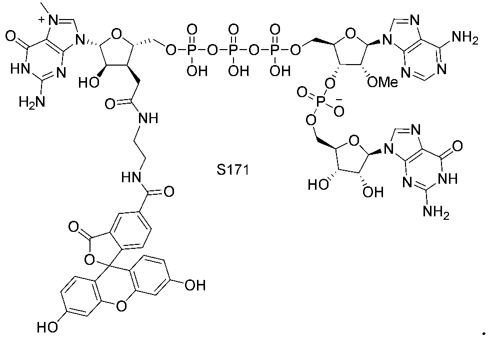

A second objective of the present disclosure is to provide a compound having a structure represented by formula IV, or a stereoisomer, pharmaceutically acceptable salt or solvate thereof: 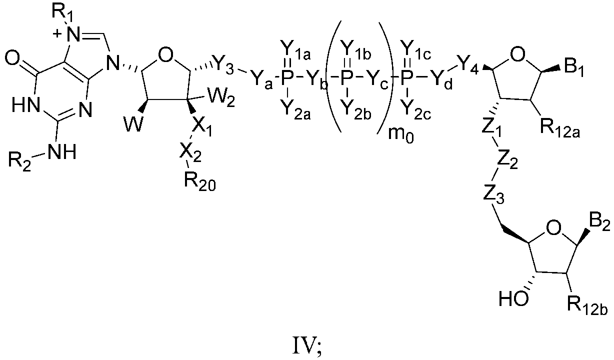
wherein R₁ is C₁-C₆ alkyl, C₂-C₆ alkenyl, C₂-C₆ alkynyl, C₃-C₆ cycloalkyl, C₃-C₆ cycloalkenyl, benzyl, R₅-substituted C₁-C₆ alkyl, R₅-substituted C₂-C₆ alkenyl, R₅-substituted C₂-C₆ alkynyl, R₅-substituted C₃-C₆ cycloalkyl, R₅-substituted C₃-C₆ cycloalkenyl or R₅-substituted benzyl;
R₂ is H, C₁-C₆ alkyl, C₂-C₆ alkenyl, C₂-C₆ alkynyl, C₃-C₈ cycloalkyl, cycloalkenyl, benzyl, aryl, heteroaryl, R₅-substituted benzyl, R₅-substituted aryl, carbonylalkyl, carbonylalkoxy or sulfonamido;
W is H, OH, OR₄, NR₄R₄, NR₄COR₄, F, Cl, N₃ or CN;
W₂ is H, OH, halogen, N₃, CN, OR₄, NR₄R₄, NR₄COR₄ or C₁-C₄ alkyl;
X₁ is (CH₂)ₙ₁, NR₄ or absent; n₁ is 1, 2 or 3;
X₂ is O, S, NR₄, CO, CO₂, CONR₄, NR₄CO, NR₄CO₂, NR₄CONR₄, SO₂, SO₂NR₄, CH₂ or absent;
R₂₀ is H, C₁-C₈ alkyl, C₂-C₈ alkenyl, C₂-C₈ alkynyl, R₅-substituted C₁-C₈ alkyl, R₅-substituted C₂-C₈ alkenyl, R₅-substituted C₂-C₈ alkynyl, aryl, R₅-substituted aryl, heteroaryl, R₅-substituted heteroaryl, halogen, CN or N₃;
R₁₂ₐ and R_{12b} are each independently H, halogen, alkoxy, C₁-C₆ alkyl, C₂-C₆ alkenyl, C₂-C₆ alkynyl, R₅-substituted C₁-C₆ alkyl, R₅-substituted C₂-C₆ alkenyl or R₅-substituted C₂-C₆ alkynyl;
or R₁₂ₐ and R_{12b} are each independently OH or R₅-substituted C₁-C₆ alkoxy;
Yₐ, Y_{b}, Y_{c} and Y_{d} are each independently O, S, CH₂, CCl₂, CF₂ or NH;
Y₁ₐ, Y_{1b} and Y_{1c} are each independently O or S;
Y₂ₐ, Y_{2b} and Y_{2c} are each independently OH, SH or BH₃;
Y₃ and Y₄ are each independently CH₂ or O;
Z₁ is O, OH, CH₂, S, NR₆, CO or SO₂;
Z₂ and Z₃ are each independently O, NR₆, CHR₇, CHCOOR₇, CHCONR₇R₇, S, CO, SO₂, PO(OH), PO(SH), P(O)VCO₂H or absent; Z₂ may form a ring with an oxygen atom connected to R₃ₐ;
Z₄ is O, CH₂, S, NR₆, CO, SO₂ or absent;
B₁ and B₂ are each independently a natural or modified pyrimidine nucleotide base or a natural or modified purine nucleotide base;
R₄ is H, C₁-C₈ alkyl, C₂-C₈ alkenyl or C₂-C₈ alkynyl;
R₅ is halogen, CN, SO₂, NO₂, D, N₃, C₁-C₈ alkyl, C₂-C₈ alkenyl, C₂-C₈ alkynyl, C₁-C₈ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₆ alkyl, COR₈ or SO₂R₈;
R₇ is H, halogen, C₁-C₈ alkyl, C₂-C₈ alkenyl or C₂-C₈ alkynyl;
R₈ is H, C₁-C₈ alkyl, C₂-C₈ alkenyl or C₂-C₈ alkynyl;
V is C₁-C₄ alkyl;
m₀ is 0, 1 or 2.

In some embodiments, R₄ is H, C₁-C₄ alkyl, C₂-C₅ alkenyl or C₂-C₅ alkynyl;
R₅ is halogen, CN, SO₂, NO₂, D, N₃, C₁-C₄ alkyl, C₂-C₅ alkenyl, C₂-C₅ alkynyl, C₁-C₄ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₄ alkyl, COR₈ or SO₂R₈;
R₇ is H, halogen, C₁-C₄ alkyl, C₂-C₅ alkenyl or C₂-C₅ alkynyl;
R₈ is H, C₁-C₄ alkyl, C₂-C₅ alkenyl or C₂-C₅ alkynyl;
more preferably, R₅ is halogen, CN, SO₂, NO₂, D, N₃, C₁-C₄ alkyl, C₂-C₅ alkenyl, C₂-C₅ alkynyl, C₁-C₄ alkoxy, pyridyl, pyrimidinyl or morpholinyl.

In some embodiments, W is H, OH, C₁-C₄ alkoxy, C₁-C₄ alkylamino, F, Cl, N₃, or CN;
W₂ is H, OH, halogen, N₃, CN, C₁-C₄ alkyl, C₁-C₄ alkoxy or C₁-C₄ alkylamino;
X₁ is (CH₂)ₙ₁, C₁-C₄ alkyl-substituted amino or absent; n₁ is 1, 2 or 3;
X₂ is O, S, CO, CO₂, CONR₄, NR₄CO, NR₄CO₂, NR₄CONR₄, SO₂ or SO₂NR₄;
R₁₂ₐ and R_{12b} are each independently OH, C₁-C₆ alkyl, C₁-C₆ alkoxy, R₅-substituted C₁-C₆ alkyl or R₅-substituted C₁-C₆ alkoxy;
R₂₀ is H, C₁-C₄ alkyl, C₂-C₅ alkenyl, R₅-substituted C₁-C₄ alkyl, R₅-substituted aryl, heteroaryl, N₃ or absent.

In some more preferred embodiments, X₁ is CH₂, C₂H₄, C₃H₆, NH, N(CH₃), N(C₂H₅), N(C₃H₇), N(C₄H₉), N(C₂H₃), N(C₃H₅), N(C₄H₇), N(C₂H), N(C₃H₃), N(C₄H₅), N(C₅H₇) or absent;
X₂ is O, S, CO, CO₂, CONH, CON(CH₃), CON(C₂H₅), CON(C₃H₇), CON(C₄H₉), CON(C₃H₅), CON(C₄H₇), CON(C₃H₃), CON(C₄H₅), CON(C₅H₇),
N(CH₃)CO, N(C₂H₅)CO, N(C₃H₇)CO, N(C₄H₉)CO, N(C₂H₃)CO, N(C₃H₅)CO, N(C₄H₇)CO, N(C₂H)CO, N(C₃H₃)CO, N(C₄H₅)CO, N(C₅H₇)CO,
N(CH₃)COO, N(C₂H₅)COO, N(C₃H₇)COO, N(C₄H₉)COO, N(C₂H₃)COO, N(C₃H₇)COO, N(C₄H₉)COO, N(C₂H)COO, N(C₃H₃)COO, N(C₄H₅)COO, N(C₅H₇)COO,
N(CH₃)CON(CH₃), N(CH₃)CON(C₂H₅), N(CH₃)CON(C₄H₉), N(CH₃)CON(C₂H₃), N(CH₃)CON(C₄H₇), N(CH₃)CON(C₃H₃), N(C₂H₅)CON(CH₃), N(C₂H₅)CON(C₂H₅), N(C₂H₅)CON(C₄H₉), N(C₂H₅)CON(C₂H₃), N(C₂Hs)CON(C₄H₉), N(C₂H₅)CON(C₃H₃), N(C₃H₇)CON(CH₃), N(C₃H₇)CON(C₂Hs), N(C₃H₇)CON(C₄H₉), N(C₃H₇)CON(C₂H₃), N(C₃H₇)CON(C₄H₉), N(C₃H₇)CON(C₃H₃),
SO₂, SO₂NH, SO₂N(CH₃), SO₂N(C₂H₅), SO₂N(C₃H₇), SO₂N(C₄H₉), SO₂N(C₂H₃), SO₂N(C₃H₅), SO₂N(C₄H₇), SO₂N(C₂H), SO₂N(C₃H₃), SO₂N(C₄H₅) or SO₂N(C₅H₇).

In some more preferred embodiments, X₁ is CH₂, C₂H₄, C₃H₆, NH, N(CH₃), N(C₂H₅), N(C₃H₇) or N(C₄H₉).

In some embodiments, R₁₂ₐ and R_{12b} are each independently OH, F, Cl, C₁-C₄ alkyl, C₁-C₄ alkoxy, C₁-C₄ haloalkyl, or C₁-C₄ haloalkoxy;
W is OH;
Yₐ, Y_{b}, Y_{c} and Y_{d} are each independently O or CH₂;
Y₁ₐ, Y_{1b} and Y_{1c} are each independently O or S;
Y₂ₐ, Y_{2b} and Y_{2c} are each independently OH;
Y₃ and Y₄ are independently CH₂ or O;
Z₁ is O, OH, CH₂, S or NH;
Z₂ and Z₃ are each independently O, NH, CHR₇, CHCOOR₇, CO, SO₂ or PO(OH);
B₁ and B₂ are each independently a natural or modified pyrimidine nucleotide base or a natural or modified purine nucleotide base;
R₄ is H or C₁-C₄ alkyl;
R₅ is halogen, OR₇, NR₇R₇, CONR₇R₇ or OCONR₇R₇;
R₆ is H;
R₇ is H or C₁-C₄ alkyl;
m₀ is 0, 1 or 2.

In some embodiments, R₁ is C₁-C₄ alkyl, C₂-C₄ alkenyl, C₂-C₄ alkynyl, C₃-C₆ cycloalkyl, C₃-C₆ cycloalkenyl, benzyl, C₁-C₄ haloalkyl, C₂-C₄ haloalkenyl, C₂-C₄ haloalkynyl, C₃-C₆ halocycloalkyl, C₃-C₆ halocycloalkenyl or halobenzyl;
R₂ is H, C₁-C₄ alkyl, C₂-C₄ alkenyl, C₂-C₄ alkynyl, C₃-C₆ cycloalkyl, C₃-C₆ cycloalkenyl, C₁-C₄ haloalkyl, C₂-C₄ haloalkenyl, C₂-C₄ haloalkynyl, C₃-C₆ halocycloalkyl, C₃-C₆ halocycloalkenyl, benzyl or halobenzyl.

In some preferred embodiments, the compound has a structure represented by any one of the following general formulas: IV-2, IV-3, IV-4, IV-5 or IV-6: 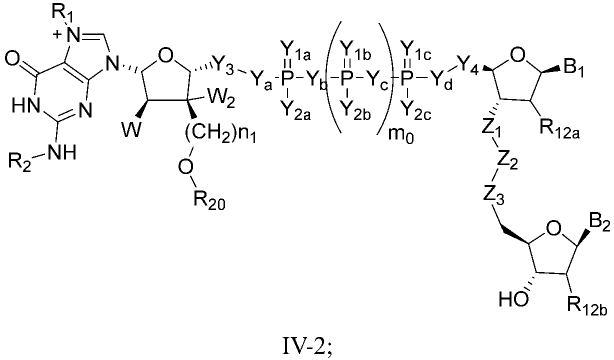 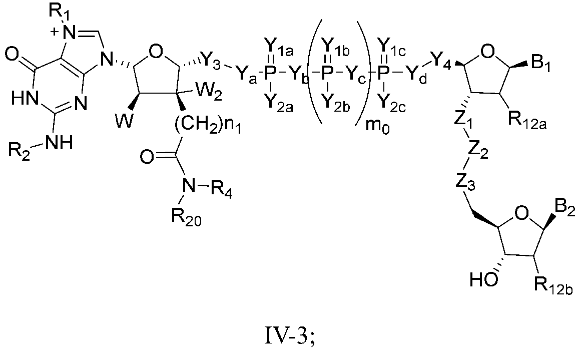 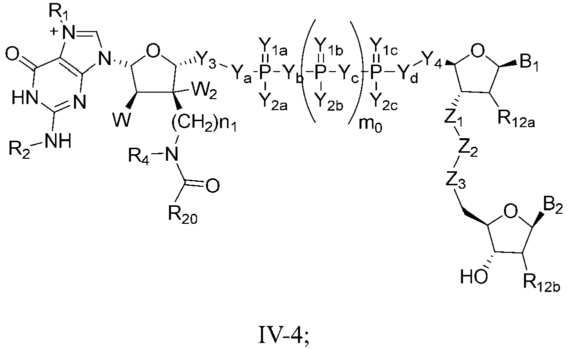 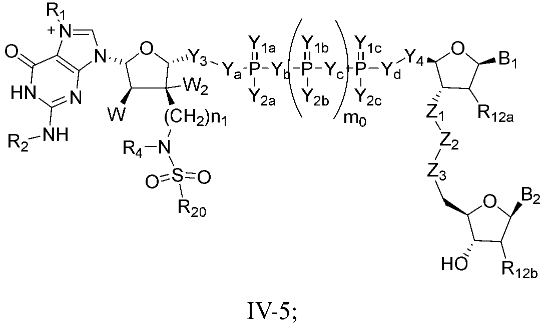 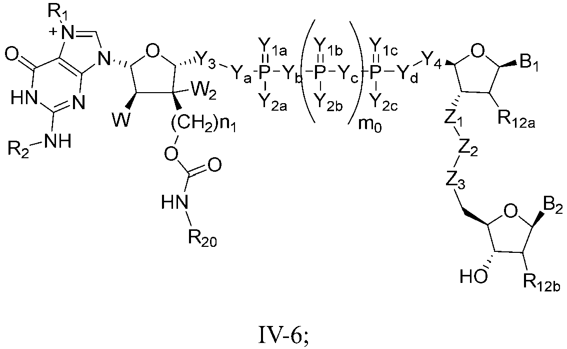 wherein R₁, R₂, R₂₀, R₁₂ₐ, R_{12b}, R₄, R₅, R₆, R₇, R₈, W, W₂, Yₐ, Y_{b}, Y_{c}, Y_{d}, Y₁ₐ, Y₁₆, Y_{1c}, Y₂ₐ, Y_{2b}, Y_{2c}, Y₃, Y₄, B₁, B₂, Z₁, Z₂, Z₃, B₁, B₂, V, n₁ and m₀ are as defined above, respectively.

In some embodiments, R₁ is methyl, ethyl, n-propyl, isopropyl, butyl, vinyl, propenyl, butenyl, ethynyl, propynyl, butynyl, benzyl, halomethyl, haloethyl, halo-n-propyl, haloisopropyl, halobutyl, halovinyl, halopropenyl, halobutenyl, haloethynyl, halopropynyl, halobutynyl or halobenzyl;
R₂ is H, methyl, ethyl, n-propyl, isopropyl, fluoromethyl, difluoromethyl, trifluoromethyl, trifluoroethyl, trifluoropropyl or trifluoroisopropyl;
W is OH, F, Cl, methyl, ethyl, methoxy or ethoxy;
W₂ is H, OH, F, Cl, N₃, CN, methyl, ethyl, propyl, isopropyl, methoxy, ethoxy, methyl-substituted amino or ethyl-substituted amino;
R₄ is H, methyl, ethyl, n-propyl, isopropyl, butyl, vinyl, propenyl, butenyl, ethynyl, propynyl or butynyl;
R₂₀ is H, C₁-C₄ alkyl, C₂-C₅ alkenyl, R₅-substituted C₁-C₄ alkyl, R₅-substituted aryl, heteroaryl, N₃ or absent;
R₅ is halogen, CN, D, N₃, C₁-C₄ alkyl, C₂-C₅ alkenyl, C₂-C₅ alkynyl, C₁-C₄ alkoxy, pyridyl, pyrimidinyl or morpholinyl.

In some embodiments, R₁₂ₐ and R_{12b} are each independently OH, F, Cl, C₁-C₄ alkyl, C₁-C₄ alkoxy, C₁-C₄ haloalkyl, or C₁-C₄ haloalkoxy;
W is OH;
Z₁ is O, OH, CH₂, S or NH;
Z₂ and Z₃ are each independently O, NH, CH₂, CHCOO, CO, SO₂ or PO(OH);
B₁ and B₂ are independently a natural or modified cytosine nucleotide base, a natural or modified uracil nucleotide base, a natural or modified adenine nucleotide base or a natural or modified guanine nucleotide base;
m₀ is 0, 1 or 2.

In some preferred embodiments, the compound has a structure represented by formula V: 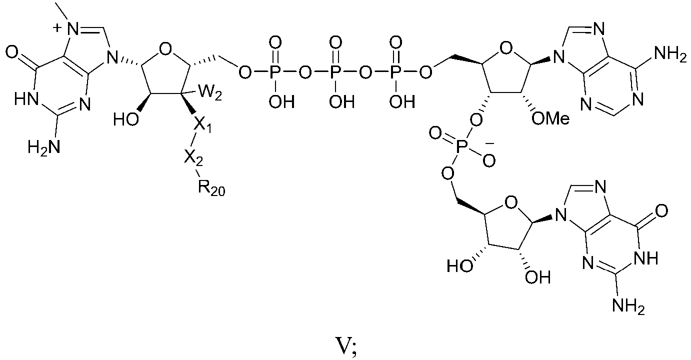
wherein W₂ is H, OH, halogen, N₃, CN, C₁-C₄ alkyl, C₁-C₄ alkoxy or C₁-C₄ alkylamino;
X₁ is (CH₂)ₙ₁, C₁-C₄ alkyl-substituted amino or absent; n₁ is 1, 2 or 3;
X₂ is O, S, CO, CO₂, CONR₄, NR₄CO, NR₄CO₂, NR₄CONR₄, SO₂ or SO₂NR₄;
R₂₀ is H, C₁-C₄ alkyl, C₂-C₅ alkenyl, R₅-substituted C₁-C₄ alkyl, R₅-substituted aryl, heteroaryl, N₃ or absent;
R₄ is H, C₁-C₄ alkyl, C₂-C₅ alkenyl or C₂-C₅ alkynyl;
R₅ is halogen, CN, SO₂, NO₂, D, N₃, C₁-C₄ alkyl, C₂-C₅ alkenyl, C₂-C₅ alkynyl, C₁-C₄ alkoxy, OR₇, SR₇, NR₇R₇, COR₇, COOR₇, OCOOR₇, CONR₇R₇, NHCOR₇, OCONR₇R₇, aryl or heteroaryl;
R₇ is H, halogen, C₁-C₄ alkyl, C₂-C₅ alkenyl or C₂-C₅ alkynyl.

In some more preferred embodiments, the compound has a structure represented by formula V-2, V-3, V-4, V-5 or V-6: 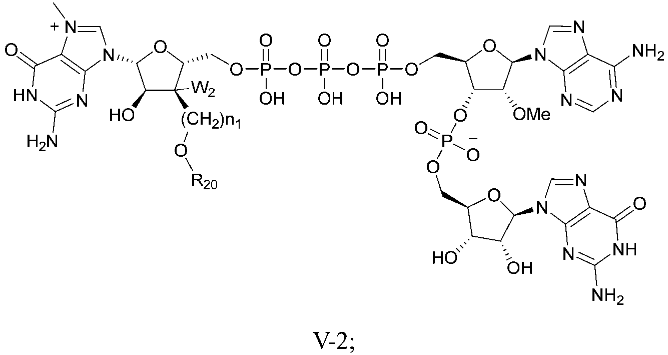 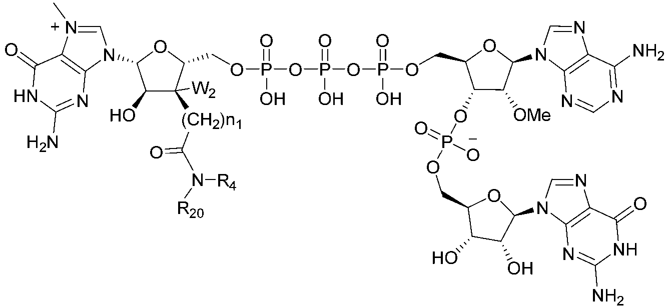 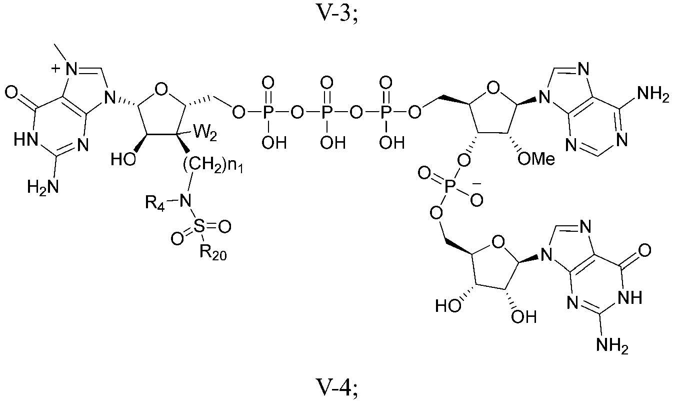 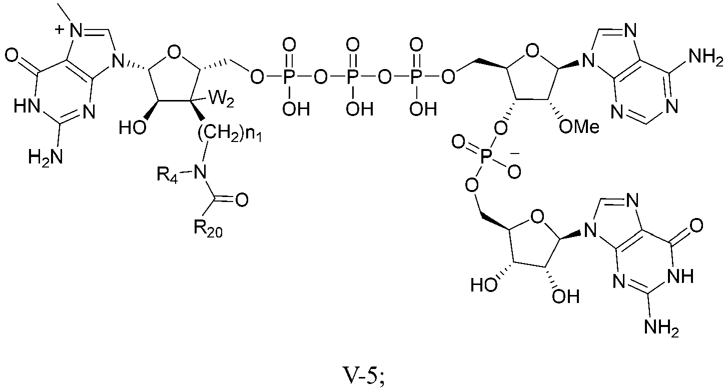 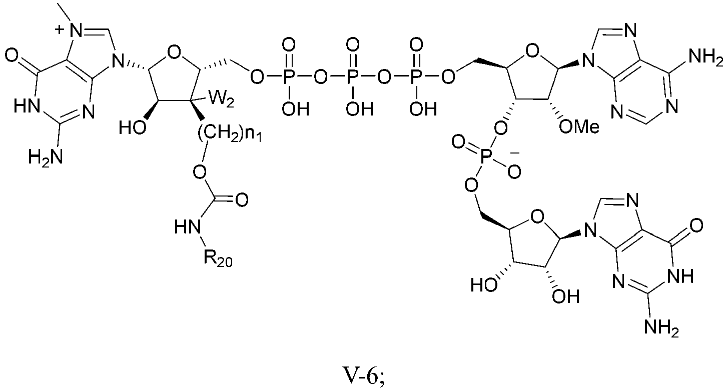
wherein W₂ is H, OH, F, Cl, N₃, CN, methyl, ethyl, propyl, isopropyl, methoxy, ethoxy, methyl-substituted amino or ethyl-substituted amino;
R₄ is H, methyl, ethyl, n-propyl, isopropyl, butyl, vinyl, propenyl, butenyl, ethynyl, propynyl or butynyl;
R₂₀ is H, C₁-C₄ alkyl, C₂-C₅ alkenyl, R₅-substituted C₁-C₄ alkyl, R₅-substituted aryl, heteroaryl, N₃ or absent;
R₅ is halogen, CN, D, N₃, C₁-C₄ alkyl, C₂-C₅ alkenyl, C₂-C₅ alkynyl, C₁-C₄ alkoxy, pyridyl, pyrimidinyl or morpholinyl.

In some embodiments, the compound is represented by any one of the following structures (non-exhaustive):

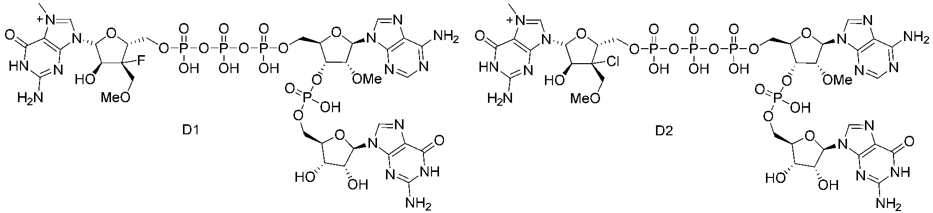

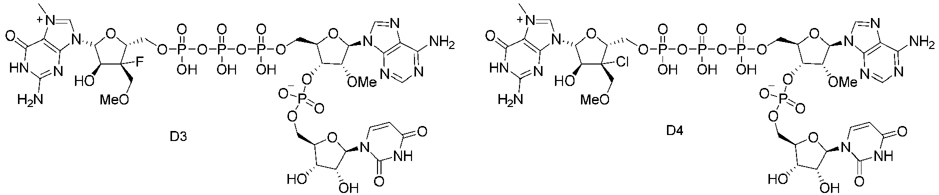

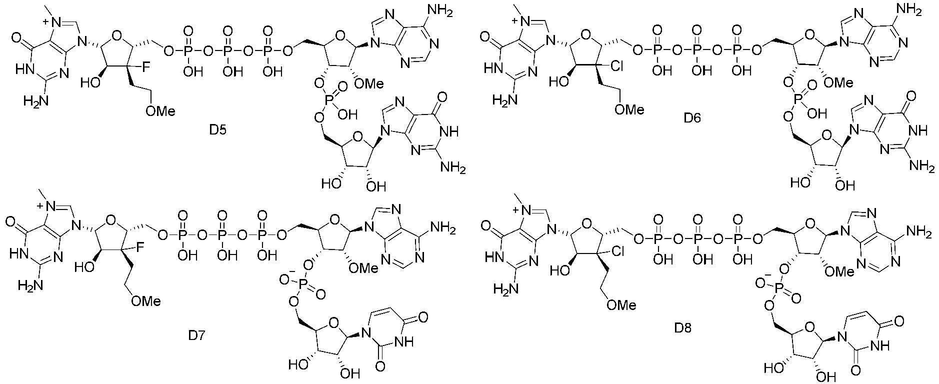

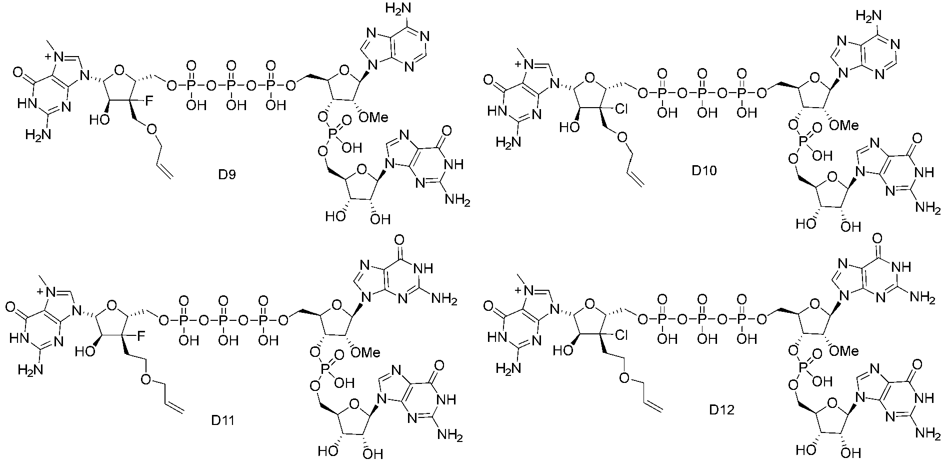

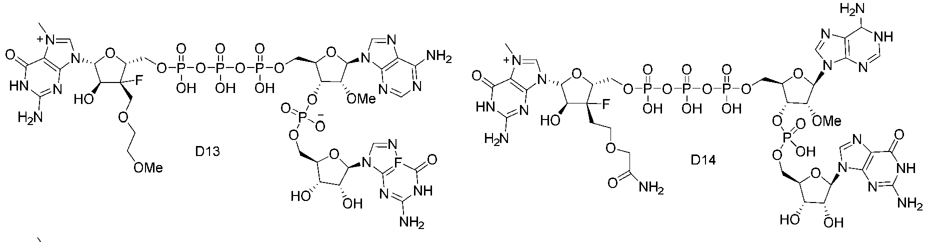

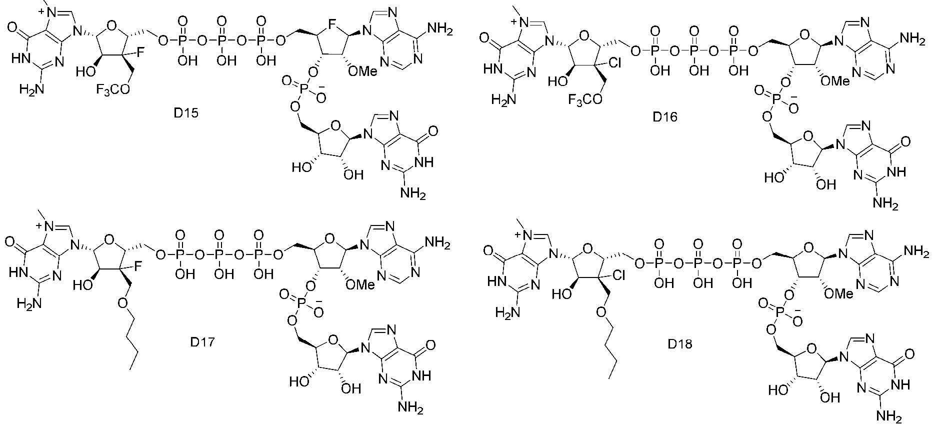

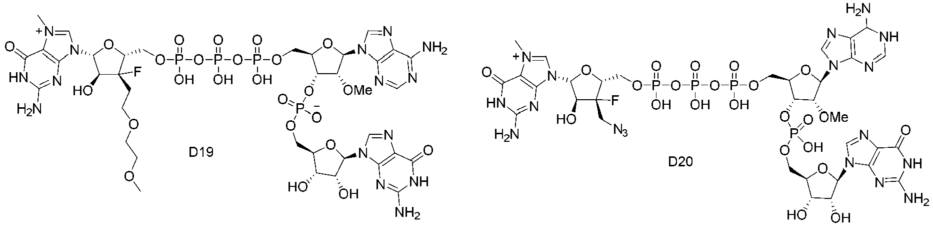

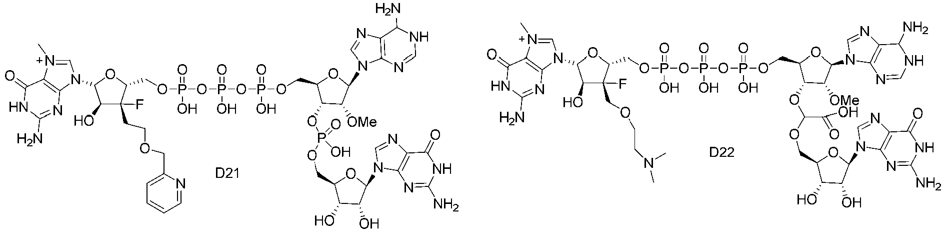

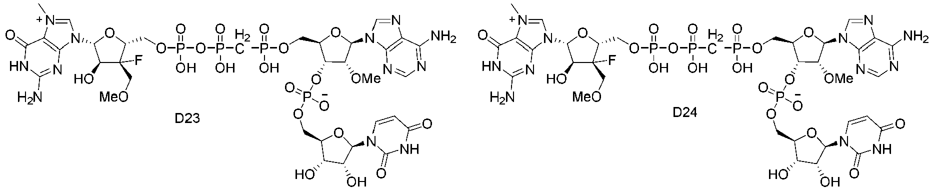

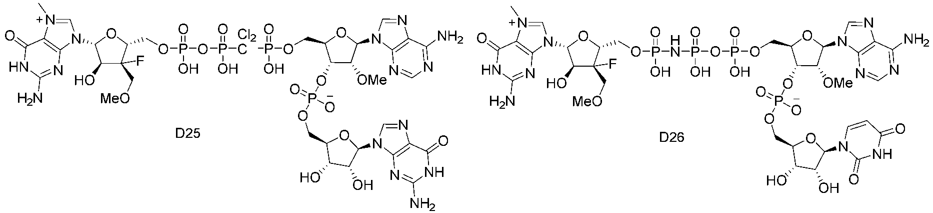

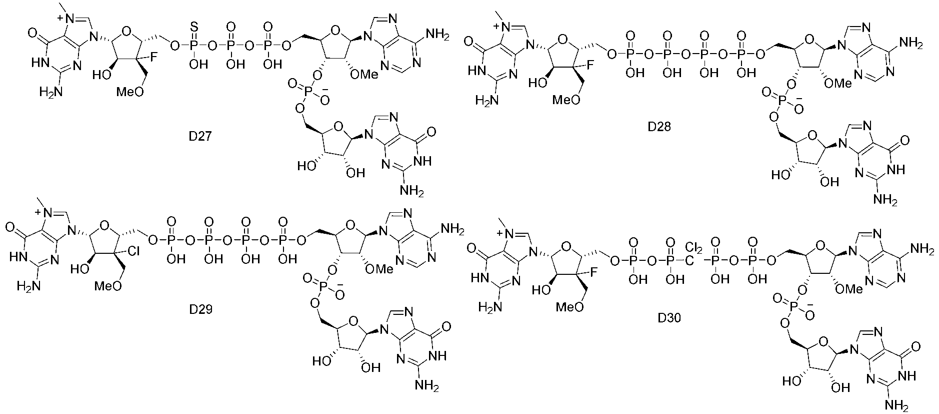

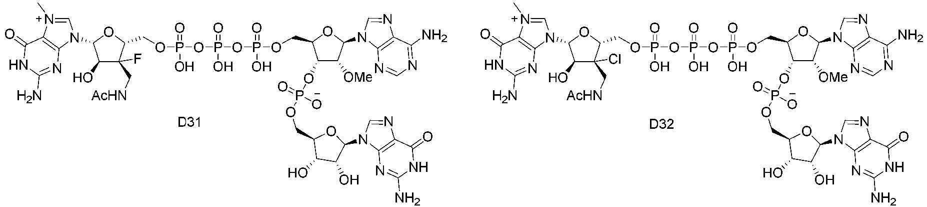

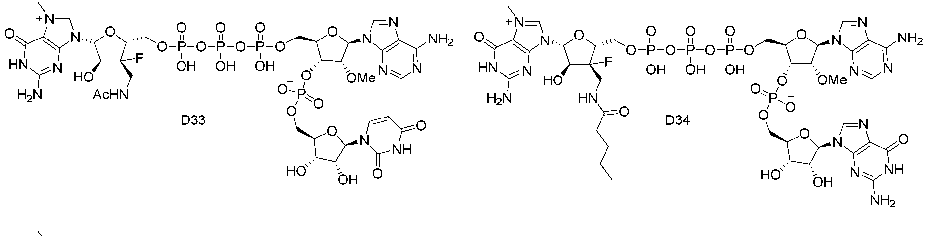

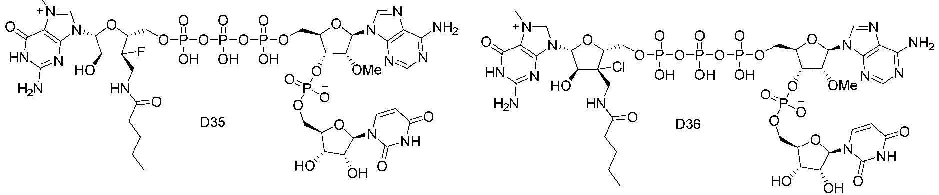

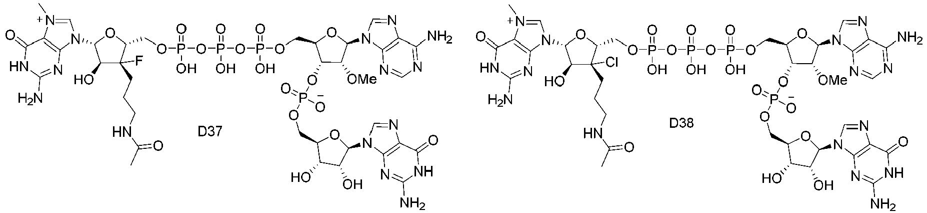

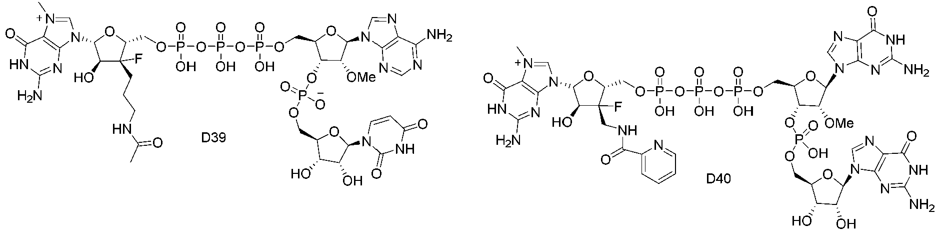

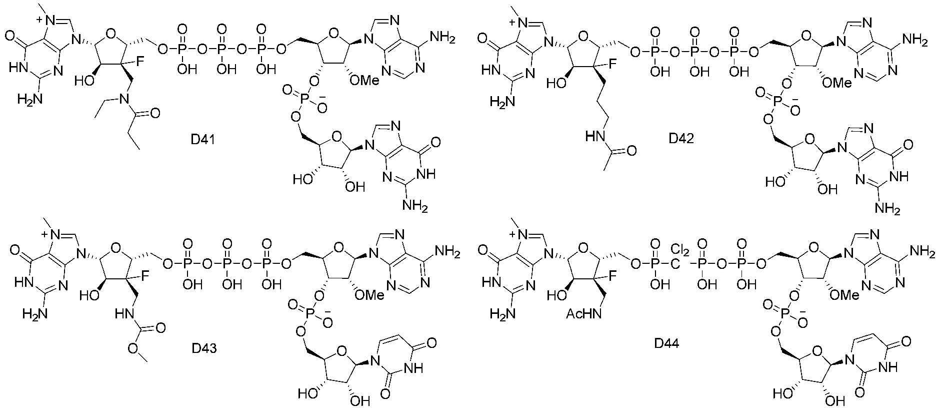

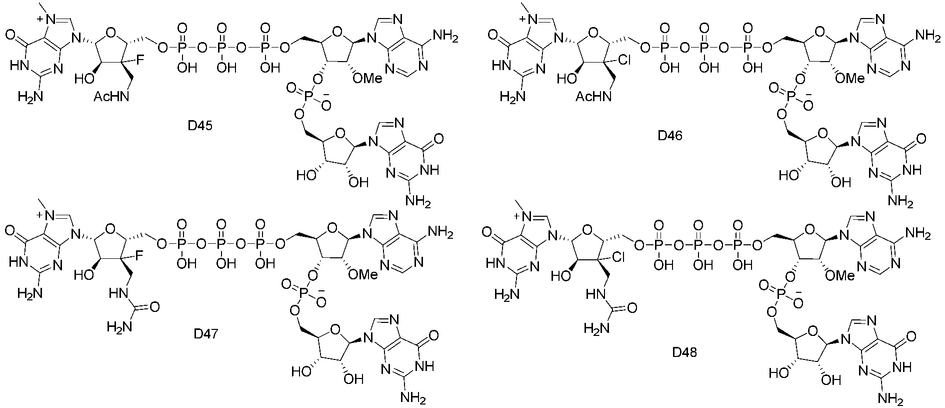

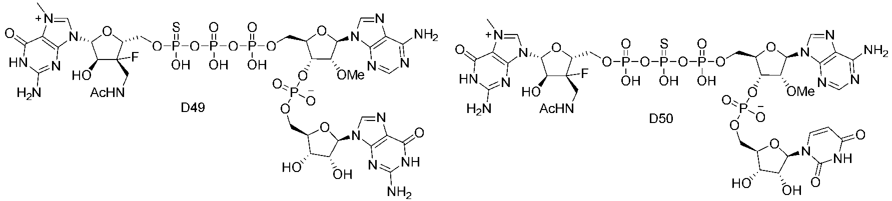

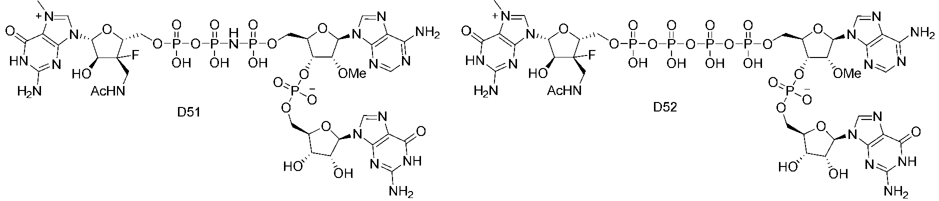

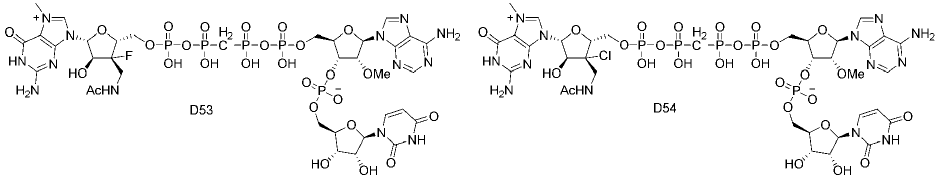

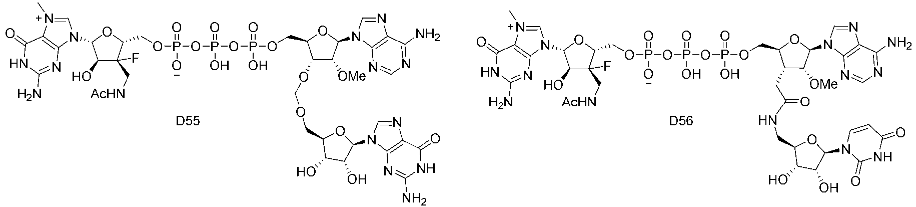

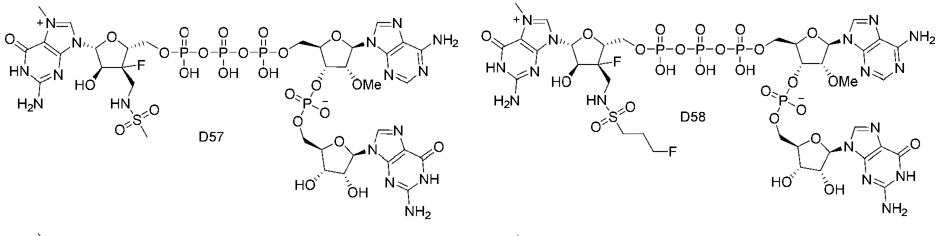

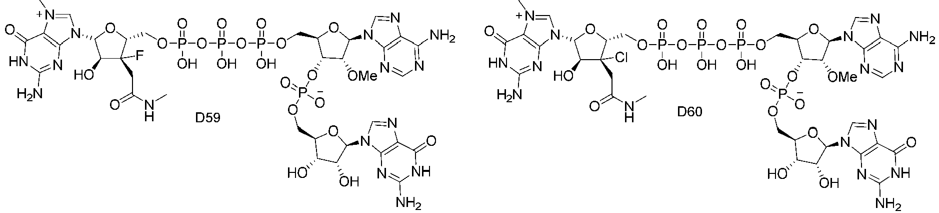

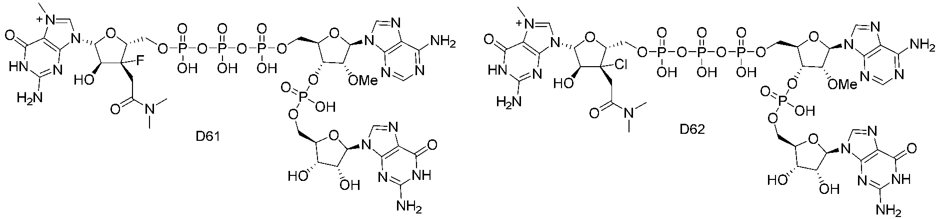

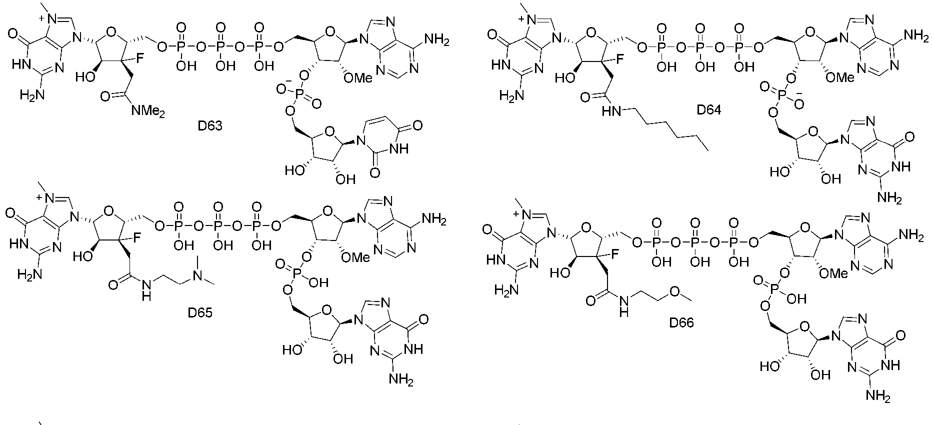

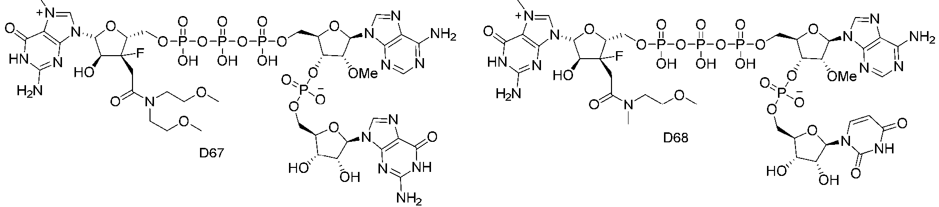

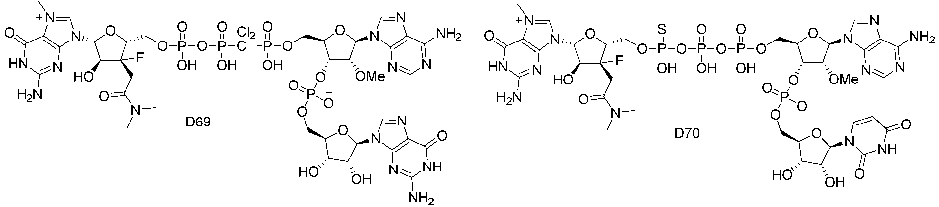

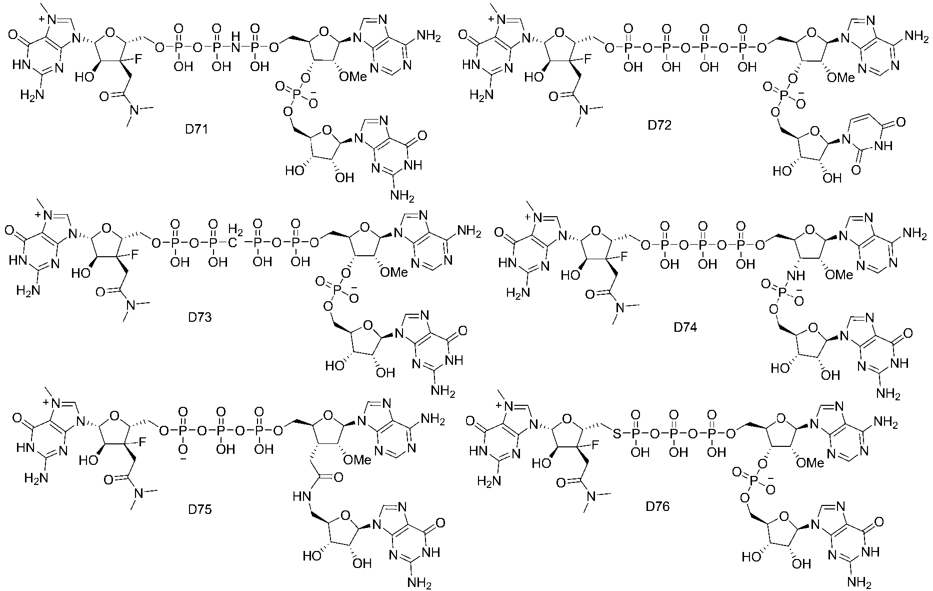

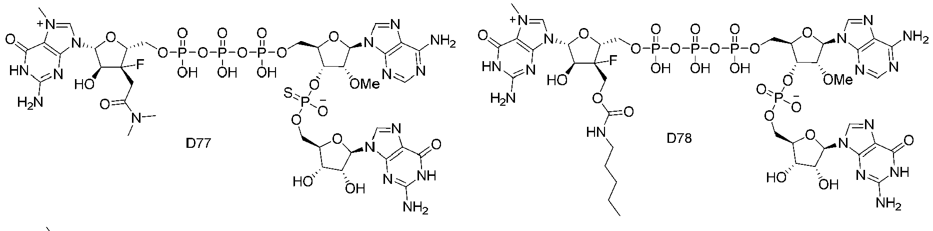

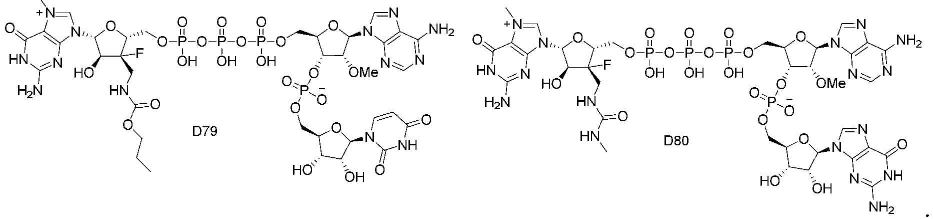

In some embodiments, the compound is represented by any one of the following structures:

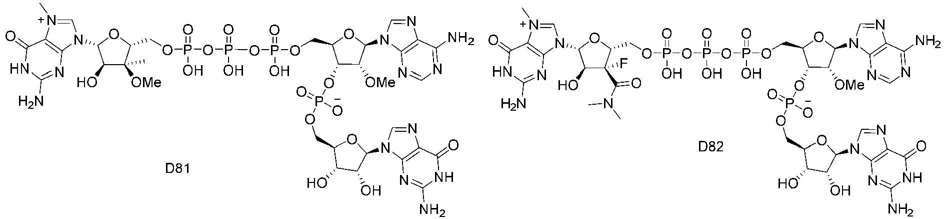

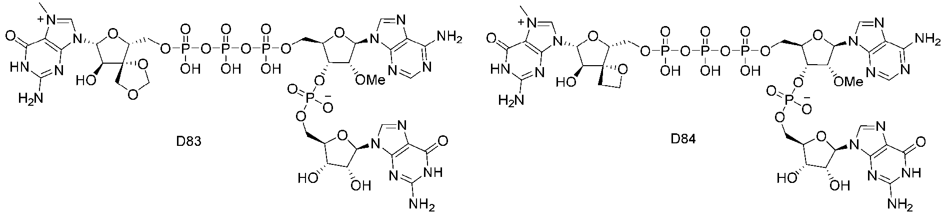

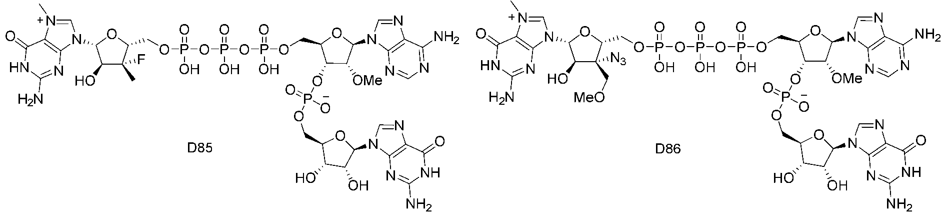

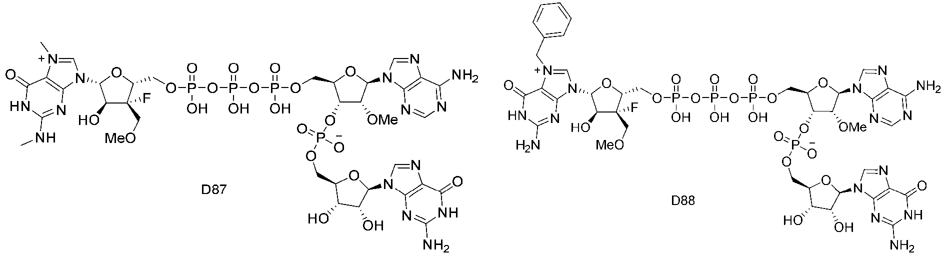

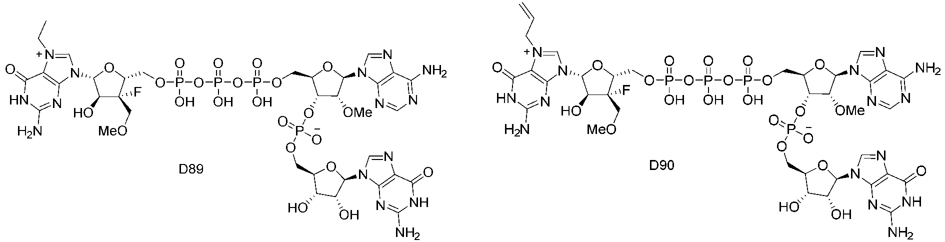

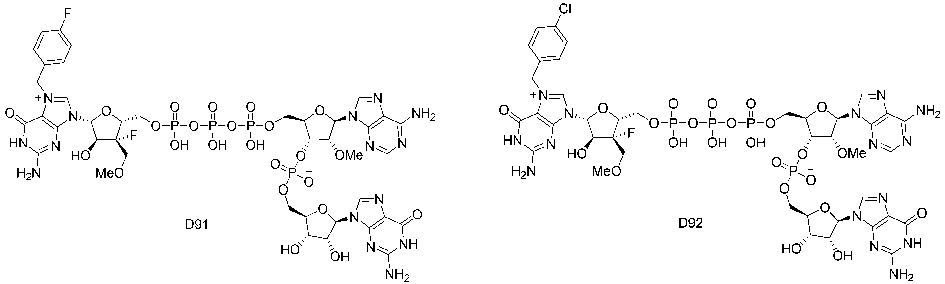

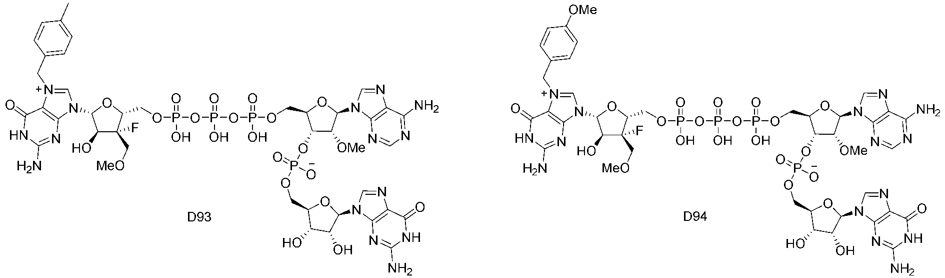

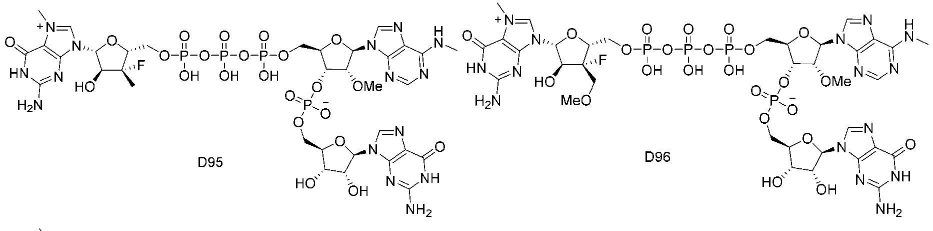

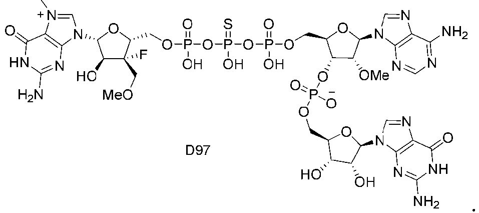

A third objective of the present disclosure is to protect a preparation method for the above compounds, wherein a preparation route for one class of compounds is:

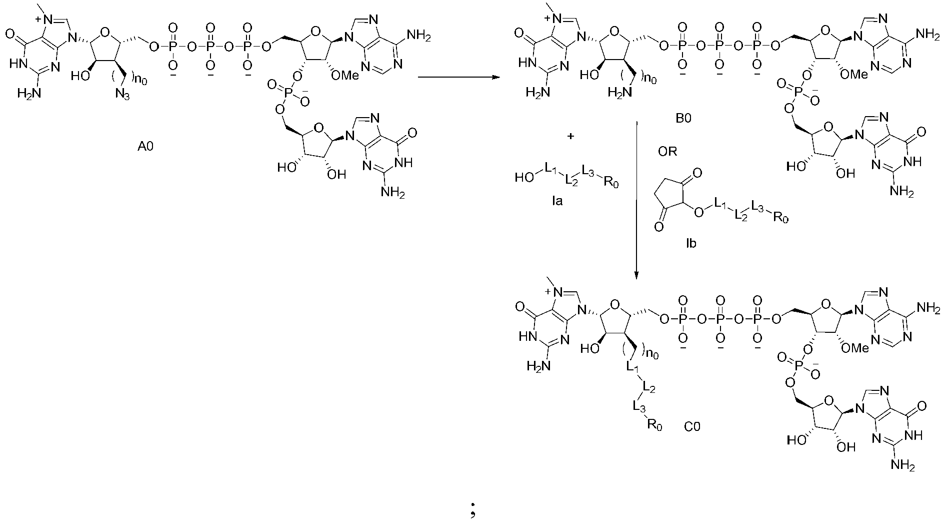

A0 undergoes a displacement reaction with hydrogen in the presence of a catalyst to obtain B0, and then B0 undergoes a condensation reaction with Ia or Ib to prepare a target compound C0.

In some embodiments, A0 and B0 may be salt compounds commonly used in chemical reactions or pharmaceutically acceptable salt compounds; more preferably, A0 and B0 are ammonium salts, sodium salts, potassium salts, Tris salts, phosphate salts, or the like.

A fourth objective of the present disclosure is to protect use of the above compound as an *in vitro* co-transcriptional RNA capping reagent.

A fifth objective of the present disclosure is to protect an RNA molecule comprising any one of the above compounds as a cap structure or a cap structure fragment.

A sixth objective of the present disclosure is to protect a pharmaceutical composition, namely a composition comprising the above RNA molecule and a pharmaceutically acceptable carrier.

A seventh objective of the present disclosure is to protect a method for synthesizing an RNA molecule, comprising the following steps:
co-incubating any one of the above compounds with a polynucleotide template for template transcription.

An eighth objective of the present disclosure is to provide a capped RNA transcription reaction system, comprising: a polynucleotide template, any one of the above compounds, NTPs and an RNA polymerase.

Compared with the prior art, the present disclosure has the following beneficial effects:
The present disclosure provides a fluorophore-labeled compound for RNA capping. This compound not only maintains its original excellent performance (such as good capping rate, as well as good stability, expression levels, and other properties of the capped mRNA), but also can be utilized for detection and tracking of dynamic changes of mRNA in cells or organisms due to its fluorophore labeling. This facilitates the study of delivery processes, pharmacokinetics, and other properties of mRNA in cells or organisms, thereby providing convenience for the research of mRNA vaccines/drugs in cells or organisms.

The present disclosure provides a compound for RNA capping. When employed for mRNA 5' capping, this compound not only exhibits good capping efficiency but also significantly enhances the performance of capped mRNA in terms of stability and expression levels. This compound exhibits favorable performance in *in vitro* conditions or in animals, outperforming positive controls.

### BRIEF DESCRIPTION OF THE DRAWINGS

FIG. 1 shows the fluorescence intensity detection results at different mRNA concentrations in Example 23.
FIG. 2 shows the fluorescence detection results at different time points after transfection of S10-GFP-mRNA into cells in Example 25.
FIG. 3 shows the fluorescence detection results 20 h after transfection of S10-GFP-mRNA into cells in Example 25.
FIG. 4 shows the *in vivo* imaging detection results 1 h after injection of S10-FLuc-mRNA-LNP into mice in Example 26.
FIG. 5 shows the imaging detection results of liver 1 h after injection of S10-FLuc-mRNA-LNP into mice in Example 26.
FIG. 6 shows the imaging detection results of lung 1 h after injection of S10-FLuc-mRNA-LNP into mice in Example 26.
FIG. 7 shows the imaging detection results of spleen 1 h after injection of S10-FLuc-mRNA-LNP into mice in Example 26.
FIG. 8 shows the *in vivo* imaging detection results 3 h after injection of S10-FLuc-mRNA-LNP into mice in Example 27.

### DETAILED DESCRIPTION OF THE PREFERRED EMBODIMENT

In order to facilitate understanding of the present disclosure, the present disclosure will be described more fully below with reference to the relevant drawings. Preferred examples of the present disclosure are shown in the accompanying drawings. However, the present disclosure may be embodied in many different forms and is not limited to the examples described herein. On the contrary, these examples are provided for the purpose of providing a more thorough and comprehensive understanding of the present disclosure.

Unless otherwise defined, all technical and scientific terms used herein have the same meaning as commonly understood by those skilled in the art belonging to the present disclosure. The terms used in the specification of the present disclosure herein are for the purpose of describing specific examples only and are not intended to limit the present disclosure.

### Terms:

Pyrimidine nucleotide bases include, but are not limited to: uracil (U), thymine (T), cytosine (C), 5-methylcytosine, 5-fluorouracil, 5-fluorocytosine, *etc.*

Purine nucleotide derivatives include, but are not limited to, adenine (A), guanine (G), 6-N-methyladenine (m6A), 6-N,N-dimethyladenine, 2-N-methylguanine, *2-N,N-*dimethylguanine, *etc.*

"Together as a single bond or a double bond" indicates that this structure is a chemical bond, and specifically optionally a single bond or a double bond, e.g., "-X₄- together as a single bond or a double bond" means that when optionally -X₄- is a single bond or a double bond, the ring of the parent nucleus of formula I is a five-membered ring, with the groups on both side of X₄ directly connected.

"Connected to form a ring by a chemical bond" means that two groups are connected by a carbon-carbon bond, a carbon-oxygen bond, a carbon-nitrogen bond, a carbon-sulfur bond, or the like to form a ring structure, with the corresponding groups reducing 1 to 2 hydrogen atoms when necessary.

"Stereoisomer" refers to a compound with the same chemical structure but differing in the spatial arrangement of atoms or groups. Stereoisomers include enantiomers, diastereomers, conformational isomers (rotational isomers), geometric isomers *(cis*/*trans* isomers), atropisomers, *etc.*

Any asymmetric atom (e.g., carbon) in the compounds disclosed in the present disclosure may exist in a racemic or enantiomerically enriched form, such as in the *(R)-,* (S)-, or (R, S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in terms of the *(R)-* or (S)-configuration.

The term "substituted" indicates that one or more hydrogen atoms in the given structure are replaced by specific substituents. Unless otherwise indicated, a substituted group may have a substituent substituted at each substitutable site of the group. When more than one site in a given structural formula can be substituted by one or more substituents selected from specific groups, the substituents may be identically or differently substituted at each site.

The term "be independently" should be understood in a broad sense, either to mean that the specific options expressed between the same symbols in different groups do not affect each other, or that the specific options expressed between the same symbols in the same groups do not affect each other.

In various sections of this specification, the substituents of the compounds disclosed in the present disclosure are disclosed according to the type or range of groups. It is specifically noted that the present disclosure includes each individual member of every independent secondary combination of these types and ranges of groups. For example, the term "C₁₋₆ alkyl" specifically refers to the independently disclosed methyl, ethyl, C₃ alkyl, C₄ alkyl, C₅ alkyl and C₆ alkyl.

In various sections of the present disclosure, the connection of substituents is described. When the structure explicitly requires a connecting group, the Markush variables listed for the group should be understood as connecting groups. For example, if the structure requires a connecting group and the Markush group definition for the variable lists "alkyl" or "aryl," it should be understood that the "alkyl" or "aryl" respectively represent a connected alkylene group or a connected arylene group.

The term "alkyl" or "alkyl group" as used in the present disclosure refers to a saturated straight-chain or branched monovalent hydrocarbon group, wherein the alkyl group may be optionally substituted by one or more substituents described in the present disclosure. The alkyl group may be optionally substituted by one or more substituents described in the present disclosure.

Examples of alkyl groups include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, - CH₂CH₂CH₂CH₃), isobutyl (i-Bu, -CH₂CH(CH₃)₂), sec-butyl (s-Bu, -CH(CH₃)CH₂CH₃), *tert-*butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH (CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, *etc.*

The term "alkenyl" refers to a straight-chain or branched monovalent hydrocarbon group containing 2 to 30 carbon atoms, with at least one unsaturated site, i.e., a carbon-carbon sp2 double bond. Such alkenyl groups include a *"cis"* and *"trans"* orientation, or an "E" and "Z" orientation. Examples of alkenyl groups include, but are not limited to, vinyl (-CH=CH₂), allyl (-CH₂CH=CH₂), *etc.* The alkenyl group may be optionally substituted by one or more substituents described in the present disclosure.

The term "alkynyl" means that there is at least one unsaturated site, that is, a carbon-carbon sp triple bond. Examples of alkynyl groups include, but are not limited to, ethynyl (-C≡CH), propargyl (-CH₂C≡CH), 1-propynyl (-C≡C-CH₃), *etc.* The alkynyl group may be optionally substituted by one or more substituents described in the present disclosure.

The term "cycloalkyl" as used in the present disclosure, unless otherwise specified, refers to a monovalent saturated or partially unsaturated (but non-aromatic) monocyclic or polycyclic hydrocarbon. In some embodiments, the cycloalkyl group may be bridged or non-bridged, spiro or non-spiro, and/or fused or non-fused bicyclic groups. In some embodiments, the cycloalkyl group contains 3 to 10 carbon atoms, i.e., C3 to C10 cycloalkyl. In some embodiments, the cycloalkyl has 3-15 (C3-15), 3-10 (C3-10) or 3-7 (C3-7) carbon atoms. In some embodiments, the cycloalkyl group is monocyclic or bicyclic. In some embodiments, the cycloalkyl group is monocyclic. In some embodiments, the cycloalkyl group is bicyclic. In some embodiments, the cycloalkyl group is tricyclic. In some embodiments, the cycloalkyl group is fully saturated. In some embodiments, the cycloalkyl group is partially saturated. In some embodiments, the cycloalkyl group is cyclopropyl, cyclobutyl, cyclopentyl, cyclohexyl, cycloheptyl, bicyclo[2.1.1]hexyl, bicyclo[2.2.1]heptyl, decahydronaphthyl, or adamantyl. When the cycloalkyl group is substituted, it may be substituted independently by one or more substituents described in the present disclosure, on any ring, i.e., any aromatic or non-aromatic ring contained in the cycloalkyl.

The term "haloalkyl" refers to an alkyl group in which at least one H is substituted by halogen, wherein the halogen is one or more of fluorine, chlorine, bromine, or iodine.

The term "alkylamino" refers to an amino group in which at least one H is substituted by an alkyl group.

The terms "heterocyclyl/heterocyclic group" and "heterocycle" are used interchangeably in the present disclosure, unless otherwise specified, to refer to a monovalent monocyclic non-aromatic ring system and/or a polycyclic system comprising at least one non-aromatic ring, wherein one or more (in some embodiments, 1, 2, 3, or 4) atoms in the monocyclic non-aromatic ring are independently selected from O, S(O)0-2, and N heteroatoms, with the remaining ring atoms being carbon atoms; and wherein in the polycyclic system, one or more (in some embodiments, 1, 2, 3, or 4) ring atoms are independently selected from O, S(O)0-2, and N heteroatoms, with the remaining ring atoms being carbon atoms. In some embodiments, the heterocycle contains 1 or 2 heteroatoms, where the heteroatoms are nitrogen atoms. In some embodiments, the heterocyclic group is polycyclic and contains one heteroatom in a non-aromatic ring, or one heteroatom in an aromatic ring, or two heteroatoms, with one in the aromatic ring and the other in the non-aromatic ring. In some embodiments, the heterocyclic group has 3 to 20, 3 to 15, 3 to 10, 3 to 8, 4 to 7 or 5 to 6 ring atoms. In some embodiments, the heterocyclic group is a monocyclic, bicyclic, tricyclic or tetracyclic system. In some embodiments, the heterocyclic group may be bridged or non-bridged, spiro or non-spiro, and/or fused or non-fused bicyclic groups. One or more nitrogen atoms and sulfur atoms may optionally be oxidized, one or more nitrogen atoms may optionally be quaternized, and one or more carbon atoms may optionally be substituted by 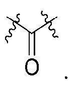 Some rings may be partially or fully saturated, or aromatic, provided that the heterocycle is not fully aromatic. The monocyclic heterocycle and polycyclic heterocycle may be attached to the main structure at any heteroatom or carbon atom that results in a stable compound. The polycyclic heterocyclic group may be attached to the main structure through any of its rings, including any aromatic or non-aromatic ring, regardless of whether the ring contains a heteroatom. In some embodiments, the heterocyclic group is a "heterocycloalkyl group", which is 1) a saturated or partially unsaturated (but non-aromatic) monovalent monocyclic group containing at least one ring heteroatom as described in the present disclosure, or 2) a saturated or partially unsaturated (but non-aromatic) monovalent bicyclic or tricyclic group, wherein at least one ring contains at least one heteroatom as described in the present disclosure. When the heterocyclic group and heterocycloalkyl group are substituted, they may be substituted on any of the rings, i.e., on any aromatic or non-aromatic ring contained in the heterocyclic group and heterocycloalkyl group. In some embodiments, such heterocyclic groups include, but are not limited to, oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothiophenyl, dihydrothiophenyl, 1,3-dioxolanyl, dithiolanyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, oxepanyl, thiepanyl, oxazepinyl, diazepinyl, thiazepinyl, benzodioxanyl, benzodioxolyl, benzofuranonyl, benzopyranonyl, benzopyranyl, dihydrobenzofuranyl, benzotetrahydrothiophenyl, benzothiopyranyl, benzoxazinyl, β-carbolinyl, chromanyl, chromonyl, cinnolinyl, coumarinyl, decahydroquinolinyl, decahydroisoquinolinyl, dihydrobenzisothiazinyl, dihydrobenzisoxazinyl, dihydrofuranyl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, 2,4-dioxo-imidazolidinyl, imidazolinyl, indolinyl, 2-oxo-indolinyl, isobenzotetrahydrofuranyl, isobenzotetrahydrothiophenyl, isochromanyl, isocoumarinyl, isodihydroindolyl (isoindolinyl ), 1-oxo-isoindolinyl, 1,3-dioxo-isoindolinyl, isothiazolidinyl, isoxazolidinyl, 3-oxo-isoxazolidinyl, morpholinyl, 3,5-dioxo-morpholinyl, octahydroindolyl, octahydroisoindolyl, 1-oxo-octahydroisoindolyl, 1,3-dioxo-hexahydroisoindolyl, oxazolidinonyl, oxazolidinyl, oxiranyl, piperazinyl, 2,6-dioxo-piperazinyl, piperidinyl, 2,6-dioxo-piperidinyl, 4-piperidonyl, 2-oxo-pyrrolidinyl, 2,5-dioxo-pyrrolidinyl, quinuclidinyl, tetrahydroisoquinolinyl, 3,5-dioxothiomorpholinyl, thiazolidinyl, 2,4-dioxo-thiazolidinyl, tetrahydroquinolinyl, phenothiazinyl, phenoxazinyl, xanthenyl, and 1,3,5-trithianyl. Examples of the -CH2- group in the heterocyclic group substituted with -C(=O)- include, but are not limited to, 2-oxopyrrolidinyl, oxo-1,3-thiazolidinyl, 2-piperidinonyl, 3,5-dioxopiperidinyl and pyrimidinedionyl. Examples of heterocyclic groups in which a sulfur atom is oxidized include, but are not limited to, cyclobutanesulfonyl and 1,1-dioxothiomorpholinyl. The heterocyclic group may be optionally substituted by one or more substituents described in the present disclosure.

In one embodiment, the heterocyclic group is a heterocyclic group composed of 3 to 8 atoms, which refers to a saturated or partially unsaturated monocyclic ring containing 3 to 8 ring atoms, wherein at least one ring atom is selected from nitrogen, sulfur and oxygen atoms. Unless otherwise specified, the heterocyclic group composed of 3 to 8 atoms may be connected to other parts through carbon atoms or nitrogen atoms, and the -CH2- group may be optionally replaced by -C(=O)-. The sulfur atom in the ring may be optionally oxidized to form an S-oxide. The nitrogen atom in the ring may be optionally oxidized to form an N-oxide. Examples of heterocyclic groups composed of 3 to 8 atoms include, but are not limited to: azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothiophenyl, dihydrothiophenyl, 1,3-dioxolanyl, dithiolanyl, tetrahydropyranyl, dihydropyranyl, 2H-pyranyl, 4H-pyranyl, tetrahydrothiopyranyl, piperidyl, morpholinyl, thiomorpholinyl, piperazinyl, dioxanyl, dithianyl, thioxanyl, homopiperazinyl, homopiperidinyl, oxacycloheptyl, thiocycloheptyl, oxazepinyl, diazepinyl and thiazepinyl. Examples of the -CH2- group in the heterocyclic group substituted with -C(=O)- include, but are not limited to, 2-oxopyrrolidinyl, oxo-1,3-thiazolidinyl, 2-piperidinonyl, 3,5-dioxopiperidinyl and pyrimidinedionyl. Examples of heterocyclic groups in which a sulfur atom is oxidized include, but are not limited to, cyclobutanesulfonyl and 1,1-dioxothiomorpholinyl. The heterocyclic groups composed of 3 to 8 atoms may be optionally substituted by one or more substituents as described in the present disclosure.

In one embodiment, the heterocyclic group is a heterocyclic group composed of 3 to 6 atoms, which refers to a saturated or partially unsaturated monocyclic ring containing 3 to 6 ring atoms, wherein at least one ring atom is selected from nitrogen, sulfur and oxygen atoms. Unless otherwise specified, the heterocyclic group composed of 3 to 6 atoms may be connected to other parts through carbon atoms or nitrogen atoms, and the -CH2- group may be optionally replaced by -C(=O)-. The sulfur atom in the ring may be optionally oxidized to form an S-oxide. The nitrogen atom in the ring may be optionally oxidized to form an N-oxide. The heterocyclic groups composed of 3 to 6 atoms may be optionally substituted by one or more substituents as described in the present disclosure.

In another embodiment, the heterocyclic group is a heterocyclic group composed of 5 to 6 atoms, which refers to a saturated or partially unsaturated monocyclic ring containing 5 to 6 ring atoms, where at least one ring atom is selected from nitrogen, sulfur, and oxygen atoms. Unless otherwise specified, the heterocyclic group composed of 5 to 6 atoms may be connected to other parts through carbon atoms or nitrogen atoms, and the -CH2- group may be optionally replaced by -C(=O)-. The sulfur atom in the ring may be optionally oxidized to form an S-oxide. The nitrogen atom in the ring may be optionally oxidized to form an N-oxide. Examples of heterocyclic groups composed of 5 to 6 atoms include, but are not limited to: pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothiophenyl, dihydrothiophenyl, 1,3-dioxolanyl, dithiolanyl, 2-oxo-pyrrolidinyl, oxo-1,3-thiazolidinyl, cyclobutanesulfonyl, tetrahydropyranyl, dihydropyranyl, 2H-pyranyl, 4H-pyranyl, tetrahydrothiopyranyl, piperidyl, morpholinyl, thiomorpholinyl, piperazinyl, dioxanyl, dithianyl, thioxanyl, 2-piperidone, 3,5-dioxopiperidinyl, pyrimidinedione, and 1,1-dioxothiomorpholinyl. The heterocyclic group composed of 5 to 6 atoms may be optionally substituted by one or more substituents as described in the present disclosure.

The term "aryl" as used in the present disclosure, unless otherwise specified, refers to a monovalent C6-C14 carbocyclic ring system containing at least one aromatic ring, where the aromatic ring system may be monocyclic, bicyclic or tricyclic. The aryl group may be attached to the main structure via any of its rings, i.e., through any aromatic or non-aromatic ring. In some embodiments, the aryl group is phenyl, naphthyl, bicyclo[4.2.0]oct-1,3,5-trienyl, indanyl, fluorenyl or tetrahydronaphthyl. When the aryl group is substituted, it may be substituted on any of the rings, i.e., on any aromatic or non-aromatic ring contained in the aryl group. In some or any embodiment, the aryl group is phenyl, naphthyl, tetrahydronaphthyl, fluorenyl, or indanyl. The aryl group may be independently and optionally substituted by one or more substituents as described in the present disclosure.

The term "heteroaryl" as used in the present disclosure, unless otherwise specified, refers to a monovalent monocyclic or polycyclic aromatic group, where at least one (in some embodiments, 1, 2, 3 or 4) ring atom is independently selected from O, S(O)0-2 and N heteroatoms in the ring. The heteroaryl group is connected to the rest of the molecule via any atom in the ring system, provided that the valency rules for the atom allow such a connection. In some embodiments, each ring in the heteroaryl group may contain 1 or 2 O atoms, 1 or 2 S atoms, and/or 1 to 4 N atoms, or combinations thereof, provided that the total number of heteroatoms in each ring is 4 or fewer, and each ring contains at least one carbon atom. In some embodiments, the heteroaryl group contains 5 to 20, 5 to 15 or 5 to 10 ring atoms. When the heteroaryl group is substituted, substitution may occur on any of the rings. In certain embodiments, monocyclic heteroaryl groups include, but are not limited to, furanyl, imidazolyl, isothiazolyl, isoxazolyl, oxadiazolyl, oxazolyl, pyrazinyl, pyrazolyl, pyridazinyl, pyridinyl, pyrimidinyl, pyrrolyl, thiadiazolyl, thiazolyl, thienyl, tetrazolyl, triazinyl, and triazolyl. In certain embodiments, bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridinyl, imidazopyridinyl, imidazothiazolyl, indolizinyl, indolyl, indazolyl, isobenzofuranyl, isobenzothienyl, isoindolyl, isoquinolinyl, isothiazolyl, naphthyridinyl, oxazolopyridinyl, phthalazinyl, pteridinyl, purinyl, pyridopyridinyl, pyrrolopyridinyl, quinolinyl, quinoxalinyl, quinazolinyl, thiadiazolopyrimidinyl, and thienopyridinyl. In certain embodiments, tricyclic heteroaryl groups include, but are not limited to, acridinyl, benzoindolyl, carbazolyl, dibenzofuranyl, perimidinyl, phenanthrolinyl, phenanthridinyl, and phenazinyl. In some or any embodiments, the heteroaryl group is phenylene, naphthylene, pyridylene, pyrimidinylene, pyrazinylene, pyridazinylene, thiazolylene, benzothiazolyl, benzo[d]isothiazolyl, imidazo[1,2-a]pyridinyl, quinolinyl, 1H-indolyl, pyrrolo[1,2-b]pyridazinyl, benzofuranyl, benzo[b]thienyl, 1H-indazolyl, benzo[d]isoxazolyl, quinazolinyl, 1H-pyrrolo[3,2-c]pyridinyl, pyrazolo[1,5-a]pyrimidinyl, imidazo[1,2-b]pyridazinyl, or pyrazolo[1,5-a]pyridinyl; each of which is optionally substituted by 1, 2, 3, or 4 groups as defined throughout the specification.

The "solvate" of the present disclosure refers to an adduct formed by one or more solvent molecules and the compound of the present disclosure. Solvents forming solvates include, but are not limited to, water, isopropanol, ethanol, methanol, dimethyl sulfoxide, ethyl acetate, acetic acid and aminoethanol. The term "hydrate" means that the solvent molecule is an adduct formed by water.

When the solvent is water, the term "hydrate" can be used. In some embodiments, one compound molecule of the present disclosure can be combined with one water molecule, such as a monohydrate; in other embodiments, one compound molecule of the present disclosure can be combined with more than one water molecule, such as a dihydrate, and in still other embodiments, one compound molecule of the present disclosure can be combined with less than one water molecule, such as a hemihydrate. It should be noted that the hydrate of the present disclosure retains the bioavailability of the compound in non-hydrated form.

The compounds used in the following examples are all commercially available unless otherwise stated; the methods used in the following examples can be implemented by conventional methods unless otherwise stated.

In the compounds of the present disclosure, when the -L₁-L₂-L₃ substituent is connected to the main skeleton of the compound via NH, the preparation route of the compound is as follows:

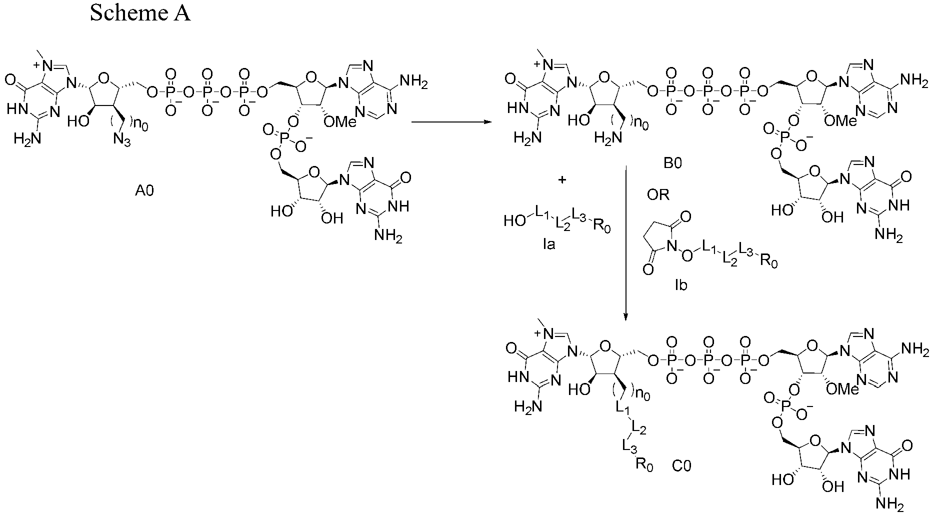

A0 undergoes a reduction reaction with hydrogen in the presence of a catalyst to obtain B0, and then B0 undergoes a condensation reaction with Ia or Ib to prepare C0. In C0, when L₁ is present, L₁ is NH; if L₁ is absent and L₂ is present, L₂ is NH; if both L₁ and L₂ are absent, the main skeleton of the compound is connected to NH in the L₃ group.

In the above reaction route, A0 and B0 may be salt compounds commonly used in chemical reactions or pharmaceutically acceptable salt compounds, such as ammonium salts, sodium salts, potassium salts, Tris salts, or phosphate salts.

For example, when -L₁-L₂-L₃- represents different groups, the preparation route is one of the following (since the process of preparing B0 from A0 is identical, it is not repeated, and the reaction route is provided starting from B0). The following examples are merely a few examples and are not exhaustive.

When R₀ is a fluorophore, the preparation route is:

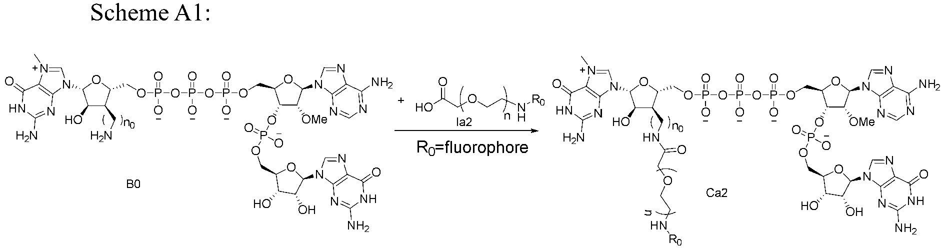

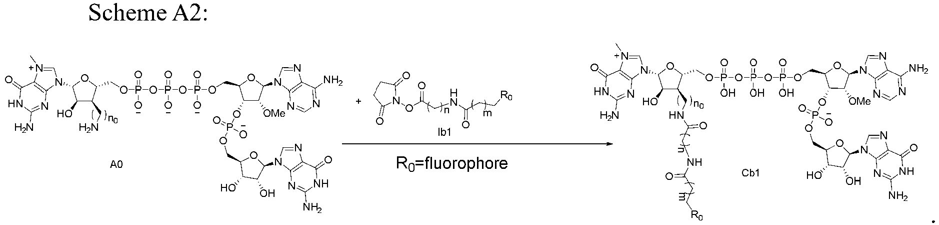

### Example 1 Synthesis of compound S1

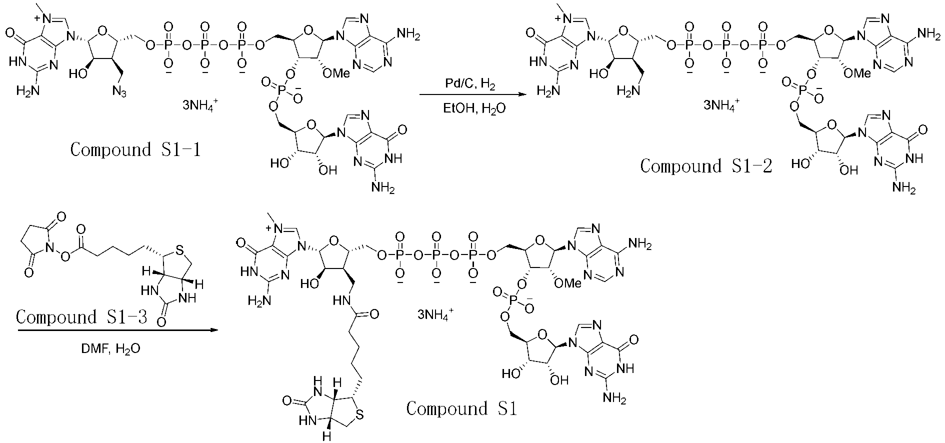

The ammonium salt of compound 1-1 was synthesized according to the method in patent CN115260264A and used as the starting material for the preparation of the compound in this example.

Compound S1-1 (350 mg) was dissolved in water (8 mL) and ethanol (2 mL), followed by the addition of palladium on carbon (500 mg). After the system was purged with hydrogen, the mixture was stirred at room temperature. After completion of the reaction, the reaction mixture was purified by anion exchange resin and lyophilized to obtain the ammonium salt of compound S1-2 (320 mg).

The ammonium salt of compound S1-2 (50 mg) was dissolved in *N,N-*dimethylformamide (1 mL) and water (1 mL), followed by the addition of compound S1-3 (38 mg, 5 equivalents), and the mixture was stirred at room temperature. After completion of the reaction, the reaction mixture was purified by anion exchange resin and lyophilized to obtain the ammonium salt of compound S1 (21 mg).

The characterization data of the ammonium salt of compound S1 are: 1382.85[M-1]⁻. ¹H NMR (400 MHz, D₂O) δ 9.09 (s, 1H), 8.46 (s, 1H), 8.16 (s, 1H), 7.96 (s, 1H), 6.06 (d, *J =* 5.8 Hz, 1H), 5.90 (s, 1H), 5.82 (d, *J =* 6.0 Hz, 1H), 4.96 - 4.92 (m, 1H), 4.85 - 4.82 (m, 1H), 4.56 (d, *J =* 4.3 Hz, 1H), 4.53 - 4.46 (m, 4H), 4.43 (t, *J =* 5.0 Hz, 1H), 4.38 - 4.36 (m, 1H), 4.30 - 4.28 (m, 3H), 4.24 - 4.21 (m, 3H), 4.18 - 4.14 (m, 1H), 4.00 (s, 3H), 3.57 - 3.49 (m, 1H), 3.40 (s, 3H), 3.23 - 3.19 (m, 1H), 3.03 - 3.01 (m, 1H), 2.83 - 2.78 (m, 1H), 2.64 - 2.59 (m, 2H), 2.25 - 2.22 (m, 2H), 1.61 - 1.51 (m, 3H), 1.44 - 1.35 (m, 1H), 1.22 - 1.15 (m, 2H); ³¹P NMR (162 MHz, D₂O) δ -0.93 (s, 1H), -11.56 (m, 2P), -22.90 (t, *J =* 17.6 Hz, 1P).

### Example 2 Synthesis of compound S3

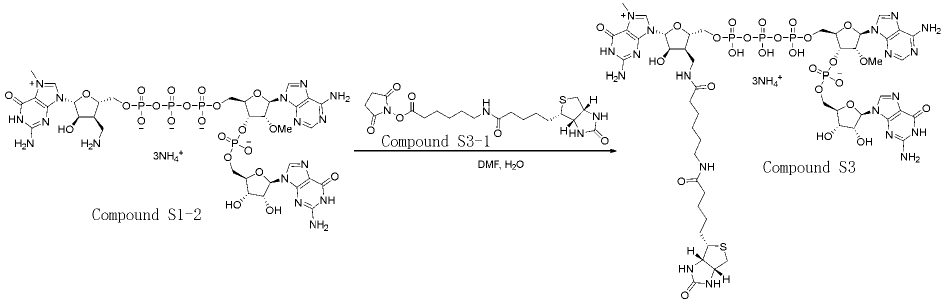

According to the above reaction route and referring to the synthetic method of compound S1, the ammonium salt of compound S2 was synthesized. Since the reaction for preparing compound S1-2 from compound S1-1 was identical, it was not listed in the preparation route. The same applies to the following examples.

The characterization data of the ammonium salt of compound S3 are: 1495.83[M-1]⁻. ¹H NMR (500 MHz, D₂O) δ 9.07 (s, 1H), 8.45 (s, 1H), 8.16 (s, 1H), 7.93 (s, 1H), 6.04 (d, *J=* 5.3 Hz, 1H), 5.82 (s, 1H), 5.76 (d, *J =* 5.9 Hz, 1H), 4.91 - 4.90 (m, 1H), 4.83 - 4.75 (m, 1H), 4.56 (d, *J=* 4.2 Hz, 1H), 4.54 - 4.51 (m, 1H), 4.48 - 4.46 (m, 3H), 4.40 (t, *J* = 4.7 Hz, 1H), 4.33 - 4.27 (m, 4H), 4.24 - 4.22 (m, 1H), 4.17 (m, 2H), 4.13 - 4.11 (m, 1H), 3.97 (s, 3H), 3.53 - 3.48 (m, 1H), 3.40 (s, 3H), 3.24 - 3.16 (m, 2H), 3.06 - 2.95 (m, 2H), 2.91 - 2.88 (m, 1H), 2.70 - 2.67 (m, 1H), 2.61 - 2.58 (m, 1H), 2.18 (t, *J =* 7.4 Hz, 2H), 2.14 (t, *J =* 7.2 Hz, 2H), 1.65 - 1.57 (m, 1H), 1.55 - 1.44 (m, 5H), 1.39 - 1.33 (m, 2H), 1.31 - 1.23 (m, 2H), 1.19 - 1.15 (m, 2H); ³¹P NMR (202 MHz, D₂O) δ -0.99 (s, 1H), -11.60 (m, 2P), -22.87 (t, *J=* 17.6 Hz, 1P).

### Example 3 Synthesis of compound S17

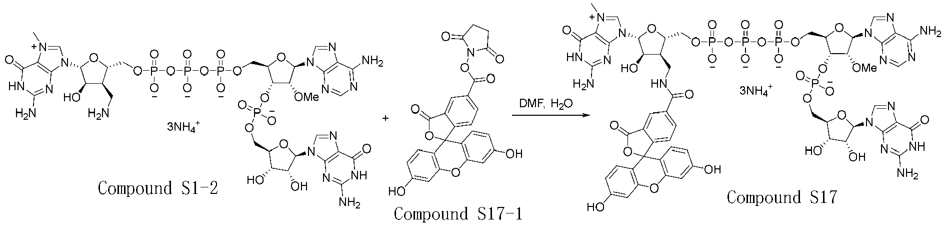

According to the above reaction route and referring to the synthetic method of compound S1, the ammonium salt of compound S17 was synthesized.

The characterization data of the ammonium salt of compound S17 are: 1514.86[M-1]⁻. ¹H NMR (400 MHz, d6-DMSO) δ 8.48 (s, 1H), 8.41 (s, 1H), 8.31 (d, 1H), 8.10 (s, 1H), 7.94 (s, 1H), 7.31 (d, *J=* 7.8 Hz, 1H), 6.70 - 6.50 (m, 6H), 5.96 (d, *J=* 6.0 Hz, 1H), 5.92 (s, 1H), 5.65 (d, *J =* 6.0 Hz, 1H), 4.87 (m, 1H), 4.61 - 4.53 (m, 3H), 4.26 - 4.21 (m, 3H), 4.08 - 3.90 (m, 9H), 3.68 (m, 2H), 3.26 (m, 4H); ³¹P NMR (162 MHz, d6-DMSO) δ -1.02 (s, 1H), -11.53 (m, 2P), -22.70 (m, 1P).

### Example 4 Synthesis of compound S19

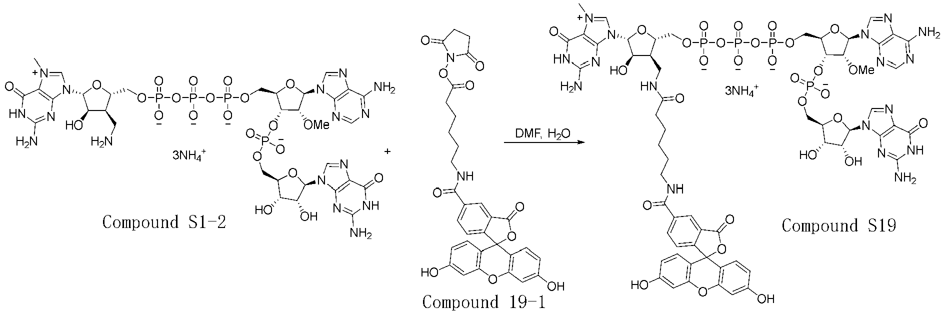

According to the above reaction route and referring to the synthetic method of compound S1, the ammonium salt of compound S 19 was synthesized.

The characterization data of the ammonium salt of compound S19 are: 1627.69[M-1]⁻. ¹H NMR (500 MHz, d6-DMSO) δ 8.50 (s, 1H), 8.39 (s, 1H), 8.21 (d, *J=* 8.0 Hz, 1H), 8.10 (s, 1H), 7.96 (s, 1H), 7.31 (d, *J=* 8.0 Hz, 1H), 6.67 (s, 2H), 6.56 (s, 4H), 5.96 (d, *J=* 6.2 Hz, 1H), 5.82 (s, 1H), 5.65 (d, *J=* 6.3 Hz, 1H), 4.87 (m, 1H), 4.62 - 4.56 (m, 2H), 4.38 - 4.33 (m, 2H), 4.21 - 3.89 (m, 12H), 3.32 - 3.20 (m, 4H), 3.01 - 3.00 (m, 2H), 2.09 (m, 2H), 1.46 (m, 4H), 1.21 (m, 2H); ³¹P NMR (202 MHz, d6-DMSO) δ -2.18 (s, 1H), -11.86 (m, 2P), -21.83 (m, 1P).

### Example 5 Synthesis of compound 5

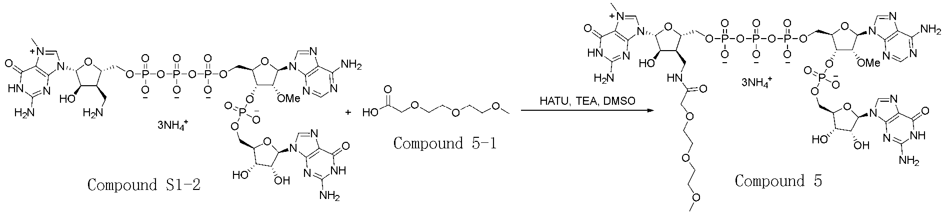

Compound 5-1 (77 mg, 5 equivalents) was dissolved in dimethyl sulfoxide (2 mL), followed by the addition of triethylamine (60 µL, 5 equivalents) and HATU (164 mg, 5 equivalents). The mixture was stirred at room temperature for 30 min. Compound S1-2 (100 mg) was dissolved in dimethyl sulfoxide (1 mL) and added to the above reaction mixture. The resulting mixture was stirred at room temperature. After completion of the reaction, the reaction mixture was purified by anion exchange resin and lyophilized to obtain the ammonium salt of compound 5 (17 mg).

The characterization data of the ammonium salt of compound 5 are: 1316.85[M-1]⁻. ¹H NMR (500 MHz, D₂O) δ 8.35 (s, 1H), 8.06 (s, 1H), 7.91 (s, 1H), 5.96 (d, *J =* 5.6 Hz, 1H), 5.78 (d, *J=* 6.0 Hz, 1H), 5.73 (s, 1H), 4.90 - 4.88 (m, 1H), 4.82 (m, 1H), 4.49 - 4.46 (m, 4H), 4.39 (t, *J=* 5.3 Hz, 1H), 4.32 - 4.20 (m, 4H), 4.17 (m, 2H), 4.13 - 4.09 (m, 1H), 4.02 (s, 2H), 3.98 (s, 3H), 3.63 - 3.53 (m, 7H), 3.48 - 3.45 (m, 2H), 3.38 (s, 3H), 3.27 (s, 3H), 3.25 - 3.21 (m, 1H), 2.54 - 2.50 (m, 1H); ³¹P NMR (202 MHz, D₂O) δ -0.98 (s, 1H), -11.68 (m, 2P), -22.94 (m, 1P).

### Example 6 Synthesis of compound 6

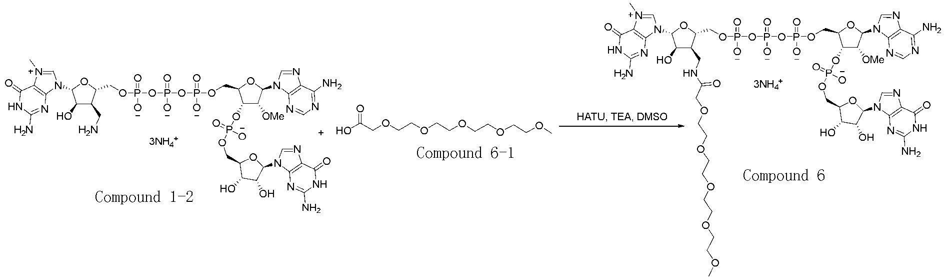

According to the above reaction route and referring to the synthetic method of compound 5, the ammonium salt of compound 6 was synthesized.

The characterization data of the ammonium salt of compound 6 are: 1404.88[M-1]⁻. ¹H NMR (500 MHz, D₂O) δ 9.05 (s, 1H), 8.43 (s, 1H), 8.15 (s, 1H), 7.92 (s, 1H), 6.04 (d, *J=* 4.8 Hz, 1H), 5.77 (s, 1H), 5.75 (d, *J* = 5.9 Hz, 1H), 4.91 (m, 1H), 4.71 (t, *J =* 5.2 Hz, 1H), 4.55 (d, *J=* 3.8 Hz, 1H), 4.49 - 4.46 (m, 3H), 4.40 (t, 1H), 4.32 - 4.28 (m, 3H), 4.24 - 4.10 (m, 4H), 4.04 (s, 2H), 3.96 (s, 3H), 3.64 - 3.54 (m, 17H), 3.44 (s, 3H), 3.32 (s, 3H), 3.30 - 3.27 (m, 1H), 2.60 - 2.55 (m, 1H); ³¹P NMR (202 MHz, D₂O) δ -1.00 (s, 1H), -11.64 (m, 2P), -22.76 (m, 1P).

### Example 7 Synthesis of compound S23

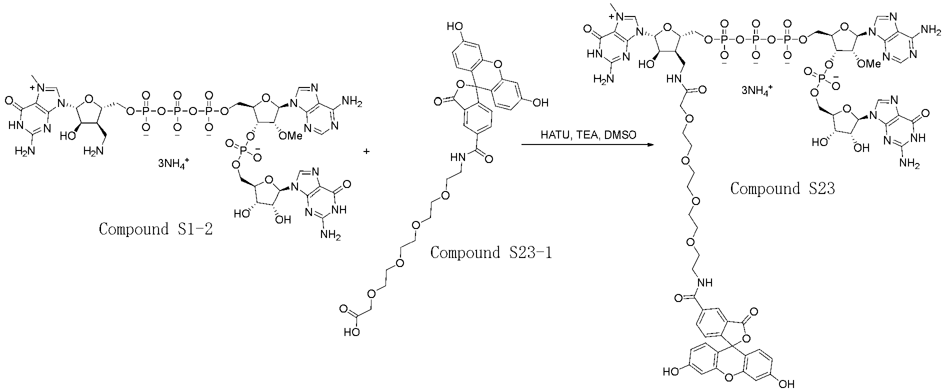

According to the above reaction route and referring to the synthetic method of compound 5, the ammonium salt of compound S23 was synthesized.

The characterization data of the ammonium salt of compound S23 are: 1747.91[M-1]⁻. ¹H NMR (500 MHz, d6-DMSO) δ 8.49 (s, 1H), 8.40 (s, 1H), 8.28 (d, 1H), 8.10 (s, 1H), 7.95 (s, 1H), 7.31 (d, *J=* 7.8 Hz, 1H), 6.70 - 6.53 (m, 6H), 5.96 (d, *J=* 6.0 Hz, 1H), 5.88 (s, 1H), 5.65 (d, *J* = 6.0 Hz, 1H), 4.88 - 4.86 (m, 1H), 4.65 - 4.55 (m, 3H), 4.28 - 4.20 (m, 3H), 4.10 - 3.89 (m, 11H), 3.68 - 3.53 (m, 16H), 3.26 (m, 6H); ³¹P NMR (202 MHz, d6-DMSO) δ -1.38 (s, 1H), -11.70 (m, 2P), -22.56 (m, 1P).

Referring to the synthesis method of compound S1, the compounds of Examples 8-15 were prepared, as shown in Table 1.

| 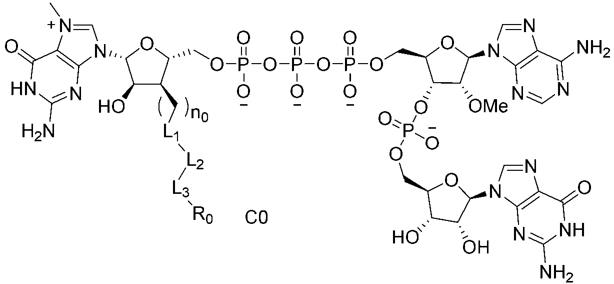 | | | |
|---|---|---|---|
| Example | no | -L₁-L₂-L₃-R₀ | Characterization data |
| 8 | 1 | 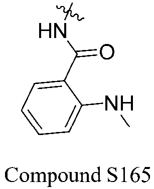 | 1290.02[M-1]⁻. ¹H NMR (500 MHz, D₂O) δ 9.08 (s, 1H), 8.35 (s, 1H), 8.06 (s, 1H), 7.90 (s, 1H), 7.28 (d, *J=* 7.8 Hz, 1H), 7.14 (t, *J=* 7.8 Hz, 1H), 6.61 (d, *J* = 8.5 Hz, 1H), 6.50 (t, *J=* 7.4 Hz, 1H), 5.96 (d, J = 5.4 Hz, 1H), 5.87 (s, 1H), 5.75 (d, *J =* 5.6 Hz, 1H), 4.89 - 4.88 (m, 1H), 4.76 - 4.74 (m, 1H), 4.66 (d, *J=* 4.3 Hz, 1H), 4.50 - 4.47 (m, 3H), 4.36 - 4.34 (m, 2H), 4.30 - 4.26 (m, 2H), 4.22 - 4.17 (m, 4H), 3.98 (s, 3H), 3.72 - 3.67 (m, 1H), 3.43 - 3.36 (m, 4H), 2.74 - 2.66 (m, 4H); ³¹P NMR (202 MHz, D₂O) δ -0.90 (s, 1P), - 11.52 (m, 2P), -22.90 (m, 1P). |
| 9 | 1 | 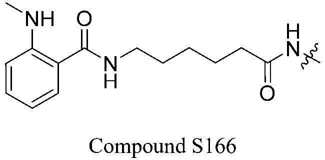 | 1403.05[M-1]⁻. ¹H NMR (500 MHz, D₂O) δ 9.06 (s, 1H), 8.44 (s, 1H), 8.14 (s, 1H), 7.92 (s, 1H), 7.32 (t, *J =* 7.1 Hz, 1H), 7.27 (d, *J=* 7.6 Hz, 1H), 6.83 (d, *J=* 8.2 Hz, 1H), 6.75 (t, *J=* 7.1 Hz, 1H), 6.02 (d, J = 5.0 Hz, 1H), 5.81 (s, 1H), 5.75 (d, *J =* 5.8 Hz, 1H), 4.91 (s, 1H), 4.76 (m, 1H), 4.57 (d, *J =* 3.6 Hz, 1H), 4.49 - 4.44 (m, 3H), 4.41 (m, 1H), 4.34 (s, 1H), 4.29 - 4.25 (m, 3H), 4.18 (s, 2H), 4.14 - 4.12 (m, 1H), 3.93 (s, 3H), 3.55 - 3.50 (m, 1H), 3.41 (s, 3H), 3.22 - 3.16 (m, 3H), 2.77 (s, 3H), 2.64 (m, 1H), 2.24 (t, *J =* 7.0 Hz, 2H), 1.59 - 1.53 (m, 2H), 1.50 - 1.55 (m, 2H), 1.27 - 1.23 (m, 2H); ³¹P NMR (202 MHz, D₂O) δ -0.98 (s, 1P), -11.56 (m, 2P), -22.83 (m, 1P). |
| 10 | 1 | 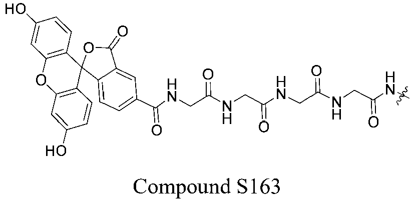 | 1742.98[M-1]⁻. ¹H NMR (500 MHz, D₂O) δ 9.04 (s, 1H), 8.42 (s, 1H), 8.26 (s, 1H), 8.08 (d, 1H), 8.01 (br, 1H), 7.88 (s, 1H), 7.16 (br, 1H), 6.74 (br, 2H), 6.48 (br, 2H), 5.93 (s, 1H), 5.71 (m, 2H), 4.89 (s, 1H), 4.76 (s, 1H), 4.66 (s, 1H), 4.51 (s, 1H), 4.44 - 4.41 (m, 3H), 4.36 (s, 1H), 4.26 - 4.21 (m, 5H), 4.14 - 4.06 (m, 3H), 4.03 - 3.99 (m, 4H), 3.89 (s, 3H), 3.89 - 3.83 (m, 2H), 3.52 - 3.45 (m, 1H), 3.38 (s, 3H), 3.31 - 3.25 (m, 1H), 2.63 - 2.58 (m, 1H); ³¹P NMR (202 MHz, D₂O) δ -1.00 (s, 1P), -11.56 (m, 2P), -22.66 (m, 1P). |
| 11 | 1 | 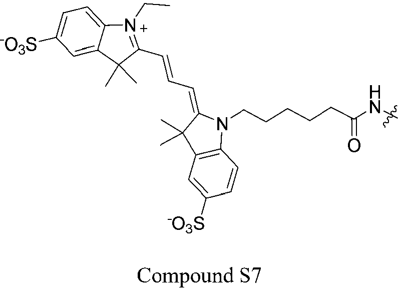 | 1769.03[M-1]⁻. ¹H NMR (500 MHz, D₂O) δ 8.37 (s, 1H), 8.32 (t, *J =* 13.6 Hz, 1H), 7.94 (m, 2H), 7.85 - 7.82 (m, 3H), 7.77 (d*, J =* 8.3 Hz, 1H), 7.28 (d, *J=* 8.3 Hz, 1H), 7.16 (d, *J* = 8.4 Hz, 1H), 6.17 (d, *J* = 13.4 Hz, 1H), 6.08 (d, *J =* 13.1 Hz, 1H), 5.91 (d, *J =* 6.0 Hz, 1H), 5.87 (s, 1H), 5.77 (d, *J =* 5.0 Hz, 1H), 4.93 (s, 1H), 4.65 (s, 1H), 4.57 (s, 1H), 4.48 - 4.45 (m, 3H), 4.38 (s, 1H), 4.29 - 4.25 (m, 4H), 4.15 (m, 3H), 3.98 (m, 2H), 3.93 (s, 3H), 3.82 - 3.80 (m, 2H), 3.48 - 3.44 (m, 1H), 3.31 (s, 3H), 3.27 - 3.25 (m, 1H), 3.21 - 3.17 (m, 1H), 2.56 (s, 1H), 2.27 (s, 2H), 1.63 - 1.54 (m, 15H), 1.28 - 1.24 (m, 6H); ³¹P NMR (202 MHz, D₂O) δ -1.22 (s, 1P), -10.69 (m, 1P), -11.46 (m, 1P), -21.90 (m, 1P). |
| 12 | 1 | 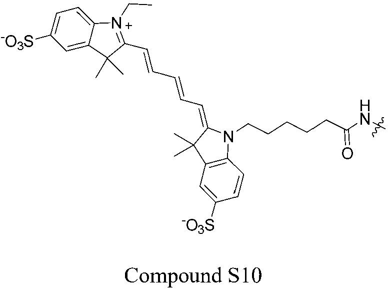 | 1795.05[M-1]⁻. ¹H NMR (500 MHz, D₂O) δ 9.18 (s, 1H), 8.63 (s, 1H), 8.24 (s, 1H), 8.03 (s, 1H), 7.97 - 7.89 (m, 2H), 7.83 - 7.76 (m, 4H), 7.28 (d, *J=* 7.9 Hz, 1H), 7.21 (d, *J =* 7.9 Hz, 1H), 6.40 (t, *J =* 12.2 Hz, 1H), 6.21 (d, *J =* 13.3 Hz, 1H), 6.08 (m, 2H), 5.89 (s, 1H), 5.77 (s, 1H), 4.97 (s, 1H), 4.76 - 4.75 (m, 1H), 4.58 (s, 1H), 4.52 - 4.49 (m, 4H), 4.37 - 4.30 (m, 4H), 4.19 (m, 3H), 4.02 (m, 5H), 3.91 (s, 2H), 3.54 - 3.50 (m, 1H), 3.43 (s, 3H), 3.39 - 3.37 (m, 1H), 2.72 (s, 1H), 2.30 (s, 2H), 1.68 - 1.58 (m, 15H), 1.33 - 1.29 (m, 6H); ³¹P NMR (202 MHz, D₂O) δ -0.93 (s, 1P), -11.42 - -11.68 (m, 2P), -21.71 (m, 1P). |
| 13 | 1 | 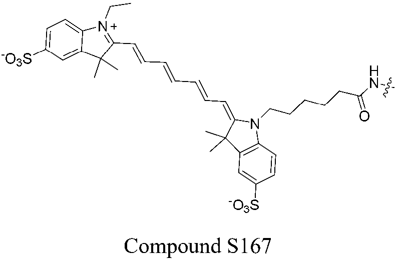 | 1821.05[M-1]-. 1H NMR (400 MHz, D₂O) δ 9.13 (s, 1H), 8.50 (s, 1H), 8.07 (s, 1H), 7.94 (s, 1H), 7.79 - 7.72 (m, 4H), 7.68 - 7.66 (m, 2H), 7.60 - 7.53 (m, 1H), 7.25 - 7.20 (m, 2H), 7.06 (d, J = 8.6 Hz, 1H), 6.14 - 6.08 (m, 2H), 5.99 (d, J = 5.9 Hz, 1H), 5.90 (s, 1H), 5.84 (d, *J =* 14.8 Hz, 1H), 5.76 (d, J = 5.5 Hz, 1H), 4.96 (s, 1H), 4.73 - 4.72 (m, 1H), 4.62 (d, J = 4.0 Hz, 1H), 4.49 - 4.43 (m, 4H), 4.35 - 4.27 (m, 4H), 4.22 - 4.16 (m, 3H), 4.03 - 4.00 (m, 2H), 3.95 (s, 3H), 3.78 (m, 2H), 3.59 - 3.53 (m, 1H), 3.37 (s, 3H), 3.33 - 3.28 (m, 1H), 2.70 (m, 1H), 2.28 (m, 2H), 1.59 - 1.46 (m, 15H), 1.30 - 1.26 (m, 6H); 31P NMR (162 MHz, D₂O) δ -0.93 (s, 1P), -11.50 (m, 2P), -22.78 (m, 1P). |
| 14 | 1 | 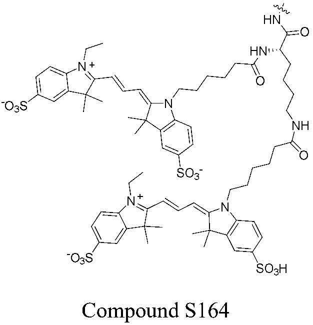 | 2509.13[M-1]⁻. ¹H NMR (500 MHz, D₂O) δ 8.50 (s, 1H), 8.40 (br, 2H), 8.09 - 8.07 (m, 1H), 7.97 (s, 1H), 7.89 (br, 8H), 7.34 - 7.29 (m, 4H), 6.34 - 6.25 (m, 4H), 6.09 (s, 1H), 5.92 (s, 1H), 5.81 (s, 1H), 5.00 (s, 1H), 4.62 (s, 1H), 4.56 - 4.52 (m, 4H), 4.35 - 4.31 (m, 4H), 4.22 (m, 3H), 4.05 (m, 12H), 3.61 (s, 1H), 3.45 - 3.35 (m, 4H), 3.28 - 3.25 (m, 1H), 2.97 (m, 2H), 2.68 (m, 1H), 2.26 - 2.23 (m, 2H), 2.14 (s, 2H), 1.64 - 1.53 (m, 32H), 1.27 - 1.10 (m, 16H); ³¹P NMR (202 MHz, D₂O) δ -0.82 (s, 1P), -11.52 (m, 2P), -22.82 (m, 1P). |
| 15 | 0 | 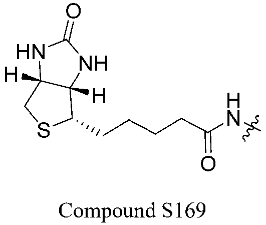 | 1369.02[M-1]⁻. ¹H NMR (500 MHz, D₂O) δ 8.39 (s, 1H), 8.13 (s, 1H), 7.95 (s, 1H), 6.03 (d, *J =* 5.6 Hz, 1H), 5.87 (s, 1H), 5.82 (d, *J=* 5.6 Hz, 1H), 4.93 (m, 1H), 4.75 (m, 1H), 4.61 (s, 1H), 4.59 - 4.56 (m, 1H), 4.53 (s, 1H), 4.50 - 4.46 (m, 4H), 4.42 (t, *J=* 4.8 Hz, 1H), 4.40 - 4.37 (m, 1H), 4.35 (s, 1H), 4.32 - 4.30 (m, 1H), 4.26 - 4.24 (m, 1H), 4.21 - 4.18 (m, 2H), 4.13 - 4.11 (m, 1H), 4.04 (s, 3H), 3.44 (s, 3H), 3.29 - 3.26 (m, 1H), 2.97 (dd, *J =* 13.2, 4.8 Hz, 1H), 2.75 (d, *J =* 13.0 Hz, 1H), 2.30 (t, *J=* 7.0 Hz, 2H), 1.70 - 1.51 (m, 4H), 1.40 - 1.36 (m, 2H); ³¹P NMR (202 MHz, D₂O) δ -0.89 (s, 1P), -11.52 - - 11.75 (m, 2P), -22.83 (t, *J=* 18.1 Hz, 1P). |

### Example 16: Preparation of compound S41

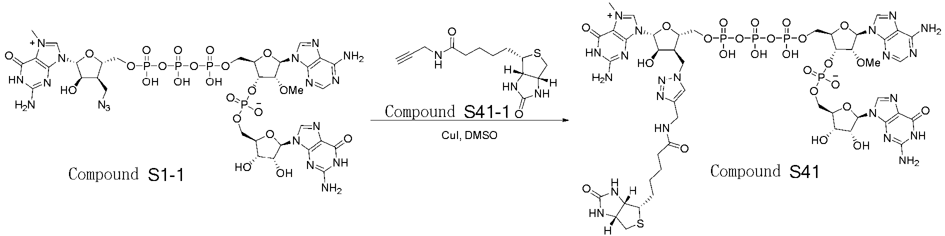

According to the above reaction route, compound S1-1 was dissolved in DMSO, followed by the addition of compound S41-1 and CuI. The mixture was stirred at room temperature. After completion of the reaction, the reaction mixture was purified by ion exchange resin to synthesize the ammonium salt of compound S41.

The characterization data of the ammonium salt of compound S41 are: 1464.04[M-1]⁻. ¹H NMR (400 MHz, D₂O) δ 9.10 (s, 1H), 8.51 (s, 1H), 8.21 (s, 1H), 7.99 (s, 1H), 7.98 (s, 1H), 6.06 (d, *J* = 5.5 Hz, 1H), 5.85 (s, 1H), 5.81 (d, *J =* 5.8 Hz, 1H), 4.96 (s, 1H), 4.74 - 4.72 (m, 1H), 4.69 - 4.67 (m, 1H), 4.61 - 4.50 (m, 5H), 4.48 - 4.45 (m, 3H), 4.42 (s, 1H), 4.36 (m, 2H), 4.32 - 4.30 (m, 2H), 4.27 - 4.25 (m, 1H), 4.21 (m, 2H), 3.99 (s, 3H), 3.95 - 3.92 (m, 1H), 3.45 (s, 3H), 3.10 - 3.06 (m, 1H), 3.01 - 2.96 (m, 1H), 2.90 (dd, *J=* 13.2, 4.7 Hz, 1H), 2.73 (d, *J =* 12.9 Hz, 1H), 2.18 (t, *J=* 7.0 Hz, 2H), 1.60 - 1.50 (m, 3H), 1.40 - 1.33 (m, 1H), 1.15 - 1.08 (m, 2H); ³¹P NMR (202 MHz, D₂O) δ -0.89 (s, 1P), -11.58 (d, *J=* 17.5 Hz, 1P), -11.84 (d, *J=* 18.2 Hz, 1P), -22.90 (t, *J =* 17.8 Hz, 1P).

### Example 17: Preparation of compound S170

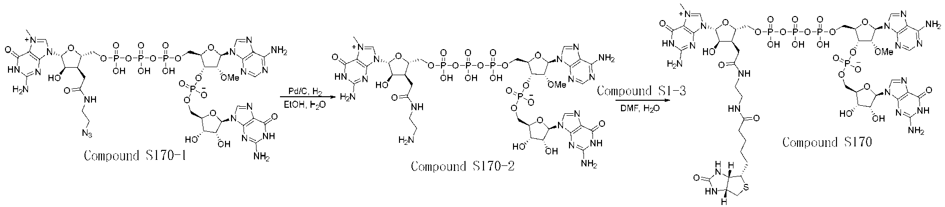

The ammonium salt of compound S170-1 was synthesized according to the method in patent CN115260264A. Referring to the synthesis method of compound S1, the ammonium salt of compound S170 was synthesized.

The characterization data of the ammonium salt of compound S170 are: 1454.02[M-1]⁻. ¹H NMR (400 MHz, D₂O) δ 9.11 (s, 1H), 8.50 (s, 1H), 8.22 (s, 1H), 7.97 (s, 1H), 6.08 (s, 1H), 5.82 - 5.81 (m, 2H), 4.94 (s, 1H), 4.56 - 4.44 (m, 6H), 4.34 (m, 3H), 4.25 (m, 2H), 4.19 (m, 2H), 4.14 - 4.12 (m, 1H), 4.01 (s, 3H), 3.44 (s, 3H), 3.29 - 3.19 (m, 5H), 2.93 - 2.91 (m, 1H), 2.73 - 2.71 (m, 1H), 2.65 (m, 1H), 2.48 - 2.46 (m, 2H), 2.16 - 2.11 (m, 2H), 1.58 - 1.43 (m, 4H), 1.27 - 1.22 (m, 3H); ³¹P NMR (202 MHz, D₂O) δ -0.91 (s, 1P), -11.58 - -11.67 (m, 2P), -22.86 (m, 1P).

### Example 18: Preparation of compound S171

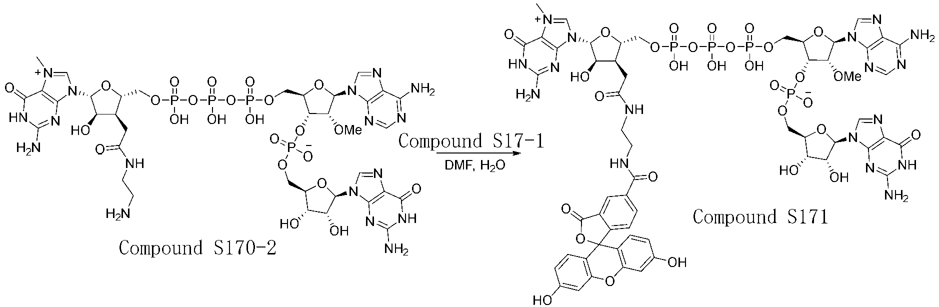

The characterization data of the ammonium salt of compound S170 are: 1586.03[M-1]⁻. ¹H NMR (500 MHz, D₂O) δ 9.03 (s, 1H), 8.41 (s, 1H), 8.09 (br, 2H), 7.88 (s, 1H), 7.61 (br, 1H), 6.82 - 6.48 (m, 7H), 5.91 (s, 1H), 5.78 (s, 1H), 5.72 (s, 1H), 4.89 (m, 1H), 4.67 - 4.64 (m, 2H), 4.45 (m, 2H), 4.40 - 4.35 (m, 2H), 4.28 (s, 2H), 4.22 - 4.15 (m, 4H), 3.90 (s, 3H), 3.50 - 3.45 (m, 4H), 3.38 (s, 3H), 2.75 (m, 1H), 2.55 (m, 2H); ³¹P NMR (202 MHz, D₂O) δ -0.97 (s, 1P), -11.57 (m, 2P), -22.72 (m, 1P).

### Example 19: Preparation of compound S168

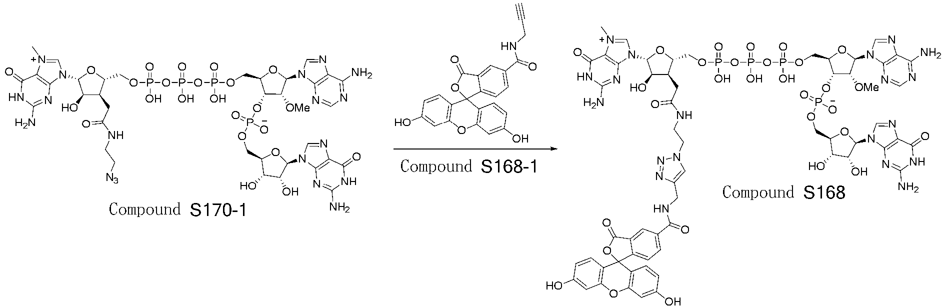

According to the above reaction route and referring to the synthetic method of compound S41, the ammonium salt of compound S 168 was synthesized.

The characterization data of compound S168 are: 1667.02[M-1]⁻. ¹H NMR (500 MHz, D₂O) δ 9.02 (s, 1H), 8.39 (s, 1H), 8.24 (s, 1H), 8.07 (s, 1H), 7.97 - 7.88 (m, 3H), 7.26 (s, 1H), 6.82 (br, 2H), 6.55 (br, 4H), 5.92 (s, 1H), 5.72 (s, 1H), 5.62 (s, 1H), 4.90 (m, 1H), 4.68 - 4.63 (m, 3H), 4.47 - 4.35 (m, 6H), 4.28 (s, 1H), 4.21 - 4.14 (m, 5H), 4.04 (s, 1H), 3.94 (s, 3H), 3.65 (m, 1H), 3.53 (m, 1H), 3.36 (s, 3H), 2.59 (m, 1H), 2.31 (m, 2H); ³¹P NMR (202 MHz, D₂O) δ -0.93 (s, 1P), -11.59 - -11.67 (m, 2P), -22.81 (m, 1P).

### Example 20 Detection of the efficiency of mRNA capping synthesis

1) The plasmids were linearized and the DNA templates were purified.
2) The mRNA was synthesized by *in vitro* transcription, using the capping structure prepared in the present disclosure, positive control CleanCap^{®}AG (3'OMe) (Trilink, N-7413), negative control (uncapped mRNA) and blank control (DNase/RNase-Free water), respectively. The reaction system is as shown in Table 1:

**Table 1. Reaction system**

| Component | Final concentration | Dosage |
|---|---|---|
| DNase/RNase-Free water | | Up to 20 µL |
| ATP Solution (100 mM) | 5 mM | 1 µL |
| CTP Solution (100 mM) | 5 mM | 1 µL |
| GTP Solution (100 mM) | 5 mM | 1 µL |
| UTP Solution (100 mM) | 5 mM | 1 µL |
| Test compound (10 mM/100 mM) | 4 mM | 8 µL/0.8 µL |
| 10×Transcription buffer | 1×Transcription buffer | 2 µL |
| DNA template | 50 µg/mL | 1 µg |
| Murine RNase Inhibitor (40 U/µL) | 1 U/µL | 0.5 µL |
| Inorganic Pyrophosphatase (0.1 U/µL) | 0.002 U/µL | 0.4 µL |
| T7 RNA Polymerase (50 U/µL) | 8 U/µL | 3.2 µL |
| Total volume | 20 µL | |

The reaction was carried out at 37°C for 2 to 3 h. TURBO DNase digestion was carried out for 15 min. The mRNA was precipitated by LiCl for 30 min or more or overnight, and the mRNA precipitate was washed with 75% ethanol, dried briefly to evaporate the ethanol, and the mRNA was redissolved with RNase-Free water.

In the reaction system listed in Table 1, since the solubility of different test compounds varies in different solutions, adjustments can be made as needed. For ease of operation, the commonly used concentrations are 10 mM and 100 mM. When the test compound exhibits good solubility, it can be prepared at an initial concentration of 100 mM, with a dosage of 0.8 µL. If the initial concentration of the test compound is 10 mM, the dosage is 8 µL. Regardless of the initial concentration, the dosages of different test compounds were the same, and the final concentrations were also the same.

For example, when testing compounds S17 and S19, their initial concentration was 10 mM, and the addition volume was 8 µL. When testing compounds 5 and 6, their initial concentration was 100 mM, and the addition volume was 0.8 µL.

3) The transcription product was purified, and the reaction yield was calculated to provide the test results of some compounds, as shown in Table 2.

**Table 2. Final product mass (µg) obtained from the final product of 20 µL mRNA synthesis reaction system**

| **Example** | **Compound** | **Final product mass** | **Example** | **Compound** | **Final product mass** |
|---|---|---|---|---|---|
| 1 | Compound S1 | 139 | 12 | Compound S10 | 99 |
| 2 | Compound S3 | 137 | 13 | Compound S167 | 87 |
| 3 | Compound S17 | 98 | 14 | Compound S164 | 12 |
| 4 | Compound S19 | 99 | 15 | Compound S169 | 131 |
| 5 | Compound 5 | 180 | 16 | Compound S41 | 126 |
| 6 | Compound 6 | 117 | 17 | Compound S170 | 96 |
| 7 | Compound S23 | 86 | 18 | Compound S171 | 92 |
| 8 | Compound S165 | 88 | 19 | Compound S168 | 99 |
| 9 | Compound S166 | 91 | | N-7413 | 122 |
| 10 | Compound S163 | 102 | | Negative control | 118 |
| 11 | Compound S7 | 86 | | Blank control | 0 |

As can be seen from Table 2, all test groups can obtain mRNA via normal transcription, with favorable yields.

### Example 21 Evaluation of expression efficiency of different capped luciferase mRNAs in HEK293T cells

1) HNE293T cells were cultured in DMEM medium containing 10% FBS and penicillin/streptomycin at 37°C and 5% CO₂.
2) The cultured HEK293T cells were spread in a 96-well plate with 1.25 × 10⁴ cells per well.
3) After the cells completely adhered to the wall, 0.5 µg of a mixture of mRNA sample and Polyplus jetMESSENGER was added to each well of cells, and cultured at 37°C and 5% CO₂ for 6 h.
4) The growth medium was removed from the cells to be tested and the cells were rinsed with PBS. After centrifugation to remove PBS, 50 µL of 1x lysis buffer was added thereto, and cells and all liquid were transferred to a microcentrifuge tube, and then centrifuged at 12000 x g at 4°C for 2 min.
5) The supernatant was transferred to a new tube. Fluorescence readings were detected using the ONE-Glo Luciferase Assay System Kit.

All test compounds, N-7413, negative control and blank control were tested under the same conditions, and some experimental results in the test results are shown in Table 3.

**Table 3. Relative fluorescence readings for capped mRNAs**

| **Example** | **Compound** | **Relative fluorescence reading** | **Example** | **Compound** | **Relative fluorescence reading** |
|---|---|---|---|---|---|
| 1 | Compound S1 | 2.41 | 12 | Compound S10 | 1.90.48 |
| 2 | Compound S3 | 2.56 | 13 | Compound S167 | 0.730.47 |
| 3 | Compound S17 | 0.20 | 14 | Compound S164 | 1.00 0.03 |
| 4 | Compound S19 | 0.17 | 15 | Compound S169 | 1.35 |
| 5 | Compound 5 | 1.86 | 16 | Compound S41 | 1.81 |
| 6 | Compound 6 | 1.90 | 17 | Compound S170 | 1.47 |
| 7 | Compound S23 | 0.73 | 18 | Compound S171 | 0.31 |
| 8 | Compound S165 | 0.34 | 19 | Compound S168 | 0.28 |
| 9 | Compound S166 | 0.87 | | N-7413 | 1.00 |
| 10 | Compound S163 | 0.30 | | Negative control | 0 |
| 11 | Compound S7 | 0.52 | | Blank control | 0 |

| | | | | | |
|---|---|---|---|---|---|
| Note: The fluorescence reading of N-7413 is used as 1 for calibration, and the ratio of the fluorescence reading of other capped mRNA transfected to the fluorescence reading of N-7413 is the relative fluorescence reading. | | | | | |

The relative fluorescence reading detection results (6 h) after transfection of capped mRNA into 293T cells are shown in Table 3. It can be seen from the table that the mRNA capped with some compounds of the present disclosure has a higher expression level, and some compounds can enable higher expression efficiency of mRNA compared to N-7413.

### Example 22 Biotin affinity assay

1) S1-30nt-mRNA (mRNA capped with compound S1, with a length of 30 nt) was prepared and purified by LiCl;
2) 500 µL of streptavidin-coated magnetic beads (2 mg) were taken, and the test tube was placed on a magnetic stand to collect the magnetic beads. The supernatant was removed and discarded, and the magnetic beads were washed;
3) The magnetic beads were resuspended in 600 µL of TES buffer, and 5.5 µg of S1-30nt-mRNA from 1) was added to the magnetic bead resuspension and mixed well; the mixture was incubated at room temperature for one hour on a shaker or vortex mixer;
4) The sample was placed on the magnetic stand for 5 min, and after the solution became clear, the supernatant was removed;
5) While keeping the sample on the magnetic stand, the magnetic beads were rinsed three times with TES buffer;
6) While keeping the sample on the magnetic stand, the magnetic beads were rinsed three times with distilled water;
7) The deionized water was removed from the beads, and 60 µL of elution buffer heated to 80°C was added and mixed well. The mixture was heated to 80°C for 3 min in a constant-temperature metal bath, then placed on the magnetic stand for 3 min. The supernatant was aspirated as the elution product.
8) Experimental results

Amount obtained: 5.5 µg; recovery rate: 100%.

Compounds S3, 169, S41, and S170 prepared in the examples were detected according to the aforementioned test procedure, and the recovery rates were all above 95%.

It can be seen that cap analogs bearing biotin groups can be bound by streptavidin magnetic beads. When mRNA is capped with such compounds, the mRNA molecules carry biotin groups and can likewise be bound by streptavidin magnetic beads, thereby enabling the purification or enrichment of the desired mRNA molecules. Not only is the obtained mRNA of high purity, but the purification or enrichment process is also simple and efficient.

Cap analogs bearing biotin groups, such as compounds S1, S3, S169, S41, and S170, are beneficial for improving the expression performance of mRNA. Additionally, during the production and preparation of mRNA, they facilitate convenient and efficient purification and enrichment of the target mRNA after capping, which is highly advantageous for the preparation and application of *in vitro* transcribed mRNA.

Example 23 Verification of the linear relationship between fluorescence-labeled mRNA concentration and fluorescence intensity
1) Some selected compounds were used to synthesize corresponding mRNAs. For example, using compound S23 (FAM-labeled), compound S166 (MANT-labeled), compound S7 (Cy3-labeled), and compound S10 (Cy5-labeled) as cap analogs, FLuc-mRNAs bearing the respective cap analogs were synthesized according to Example 20. Further purification was performed using LiCl precipitation and an RNA purification kit, and the resulting mRNA stock solutions were designated as S23-mRNA, S166-mRNA, S7-mRNA, and S10-mRNA, respectively;
2) The above mRNA stock solutions were serially diluted to sample solutions with concentrations of 200 ng/µL, 100 ng/µL, 50 ng/µL, 25 ng/µL, 12.5 ng/µL, and 6.25 ng/µL. Then, 20 µL of each dilution was added into a 96-well plate;
3) The linear correlation between concentration and fluorescence value was detected using a microplate reader, and the excitation/emission wavelengths were as follows: S23-mRNA: 492/518 nm; S166-mRNA: 356/448 nm; S7-mRNA: 570/650 nm; S10-mRNA: 640/675 nm.

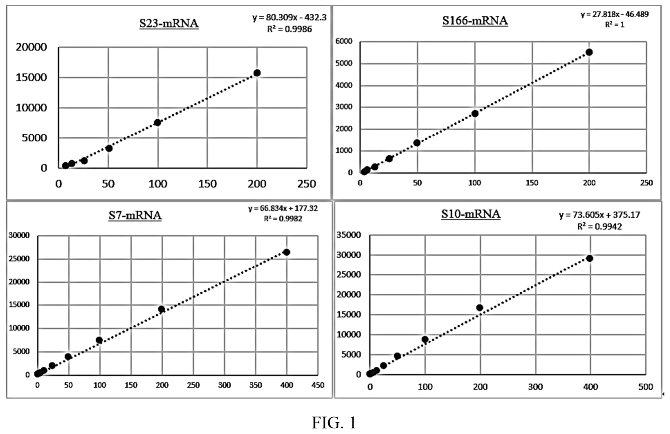

The detection results are shown in FIG. 1, which are the fluorescence intensity detection results at different mRNA concentrations. As shown in FIG. 1, the mRNA prepared with the cap analog labeled by the fluorophore exhibits a linear relationship between mRNA concentration and fluorescence intensity at the corresponding wavelength.

### Example 24 Preparation of mRNA-LNP

### Preparation process:

1) The components of LNP included: cationic lipid (SM102), cholesterol, DSPC, and DMG-PEG2000, with molar ratios of 48.5%, 38.9%, 11.1%, and 1.5%, respectively.
2) Lipid-ethanol solution: The required cationic lipid, cholesterol, DSPC, and DMG-PEG2000 were mixed and dissolved in anhydrous ethanol according to the molar ratio to prepare a lipid-ethanol solution with a total lipid concentration of 8 mM for later use.
3) mRNA-acetate buffer: The mRNA stock solution (prepared according to Example 20) was diluted with acetate buffer (200 mM, pH 5.0) to an appropriate concentration for later use.
4) Encapsulation: The mRNA-buffer phase and the lipid-ethanol phase were mixed using a microfluidic instrument (syringe pump: SPM, DK INFUSETEK CO., LTD; mixing chip: LNP-B0, FluidicLab) with a nitrogen-to-phosphorus ratio of 4.8 or other values, a flow rate ratio of 3:1 (buffer phase/ethanol phase), and a total flow rate of 3.6 mL/min. The mixed sample was dialyzed in 34 times the volume of Tris saline buffer (20.5 mM, pH 7.5, containing 8.95% sucrose) at 4°C overnight (25 or 30 kDa MwCO) to obtain mRNA-encapsulated LNPs.

### Example 25 Cell confocal experiment

Using compound S10 as a cap analog, compound S10-capped GFP-mRNA (denoted as S10-GFP-mRNA) was prepared by referring to the procedure in Example 20; then encapsulation was performed by referring to the procedure in Example 24 to obtain the encapsulated LNPs, denoted as S10-GFP-mRNA-LNP. When the cap analog was compound X, the prepared mRNA was denoted as X-GFP-mRNA; and the encapsulated LNPs were denoted as X-GFP-mRNA-LNP.

### Experimental methods

1) A 35 mm culture dish was prepared for Huh7 cell plating, with approximately 1.0×10⁵ cells per dish. The dish was placed in a 37°C, 5% CO₂ incubator and cultured for 18 h before LNP transfection;
2) The pre-encapsulated X-GFP-mRNA-LNP was taken out before transfection, and 1 µg of X-GFP-mRNA was added per dish containing 1.0×10⁵ cells. After gentle mixing, the dish was incubated in a 37°C, 5% CO₂ incubator;
3) After the incubation period, the culture medium in the cell culture dish was gently aspirated, washed twice with PBS, and 300 µL of fixative (4% paraformaldehyde) was added to the center of the dish for fixation treatment. The cells were fixed for 15 min, followed by two washes with PBS after fixation;
4) Nuclear staining: 100 µL of DAPI staining solution was added to the culture dish with fixed cells, sufficient to cover the sample, and incubated at room temperature for 3-5 min. The DAPI staining solution was aspirated, and the cells were washed 2-3 times with PBS, each wash lasting 3-5 min;
5) Before confocal observation, a small amount of PBS was added to the fixed or unfixed culture dish to maintain a moist environment within the dish;
6) The culture dish was placed under a confocal microscope, and the fluorescence expression and intracellular localization distribution of DAPI, GFP, and compound X were observed using excitation/emission wavelengths of 410 nm/410-585 nm, 488 nm/494-572 nm, and the excitation/emission wavelengths of the fluorophore in compound X, respectively.

Taking compound S10 as an example, its structure contains a Cy5 group with an excitation wavelength/emission wavelength of 643 nm/638-759 nm.

When the compound carries different fluorophores, such as FAM, MANT, Cy3, or Cy5 groups, the corresponding excitation/emission wavelengths of the fluorophores are selected.

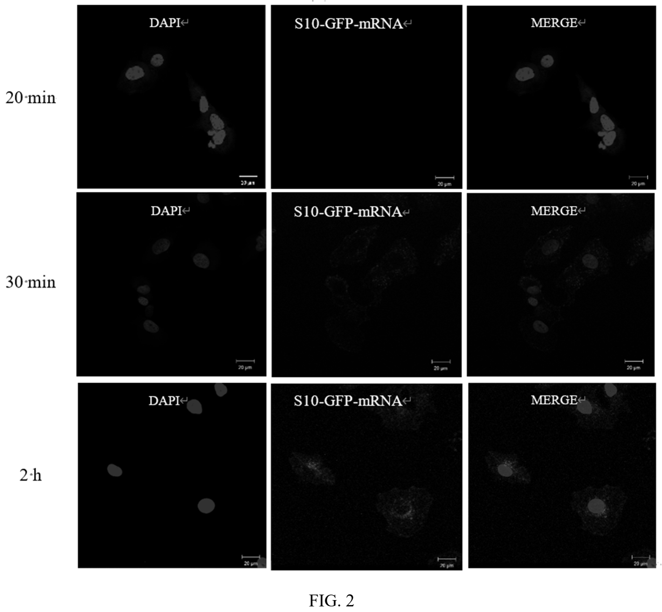

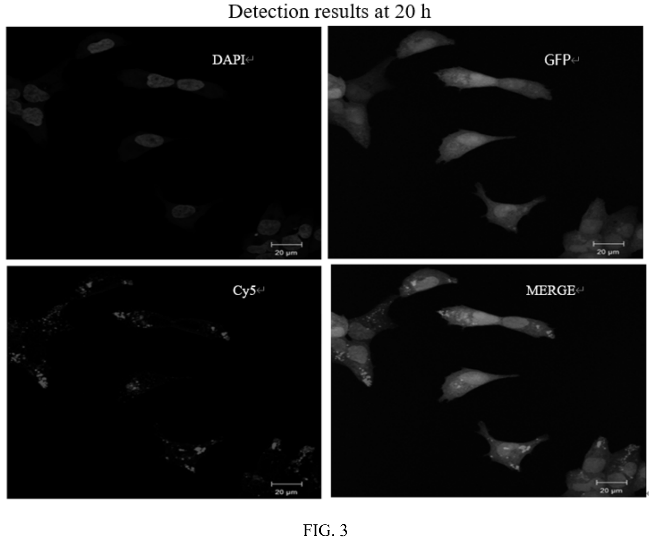

Taking S 10-GFP-mRNA as an example, the fluorescence expression and intracellular distribution were detected by referring to the above procedure. The detected results are shown in FIG. 2 and FIG. 3.

Among them, the results after staining and fixation for 20 min, 30 min, and 2 h are shown in FIG. 2 (imaging results of the same region at the same time point); the detection results after staining and fixation for 20 h are shown in FIG. 3 (imaging results of the same region).

As shown in FIG. 2 and FIG. 3, 30 min after transfection with S10-GFP-mRNA (labeled with the Cy5 group), the distribution of Cy5 fluorescence in Huh7 cells can be observed under a confocal microscope, and the fluorescence becomes clear 2 h post-transfection. At 20 h post-transfection, obvious GFP expression can be observed, with the Cy5 fluorescence exhibiting an aggregated distribution within the cells. This indicates that the Cy5-labeled mRNA can serve as a tracer to observe mRNA distribution at different time points after transfection, while the mRNA can be expressed normally.

### Example 26 In vivo imaging experiment in mice 1 (Cy5 fluorescence detection)

Using compound S10 (bearing a Cy5 group) as a cap analog, compound S10-capped FLuc-mRNA (denoted as S10-FLuc-mRNA) was prepared by referring to the procedure in Example 20; then encapsulation was performed by referring to the procedure in Example 24 to obtain the encapsulated LNPs, denoted as S10-FLuc-mRNA-LNP.

### Experimental methods

1) The S10-FLuc-mRNA-LNP sample was diluted to 200 ng/µL for later use;
2) Healthy mice were randomly divided into two groups, with three mice in each group; the mice in the experimental group were injected (IV) with 0.2 mL of sample (40 µg/mouse) via the tail vein, and the mice in the control group were injected (IV) with 0.2 mL of normal saline via the tail vein. *In vivo* imaging was performed after 1 h, with an excitation wavelength/emission wavelength of 640 nm/680 nm.
3) After the *in vivo* imaging detection, the liver, lung, and spleen of the mice were excised and imaged using the imaging system, with an excitation/emission wavelength of 640 nm/680 nm.

### Experimental results

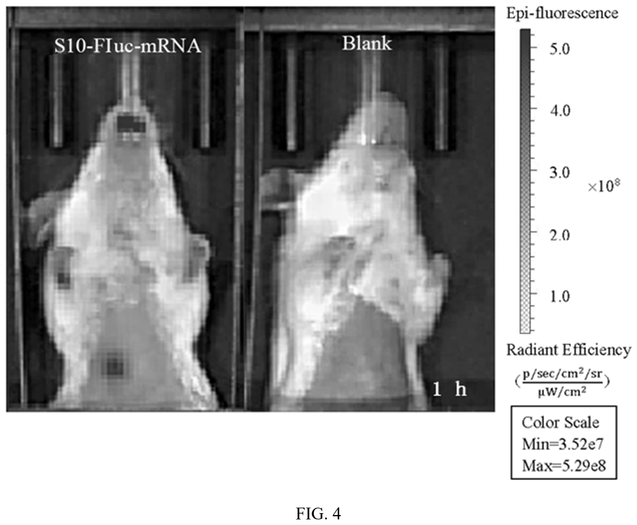

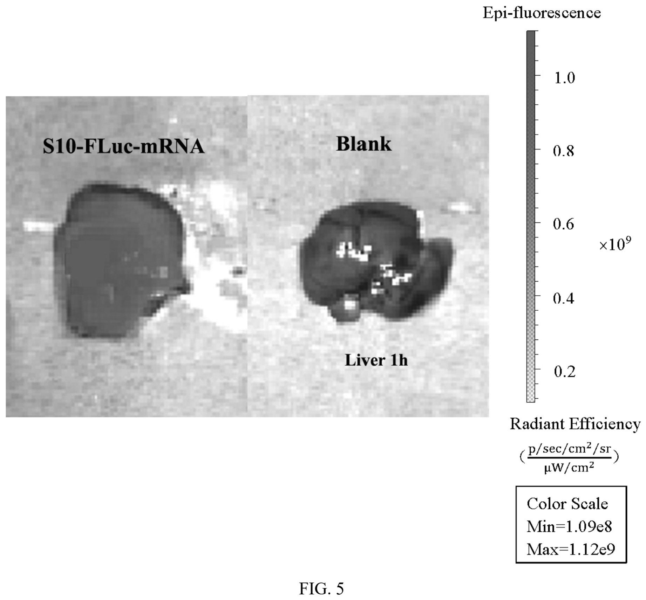

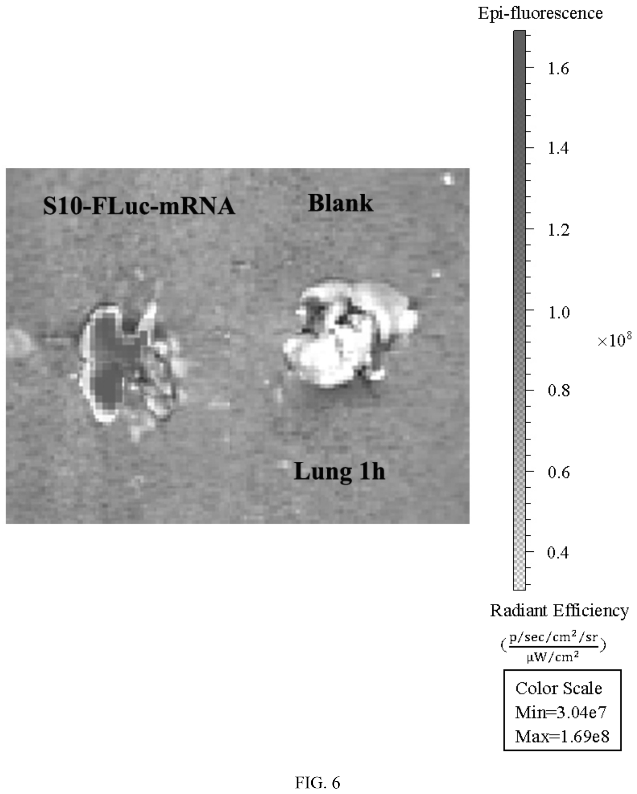

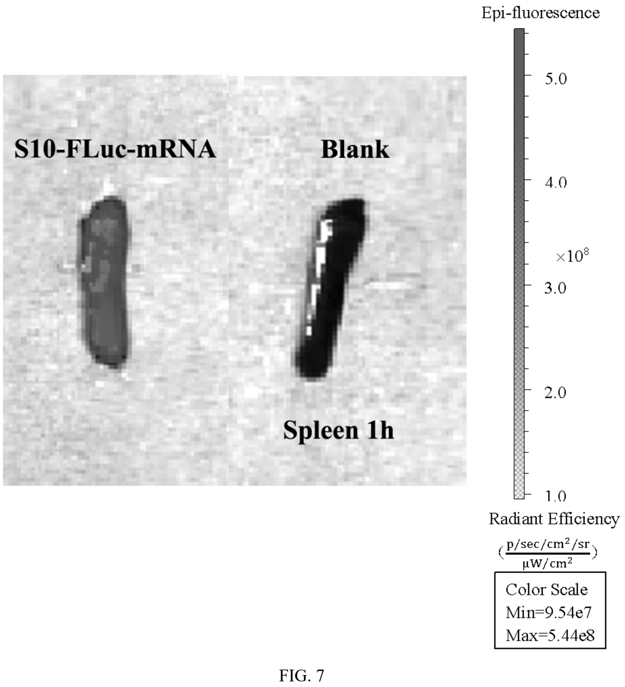

The detection results are shown in FIGS. 4, 5 to 7. FIG. 4 shows the *in vivo* imaging detection results of mice 1 h after injection; FIGS. 5 to 7 show the imaging detection results of the liver, lung, and spleen of the mice, respectively.

As shown in FIG. 4, obvious Cy5 fluorescence can be observed in the live mice 1 h after IV injection of S10-FLuc-mRNA-LNP; as shown in FIGS. 5 to 7, S10-FLuc-mRNA-LNP is primarily distributed in the liver, lung, and spleen of the mice after injection.

### Example 27 In vivo imaging experiment in mice 2 (Fluc fluorescence detection)

The test sample was the same as in Example 26, namely S10-FLuc-mRNA-LNP.

### Experimental methods

The S10-FLuc-mRNA-LNP sample was diluted to 50 ng/µL, and the fluorescent substrate (D-Luciferin, which could catalyze FLuc to emit yellow-green fluorescence) was prepared as a 15 mg/mL sample for later use;
1) Three healthy mice were selected, and each mouse was injected (IV) with 0.2 mL of S10-FLuc-mRNA-LNP sample (10 µg/mouse) via the tail vein. After 3 h, 0.2 mL of the fluorescent substrate was intraperitoneally injected (IP), followed by abdominal *in vivo* imaging with an excitation wavelength/emission wavelength of 560/590 nm.

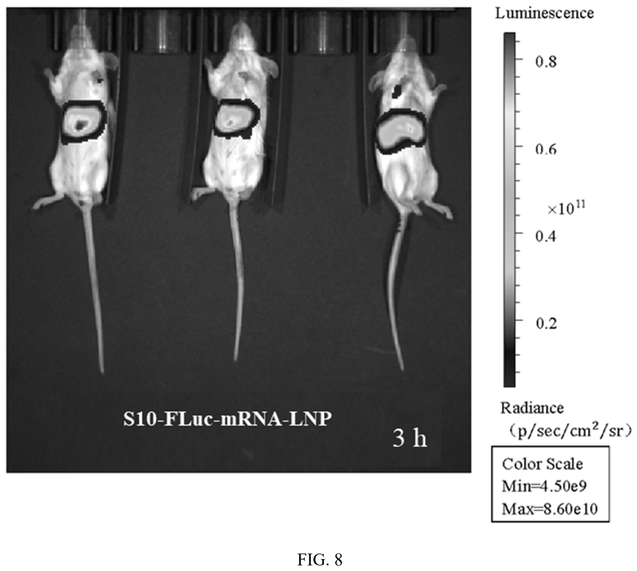

The detection results are shown in FIG. 8. As can be seen from FIG. 8, 3 h after IV injection of S10-FLuc-mRNA-LNP encoding FLuc protein, obvious FLuc fluorescence can be observed in the *in vivo* imaging of mice, indicating that after injection of S10-FLuc-mRNA-LNP into the mice, the S10-FLuc-mRNA therein can normally express the FLuc protein in mice *in vivo.*

### Example 28 Synthesis of compound D20-1

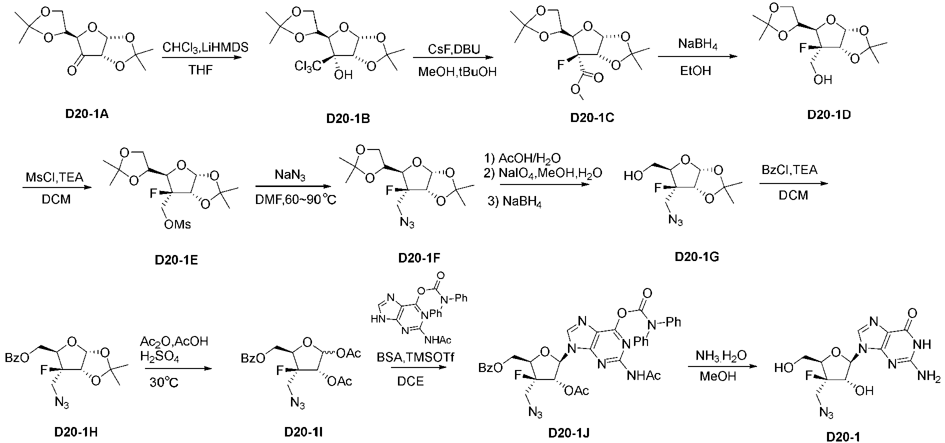

Compound D20-1A (97.00 g, 375.56 mmol) and CHCl₃ (170 mL) were added to anhydrous tetrahydrofuran at room temperature, and the mixture was cooled to -78°C. Lithium bis(trimethylsilyl)amide (500 mL) was slowly added dropwise to the system, and the reaction was stirred and reacted at the same temperature for 4 h. The reaction mixture was poured into a saturated aqueous sodium bicarbonate solution (700 mL) at 0°C, and the phases were separated. The organic phase was washed with saturated brine, dried over anhydrous sodium sulfate, concentrated under reduced pressure, and purified by slurrying with ethanol to obtain compound D20-1B as a white solid (73.79 g, 52.3%).

At room temperature, cesium fluoride (296.20 g, 1.95 mol) was added to a mixed solution of compound D20-1B (73.79 g, 195.4 mmol) in methanol (500 mL) and tert-butanol (500 mL), followed by the addition of DBU (117 mL, 781.0 mmol). The mixture was stirred and reacted for 1 h. A saturated ammonium chloride solution (150 mL) was added to the reaction system, and the mixture was stirred for 10 min. Water (2 L) and ethyl acetate (1 L) were added, and the mixture was stirred for 5 min. The phases were separated, and the aqueous phase was extracted with ethyl acetate (500 mL × 2). The organic phases were combined, concentrated under reduced pressure and purified by slurrying with ethanol to obtain compound D20-1C as a white solid (48.50 g, 77.5%).

At 0°C, sodium borohydride (6.73 g, 177.95 mmol) was added in batches to a solution of compound D20-1C (28.5 g, 88.98 mmol) in ethanol (290 mL), and the reaction was stirred at room temperature. A saturated ammonium chloride solution (100 mL) was added to the reaction system. The reaction mixture was concentrated and extracted with dichloromethane (100 mL) and water (100 mL). The aqueous phase was extracted with dichloromethane (100 mL × 2). The organic phases were combined, washed with saturated brine, dried over anhydrous sodium sulfate, and concentrated under reduced pressure to obtain compound D20-1D as a colorless oil (27.13 g,100%), which was used directly in the next step without purification.

Under an ice bath and a nitrogen atmosphere, methanesulfonyl chloride (7.0 mL, 91.14 mmol) was slowly added dropwise to a solution of compound D20-1D (22.20 g, 75.95 mmol) and triethylamine (21.1 mL, 151.90 mmol) in dichloromethane (220 mL). After completion of the addition, the reaction mixture was stirred and reacted at room temperature for 1 h. Water (200 mL) was added to the system, stirred for 2 min, and the phases were separated. The organic phase was washed with water (100 mL), dried over anhydrous sodium sulfate, and concentrated under reduced pressure. The residue was purified by slurrying with petroleum ether (200 mL) to obtain compound D20-1E as a pale yellow solid (26.58 g, 94.5%).

At room temperature, sodium azide (2.19 g, 33.75 mmol) was added to a solution of compound D20-1E (5.00 g, 13.50 mmol) in anhydrous N,N-dimethylformamide (25 mL). The mixture was heated to 120°C, stirred and reacted overnight. After cooling to room temperature, water (150 mL) was added, and the mixture was extracted with methyl tert-butyl ether (60 mL × 2). The organic phases were combined, washed with water (100 mL), dried over anhydrous sodium sulfate, and concentrated under reduced pressure to obtain compound D20-1F as a colorless oil (4.28 g, 100%). The crude product was directly used in the next step without further purification.

At room temperature, acetic acid (35 mL) and water (15 mL) were mixed well and added to compound D20-1F (4.28 g, 13.50 mmol). The mixture was heated to 50°C, stirred and reacted for 2 h. The reaction mixture was concentrated under reduced pressure, followed by the addition of a saturated aqueous sodium bicarbonate solution (19 mL) and methanol (19 mL). The mixture was stirred for 2 min. Sodium periodate (3.18 g, 14.85 mmol) was added to the system, and the mixture was stirred and reacted for 30 min. The reaction mixture was subjected to suction filtration, and the filtrate was collected and cooled to 0°C. Sodium borohydride (1.02 g, 27.00 mmol) was added to the system in batches, and the reaction mixture was stirred at the same temperature for 30 min. A saturated aqueous ammonium chloride solution (10 mL) was added to quench the reaction. The majority of the reaction mixture was concentrated under reduced pressure, followed by the addition of water (50 mL) and dichloromethane (60 mL × 2) for extraction. The organic phases were combined, dried over anhydrous sodium sulfate, and the reaction mixture was concentrated under reduced pressure to obtain compound D20-1G as a colorless oil (3.34 g, 100%). The crude product was directly used in the next step without further purification.

Under an ice bath and a nitrogen atmosphere, benzoyl chloride (2.35 mL, 20.25 mmol) was slowly added dropwise to a solution of compound D20-1G (3.34 g, 13.50 mmol) and triethylamine (3.75 mL, 27.00 mmol) in dichloromethane (33 mL). After completion of the addition, the mixture was stirred and reacted at room temperature for 30 min. Water (100 mL) was added to the system, and the phases was separated. The organic phase was concentrated under reduced pressure. The residue was purified by silica gel column chromatography (petroleum ether/ethyl acetate = 15/1) to obtain compound D20-1H as a colorless oil (3.64 g, 76.8%).

At room temperature, concentrated sulfuric acid (83 µL, 1.55 mmol) was diluted with acetic acid (1 mL) and then added to a solution of compound D20-1H (3.64 g, 10.36 mmol) in acetic anhydride (2.5 mL, 25.90 mmol) and acetic acid (36 mL). The temperature was raised to 30°C, and the reaction was stirred for 5 h. The reaction mixture was poured into water (200 mL), extracted with ethyl acetate (100 mL), then washed sequentially with saturated aqueous sodium bicarbonate solution (100 mL × 2) and saturated aqueous sodium chloride solution (100 mL), dried over anhydrous sodium sulfate, and concentrated under reduced pressure. The residue was purified by silica gel column chromatography (petroleum ether/ethyl acetate = 4/1) to obtain compound D20-1I as a colorless oil (3.40 g, 82.9%).

At room temperature under a nitrogen atmosphere, N, O-bis(trimethylsilyl)acetamide (12 mL, 44.01 mmol) was added to a suspension of 2-acetylamino-9H-purine-6-yl diphenylcarbamate (6.84 g, 17.60 mmol) in 1,2-dichloroethane (70 mL). The mixture was heated to 70°C and stirred for 2 h. Trimethylsilyl trifluoromethanesulfonate (3.4 mL, 19.07 mmol) was added to the system, and then compound D20-1I (0.50 g, 1.26 mmol) was dissolved in 1,2-dichloroethane (40 mL) and added to the system. The mixture was heated to 80°C, stirred and reacted for 2 h. After cooling to room temperature, water (100 mL) was added to the system. The phases were separated, and the aqueous phase was extracted with dichloromethane (50 mL). The phases were separated, and the organic phases were combined, washed with saturated sodium chloride solution (100 mL), dried over anhydrous sodium sulfate, and concentrated under reduced pressure. The residue was purified by silica gel column chromatography (petroleum ether/ethyl acetate = 1/4) to obtain compound D20-1J as a white solid (2.78 g, 26.2%).

Aqueous ammonia (6 mL) was added to a solution of compound D20-1J (2.78 g, 3.84 mmol) in methanol (30 mL) at room temperature, and the mixture was heated to 50°C and stirred overnight. The reaction mixture was concentrated under reduced pressure, and purified by slurrying with acetonitrile (30 mL) to obtain compound D20-1 as a white solid (1.15 g, 88.5%).

The characterization data of compound D20-1 are: MS (ESI, pos. ion) m/z: 341.15 [M+H]+; ¹H NMR (500 MHz, DMSO) δ 9.71 (s, 1H), 7.76 (s, 1H), 6.55 (s, 3H), 5.66 (s, 1H), 5.06 (s, 1H), 4.69-4.63 (m, 1H), 4.10-4.01 (m, 1H), 3.90-3.67 (m, 3H), 3.66-3.60 (m, 1H).

The nucleoside intermediates required in the following examples can be synthesized by referring to the method of compound D20-1.

### Example 29-Example 38 Synthesis of compounds

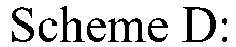

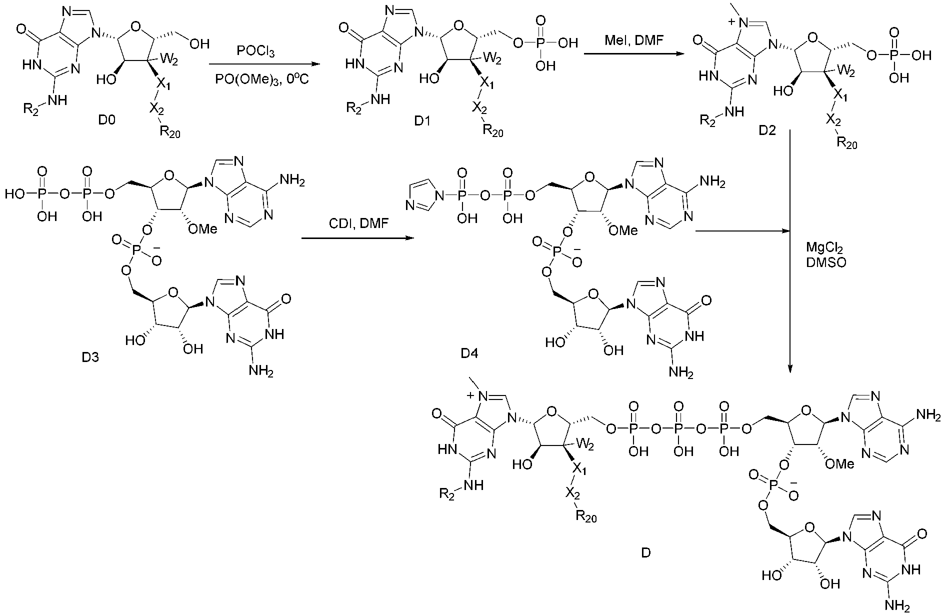

According to the above synthetic route, nucleoside D0 was dissolved in trimethyl phosphate, and phosphorus oxychloride was added dropwise at 0°C. After completion of the reaction, the reaction mixture was quenched with 1M TEAB solution, then directly purified by an ion exchange column. The product was collected, concentrated and lyophilized to obtain the triethylamine salt of compound D1.

Compound D1 was dissolved in DMF, and iodomethane was added. After the reaction, the reaction mixture was concentrated, diluted with water, and purified by an ion exchange column to obtain the triethylamine salt of compound D2.

Compound D3 was suspended in DMF, and CDI was added. After the reaction, an acetone solution of sodium iodide was added for precipitation. The mixture was filtered, and the filter cake was subjected to rotary evaporation until dryness to obtain compound D4.

Compound D2, compound D4, and anhydrous magnesium chloride were added to DMSO and stirred at room temperature. After the reaction, the mixture was diluted with water and purified by an ion exchange column to obtain product D.

Examples 29-38 were prepared by referring to the above synthetic route, wherein D0 was synthesized by referring to the method for compound D20-1 in Example 28. The compounds prepared in Examples 29-38 and their characterization parameters are shown in the table below.

| Example | Structure | Characterization data |
|---|---|---|
| 29 | 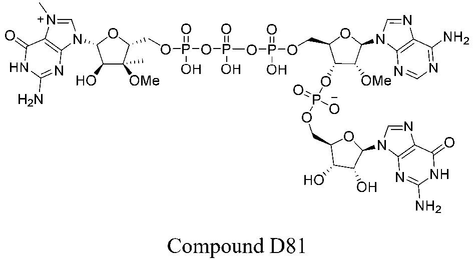 | 1172.02[M-1]⁻. ¹H NMR (500 MHz, D₂O) δ 9.08 (s, 1H), 8.38 (s, 1H), 8.15 (s, 1H), 7.92 (s, 1H), 5.98 *(d, J=* 4.9 Hz, 1H), 5.81 (d, *J=* 7.4 Hz, 1H), 5.77 (d, *J=* 5.7 Hz, 1H), 4.88 - 4.86 (m, 1H), 4.75 - 4.73 (m, 1H), 4.46 - 4.44 (m, 3H), 4.39 (d, *J =* 7.4 Hz, 1H), 4.36 - 4.34 (m, 1H), 4.30 - 4.27 (m, 3H), 4.20 - 4.13 (m, 3H), 4.05 - 3.99 (m, 4H), 3.43 (s, 3H), 3.29 (s, 3H), 1.39 (s, 3H); ³¹P NMR (202 MHz, D₂O) δ -0.93 (s, 1P), -11.60 (m, 1P), -12.01 (m, 1P), -22.84 (m, 1P). |
| 30 | 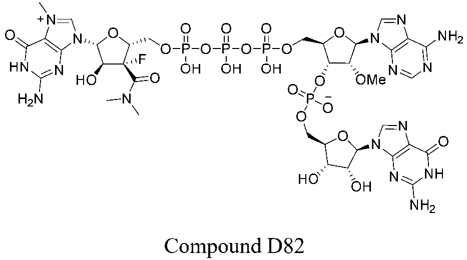 | 1217.04[M-1]⁻. ¹H NMR (500 MHz, D₂O) δ 9.13 (s, 1H), 8.49 (s, 1H), 8.22 (s, 1H), 7.98 (s, 1H), 6.03 (d, *J =* 5.5 Hz, 1H), 5.90 (d, *J= 2.4* Hz, 1H), 5.83 (d, *J=* 5.7 Hz, 1H), 5.07 (d, *J=* 20.0 Hz, 1H), 4.99 (dd, *J=* 14.8, 2.6 Hz, 1H), 4.94 - 4.92 (m, 1H), 4.76 (m, 1H), 4.51 - 4.49 (m, 2H), 4.44 - 4.35 (m, 5H), 4.26 - 4.17 (m, 3H), 4.02 (s, 3H), 3.44 (s, 3H), 3.14 (d, *J=* 3.8 Hz, 3H), 2.99 (s, 3H); ³¹P NMR (202 MHz, D₂O) δ -0.89 (s, 1P), - 11.58 - -11.68 (m, 2P), -22.80 - -22.98 (m, 1P). |
| 31 | 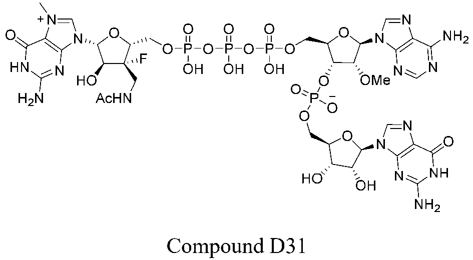 | 1217.04[M-1]⁻. ¹H NMR (500 MHz, D₂O) δ 8.38 (s, 1H), 8.05 (s, 1H), 7.96 (s, 1H), 5.98 (d, *J =* 6.0 Hz, 1H), 5.84 (d, *J =* 5.7 Hz, 1H), 5.78 (s, 1H), 4.92 (m, 1H), 4.60 - 4.50 (m, 3H), 4.44 (d, *J=* 11.0 Hz, 1H), 4.40 - 4.33 (m, 5H), 4.27 - 4.18 (m, 3H), 3.99 (s, 3H), 3.81 (dd, *J =* 32.0, 15.6 Hz, 1H), 3.66 (t, *J =* 15.6 Hz, 1H), 3.41 (s, 3H), 2.01 (s, 3H); ³¹P NMR (202 MHz, D₂O) δ 1.70 (s, 1P), -8.98 - -9.15 (m, 2P), -20.33 - -20.51 (m, 1P). |
| 32 | 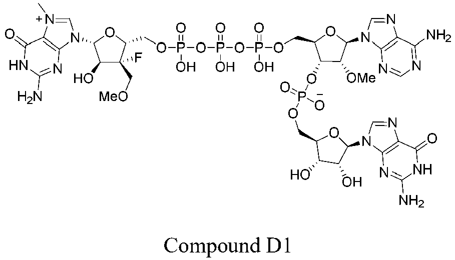 | 1190.02[M-1]⁻. ¹H NMR (500 MHz, D₂O) δ 8.90 (s, 1H), 8.46 (s, 1H), 8.16 (s, 1H), 7.94 (s, 1H), 6.03 (*d, J =* 5.3 Hz, 1H), 5.78 (s, 1H), 5.76 (d, *J =* 5.7 Hz, 1H), 4.92 - 4.89 (m, 1H), 4.75 - 4.73 (m, 1H), 4.59 *(d, J =* 11.9 Hz, 1H), 4.54 - 4.47 (m, 2H), 4.46 - 4.44 (m, 1H), 4.40 - 4.38 (m, 1H), 4.36 - 4.31 (m, 4H), 4.24 - 4.14 (m, 3H), 3.95 (s, 3H), 3.88 - 3.77 (m, 2H), 3.42 (s, 3H), 3.37 (s, 3H); ³¹P NMR (202 MHz, D₂O) δ -0.91 (s, 1P), -11.54 - -11.74 (m, 2P), -22.91 - - 23.09 (m, 1P). |
| 33 | 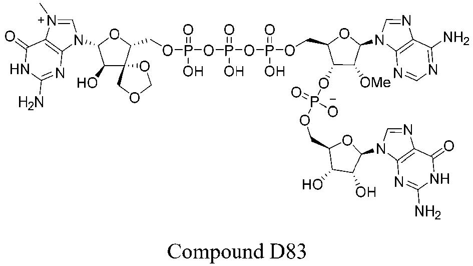 | 1186.05[M-1]⁻. ¹H NMR (500 MHz, D₂O) δ 8.39 (s, 1H), 8.08 (s, 1H), 7.96 (s, 1H), 6.00 (d, *J* = 6.1 Hz, 1H), 5.85 (d, *J =* 5.8 Hz, 1H), 5.78 (s, 1H), 5.03 (s, 1H), 4.94 (m, 1H), 4.56 (m, 2H), 4.52 (m, 1H), 4.45 - 4.35 (m, 7H), 4.29 - 4.22 (m, 5H), 4.05 - 4.02 (m, 4H), 3.41 (s, 3H), 3.37 (s, 3H); ³¹P NMR (202 MHz, D₂O) δ -0.82 (s, 1P), -11.41 - -11.70 (m, 2P), -23.01 (m, 1P). |
| 34 | 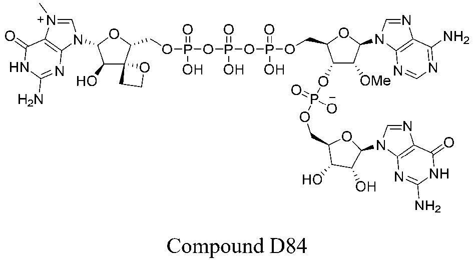 | 1190.02[M-1]⁻. ¹H NMR (500 MHz, D₂O) δ 8.92 (s, 1H), 8.53 (s, 1H), 8.25 (s, 1H), 8.00 (s, 1H), 6.07 (d, *J =* 5.0 Hz, 1H), 5.80 (d, *J =* 5.3 Hz, 1H), 5.74 (s, 1H), 4.94 (m, 1H), 4.84 - 4.79 (m, 1H), 4.70 (s, 1H), 4.64 - 4.62 (m, 1H), 4.57 - 4.49 (m, 5H), 4.45 - 4.43 (m, 2H), 4.38 - 4.35 (m, 2H), 4.28 - 4.17 (m, 3H), 3.98 (s, 3H), 3.47 (s, 3H), 2.96 - 2.91 (m, 1H), 2.80 - 2.74 (m, 1H); ³¹P NMR (202 MHz, D₂O) δ -0.90 (s, 1P), -11.33 - -11.64 (m, 2P), -22.84 (m, 1P). |
| 35 | 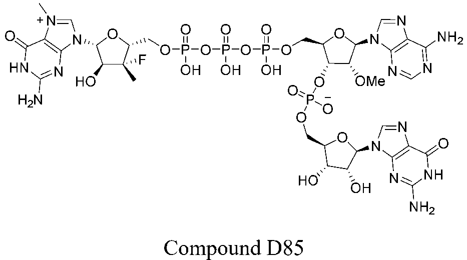 | 1160.04[M-1]⁻. ¹H NMR (500 MHz, D₂O) δ 8.93 (s, 1H), 8.51 (s, 1H), 8.23 (s, 1H), 8.00 (s, 1H), 6.07 (d, *J =* 5.2 Hz, 1H), 5.82 - 5.81 (m, 2H), 4.96 - 4.94 (m, 1H), 4.53 (s, 1H), 4.51 - 4.49 (m, 1H), 4.47 - 4.41 (m, 5H), 4.35 (m, 2H), 4.28 - 4.18 (m, 3H), 4.00 (s, 3H), 3.46 (s, 3H), 1.55 (d, *J =* 23.4 Hz, 3H); ³¹P NMR (202 MHz, D₂O) δ -0.88 (s, 1P), -11.37 (d, *J= 19.0* Hz, 1P), -11.62 *(d, J=* 17.9 Hz, 1P), -22.91 - -23.09 (t, *J=* 18.4 Hz, 1P). |
| 36 | 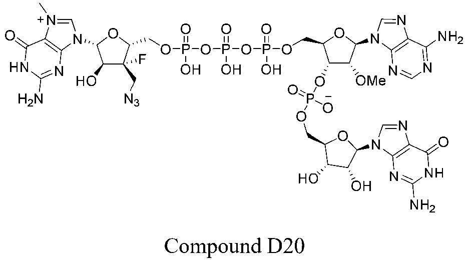 | 1201.05[M-1]⁻. ¹H NMR (500 MHz, D₂O) δ 8.86 (s, 1H), 8.44 (s, 1H), 8.13 (s, 1H), 7.96 (s, 1H), 6.03 *(d, J=* 5.6 Hz, 1H), 5.81 - 5.80 (m, 2H), 4.93 (m, 1H), 4.76 - 4.75 (m, 1H), 4.66 *(d, J=* 11.9 Hz, 1H), 4.56 - 4.47 (m, 3H), 4.42 - 4.36 (m, 3H), 4.33 (m, 2H), 4.26 - 4.16 (m, 3H), 3.98 (s, 3H), 3.93 (d, *J =* 15.0 Hz, 1H), 3.73 (dd, J= 32.5, 14.2 Hz, 1H), 3.42 (s, 3H); ³¹P NMR (202 MHz, D₂O) δ - 0.91 (s, 1P), -11.62 - -11.71 (m, 2P), - 22.92 - -23.10 (m, 1P). |
| 37 | 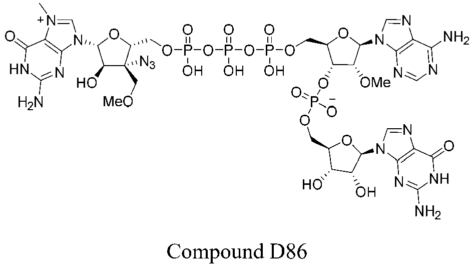 | 1213.03[M-1]⁻. ¹H NMR (500 MHz, D₂O) δ 8.21 (s, 1H), 7.90 (s, 1H), 7.79 (s, 1H), 5.83 (d, *J =* 4.7 Hz, 1H), 5.69 (d, *J =* 4.7 Hz, 1H), 5.57 (s, 1H), 4.53 (s, 1H), 4.45 (s, 1H), 4.38 - 4.35 (m, 2H), 4.25 - 4.07 (m, 10H), 3.90 (s, 3H), 3.80 - 3.71 (m, 2H), 3.34 (s, 3H), 3.29 (s, 3H); ³¹P NMR (202 MHz, D₂O) δ -1.03 (s, 1P), -11.50 (d, *J =* 17.6 Hz, 1P), -11.72 (*d, J =* 18.1 Hz, 1P), -22.63 - -22.81 (m, 1P). |
| 38 | 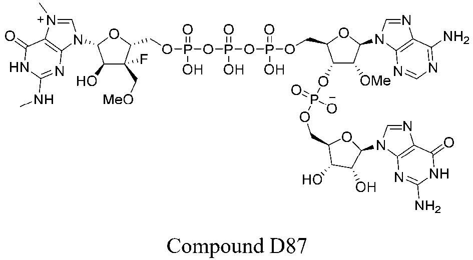 | 1204.02[M-1]⁻. ¹H NMR (500 MHz, D₂O) δ 8.85 (s, 1H), 8.41 (s, 1H), 8.11 (s, 1H), 7.94 (s, 1H), 5.98 (d, *J =* 5.6 Hz, 1H), 5.82 (s, 1H), 5.78 (d, *J = 5.5* Hz, 1H), 4.90 (m, 1H), 4.75 - 4.72 (m, 1H), 4.67 (d, *J =* 11.6 Hz, 1H), 4.59 - 4.50 (m, 2H), 4.46 - 4.44 (m, 1H), 4.36 - 4.31 (m, 5H), 4.24 - 4.14 (m, 3H), 3.96 (s, 3H), 3.87 - 3.79 (m, 2H), 3.40 (s, 3H), 3.38 (s, 3H), 2.85 (s, 3H); ³¹P NMR (202 MHz, D₂O) δ - 0.94 (s, 1P), -11.59 - -11.68 (m, 2P), - 23.04 (m, 1P). |

### Example 39-Example 45 Synthesis of compounds

The compounds of Examples 39-45 were prepared by referring to the synthetic procedure of Example 32, with the difference that, during the preparation of compound D2 from compound D1 by referring to the synthetic route of Scheme D, iodomethane was replaced by different haloalkanes such as benzyl bromide, iodoethane, allyl bromide, 4-fluorobenzyl bromide, 4-chlorobenzyl bromide, 4-methylbenzyl bromide, and 4-methoxybenzyl bromide, thereby obtaining the desired target compounds, such as the compounds of Examples 39-45, whose structures and characterization data are shown in the table below.

| Example | Structure | Characterization data |
|---|---|---|
| 39 | 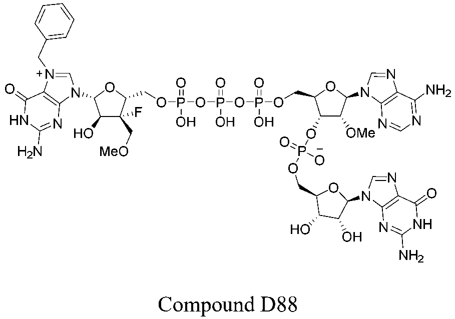 | 1266.03[M-1]⁻. ¹H NMR (500 MHz, D₂O) δ 9.26 (s, 1H), 8.50 (s, 1H), 8.20 (s, 1H), 7.98 (s, 1H), 7.14 - 7.09 (m, 5H), 6.03 (d, *J =* 5.5 Hz, 1H), 5.83 (s, 1H), 5.78 (d, *J =* 5.6 Hz, 1H), 5.43 - 5.32 (m, 2H), 5.00 - 4.97 (m, 1H), 4.76 - 4.74 (m, 1H), 4.66 - 4.58 (m, 2H), 4.54 (m, 1H), 4.50 - 4.48 (m, 1H), 4.47 - 4.36 (m, 5H), 4.30 - 4.18 (m, 3H), 3.91 - 3.83 (m, 2H), 3.46 (s, 3H), 3.41 (s, 3H); ³¹P NMR (202 MHz, D₂O) δ -0.88 (s, 1P), -11.46 - -11.69 (m, 2P), -22.70 - -22.89 (m, 1P). |
| 40 | 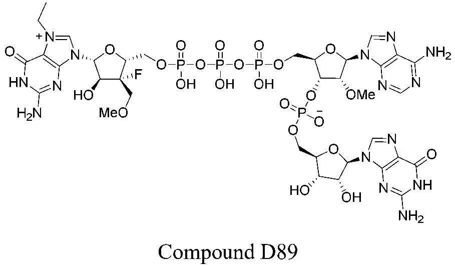 | 1204.04[M-1]⁻. ^{l}H NMR (500 MHz, D₂O) δ 8.93 (s, 1H), 8.47 (s, 1H), 8.16 (s, 1H), 7.98 (s, 1H), 6.02 (d, *J =* 5.9 Hz, 1H), 5.83 - 5.82 (m, 2H), 4.96 - 4.93 (m, 1H), 4.77 - 4.75 (m, 1H), 4.64 (d, *J =* 12.2 Hz, 1H), 4.60 - 4.52 (m, 2H), 4.51 - 4.49 (m, 1H), 4.44 - 4.42 (m, 1H), 4.40 - 4.32 (m, 6H), 4.28 - 4.24 (m, 1H), 4.22 - 4.16 (m, 2H), 3.92 - 3.80 (m, 2H), 3.42 (s, 3H), 3.41 (s, 3H), 1.46 *(t, J =* 7.2 Hz, 3H); ³¹P NMR (202 MHz, D₂O) δ -0.91 (s, 1P), -11.56 - -11.73 (m, 2P), -22.96 - - 23.15 (m, 1P). |
| 41 | 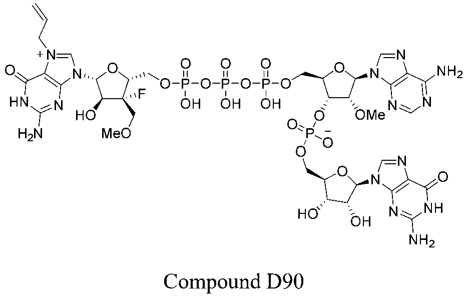 | 1216.03[M-1]⁻. ¹H NMR (500 MHz, D₂O) δ 8.96 (s, 1H), 8.45 (s, 1H), 8.14 (s, 1H), 7.97 (s, 1H), 6.05 - 5.97 (m, 2H), 5.83 (d, *J =* 5.9 Hz, 1H), 5.81 (s, 1H), 5.34 (d, *J =* 10.4 Hz, 1H), 5.26 (d, *J = 17.1* Hz, 1H), 4.96 - 4.93 (m, 3H), 4.82 - 4.79 (m, 1H), 4.63 (*d, J =* 11.9 Hz, 1H), 4.60 - 4.49 (m, 3H), 4.42 - 4.40 (m, 1H), 4.38 - 4.31 (m, 4H), 4.27 - 4.23 (m, 1H), 4.22 - 4.16 (m, 2H), 3.92 - 3.80 (m, 2H), 3.41 (s, 3H), 3.40 (s, 3H); ³¹P NMR (202 MHz, D₂O) δ - 0.90 (s, 1P), -11.58 - -11.74 (m, 2P), - 22.99 - -23.17 (m, 1P). |
| 42 | 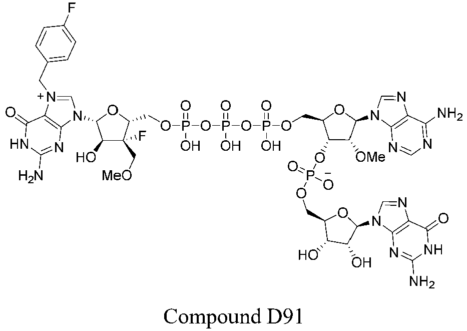 | 1284.03[M-1]⁻. ¹H NMR (500 MHz, D₂O) δ 8.35 (s, 1H), 8.04 (s, 1H), 7.93 (s, 1H), 7.21 - 7.18 (m, 2H), 6.91 - 6.88 (m, 2H), 5.92 (d, *J =* 6.2 Hz, 1H), 5.82 (s, 1H), 5.78 (d, *J =* 5.7 Hz, 1H), 5.41 - 5.35 (m, 2H), 4.96 (m, 1H), 4.75 - 4.74 (m, 1H), 4.63 - 4.56 (m, 2H), 4.52 (m, 1H), 4.49 - 4.47 (m, 1H), 4.42 - 4.37 (m, 3H), 4.34 - 4.32 (m, 2H), 4.27 - 4.17 (m, 3H), 3.89 - 3.80 (m, 2H), 3.39 (s, 6H); ³¹P NMR (202 MHz, D₂O) δ -0.85 (s, 1P), -11.57 - - 11.66 (m, 2P), -22.92 - -23.11 (m, 1P). |
| 43 | 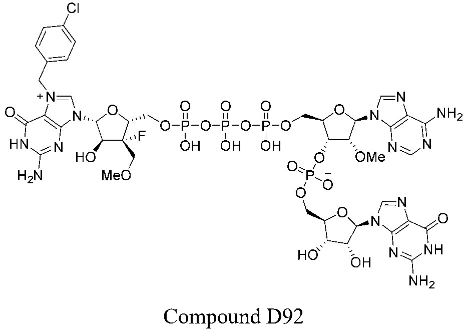 | 1300.02[M-1]⁻. ¹H NMR (500 MHz, D₂O) δ 8.36 (s, 1H), 8.07 (s, 1H), 7.93 (s, 1H), 7.12 (s, 4H), 5.92 (d, *J =* 6.3 Hz, 1H), 5.84 (s, 1H), 5.78 (d, *J = 5.8* Hz, 1H), 5.44 - 5.37 (m, 2H), 4.96 (m, 1H), 4.75 - 4.74 (m, 1H), 4.64 - 4.57 (m, 2H), 4.53 (s, 1H), 4.49 - 4.47 (m, 1H), 4.43 - 4.39 (m, 3H), 4.34 - 4.31 (m, 2H), 4.27 - 4.16 (m, 3H), 3.90 - 3.82 (m, 2H), 3.40 (s, 3H), 3.39 (s, 3H); ³¹P NMR (202 MHz, D₂O) δ - 0.84 (s, 1P), -11.63 (m, 2P), -23.04 (m, 1P). |
| 44 | 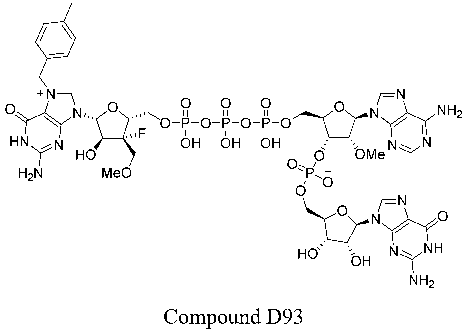 | 1280.06[M-1]⁻. ¹H NMR (500 MHz, D₂O) δ 9.19 (s, 1H), 8.47 (s, 1H), 8.17 (s, 1H), 7.95 (s, 1H), 6.99 - 6.91 (m, 4H), 5.96 (d, *J =* 5.6 Hz, 1H), 5.84 (s, 1H), 5.75 (d, *J =* 5.7 Hz, 1H), 5.37 - 5.30 (m, 2H), 4.96 - 4.95 (m, 1H), 4.79 - 4.76 (m, 1H), 4.64 - 4.56 (m, 2H), 4.51 (s, 1H), 4.47 - 4.45 (m, 1H), 4.44 - 4.38 (m, 3H), 4.36 - 4.32 (m, 2H), 4.26 - 4.14 (m, 3H), 3.89 - 3.81 (m, 2H), 3.41 (s, 3H), 3.38 (s, 3H), 2.09 (s, 3H); ³¹P NMR (202 MHz, D₂O) δ -0.90 (s, 1P), -11.53 - -11.76 (m, 2P), -22.88 - -22.98 (m, 1P). |
| 45 | 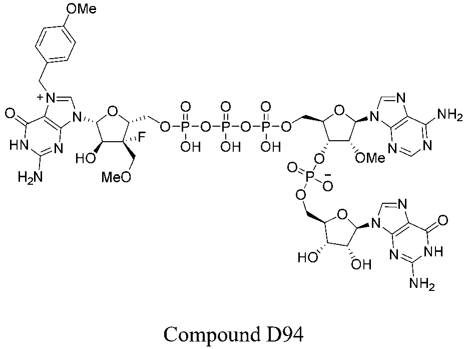 | 1296.05[M-1]⁻. ¹H NMR (500 MHz, D₂O) δ 8.36 (s, 1H), 8.07 (s, 1H), 7.94 (s, 1H), 7.08 (d, *J =* 8.5 Hz, 2H), 6.65 (d, *J =* 8.5 Hz, 2H), 5.93 (d, *J =* 6.2 Hz, 1H), 5.82 (s, 1H), 5.78 (d, *J =* 5.5 Hz, 1H), 5.35 - 5.28 (m, 2H), 4.97 (m, 1H), 4.76 - 4.74 (m, 1H), 4.64 - 4.57 (m, 2H), 4.54 (s, 1H), 4.49 - 4.47 (m, 1H), 4.42 - 4.34 (m, 5H), 4.28 - 4.16 (m, 3H), 3.90 - 3.81 (m, 2H), 3.65 (s, 3H), 3.39 (s, 6H); ³¹P NMR (202 MHz, D₂O) δ -0.85 (s, 1P), -11.63 (m, 2P), - 22.03 (m, 1P). |

### Example 46-Example 49 Synthesis of compounds

Example 46 was prepared by referring to the procedure of Example 35, and Examples 47-49 were prepared by referring to the procedure of Example 32, with the difference that, referring to the synthesis route of Scheme D, compound D3 was replaced by different dinucleotide diphosphate fragments to obtain

### Examples 46-49.

| Example | Structure | Characterization data |
|---|---|---|
| 46 | 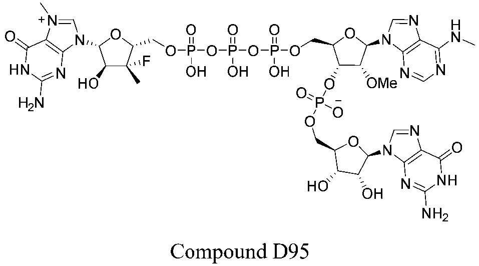 | 1174.04[M-1]⁻. ¹H NMR (500 MHz, D₂O) δ 8.30 (s, 1H), 8.01 (s, 1H), 7.91 (s, 1H), 5.96 (d, *J* = 5.8 Hz, 1H), 5.81 (d, *J =* 5.7 Hz, 1H), 5.68 (s, 1H), 4.92 - 4.89 (m, 1H), 4.74 - 4.72 (m, 1H), 4.52 - 4.51 (m, 1H), 4.47 (t, *J =* 4.5 Hz, 1H), 4.45 - 4.33 (m, 6H), 4.30 (d, *J* = 12.2 Hz, 1H), 4.27 - 4.16 (m, 3H), 3.94 (s, 3H), 3.42 (s, 3H), 3.04 (s, 3H), 1.51 (d, *J =* 23.4 Hz, 3H); ³¹P NMR (202 MHz, D₂O) δ -0.86 (s, 1P), -11.44 - -11.63 (m, 2P), -22.93 - -23.12 (t, *J* = 18.4 Hz, 1P). |
| 47 | 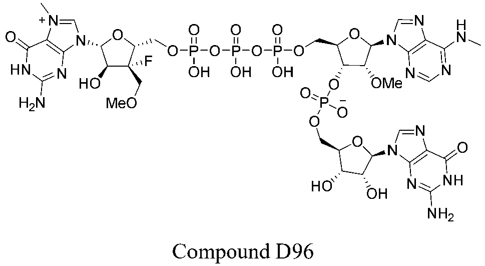 | 1204.02[M-1]⁻. ¹H NMR (500 MHz, D₂O) δ 8.81 (s, 1H), 8.38 (s, 1H), 8.12 (s, 1H), 7.94 (s, 1H), 6.01 (d*, J =* 5.7 Hz, 1H), 5.81 - 5.80 (m, 2H), 4.93 - 4.91 (m, 1H), 4.76 - 4.74 (m, 1H), 4.61 (d*, J =* 11.8 Hz, 1H), 4.59 - 4.52 (m, 2H), 4.47 (t, *J* = 4.2 Hz, 1H), 4.41 (t*, J =* 5.0 Hz, 1H), 4.38 - 4.33 (m, 4H), 4.25 - 4.16 (m, 3H), 3.97 (s, 3H), 3.91 - 3.79 (m, 2H), 3.43 (s, 3H), 3.40 (s, 3H), 3.09 (br, 3H); ³¹P NMR (202 MHz, D₂O) δ -0.91 (s, 1P), -11.54 - - 11.74 (m, 2P), -22.91 - -23.09 (m, 1P). |
| 48 | 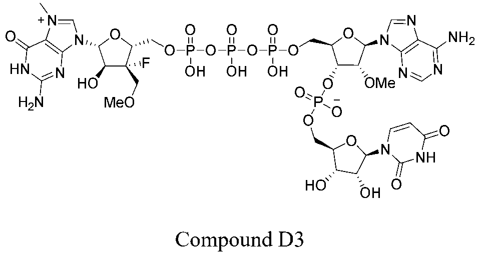 | 1151.04[M-1]⁻. ¹H NMR (500 MHz, D₂O) δ 8.81 (s, 1H), 8.44 (s, 1H), 8.12 (s, 1H), 7.83 (d, *J* = 8.1 Hz, 1H), 6.02 (d, *J =* 5.9 Hz, 1H), 5.88 (d, *J =* 4.6 Hz, 1H), 5.78 - 5.77 (m, 2H), 4.91 (m, 1H), 4.76 - 4.75 (m, 1H), 4.58 - 4.49 (m, 3H), 4.43 - 4.41 (m, 1H), 4.33 - 4.29 (m, 3H), 4.28 - 4.20 (m, 4H), 4.13 - 4.11 (m, 1H), 3.96 (s, 3H), 3.88 - 3.76 (m, 2H), 3.43 (s, 3H), 3.37 (s, 3H); ³¹P NMR (202 MHz, D₂O) δ - 1.07 (s, 1P), -11.61 (m, 2P), -22.92 (m, 1P). |
| 49 | 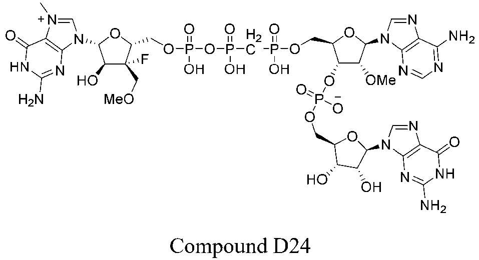 | 1188.02[M-1]⁻. ¹H NMR (500 MHz, D₂O) δ 9.01 (s, 1H), 8.51 (s, 1H), 8.17 (s, 1H), 7.95 (s, 1H), 6.03 (d*, J = 5.5* Hz, 1H), 5.85 (s, 1H), 5.80 (d, *J =* 5.7 Hz, 1H), 4.93 - 4.90 (m, 1H), 4.76 - 4.74 (m, 1H), 4.65 (d, *J =* 11.9 Hz, 1H), 4.57 (dt, *J =* 26.5, 5.2 Hz, 1H), 4.49 - 4.47 (m, 2H), 4.44 - 4.42 (m, 1H), 4.36 - 4.33 (m, 3H), 4.24 - 4.16 (m, 4H), 3.98 (s, 3H), 3.88 - 3.80 (m, 2H), 3.43 (s, 3H), 3.39 (s, 3H), 2.51 - 2.35 (m, 2H); ³¹P NMR (202 MHz, D₂O) δ 16.78 (d, *J =* 7.7 Hz, 1P), 7.61 - 7.44 (m, 1P), -0.92 (s, 1P), -11.25 (d, *J =* 26.8 Hz, 1P). |

### Example 50 Synthesis of compound D23

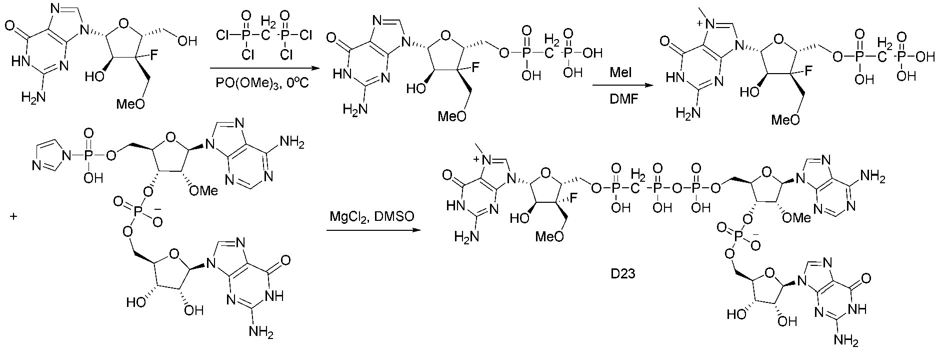

According to the synthetic route described above and referring to the method in patent CN115260264A, the ammonium salt of compound D23 was synthesized. The characterization data were as follows: 1188.02 [M-1]⁻. ¹H NMR (500 MHz, D₂O) δ 9.06 (s, 1H), 8.46 (s, 1H), 8.17 (s, 1H), 7.93 (s, 1H), 6.04 (d, *J = 5.2* Hz, 1H), 5.83 (s, 1H), 5.78 *(d, J =* 5.8 Hz, 1H), 4.91 - 4.87 (m, 1H), 4.76 - 4.75 (m, 1H), 4.66 (d, *J =* 12.3 Hz, 1H), 4.55 (dt, *J =* 25.5, 5.5 Hz, 1H), 4.49 - 4.46 (m, 2H), 4.38 - 4.35 (m, 2H), 4.32 - 4.30 (m, 3H), 4.21 - 4.15 (m, 3H), 3.96 (s, 3H), 3.88 - 3.80 (m, 2H), 3.43 (s, 3H), 3.39 (s, 3H), 2.53 - 2.37 (m, 2H); ³¹P NMR (202 MHz, D₂O) δ 17.39 (d, *J =* 8.3 Hz, 1P), 7.62 - 7.45 (m, 1P), -0.90 (s, 1P), -11.42 (d, *J =* 25.1 Hz, 1P).

### Example 51 Synthesis of compound D28

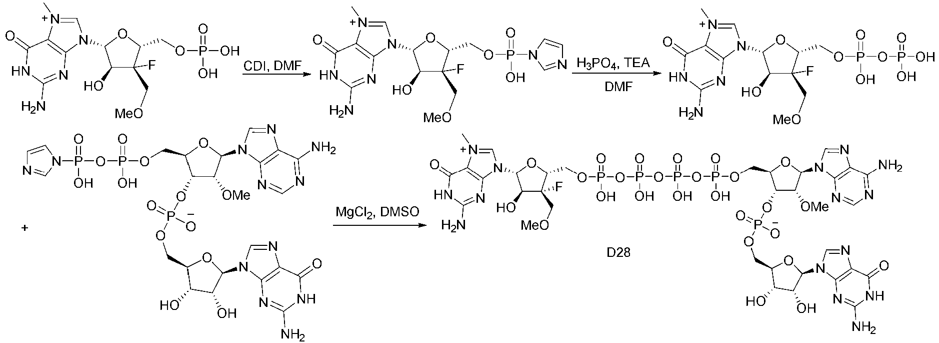

According to the above synthetic route and referring to the method in patent CN115260264A, the ammonium salt of compound D28 was synthesized. The characterization data are: 1270.06 [M-1]⁻. ¹H NMR (500 MHz, D₂O) δ 8.41 (s, 1H), 8.10 (s, 1H), 7.90 (s, 1H), 5.95 (d, *J =* 6.1 Hz, 1H), 5.87 (s, 1H), 5.79 (d, *J =* 5.8 Hz, 1H), 4.92 - 4.89 (m, 1H), 4.83 - 4.82 (m, 1H), 4.66 - 4.58 (m, 2H), 4.50 - 4.47 (m, 2H), 4.41 - 4.36 (m, 3H), 4.32 - 4.28 (m, 2H), 4.23 - 4.14 (m, 3H), 3.98 (s, 3H), 3.90 - 3.82 (m, 2H), 3.39 (s, 3H), 3.36 (s, 3H); ³¹P NMR (202 MHz, D₂O) δ -0.94 (s, 1P), -11.58 - -11.74 (m, 2P), -22.96 - -23.05 (m, 2P).

### Example 52 Synthesis of compound D97

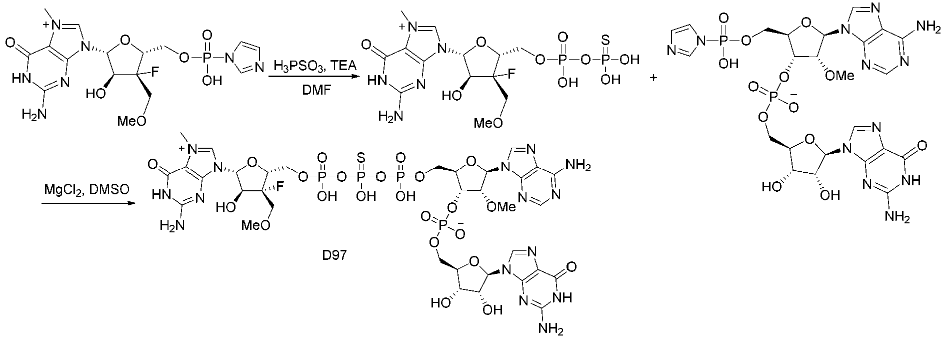

According to the above synthetic route and referring to the method in patent CN115260264A, the ammonium salt of compound D97 was synthesized. The characterization data are: 1206.03 [M-1]⁻. ¹H NMR (500 MHz, D₂O) δ 8.84 (s, 1H), 8.49 - 8.44 (m, 1H), 8.11 - 8.10 (m, 1H), 7.91 (s, 1H), 6.00 - 5.97 (m, 1H), 5.80 - 5.78 (m, 2H), 4.93 (m, 1H), 4.75 - 4.74 (m, 1H), 4.62 - 4.56 (m, 2H), 4.50 (s, 1H), 4.44 - 4.42 (m, 2H), 4.34 - 4.29 (m, 4H), 4.23 - 4.15 (m, 3H), 3.96 (s, 3H), 3.85 - 3.78 (m, 2H), 3.37 - 3.34 (m, 6H); ³¹P NMR (202 MHz, D₂O) δ 30.00 (m, 1P), -0.97 (m, 1P), -12.54 (m, 2P).

### Example 53 Detection of the efficiency of mRNA capping synthesis

The assay was performed according to the method described in Example 20. The assay results of some compounds are shown in Table 4.

**Table 4. Final product mass (µg) obtained from the final product of 20 µL mRNA synthesis reaction system**

| Example | Compound | Final product mass | Example | Compound | Final product mass |
|---|---|---|---|---|---|
| 29 | Compound D81 | 138 | 42 | Compound D91 | 113 |
| 30 | Compound D82 | 146 | 43 | Compound D92 | 109 |
| 31 | Compound D31 | 133 | 44 | Compound D93 | 105 |
| 32 | Compound D1 | 104 | 45 | Compound D94 | 116 |
| 33 | Compound D83 | 106 | 46 | Compound D95 | 111 |
| 34 | Compound D84 | 102 | 47 | Compound D96 | 94 |
| 35 | Compound D85 | 120 | 49 | Compound D24 | 83 |
| 36 | Compound D20 | 113 | 50 | Compound D23 | 96 |
| 37 | Compound D86 | 111 | 51 | Compound D28 | 92 |
| 38 | Compound D87 | 98 | 52 | Compound D97 | 91 |
| 39 | Compound D88 | 114 | | N-7413 | 122 |
| 40 | Compound D89 | 110 | | Negative control | 118 |
| 41 | Compound D90 | 100 | | Blank control | 0 |

As can be seen from Table 4, all test groups can obtain mRNA via normal transcription, with favorable yields.

### Example 54 Evaluation of expression efficiency of different capped luciferase mRNAs in HEK293T cells

The assay was performed according to the method described in Example 21. All test compounds, N-7413, negative control and blank control were tested under the same conditions, and some experimental results in the test results are shown in Table 5.

**Table 5 Relative fluorescence readings for capped mRNAs**

| Example | Compound | Relative fluorescence reading | Example | Compound | Relative fluorescence reading |
|---|---|---|---|---|---|
| 29 | Compound D81 | 0.51 | 42 | Compound D91 | 1.93 |
| 30 | Compound D82 | 1.63 | 43 | Compound D92 | 1.78 |
| 31 | Compound D31 | 1.73 | 44 | Compound D93 | 1.95 |
| 32 | Compound D1 | 2.07 | 45 | Compound D94 | 1.85 |
| 33 | Compound D83 | 1.48 | 46 | Compound D95 | 0.83 |
| 34 | Compound D84 | 1.04 | 47 | Compound D96 | 2.96 |
| 35 | Compound D85 | 1.6 | 49 | Compound D24 | 1.08 |
| 36 | Compound D20 | 1.23 | 50 | Compound D23 | 0.93 |
| 37 | Compound D86 | 1.35 | 51 | Compound D28 | 1.12 |
| 38 | Compound D87 | 1.55 | 52 | Compound D97 | 2.05 |
| 39 | Compound D88 | 2.04 | | N-7413 | 1.00 |
| 40 | Compound D89 | 1.52 | | Negative control | 0 |
| 41 | Compound D90 | 1.44 | | Blank control | 0 |

| | | | | | |
|---|---|---|---|---|---|
| Note: The fluorescence reading of N-7413 is calibrated as 1, and the ratio of the fluorescence reading of other capped mRNA transfected to the fluorescence reading of N-7413 is the relative fluorescence reading. | | | | | |

The relative fluorescence reading results (6 h) after transfection of capped mRNA into 293T cells are shown in Table 5. It can be seen from the table that the mRNA capped by the compound of the present disclosure has a higher expression level, and some compounds can enable higher expression efficiency of mRNA compared to N-7413.

### Example 55 PK test of capped Gluc mRNA-LNP in SD rats

### 1) Preparation of mRNA

The capped Gluc mRNA was prepared according to the method described in Example 20. Different compounds were used as cap analogs for preparation. When compound X was used, the corresponding target product was denoted as X-Gluc mRNA.

### 2) Encapsulation of mRNA

The encapsulation was performed according to the method described in Example 24 to obtain LNPs encapsulating mRNA.

When the cap analog was compound X, the prepared mRNA was denoted as X-Gluc-mRNA; and the encapsulated LNPs were denoted as X-Gluc-mRNA-LNP. Taking compound D1 as an example, the prepared mRNA was denoted as D 1-Gluc-mRNA; and the encapsulated LNPs were denoted as D1-Gluc-mRNA-LNP.

Gluc mRNA prepared using LZCap^{®}(3'Acm) AG as the cap analog served as the control group. The structure of LZCap^{®}AG(3'Acm) was as follows:

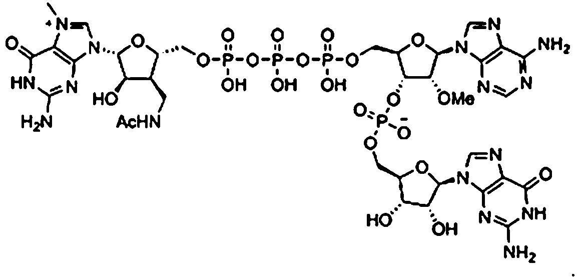

### 3) In vivo testing in rats

Male SD rats were divided into groups with 3 rats per group. The prepared capped X-Gluc-mRNA-LNP was administered intravenously at a dose of 0.05 mg/kg (mRNA). Blood samples were collected at 0, 1, 2, 4, 6, 8, 10, 24, 32, and 48 h (0.3 mL per time point), anticoagulated with K₂EDTA/heparin sodium, and placed in an ice bath. The plasma was centrifuged within 30 min and stored at -70°C. The fluorescence intensity was measured using a Gaussia luciferase assay kit, and the AUC was calculated to obtain the experimental detection data.

The experimental detection data for some compounds are shown in Table 6.

**Table 6 PK test data of rats in some test groups**

| Test group | Cap analog | Relative AUC | Test group | Cap analog | Relative AUC |
|---|---|---|---|---|---|
| D1-Gluc-mRNA-LNP | Compound D1 | 1.62 | D93-Gluc-mRNA-LNP | Compound D93 | 1.82 |
| D88-Gluc-mRNA-LNP | Compound D88 | 1.98 | D94-Gluc-mRNA-LNP | Compound D94 | 1.30 |
| D91-Gluc-mRNA-LNP | Compound D91 | 1.77 | Control group | LZCap^{®}AG(3'Acm) | 1.00 |
| D92-Gluc-mRNA-LNP | Compound D92 | 1.58 | | | |

As shown in Table 6, the samples of the test group in the present disclosure can express the target protein well in rats, and the cumulative expression levels at 48 h for most mRNA samples prepared using the compounds as cap analogs are higher than those of the control group, indicating that the compounds provided by the present disclosure as cap analogs are beneficial to increasing the expression level of mRNA in rats.

The technical features of the above examples can be combined arbitrarily. To simplify the description, not all possible combinations of the technical features in the above examples are described. However, as long as there is no contradiction in the combination of these technical features, they should be considered to be within the scope of the present specification.

The above examples only express several embodiments of the present disclosure, and their descriptions are relatively specific and detailed, but they should not be understood as limiting the scope of the present disclosure. It should be noted that, for those of ordinary skill in the art, several modifications and improvements can be made without departing from the concept of the present disclosure, and these all belong to the protection scope of the present disclosure. Therefore, the scope of protection of the patent of the present disclosure should be determined by the appended claims.

## Claims

1. A compound having a structure represented by formula I, or a stereoisomer, pharmaceutically acceptable salt or solvate thereof: 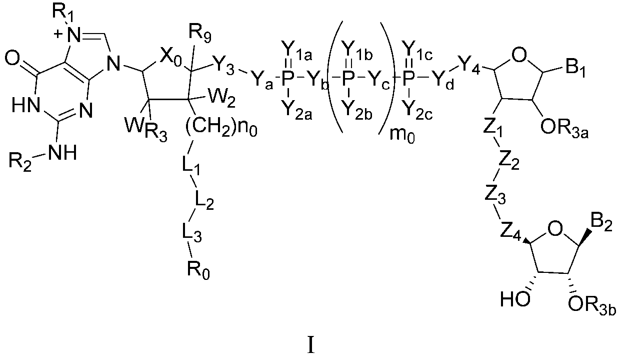 wherein
R₁ is C₁-C₆ alkyl, C₂-C₆ alkenyl, C₂-C₆ alkynyl, C₃-C₆ cycloalkyl, C₃-C₆ cycloalkenyl, benzyl, R₅-substituted C₁-C₆ alkyl, R₅-substituted C₂-C₆ alkenyl, R₅-substituted C₂-C₆ alkynyl, R₅-substituted C₃-C₆ cycloalkyl, R₅-substituted C₃-C₆ cycloalkenyl or R₅-substituted benzyl;
R₂ is H, C₁-C₆ alkyl, C₂-C₆ alkenyl, C₂-C₆ alkynyl, C₃-C₈ cycloalkyl, cycloalkenyl, benzyl, aryl, heteroaryl, R₅-substituted benzyl, R₅-substituted aryl, carbonylalkyl, carbonylalkoxy or sulfonamido;
R₃ is H, OH, C₁-C₆ alkyl, C₂-C₆ alkenyl, C₂-C₆ alkynyl, R₅-substituted C₁-C₆ alkyl, R₅-substituted C₂-C₆ alkenyl, R₅-substituted C₂-C₆ alkynyl, halogen or absent;
R₃ₐ and R_{3b} are each independently H, halogen, C₁-C₆ alkyl, C₂-C₆ alkenyl, C₂-C₆ alkynyl, R₅-substituted C₁-C₆ alkyl, R₅-substituted C₂-C₆ alkenyl or R₅-substituted C₂-C₆ alkynyl;
R₉ is H, halogen, C₁-C₃ alkyl or R₄-substituted C₁-C₃ alkyl;
R₀ is H, a fluorophore or a steroid structural group;
X₀ is O, S, NR₄, CH₂, CF₂, CHF, CCH₂ or CCF₂;
W is H, OH, OR₄, NR₄R₄, NR₄COR₄, F, Cl, N₃ or CN;
W₂ is H, OH, halogen, N₃, CN, OR₄, NR₄R₄, NR₄COR₄ or C₁-C₄ alkyl;
L₁ is O, S, NH, carbonyl or absent;
L₂ is NH, carbonyl or absent;
L₃ is -X-(CH₂)nNH-, -X-(CH₂)nNH(CH₂)mNH-, -X-(CH₂)nNHCO(CH₂)mNH-, -X-(CH₂)nCONH(CH₂)mNH-, -X-(CH₂)n(OC₂H₄)mNH-, -X-(CH₂)n(OC₂H₄)mOCH₂-, -X-(C(O)CHRaNH)n- or absent; wherein X is NH, O, 5-membered heteroaryl, 6-membered heteroaryl or absent; n and m are each independently an integer from 0 to 10; Ra is a side chain group in an amino acid, and Ra at each occurrence may be the same or different;
Yₐ, Y_{b}, Y_{c} and Y_{d} are each independently O, S, CH₂, CCl₂, CF₂ or NH;
Y₁ₐ, Y1_{b} and Y_{1c} are each independently O or S;
Y₂ₐ, Y_{2b} and Y_{2c} are each independently OH, SH or BH₃;
Y₃ and Y₄ are each independently CH₂ or O;
Z₁ is O, OH, CH₂, S, NR₆, CO or SO₂;
Z₂ and Z₃ are each independently O, NR₆, CHR₇, CHCOOR₇, CHCONR₇R₇, S, CO, SO₂, PO(OH), PO(SH), P(O)VCO₂H or absent; Z₂ may form a ring with an oxygen atom connected to R3a;
Z₄ is O, CH₂, S, NR₆, CO, SO₂ or absent;
B₁ and B₂ are each independently a natural or modified pyrimidine nucleotide base or a natural or modified purine nucleotide base;
R₄ is H, C₁-C₈ alkyl, C₂-C₈ alkenyl or C₂-C₈ alkynyl;
R₅ is halogen, CN, SO₂, NO₂, D, N₃, C₁-C₈ alkyl, C₂-C₈ alkenyl, C₂-C₈ alkynyl, C₁-C₈ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₆ alkyl, COR₈ or SO₂R₈;
R₇ is H, halogen, C₁-C₈ alkyl, C₂-C₈ alkenyl or C₂-C₈ alkynyl;
R₈ is H, C₁-C₈ alkyl, C₂-C₈ alkenyl or C₂-C₈ alkynyl;
V is C₁-C₄ alkyl;
n₀ is an integer from 0 to 6;
m₀ is 0, 1 or 2.

2. A compound having a structure represented by formula II, or a stereoisomer, pharmaceutically acceptable salt or solvate thereof: 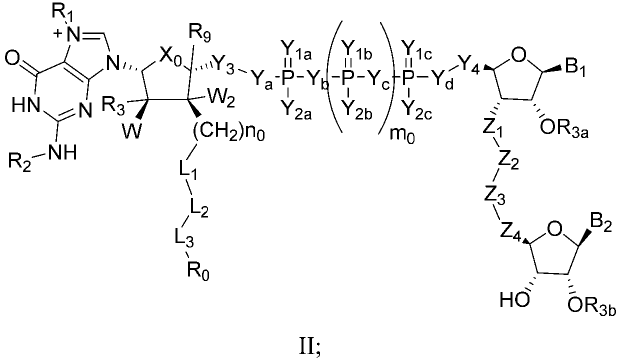 wherein R₁, R₂, R₃, R₃ₐ, R_{3b}, R₄, R₅, R₆, R₇, R₈, R₉, R₀, Ra, L₁, L₂, L₃, X, X₀, W, W₂, Yₐ, Y_{b}, Y_{c}, Y_{d}, Y₁ₐ, Y_{1b}, Y_{1c}, Y₂ₐ, Y_{2b}, Y_{2c}, Y₃, Y₄, B₁, B₂, Z₁, Z₂, Z₃, Z₄, V, n₀, m₀, n, and m are as defined in claim 1, respectively.

3. The compound according to claim 1 or 2, or a stereoisomer, pharmaceutically acceptable salt or solvate thereof, wherein when R₀ is a fluorophore, the fluorophore is a group formed by any one of the following dyes: biotin, Cy3, Cy5, Cy7, Cy5.5, DY-776, DY-751, DY-647P1, AF647, AF555, 5-FAM, 6-FAM, 6-TET, 5-SIMA, 6-JCE, ATTO 700, ATTO 680, ATTO 655, Texas Red, DEAC, AMCA, ANT, MANT, DY-480XL, DY-485XL, ATTO 425, ATTO 390, ATTO 465, ATTO495, BDP-FL, ATTO 647N, ATTO 633, ATTO Rho14, ATTO Rho13, ATTO Rho12, ATTO Rho11, ATTO Thio12, ATTO 620, ATTO Rho101, ATTO 550, ATTO Rho6G, ATTO Rho13, ATTO 532, 5/6-TARMA, 6-ROX, ATTO 565, ATTO 590, AF594, 5/6-RHOX, AF488 or AF546;
or; when R₀ is a steroid structural group, the steroid structural group is a group formed by any one of the following steroid structures: cholic acid, deoxycholic acid, ursodeoxycholic acid, chenodeoxycholic acid, hyodeoxycholic acid or glycoursodeoxycholic acid.

4. The compound according to claim 3, or a stereoisomer, pharmaceutically acceptable salt or solvate thereof, wherein when L₁ is absent, L₂ is NH, carbonyl or absent; or, when L₁ is NH, L₂ is carbonyl or absent; or, when L₁ is carbonyl, L₂ is NH or absent.

5. The compound according to claim 4, or a stereoisomer, pharmaceutically acceptable salt or solvate thereof, wherein X is NH, O, 5-membered nitrogen-containing aryl, 6-membered nitrogen-containing aryl or absent; more preferably, X is NH, O, 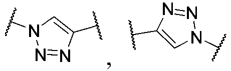 or absent.

6. The compound according to claim 5, or a stereoisomer, pharmaceutically acceptable salt or solvate thereof, wherein L₃ is -(CH₂)nNH-, -(CH₂)nNH(CH₂)mNH-, - (CH₂)nNHCO(CH₂)mNH-, -(CH₂)nCONH(CH₂)mNH-, -(CH₂)n(OC₂H₄)mNH-, -O-(CH₂)nNH-, -O-(CH₂)nNHCO(CH₂)mNH-, -O-(CH₂)nCONH(CH₂)mNH-, -O-(CH₂)n(OC₂H₄)mNH-, -NH-(CH₂)nNH-, -NH-(CH₂)nNHCO(CH₂)mNH-, -NH-(CH₂)nCONH(CH₂)mNH-, -NH-(CH₂)n(OC₂H₄)mNH-, 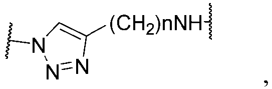 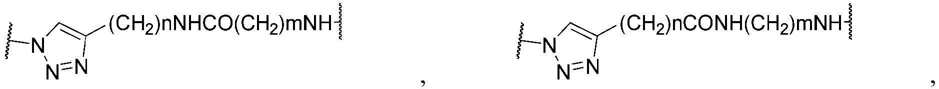 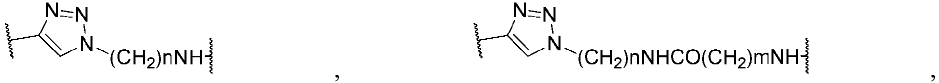 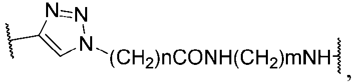 -(CH₂)n(OC₂H₄)mOCH₂-, -O-(CH₂)n(OC₂H₄)mOCH₂-, - NH-(CH₂)n(OC₂H₄)mOCH₂-, -(C(O)CHRaNH)n- or absent; wherein Ra is H, CH₃, CH(CH₃)₂, CH₂CH(CH₃)₂, CH(CH₃)CH₂CH₃, CH₂C₆H₅, CH₂C₈NH₆, CH₂C₆H₄OH, CH₂COOH, CH₂CONH₂, (CH₂)₂COOH, (CH₂)₄NH₂, (CH₂)₂CONH₂, (CH₂)₂SCH₃, CH₂OH, CH(CH₃)OH, CH₂SH, C₃H₆, CH₂C₃H₃N₂ or (CH₂)₃NHC(NH)NH₂; Ra at each occurrence may be the same or different;
wherein n and m are each independently 0, 1, 2, 3, 4, 5, 6, 7, 8, 9 or 10.

7. The compound according to claim 6, or a stereoisomer, pharmaceutically acceptable salt or solvate thereof, wherein -L₁-L₂-L₃- group is any one of the following groups: - (CH₂)n(OC₂H₄)mOCH₂-, -NHCO(CH₂)n(OC₂H₄)mOCH₂-, -CONH(CH₂)n(OC₂H₄)mOCH₂-, -(CH₂)nNH-, -(CH₂)nNH(CH₂)mNH-, -(CH₂)nCONH(CH₂)mNH-, - (CH₂)nNHCO(CH₂)mNH-, -NHCO(CH₂)nNHCO(CH₂)mNH-, -NHCONH(CH₂)nNH-, - NHCOO(CH₂)nNH-, -(CH₂)n(OC₂H₄)mNH-, -NHCO(CH₂)n(OC₂H₄)mNH-, - NHCONH(CH₂)n(OC₂H₄)mNH-, -CONH(CH₂)n(OC₂H₄)mNH-, 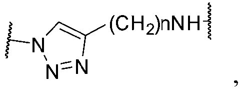 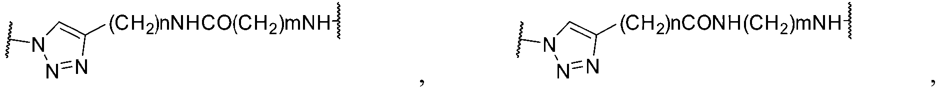 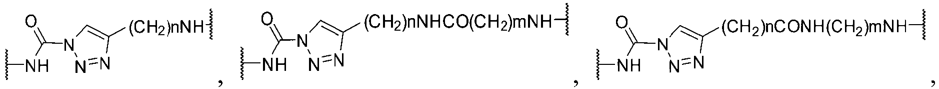 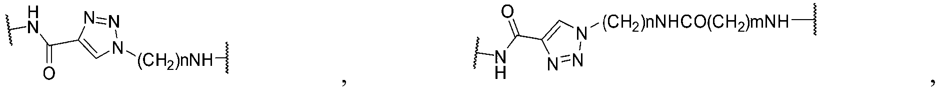 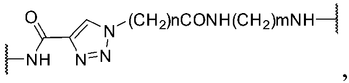 -NH-(C(O)CHRaNH)n- or absent;
wherein n and m are each independently 0, 1, 2, 3, 4, 5, 6, 7, 8, 9 or 10; and n and m at each occurrence may be the same or different.

8. The compound according to claim 7, or a stereoisomer, pharmaceutically acceptable salt or solvate thereof, wherein when R₀ is H, the -L₁-L₂-L₃- group is -(CH₂)n(OC₂H₄)mOCH₂-, -NHCO(CH₂)n(OC₂H₄)mOCH₂- or -CONH(CH₂)n(OC₂H₄)mOCH₂-;
wherein n and m are each independently 0, 1, 2, 3, 4, 5, 6, 7, 8, 9 or 10; and n and m at each occurrence may be the same or different; n₀ is 0, 1, 2, 3 or 4;
when R₀ is a fluorophore or a steroid structural group, the -L₁-L₂-L₃- group is any one of the following groups: -(CH₂)nNH-, -(CH₂)nNH(CH₂)mNH-, -(CH₂)nCONH(CH₂)mNH-, - (CH₂)nNHCO(CH₂)mNH-, -NHCO(CH₂)nNHCO(CH₂)mNH-, -NHCONH(CH₂)nNH-, - NHCOO(CH₂)nNH-, -(CH₂)n(OC₂H₄)mNH-, -NHCO(CH₂)n(OC₂H₄)mNH-, - NHCONH(CH₂)n(OC₂H₄)mNH-, -CONH(CH₂)n(OC₂H₄)mNH-, 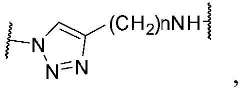 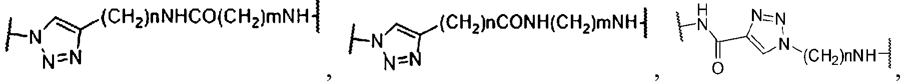 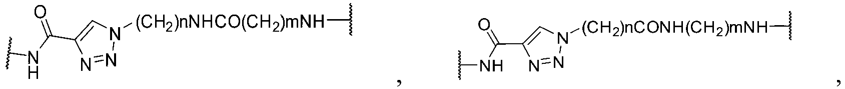 -NH-(C(O)CHRaNH)n- or absent;
wherein n and m are each independently 0, 1, 2, 3, 4, 5, 6, 7, 8, 9 or 10; and n and m at each occurrence may be the same or different; n₀ is 0, 1, 2, 3 or 4.

9. The compound according to claim 6, or a stereoisomer, pharmaceutically acceptable salt or solvate thereof, wherein R₄ is H, C₁-C₄ alkyl, C₂-C₄ alkenyl or C₂-C₄ alkynyl;
R₅ is halogen, CN, SO₂, NO₂, D, N₃, C₁-C₄ alkyl, C₂-C₄ alkenyl, C₂-C₄ alkynyl, C₁-C₄ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₄ alkyl, COR₈ or SO₂R₈;
R₇ is H, halogen, C₁-C₄ alkyl, C₂-C₄ alkenyl or C₂-C₄ alkynyl;
R₈ is H, halogen, C₁-C₄ alkyl, C₂-C alkenyl or C₂-C₅ alkynyl;
R₉ is H, halogen or C₁-C₃ alkyl.

10. The compound according to claim 9, or a stereoisomer, pharmaceutically acceptable salt or solvate thereof, wherein Z₁ is O, CH₂, S or NH;
Z₂ and Z₃ are each independently O, NH, CHR₇, CHCOOR₇, CHCONR₇R₇, S, CO, SO₂, PO(OH), PO(SH) or absent;
Z₄ is O, CH₂, S, NH or absent;
B₁ and B₂ are each independently a natural or modified cytosine nucleotide base, a natural or modified uracil nucleotide base, a natural or modified adenine nucleotide base or a natural or modified guanine nucleotide base.

11. The compound according to claim 10, or a stereoisomer, pharmaceutically acceptable salt or solvate thereof, wherein Z₂ is methylene, ethylene, CO, SO₂, PO(OH) or absent;
Z₃ is O, CH₂ or NH;
Z₄ is CH₂ or NH.

12. The compound according to claim 9, or a stereoisomer, pharmaceutically acceptable salt or solvate thereof, wherein Yₐ, Y_{b}, Y_{c} and Y_{d} are all O or at most one is S, CH₂, CCl₂, CF₂ or NH;
Y₁ₐ, Y_{1b} and Y_{1c} are all O or at most one is S;
Y₂ₐ, Y_{2b} and Y_{2c} are all OH or at most one is SH or BH₃;
Y₃ and Y₄ are each independently CH₂.

13. The compound according to claim 9, or a stereoisomer, pharmaceutically acceptable salt or solvate thereof, wherein R₁ is C₁-C₄ alkyl, C₂-C₄ alkenyl, C₂-C₄ alkynyl, C₃-C₆ cycloalkyl, C₃-C₆ cycloalkenyl, benzyl, C₁-C₄ haloalkyl, C₂-C₄ haloalkenyl, C₂-C₄ haloalkynyl, C₃-C₆ halocycloalkyl, C₃-C₆ halocycloalkenyl or halobenzyl;
R₂ is H, C₁-C₄ alkyl, C₂-C₄ alkenyl, C₂-C₄ alkynyl, C₃-C₆ cycloalkyl, C₃-C₆ cycloalkenyl, C₁-C₄ haloalkyl, C₂-C₄ haloalkenyl, C₂-C₄ haloalkynyl, C₃-C₆ halocycloalkyl, C₃-C₆ halocycloalkenyl, benzyl or halobenzyl;
R₃ is H, OH, C₁-C₄ alkyl, C₂-C₄ alkenyl, C₂-C₄ alkynyl, C₁-C₄ haloalkyl, C₂-C₄ haloalkenyl, C₂-C₄ haloalkynyl, halogen or absent;
R₃ₐ and R_{3b} are each independently H, C₁-C₄ alkyl, C₂-C₄ alkenyl, C₂-C₄ alkynyl, C₁-C₄ haloalkyl, C₂-C₄ haloalkenyl or C₂-C₄ haloalkynyl;
W is H, OH, OR₄, NR₄R₄, F, Cl or CN;
R₄ is H, methyl, ethyl, n-propyl, isopropyl, butyl, vinyl, propenyl, butenyl, ethynyl, propynyl or butynyl;
R₅ is halogen, CN, D, N₃, C₁-C₄ alkyl, C₂-C₄ alkenyl, C₂-C₄ alkynyl, OR₇, SR₇, NR₇R₇, COR₇, COOR₇, pyridyl, pyrimidinyl or morpholinyl;
R₇ is H, methyl, ethyl, n-propyl, isopropyl, butyl, vinyl, propenyl, butenyl, ethynyl, propynyl or butynyl.

14. The compound according to claim 13, or a stereoisomer, pharmaceutically acceptable salt or solvate thereof, wherein W is OH, F, Cl, methoxy or ethoxy;
R₁ is methyl, ethyl, n-propyl, isopropyl, fluoromethyl, difluoromethyl, trifluoromethyl, trifluoroethyl, trifluoropropyl or trifluoroisopropyl;
R₂ is H, methyl, ethyl, n-propyl, isopropyl, fluoromethyl, difluoromethyl, trifluoromethyl, trifluoroethyl, trifluoropropyl or trifluoroisopropyl.

15. The compound according to claim 2, or a stereoisomer, pharmaceutically acceptable salt or solvate thereof, wherein the compound has a structure represented by formula III: 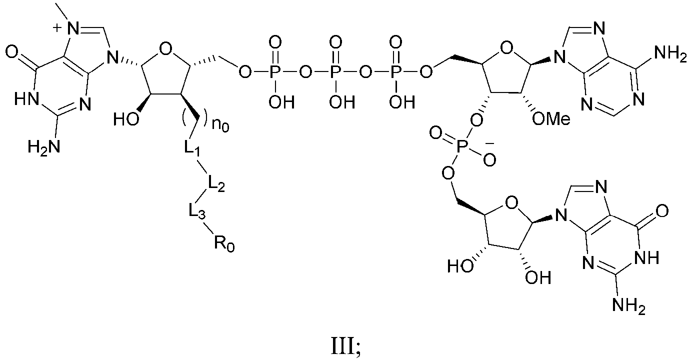
wherein L₁ is O, S, NH, carbonyl or absent;
L₂ is NH, carbonyl or absent;
L₃ is -X-(CH₂)nNH-, -X-(CH₂)nNH(CH₂)mNH-, -X-(CH₂)nNHCO(CH₂)mNH-, -X-(CH₂)nCONH(CH₂)mNH-, -X-(CH₂)n(OC₂H₄)mNH-, -X-(CH₂)n(OC₂H₄)mOCH₂-, -X-(C(O)CHRaNH)n- or absent; wherein X is NH, O, 5-membered heteroaryl, 6-membered heteroaryl or absent; n and m are each independently an integer from 0 to 10; Ra is a side chain group in an amino acid, and Ra at each occurrence may be the same or different;
R₀ is H, a fluorophore or a steroid structural group;
n₀ is an integer from 0 to 6.

16. The compound according to claim 15, or a stereoisomer, pharmaceutically acceptable salt or solvate thereof, wherein when L₁ is absent, L₂ is imino, carbonyl or absent; or, when L₁ is imino, L₂ is carbonyl or absent; or, when L₁ is carbonyl, L₂ is imino or absent.

17. The compound according to claim 16, or a stereoisomer, pharmaceutically acceptable salt or solvate thereof, wherein X is NH, O, 5-membered nitrogen-containing aryl, 6-membered nitrogen-containing aryl or absent; more preferably, X is NH, O, 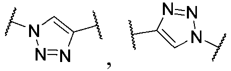 or absent.

18. The compound according to claim 17, or a stereoisomer, pharmaceutically acceptable salt or solvate thereof, wherein L₃ is -(CH₂)nNH-, -(CH₂)nNH(CH₂)mNH-, - (CH₂)nNHCO(CH₂)mNH-, -(CH₂)nCONH(CH₂)mNH-, -(CH₂)n(OC₂H₄)mNH-, -O-(CH₂)nNH-, -O-(CH₂)nNHCO(CH₂)mNH-, -O-(CH₂)nCONH(CH₂)mNH-, -O-(CH₂)n(OC₂H₄)mNH-, -NH-(CH₂)nNH-, -NH-(CH₂)nNHCO(CH₂)mNH-, -NH-(CH₂)nCONH(CH₂)mNH-, -NH-(CH₂)n(OC₂H₄)mNH-, 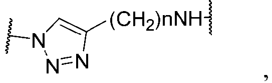 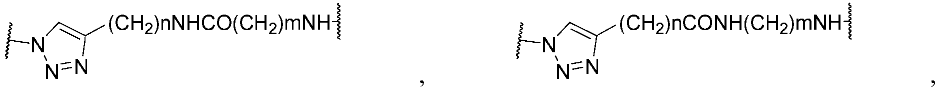 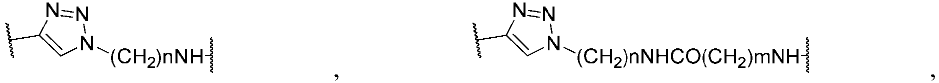 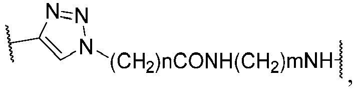 -(CH₂)n(OC₂H₄)mOCH₂-, -O-(CH₂)n(OC₂H₄)mOCH₂-, - NH-(CH₂)n(OC₂H₄)mOCH₂-, -(C(O)CHRaNH)n- or absent; wherein Ra is H, CH₃, CH(CH₃)₂, CH₂CH(CH₃)₂, CH(CH₃)CH₂CH₃, CH₂C₆H₅, CH₂C₈NH₆, CH₂C₆H₄OH, CH₂COOH, CH₂CONH₂, (CH₂)₂COOH, (CH₂)₄NH₂, (CH₂)₂CONH₂, (CH₂)₂SCH₃, CH₂OH, CH(CH₃)OH, CH₂SH, C₃H₆, CH₂C₃H₃N₂ or (CH₂)₃NHC(NH)NH₂; Ra at each occurrence may be the same or different;
wherein n and m are each independently 0, 1, 2, 3, 4, 5, 6, 7, 8, 9 or 10.

19. The compound according to claim 18, or a stereoisomer, pharmaceutically acceptable salt or solvate thereof, wherein -L₁-L₂-L₃- group is any one of the following groups: - (CH₂)n(OC₂H₄)mOCH₂-, -NHCO(CH₂)n(OC₂H₄)mOCH₂-, -CONH(CH₂)n(OC₂H₄)mOCH₂-, -(CH₂)nNH-, -(CH₂)nNH(CH₂)mNH-, -(CH₂)nCONH(CH₂)mNH-, - (CH₂)nNHCO(CH₂)mNH-, -NHCO(CH₂)nNHCO(CH₂)mNH-, -NHCONH(CH₂)nNH-, - NHCOO(CH₂)nNH-, -(CH₂)n(OC₂H₄)mNH-, -NHCO(CH₂)n(OC₂H₄)mNH-, - NHCONH(CH₂)n(OC₂H₄)mNH-, -CONH(CH₂)n(OC₂H₄)mNH-, 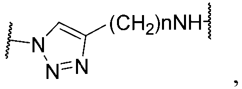 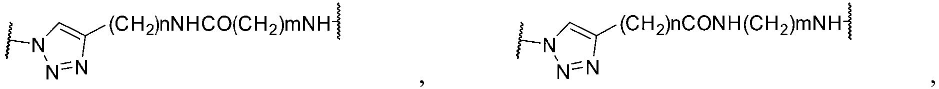 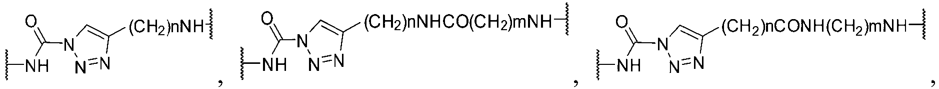 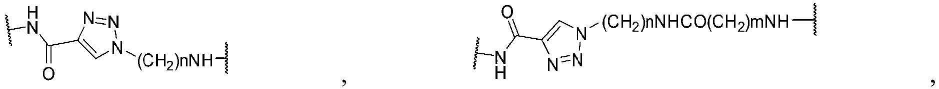 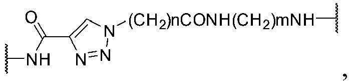 -NH-(C(O)CHRaNH)n- or absent;
wherein n and m are each independently 0, 1, 2, 3, 4, 5, 6, 7, 8, 9 or 10; and n and m at each occurrence may be the same or different.

20. The compound according to claim 19, or a stereoisomer, pharmaceutically acceptable salt or solvate thereof, wherein when R₀ is H, the -L₁-L₂-L₃- group is -(CH₂)n(OC₂H₄)mOCH₂-, -NHCO(CH₂)n(OC₂H₄)mOCH₂- or -CONH(CH₂)n(OC₂H₄)mOCH₂-;
when R₀ is a fluorophore or a steroid structural group, the -L₁-L₂-L₃- group is any one of the following groups: -(CH₂)nNH-, -(CH₂)nNH(CH₂)mNH-, -(CH₂)nCONH(CH₂)mNH-, - (CH₂)nNHCO(CH₂)mNH-, -NHCO(CH₂)nNHCO(CH₂)mNH-, -NHCONH(CH₂)nNH-, - NHCOO(CH₂)nNH-, -(CH₂)n(OC₂H₄)mNH-, -NHCO(CH₂)n(OC₂H₄)mNH-, - NHCONH(CH₂)n(OC₂H₄)mNH-, -CONH(CH₂)n(OC₂H₄)mNH-, 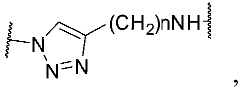 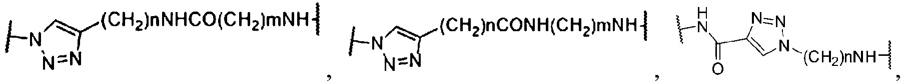 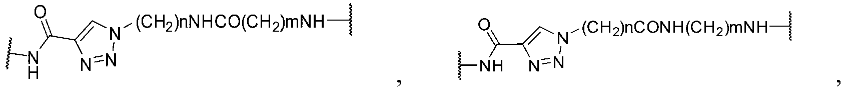 -NH-(C(O)CHRaNH)n- or absent;
wherein n and m are each independently 0, 1, 2, 3, 4, 5, 6, 7, 8, 9 or 10; and n and m at each occurrence may be the same or different; n₀ is 0, 1, 2, 3 or 4.

21. The compound according to claim 20, or a stereoisomer, pharmaceutically acceptable salt or solvate thereof, wherein the compound is represented by any one of the following structures, wherein R₀ is the fluorophore or steroid structural group defined in claim 3:

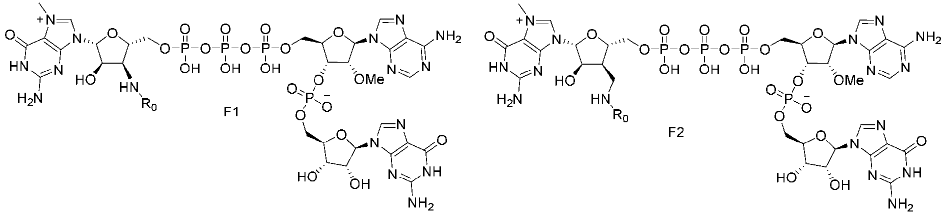

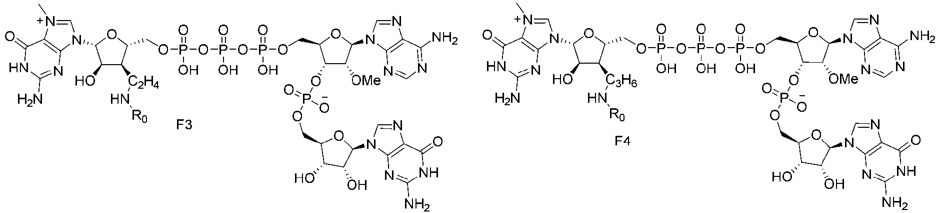

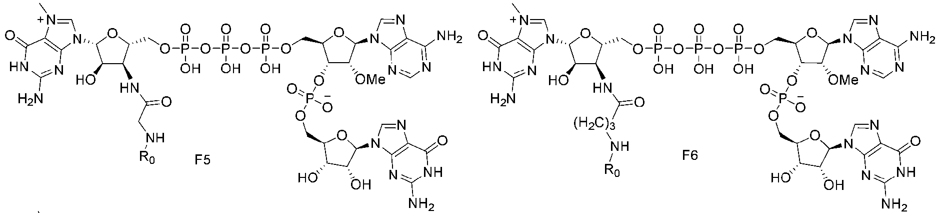

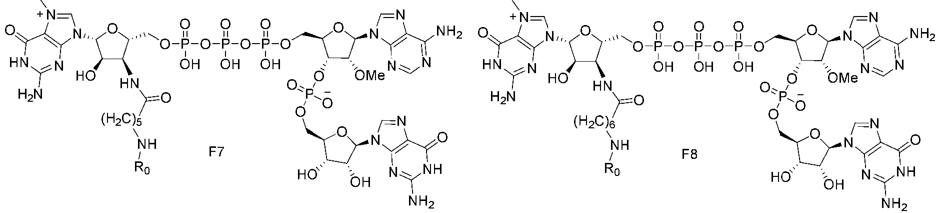

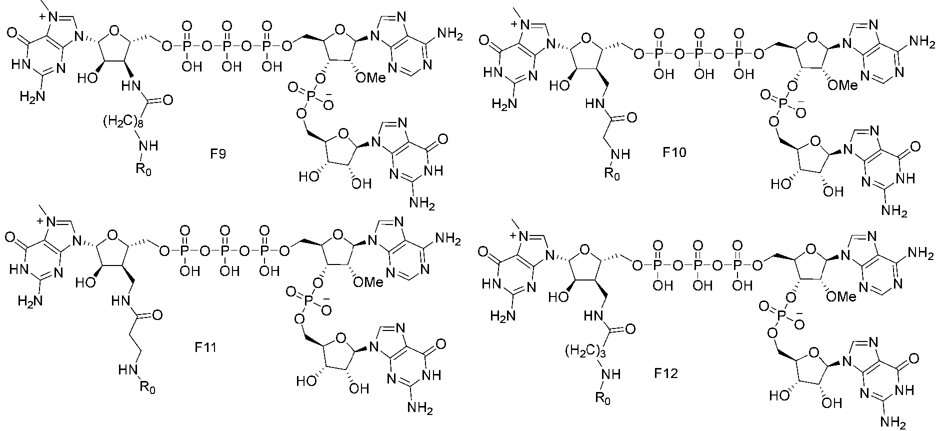

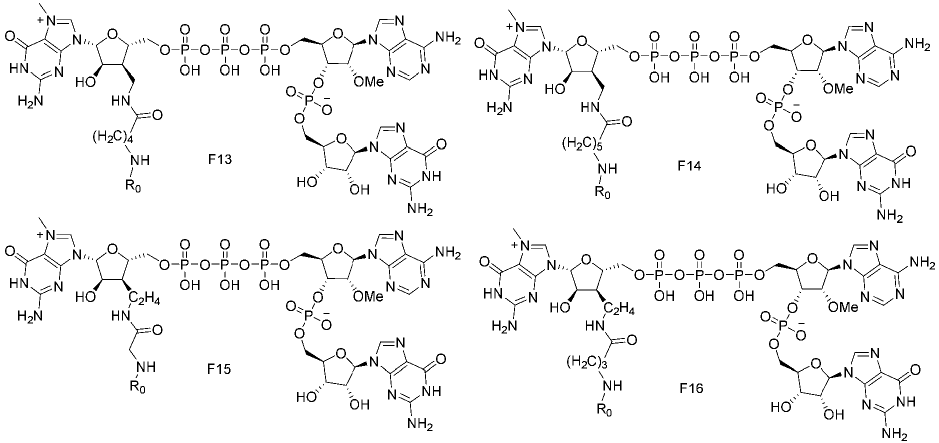

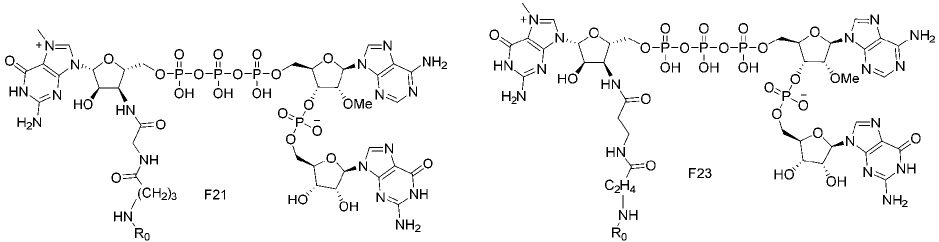

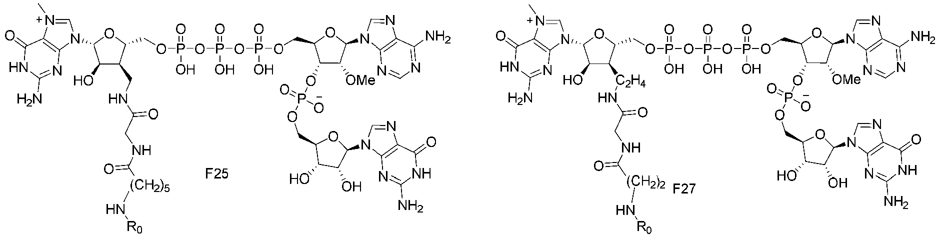

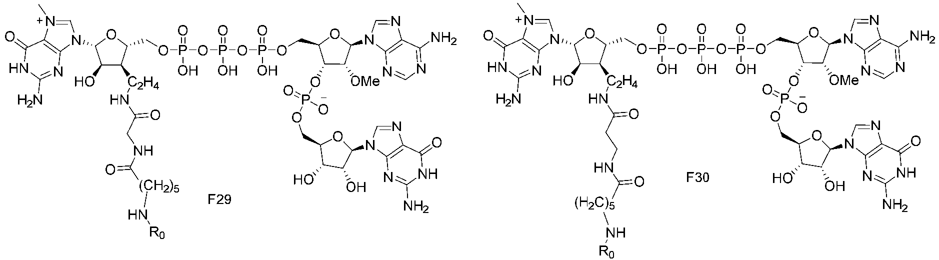

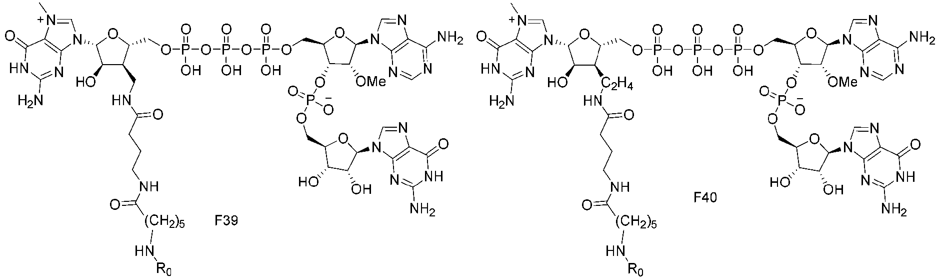

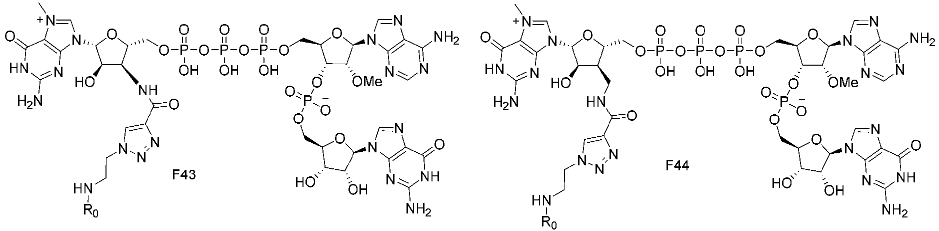

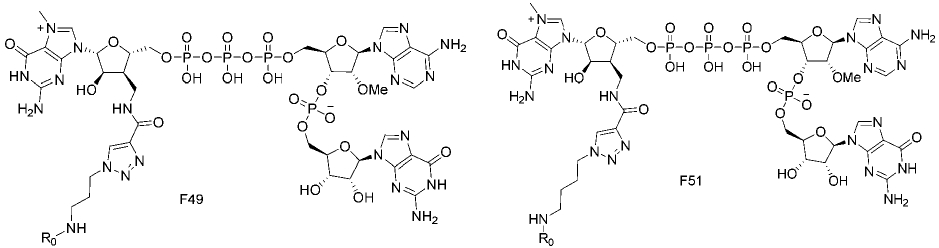

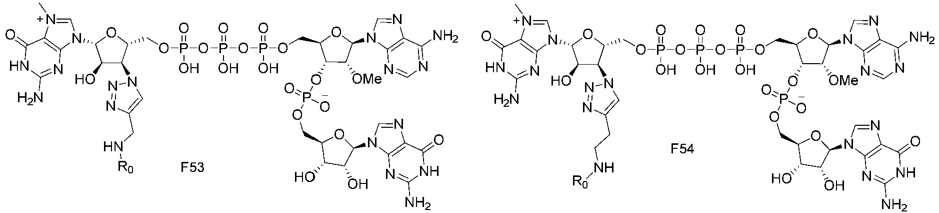

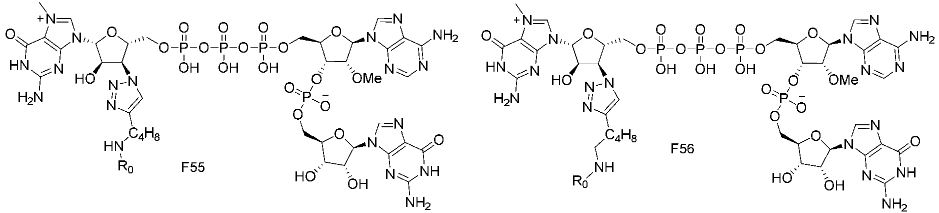

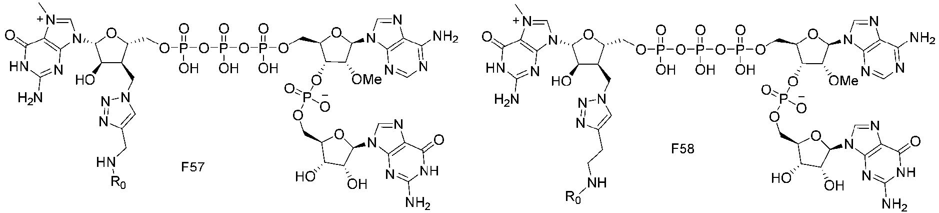

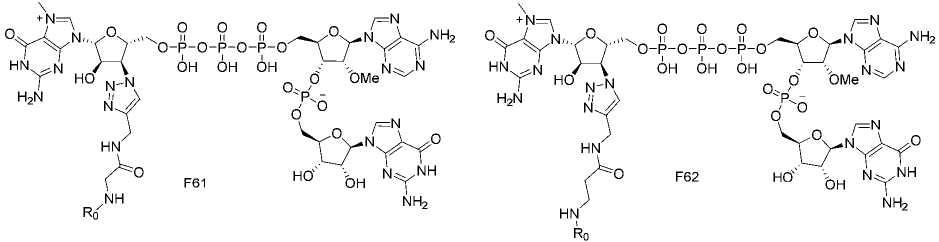

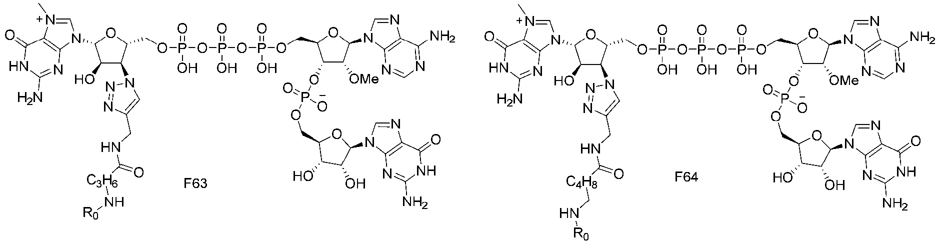

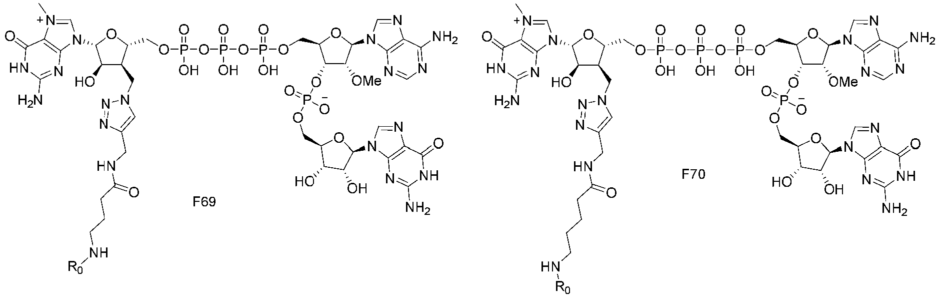

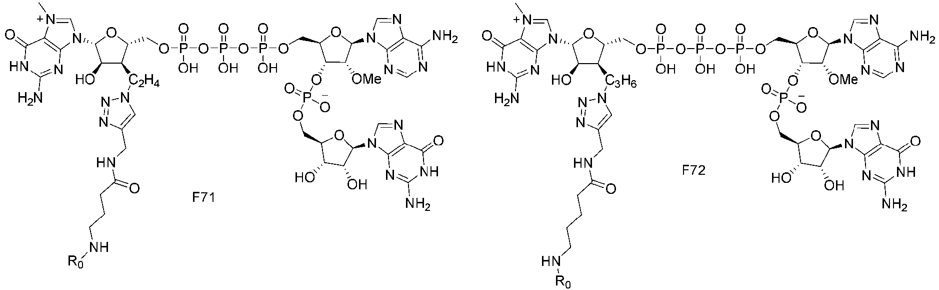

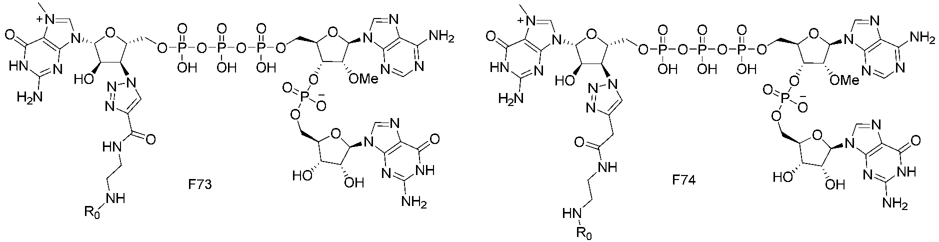

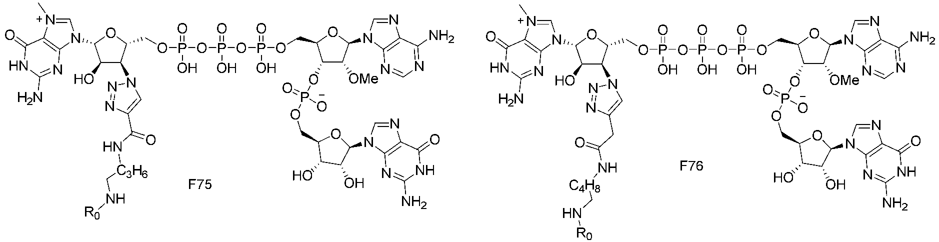

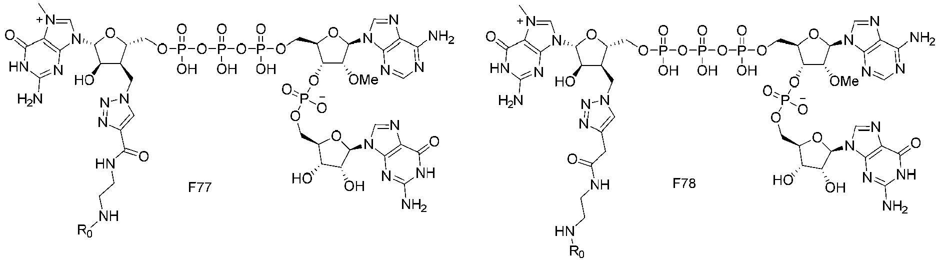

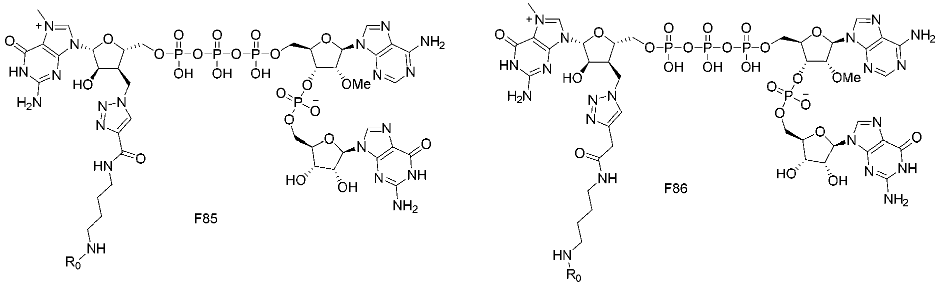

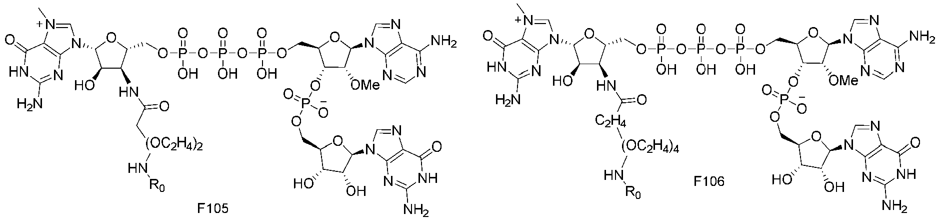

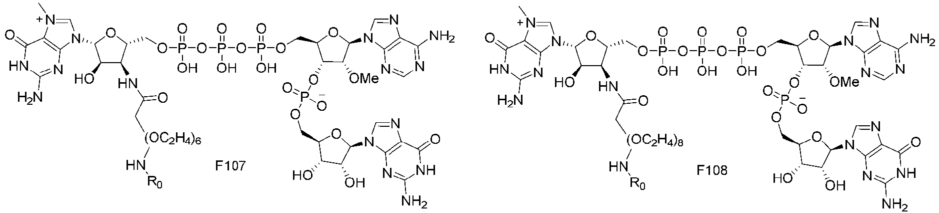

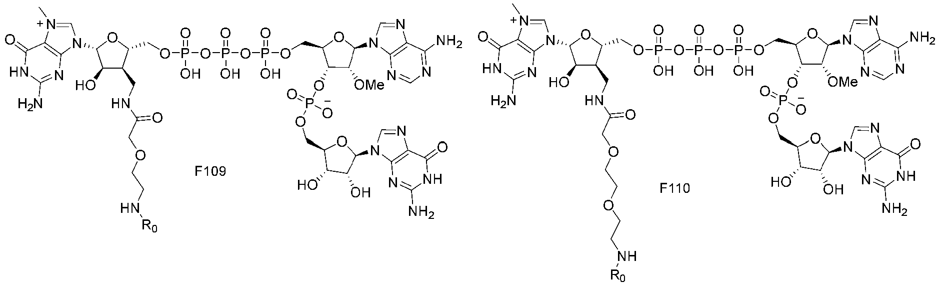

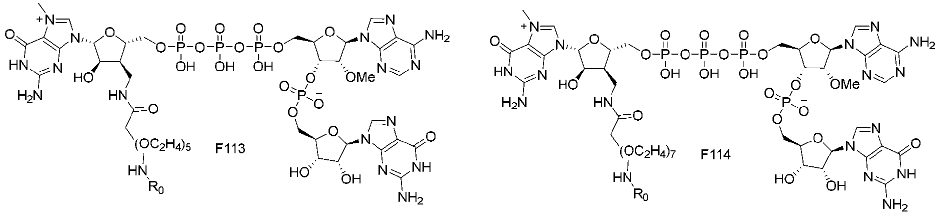

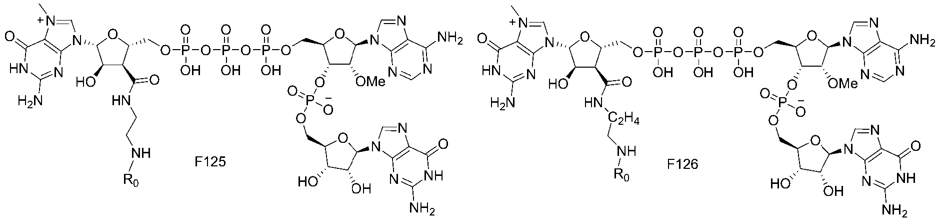

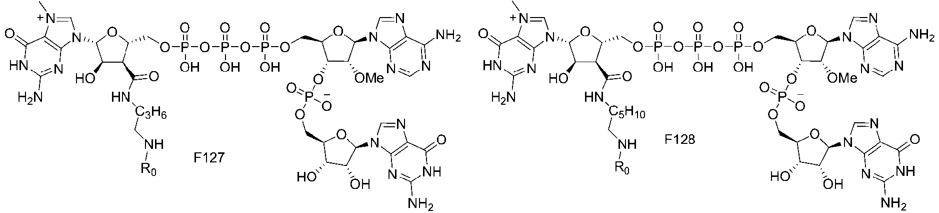

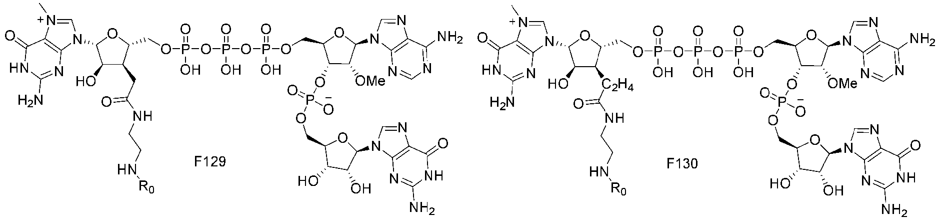

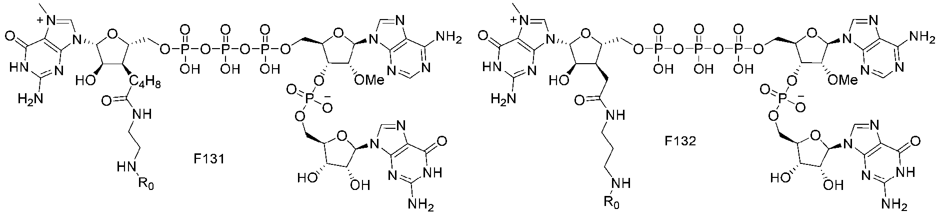

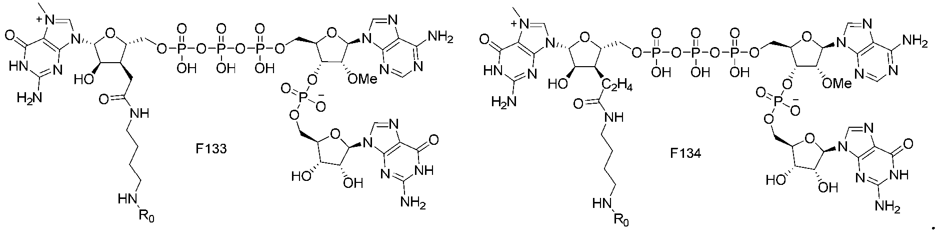

22. The compound according to claim 21, or a stereoisomer, pharmaceutically acceptable salt or solvate thereof, wherein R₀ is a fluorophore formed by biotin, Cy3, Cy5, Cy7, 5-FAM or 6-FAM, or a steroid structural group formed by cholic acid, deoxycholic acid, ursodeoxycholic acid, chenodeoxycholic acid, hyodeoxycholic acid or glycoursodeoxycholic acid.

23. The compound according to claim 22, or a stereoisomer, pharmaceutically acceptable salt or solvate thereof, wherein the compound has any one of the following structures:

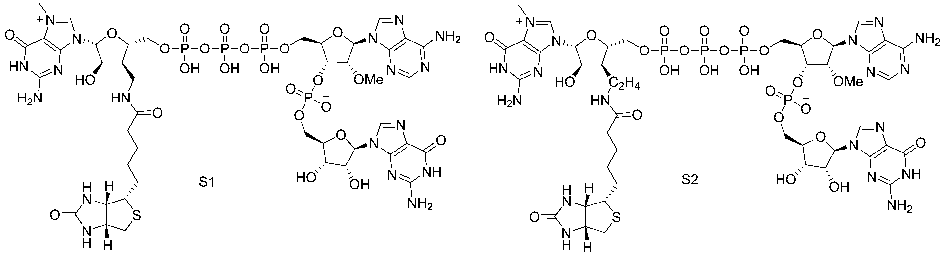

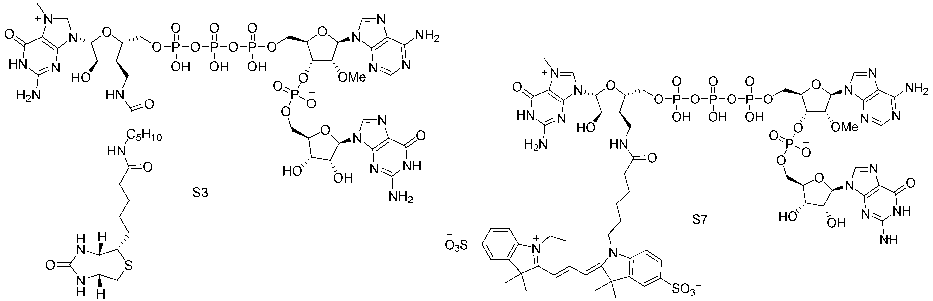

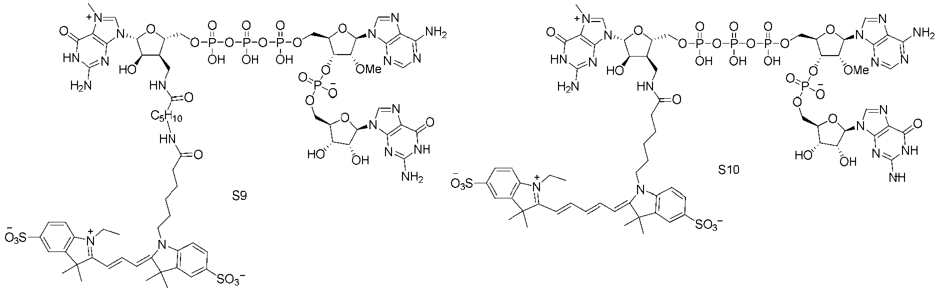

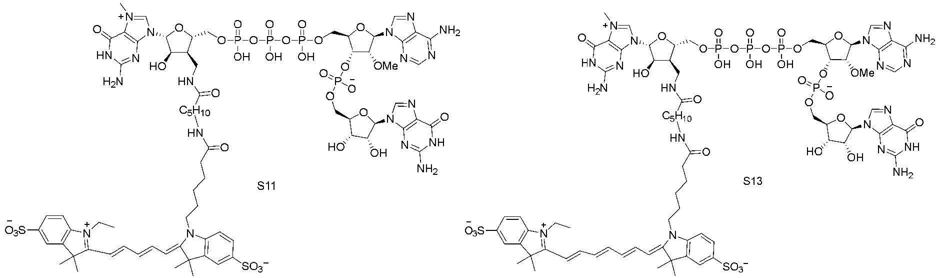

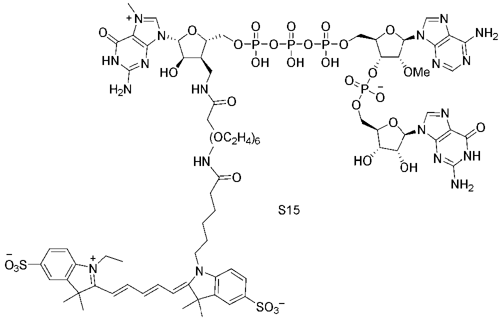

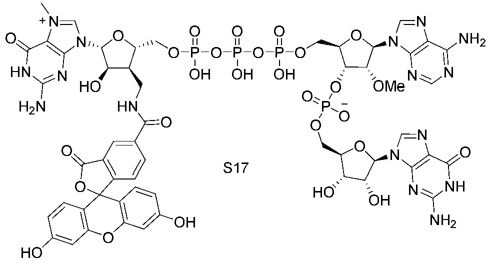

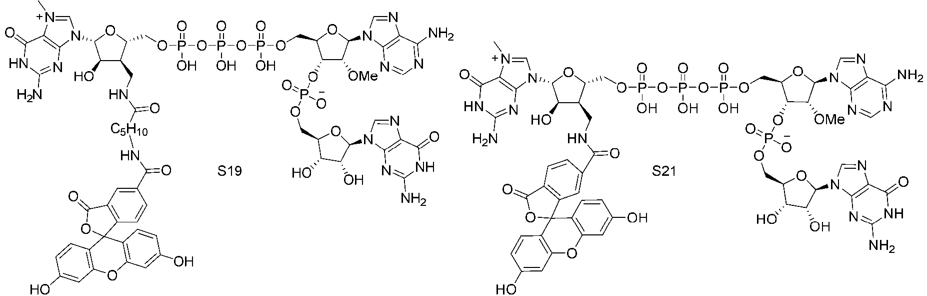

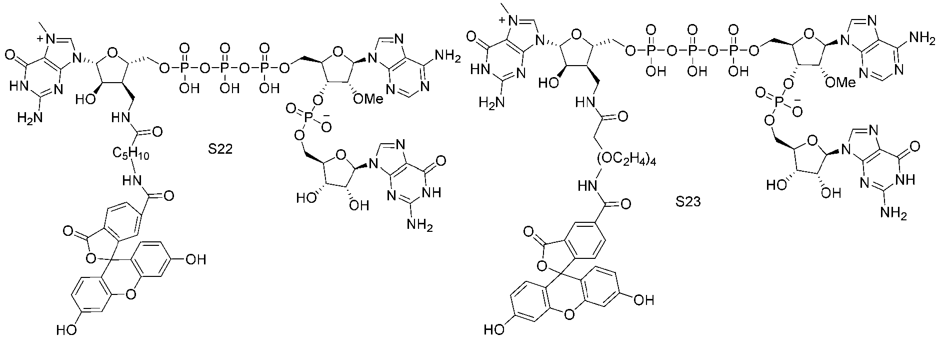

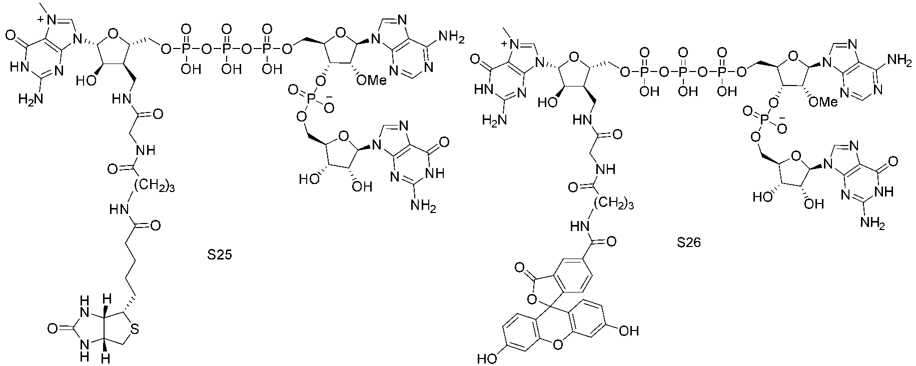

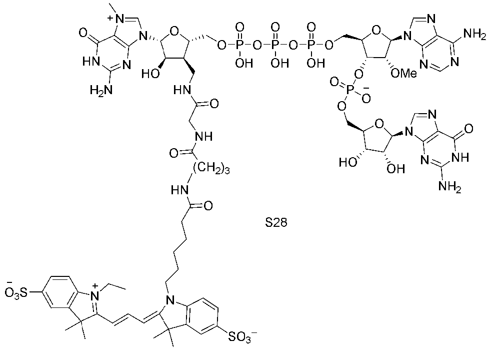

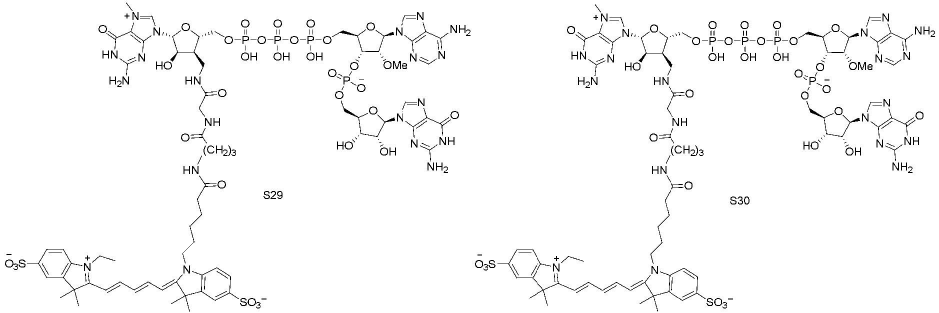

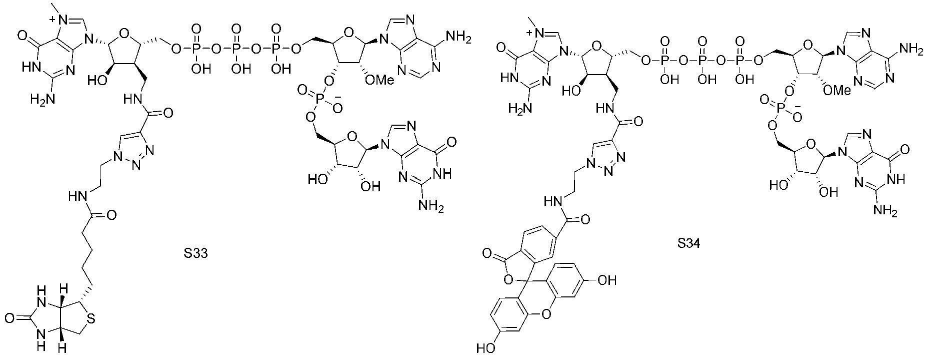

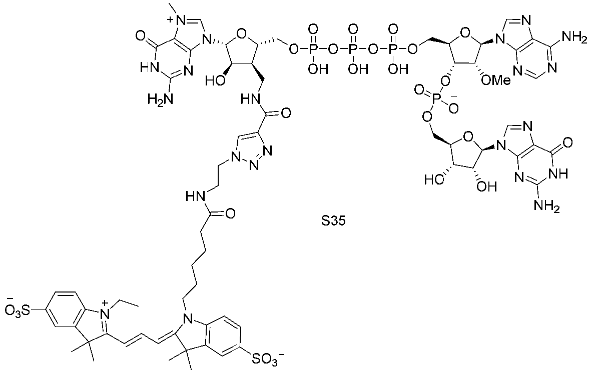

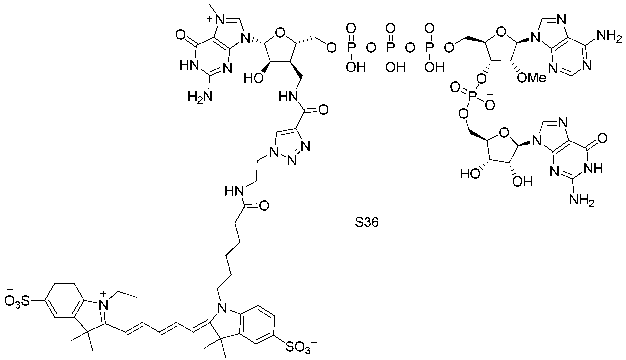

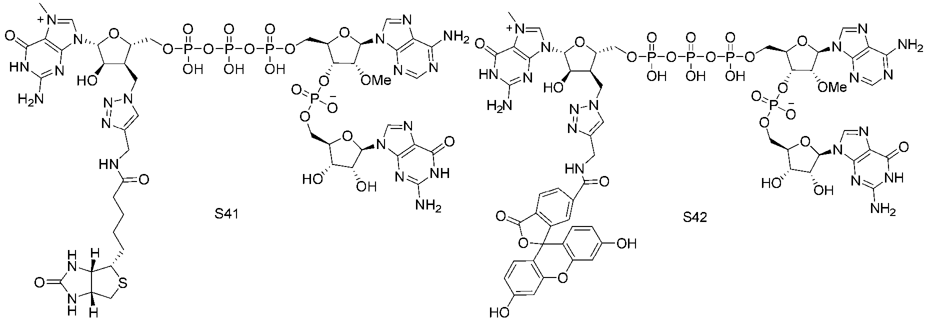

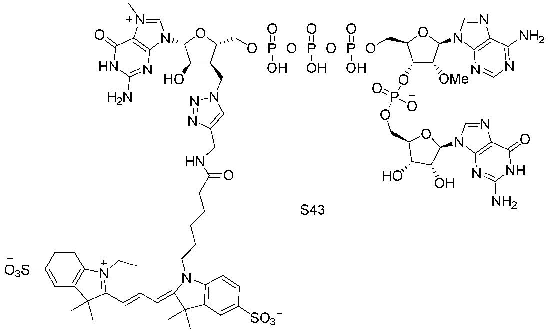

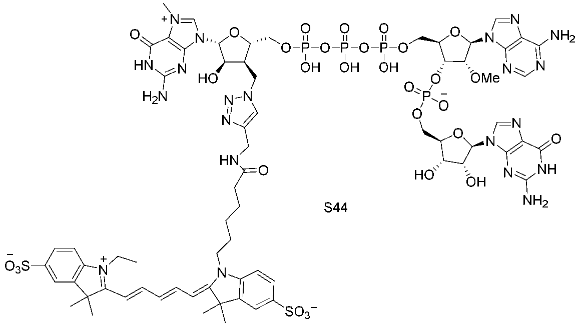

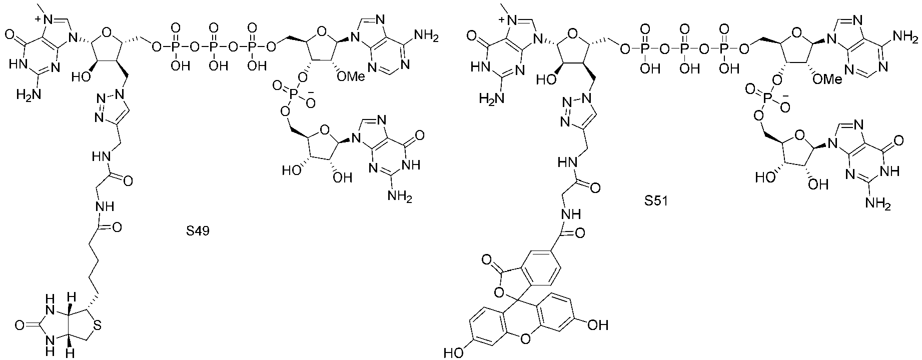

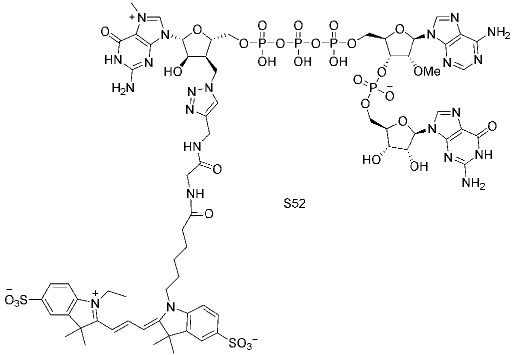

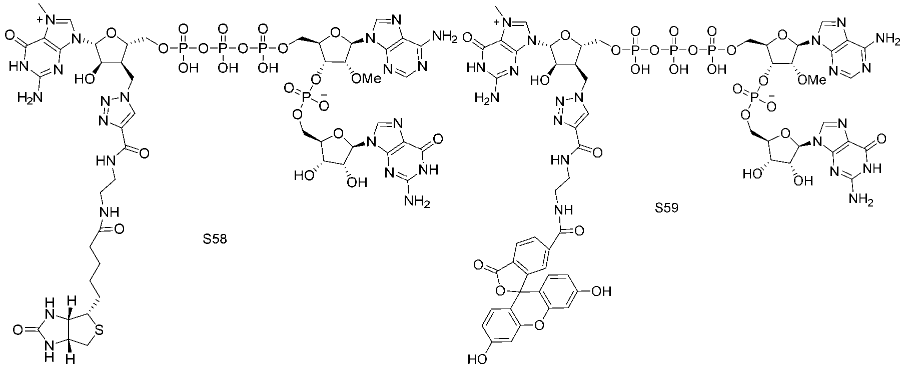

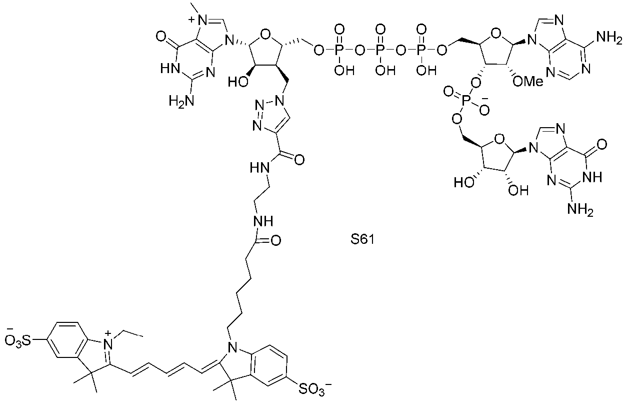

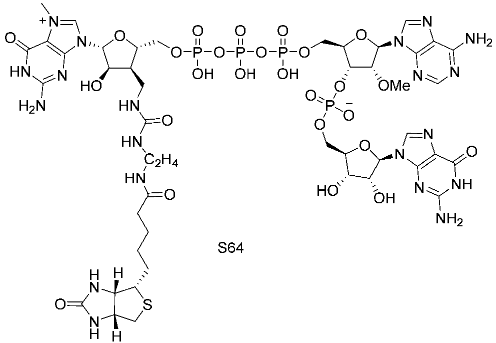

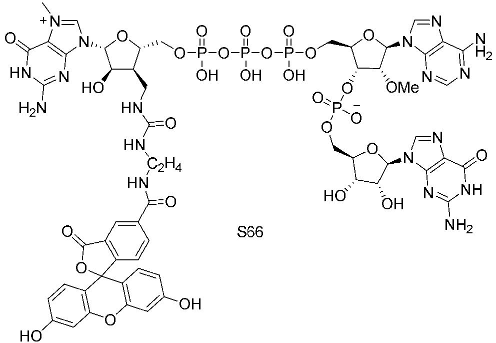

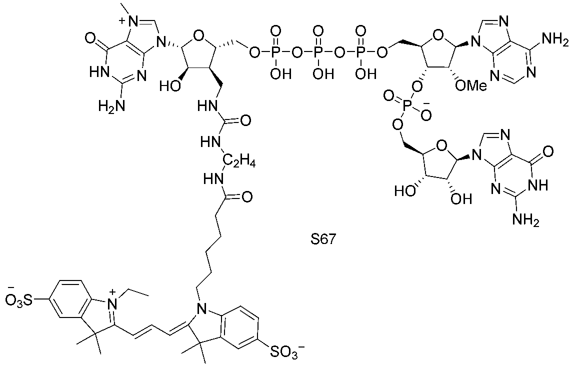

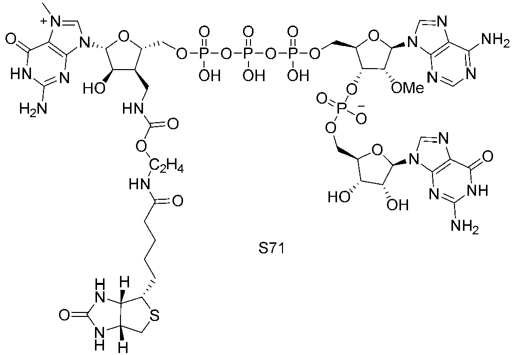

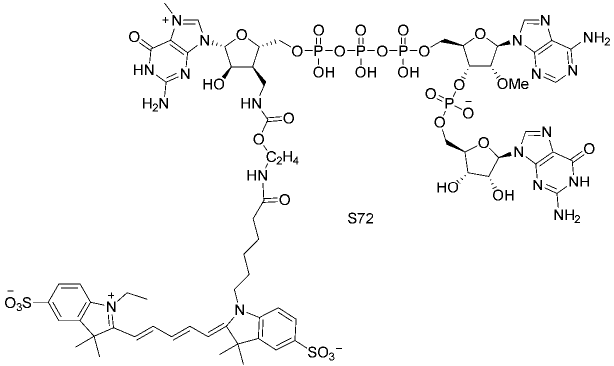

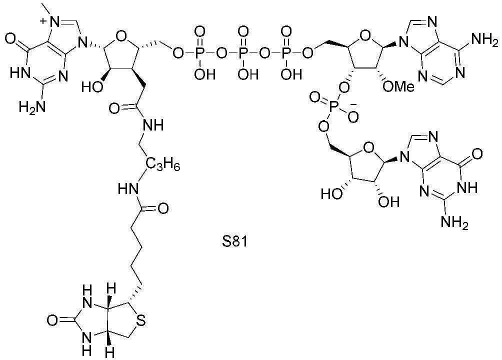

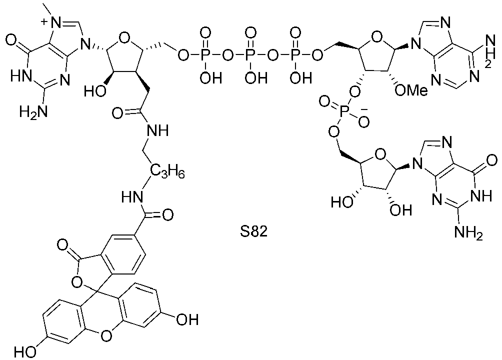

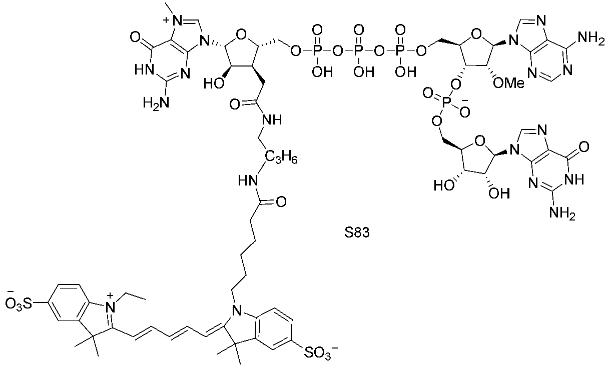

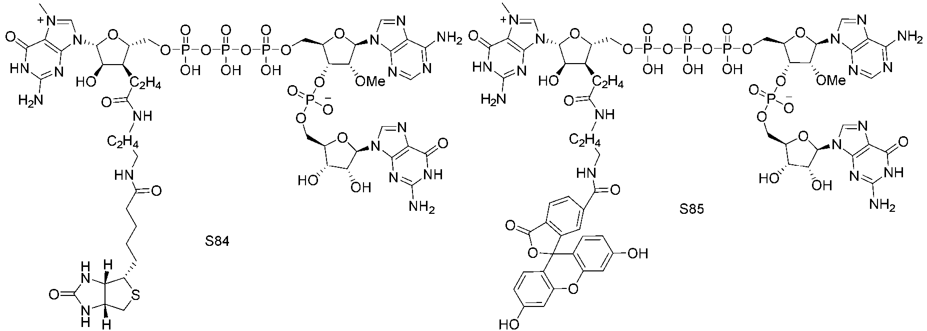

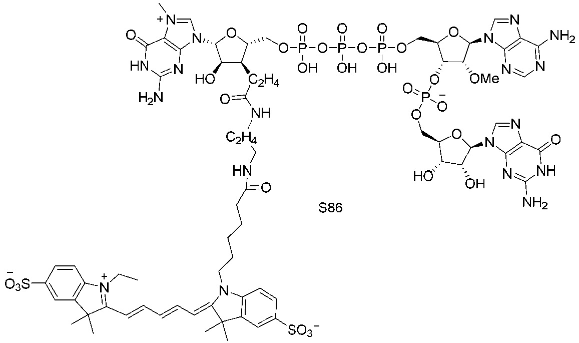

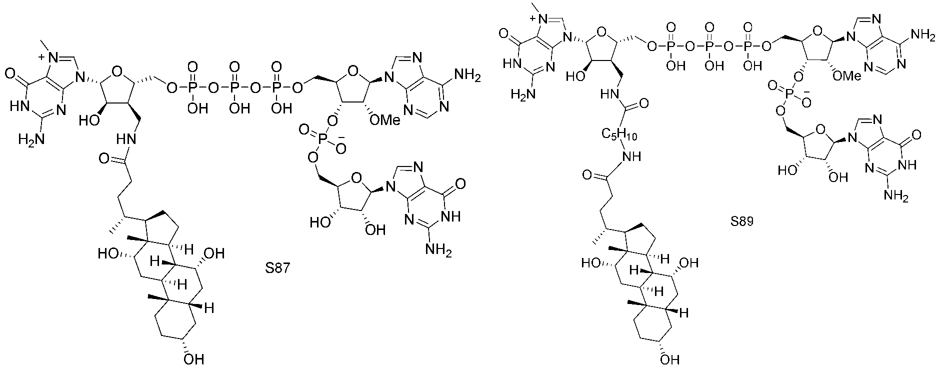

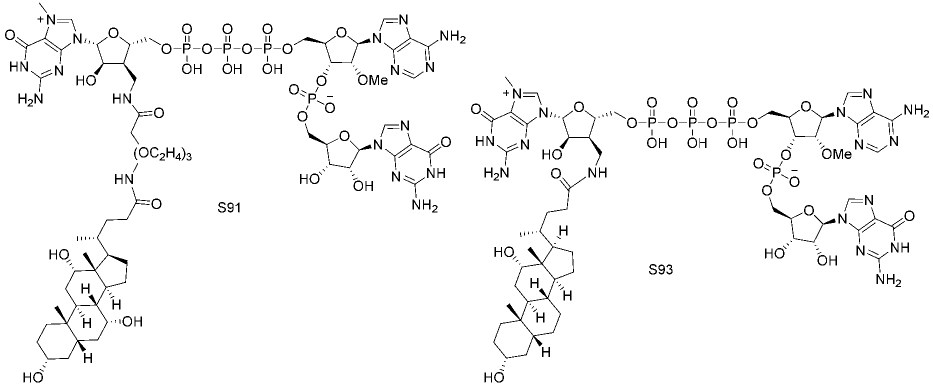

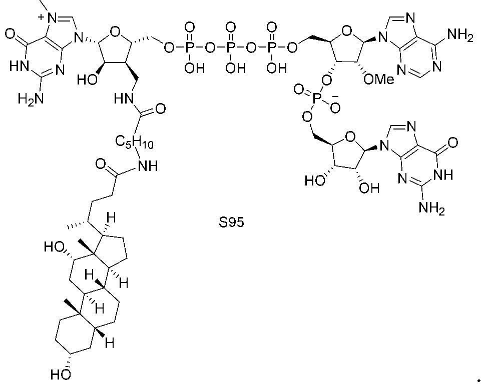

24. A compound having any one of the following structures, or a stereoisomer, pharmaceutically acceptable salt or solvate thereof, wherein the compound has a structure of:

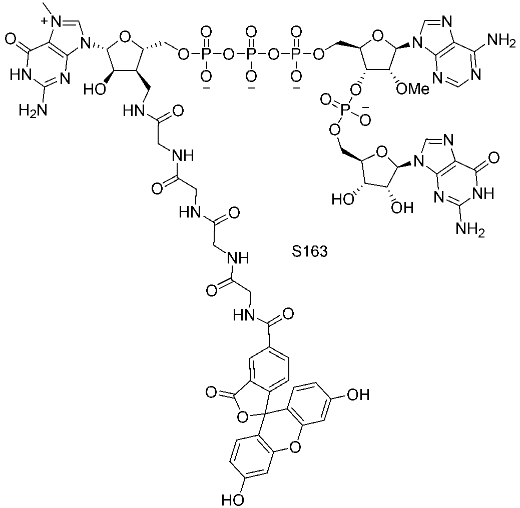

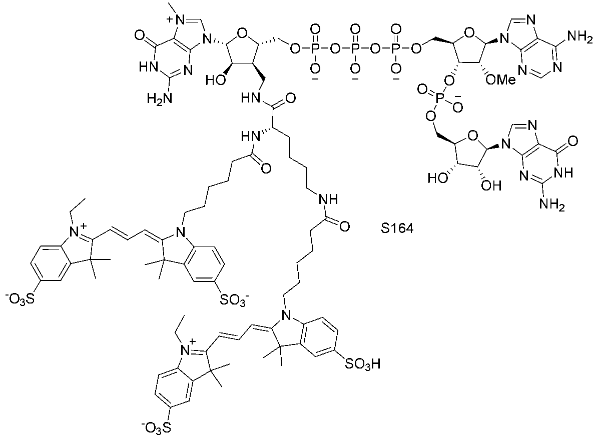

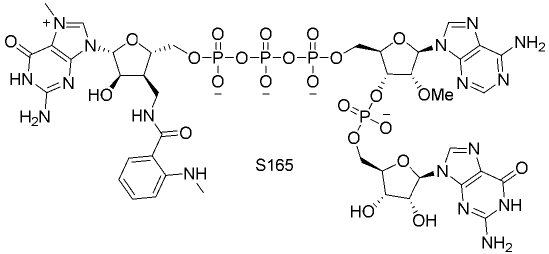

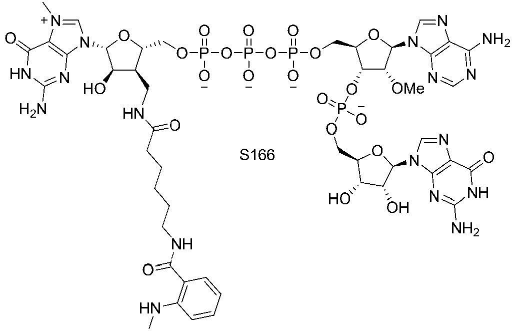

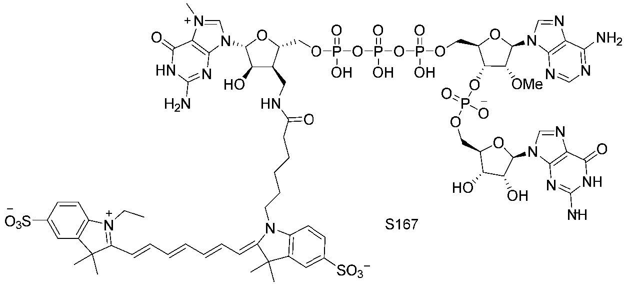

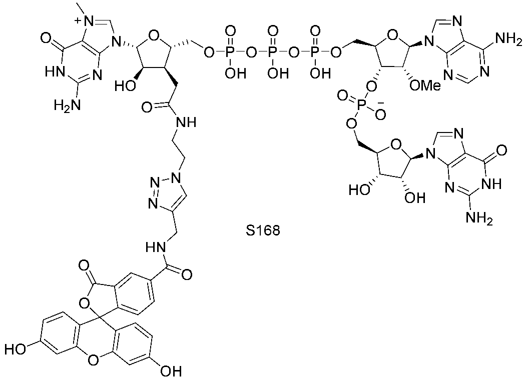

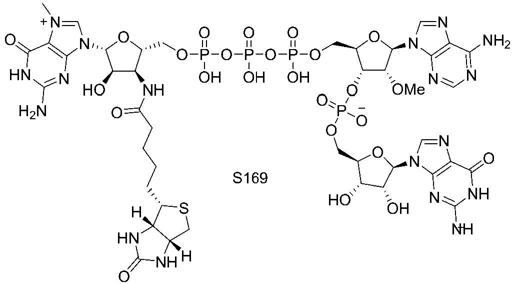

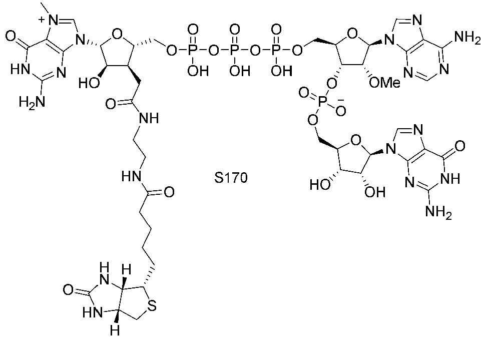

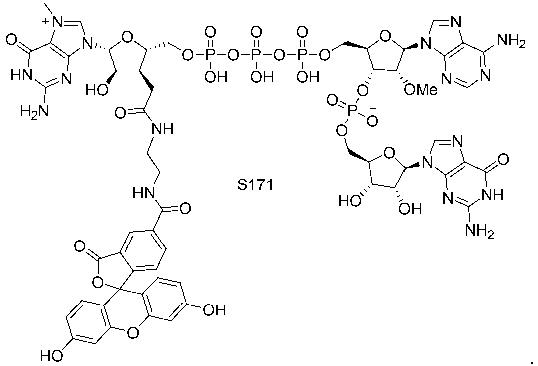

25. A compound having a structure represented by formula IV, or a stereoisomer, pharmaceutically acceptable salt or solvate thereof: 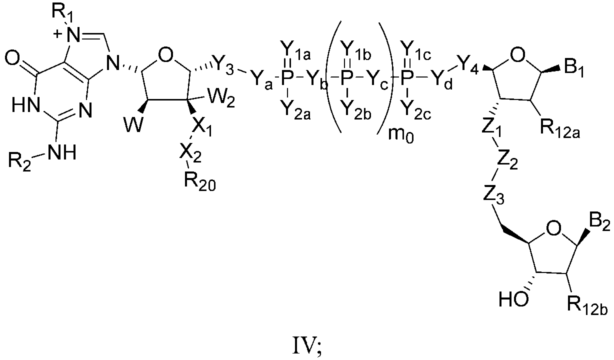
wherein R₁ is C₁-C₆ alkyl, C₂-C₆ alkenyl, C₂-C₆ alkynyl, C₃-C₆ cycloalkyl, C₃-C₆ cycloalkenyl, benzyl, R₅-substituted C₁-C₆ alkyl, R₅-substituted C₂-C₆ alkenyl, R₅-substituted C₂-C₆ alkynyl, R₅-substituted C₃-C₆ cycloalkyl, R₅-substituted C₃-C₆ cycloalkenyl or R₅-substituted benzyl;
R₂ is H, C₁-C₆ alkyl, C₂-C₆ alkenyl, C₂-C₆ alkynyl, C₃-C₈ cycloalkyl, cycloalkenyl, benzyl, aryl, heteroaryl, R₅-substituted benzyl, R₅-substituted aryl, carbonylalkyl, carbonylalkoxy or sulfonamido;
W is H, OH, OR₄, NR₄R₄, NR₄COR₄, F, Cl, N₃ or CN;
W₂ is H, OH, halogen, N₃, CN, OR₄, NR₄R₄, NR₄COR₄ or C₁-C₄ alkyl;
X₁ is (CH₂)ₙ₁, NR₄ or absent; n₁ is 1, 2 or 3;
X₂ is O, S, NR₄, CO, CO₂, CONR₄, NR₄CO, NR₄CO₂, NR₄CONR₄, SO₂, SO₂NR₄, CH₂ or absent;
R₂₀ is H, C₁-C₈ alkyl, C₂-C₈ alkenyl, C₂-C₈ alkynyl, R₅-substituted C₁-C₈ alkyl, R₅-substituted C₂-C₈ alkenyl, R₅-substituted C₂-C₈ alkynyl, aryl, R₅-substituted aryl, heteroaryl, R₅-substituted heteroaryl, halogen, CN or N₃;
R₁₂ₐ and R_{12b} are each independently H, halogen, alkoxy, C₁-C₆ alkyl, C₂-C₆ alkenyl, C₂-C₆ alkynyl, R₅-substituted C₁-C₆ alkyl, R₅-substituted C₂-C₆ alkenyl or R₅-substituted C₂-C₆ alkynyl;
or R₁₂ₐ and R_{12b} are each independently OH or R₅-substituted C₁-C₆ alkoxy;
Yₐ, Y_{b}, Y_{c} and Y_{d} are each independently O, S, CH₂, CCl₂, CF₂ or NH;
Y₁ₐ, Y1_{b} and Y_{1c} are each independently O or S;
Y₂ₐ, Y_{2b} and Y_{2c} are each independently OH, SH or BH₃;
Y₃ and Y₄ are each independently CH₂ or O;
Z₁ is O, OH, CH₂, S, NR₆, CO or SO₂;
Z₂ and Z₃ are each independently O, NR₆, CHR₇, CHCOOR₇, CHCONR₇R₇, S, CO, SO₂, PO(OH), PO(SH), P(O)VCO₂H or absent; Z₂ may form a ring with an oxygen atom connected to R3a;
Z₄ is O, CH₂, S, NR₆, CO, SO₂ or absent;
B₁ and B₂ are each independently a natural or modified pyrimidine nucleotide base or a natural or modified purine nucleotide base;
R₄ is H, C₁-C₈ alkyl, C₂-C₈ alkenyl or C₂-C₈ alkynyl;
R₅ is halogen, CN, SO₂, NO₂, D, N₃, C₁-C₈ alkyl, C₂-C₈ alkenyl, C₂-C₈ alkynyl, C₁-C₈ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₆ alkyl, COR₈ or SO₂R₈;
R₇ is H, halogen, C₁-C₈ alkyl, C₂-C₈ alkenyl or C₂-C₈ alkynyl;
R₈ is H, C₁-C₈ alkyl, C₂-C₈ alkenyl or C₂-C₈ alkynyl;
V is C₁-C₄ alkyl;
m₀ is 0, 1 or 2.

26. The compound according to claim 25, or a stereoisomer, pharmaceutically acceptable salt or solvate thereof, wherein R₄ is H, C₁-C₄ alkyl, C₂-C₅ alkenyl or C₂-C₅ alkynyl;
R₅ is halogen, CN, SO₂, NO₂, D, N₃, C₁-C₄ alkyl, C₂-C₅ alkenyl, C₂-C₅ alkynyl, C₁-C₄ alkoxy, OR₇, SR₇, NR₇R₇, COR₇, COOR₇, OCOOR₇, CONR₇R₇, NHCOR₇, OCONR₇R₇, aryl or heteroaryl;
R₆ is H, C₁-C₄ alkyl, COR₈ or SO₂R₈;
R₇ is H, halogen, C₁-C₄ alkyl, C₂-C₅ alkenyl or C₂-C₅ alkynyl;
R₈ is H, C₁-C₄ alkyl, C₂-C₅ alkenyl or C₂-C₅ alkynyl;
more preferably, R₅ is halogen, CN, SO₂, NO₂, D, N₃, C₁-C₄ alkyl, C₂-C₅ alkenyl, C₂-C₅ alkynyl, C₁-C₄ alkoxy, pyridyl, pyrimidinyl or morpholinyl.

27. The compound according to claim 26, or a stereoisomer, pharmaceutically acceptable salt or solvate thereof, wherein W is H, OH, C₁-C₄ alkoxy, C₁-C₄ alkylamino, F, Cl, N₃ or CN;
W₂ is H, OH, halogen, N₃, CN, C₁-C₄ alkyl, C₁-C₄ alkoxy or C₁-C₄ alkylamino;
X₁ is (CH₂)ₙ₁, C₁-C₄ alkyl-substituted amino or absent; n₁ is 1, 2 or 3;
X₂ is O, S, CO, CO₂, CONR₄, NR₄CO, NR₄CO₂, NR₄CONR₄, SO₂ or SO₂NR₄;
R₁₂ₐ and R_{12b} are each independently OH, C₁-C₆ alkyl, C₁-C₆ alkoxy, R₅-substituted C₁-C₆ alkyl or R₅-substituted C₁-C₆ alkoxy;
R₂₀ is H, C₁-C₄ alkyl, C₂-C₅ alkenyl, R₅-substituted C₁-C₄ alkyl, R₅-substituted aryl, heteroaryl, N₃ or absent.

28. The compound according to claim 26, or a stereoisomer, pharmaceutically acceptable salt or solvate thereof, wherein R₁₂ₐ and R_{12b} are each independently OH, F, Cl, C₁-C₄ alkyl, C₁-C₄ alkoxy, C₁-C₄ haloalkyl or C₁-C₄ haloalkoxy;
W is OH;
Yₐ, Y_{b}, Y_{c} and Y_{d} are each independently O or CH₂;
Y₁ₐ, Y_{1b} and Y_{1c} are each independently O or S;
Y₂ₐ, Y_{2b} and Y_{2c} are each independently OH;
Y₃ and Y₄ are independently CH₂ or O;
Z₁ is O, OH, CH₂, S or NH;
Z₂ and Z₃ are each independently O, NH, CHR₇, CHCOOR₇, CO, SO₂ or PO(OH);
B₁ and B₂ are each independently a natural or modified pyrimidine nucleotide base or a natural or modified purine nucleotide base;
R₄ is H or C₁-C₄ alkyl;
R₅ is halogen, OR₇, NR₇R₇, CONR₇R₇ or OCONR₇R₇;
R₆ is H;
R₇ is H or C₁-C₄ alkyl;
m₀ is 0, 1 or 2.

29. The compound according to claim 28, or a stereoisomer, pharmaceutically acceptable salt or solvate thereof, wherein R₁ is C₁-C₄ alkyl, C₂-C₄ alkenyl, C₂-C₄ alkynyl, C₃-C₆ cycloalkyl, C₃-C₆ cycloalkenyl, benzyl, C₁-C₄ haloalkyl, C₂-C₄ haloalkenyl, C₂-C₄ haloalkynyl, C₃-C₆ halocycloalkyl, C₃-C₆ halocycloalkenyl or halobenzyl;
R₂ is H, C₁-C₄ alkyl, C₂-C₄ alkenyl, C₂-C₄ alkynyl, C₃-C₆ cycloalkyl, C₃-C₆ cycloalkenyl, C₁-C₄ haloalkyl, C₂-C₄ haloalkenyl, C₂-C₄ haloalkynyl, C₃-C₆ halocycloalkyl, C₃-C₆ halocycloalkenyl, benzyl or halobenzyl.

30. The compound according to claim 27, or a stereoisomer, pharmaceutically acceptable salt or solvate thereof, wherein the compound has a structure represented by any one of the following general formulas: IV-2, IV-3, IV-4, IV-5 or IV-6: 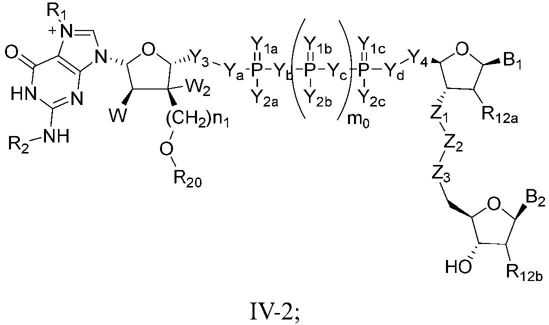 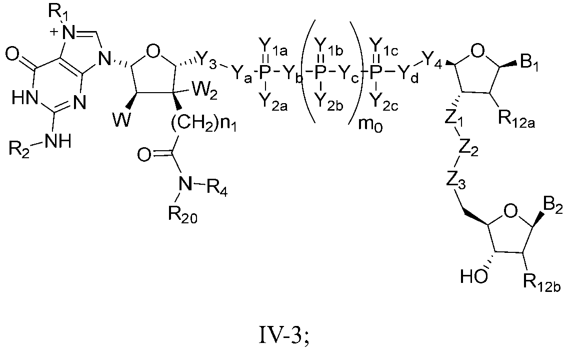 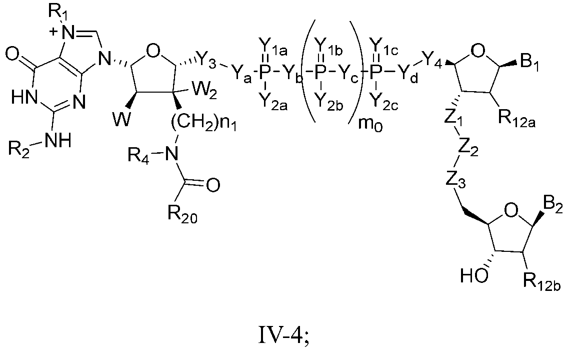 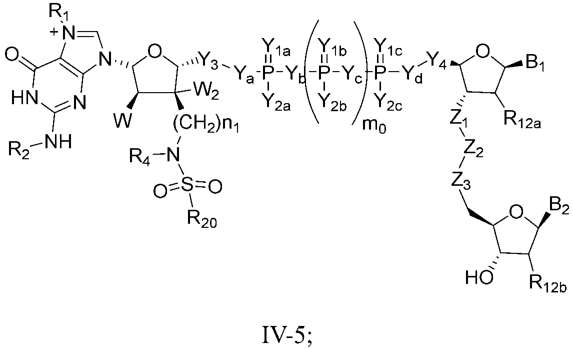 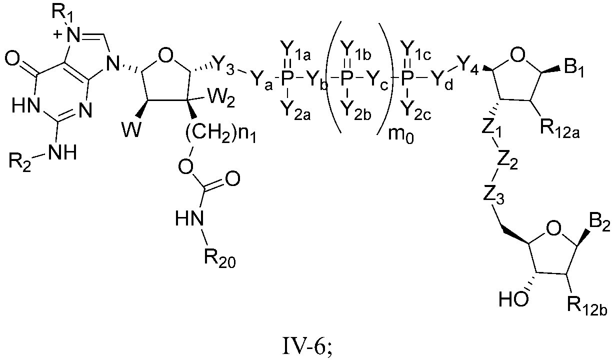 wherein R₁, R₂, R₂₀, R₁₂ₐ, R_{12b}, R₄, R₅, R₆, R₇, R₈, W, W₂, Yₐ, Y_{b}, Y_{c}, Y_{d}, Y₁ₐ, Y_{1b}, Y_{1c}, Y₂ₐ, Y_{2b}, Y_{2c}, Y₃, Y₄, B₁, B₂, Z₁, Z₂, Z₃, B₁, B₂, V, n₁, and m₀ are as defined in claim 30, respectively.

31. The compound according to claim 30, or a stereoisomer, pharmaceutically acceptable salt or solvate thereof, wherein R₁ is methyl, ethyl, n-propyl, isopropyl, butyl, vinyl, propenyl, butenyl, ethynyl, propynyl, butynyl, benzyl, halomethyl, haloethyl, halo-n-propyl, haloisopropyl, halobutyl, halovinyl, halopropenyl, halobutenyl, haloethynyl, halopropynyl, halobutynyl or halobenzyl;
R₂ is H, methyl, ethyl, n-propyl, isopropyl, fluoromethyl, difluoromethyl, trifluoromethyl, trifluoroethyl, trifluoropropyl or trifluoroisopropyl;
W is OH, F, Cl, methyl, ethyl, methoxy or ethoxy;
W₂ is H, OH, F, Cl, N₃, CN, methyl, ethyl, propyl, isopropyl, methoxy, ethoxy, methyl-substituted amino or ethyl-substituted amino;
R₄ is H, methyl, ethyl, n-propyl, isopropyl, butyl, vinyl, propenyl, butenyl, ethynyl, propynyl or butynyl;
R₂₀ is H, C₁-C₄ alkyl, C₂-C₅ alkenyl, R₅-substituted C₁-C₄ alkyl, R₅-substituted aryl, heteroaryl, N₃ or absent;
R₅ is halogen, CN, D, N₃, C₁-C₄ alkyl, C₂-C₅ alkenyl, C₂-C₅ alkynyl, C₁-C₄ alkoxy, pyridyl, pyrimidinyl or morpholinyl.

32. The compound according to claim 31, or a stereoisomer, pharmaceutically acceptable salt or solvate thereof, wherein R₁₂ₐ and R_{12b} are each independently OH, F, Cl, C₁-C₄ alkyl, C₁-C₄ alkoxy, C₁-C₄ haloalkyl or C₁-C₄ haloalkoxy;
W is OH;
Z₁ is O, OH, CH₂, S or NH;
Z₂ and Z₃ are each independently O, NH, CH₂, CHCOO, CO, SO₂ or PO(OH);
B₁ and B₂ are independently a natural or modified cytosine nucleotide base, a natural or modified uracil nucleotide base, a natural or modified adenine nucleotide base or a natural or modified guanine nucleotide base;
m₀ is 0, 1 or 2.

33. The compound according to claim 30, or a stereoisomer, pharmaceutically acceptable salt or solvate thereof, wherein the compound has a structure represented by formula V: 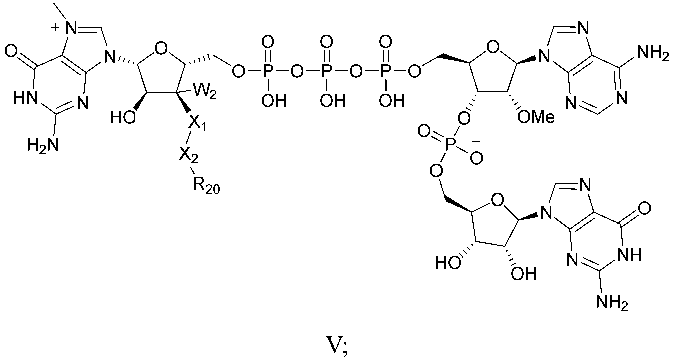
wherein W₂ is H, OH, halogen, N₃, CN, C₁-C₄ alkyl, C₁-C₄ alkoxy or C₁-C₄ alkylamino;
X₁ is (CH₂)ₙ₁, C₁-C₄ alkyl-substituted amino or absent; n₁ is 1, 2 or 3;
X₂ is O, S, CO, CO₂, CONR₄, NR₄CO, NR₄CO₂, NR₄CONR₄, SO₂ or SO₂NR₄;
R₂₀ is H, C₁-C₄ alkyl, C₂-C₅ alkenyl, R₅-substituted C₁-C₄ alkyl, R₅-substituted aryl, heteroaryl, N₃ or absent;
R₄ is H, C₁-C₄ alkyl, C₂-C₅ alkenyl or C₂-C₅ alkynyl;
R₅ is halogen, CN, SO₂, NO₂, D, N₃, C₁-C₄ alkyl, C₂-C₅ alkenyl, C₂-C₅ alkynyl, C₁-C₄ alkoxy, OR₇, SR₇, NR₇R₇, COR₇, COOR₇, OCOOR₇, CONR₇R₇, NHCOR₇, OCONR₇R₇, aryl or heteroaryl;
R₇ is H, halogen, C₁-C₄ alkyl, C₂-C₅ alkenyl or C₂-C₅ alkynyl.

34. The compound according to claim 33, or a stereoisomer, pharmaceutically acceptable salt or solvate thereof, wherein the compound has a structure represented by formula V-2, V-3, V-4, V-5 or V-6: 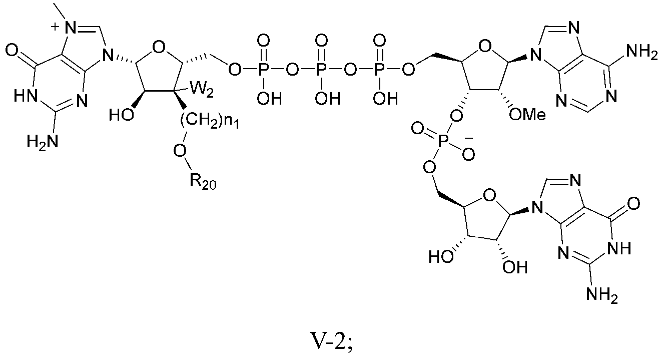 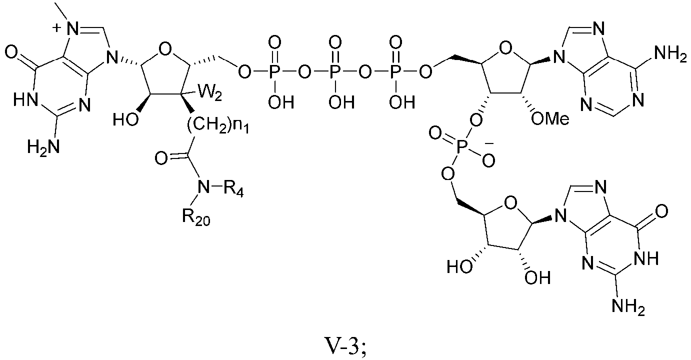 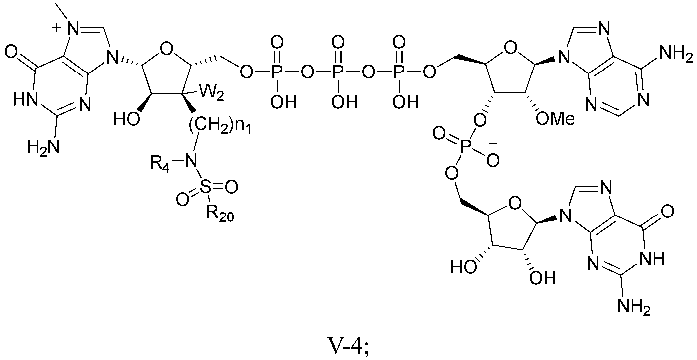 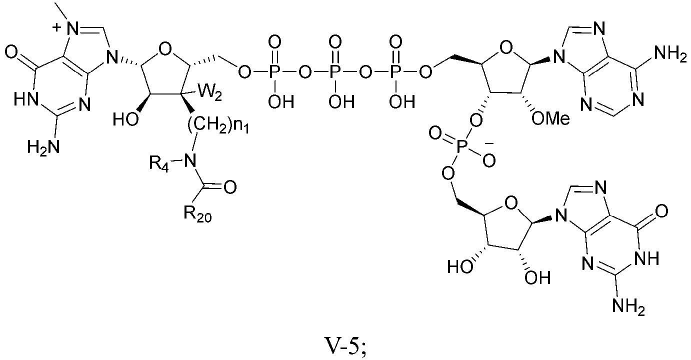 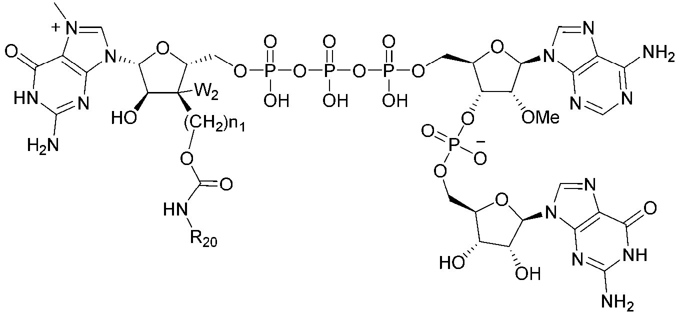 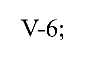
wherein W₂ is H, OH, F, Cl, N₃, CN, methyl, ethyl, propyl, isopropyl, methoxy, ethoxy, methyl-substituted amino or ethyl-substituted amino;
R₄ is H, methyl, ethyl, n-propyl, isopropyl, butyl, vinyl, propenyl, butenyl, ethynyl, propynyl or butynyl;
R₂₀ is H, C₁-C₄ alkyl, C₂-C₅ alkenyl, R₅-substituted C₁-C₄ alkyl, R₅-substituted aryl, heteroaryl, N₃ or absent;
R₅ is halogen, CN, D, N₃, C₁-C₄ alkyl, C₂-C₅ alkenyl, C₂-C₅ alkynyl, C₁-C₄ alkoxy, pyridyl, pyrimidinyl or morpholinyl.

35. The compound according to claim 25, or a stereoisomer, pharmaceutically acceptable salt or solvate thereof, wherein the compound is represented by any one of the following structures:

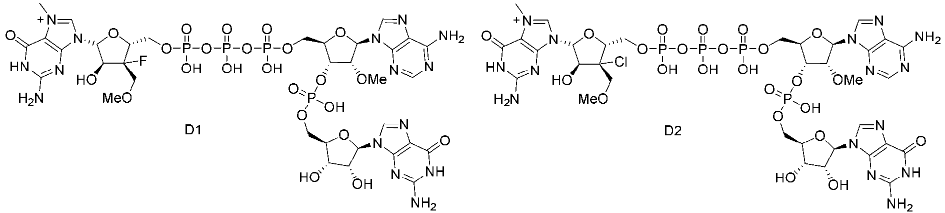

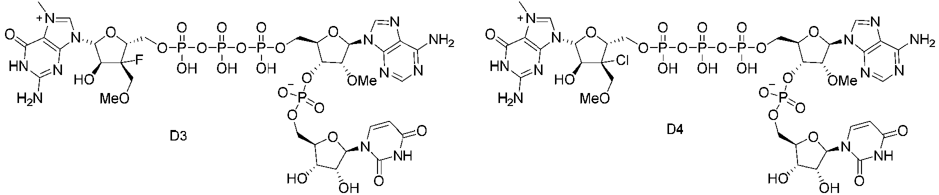

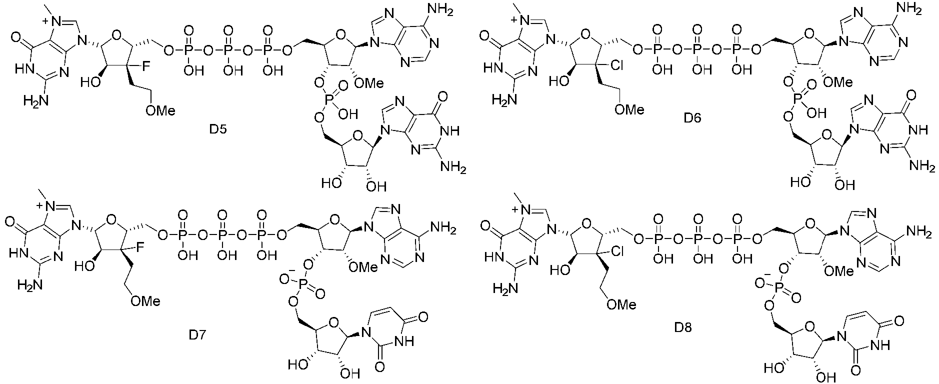

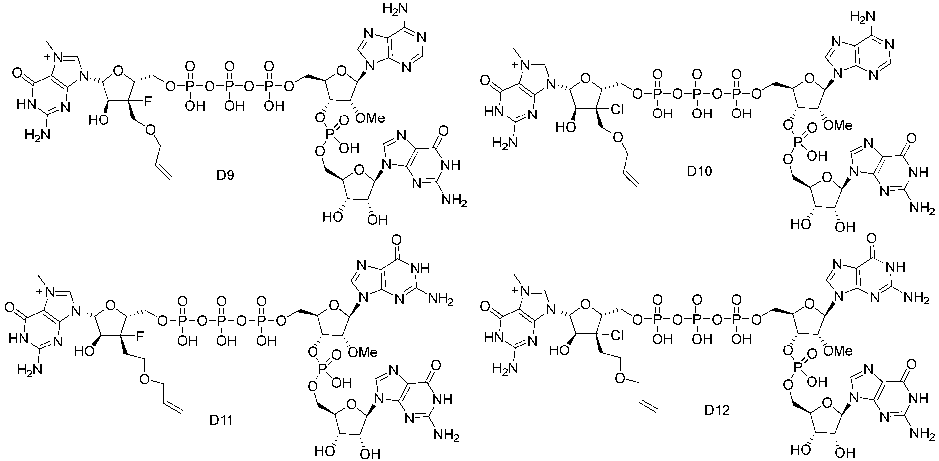

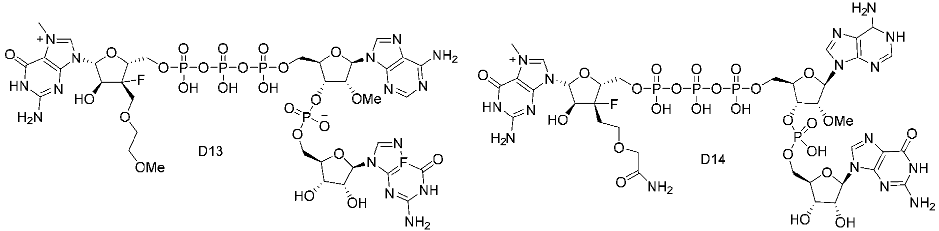

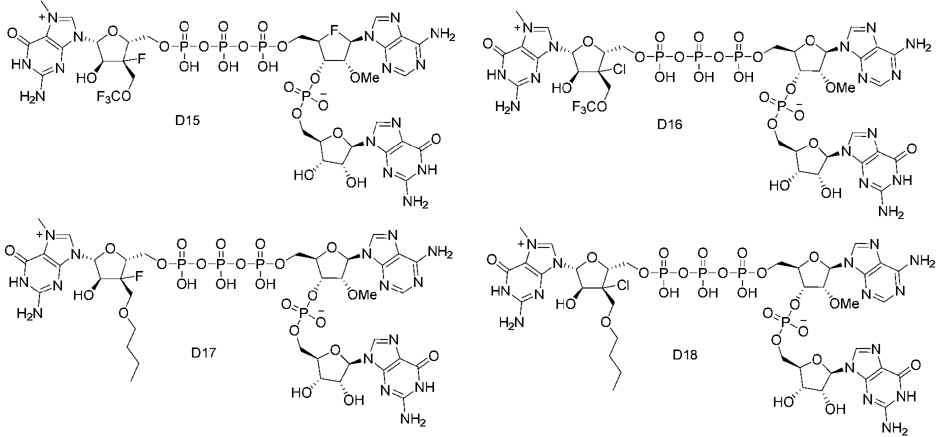

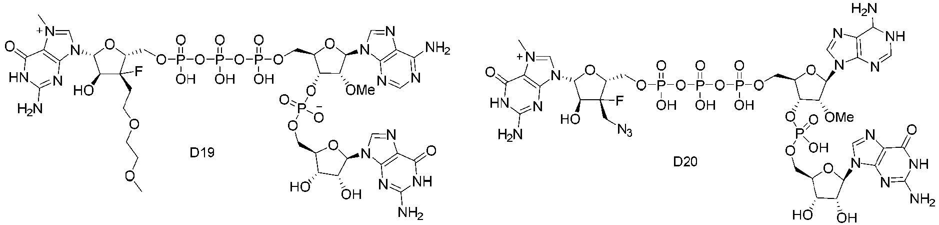

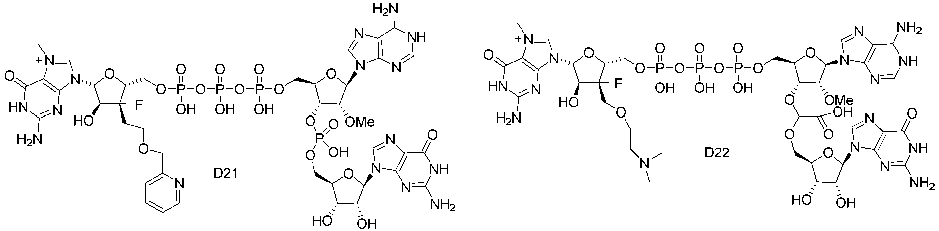

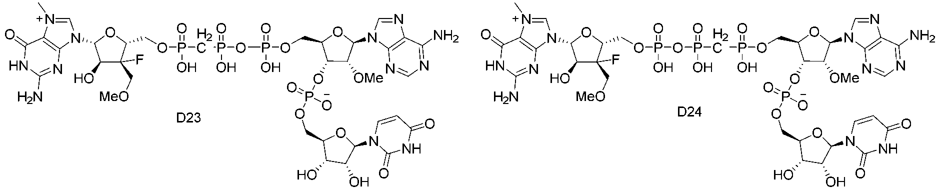

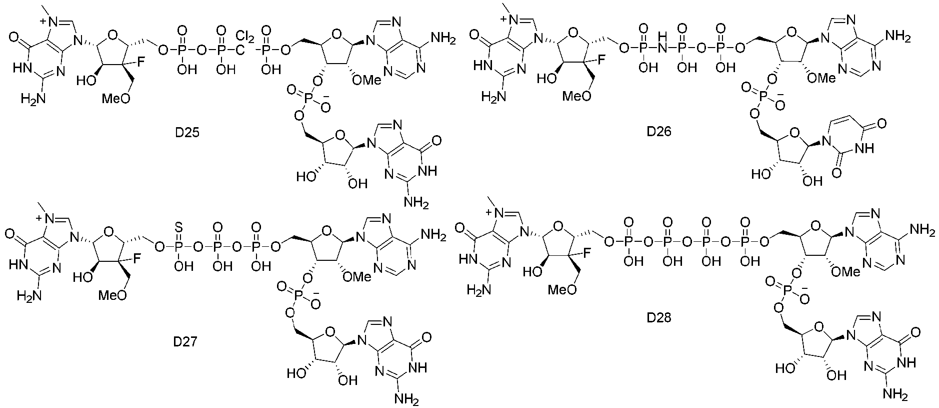

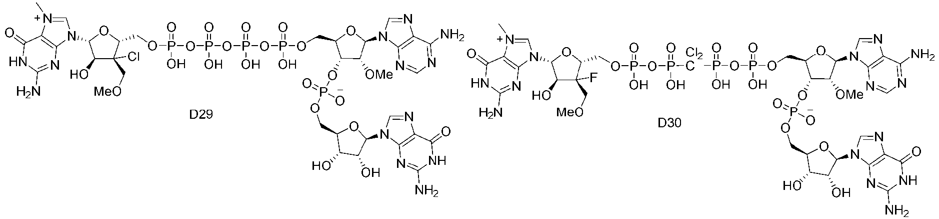

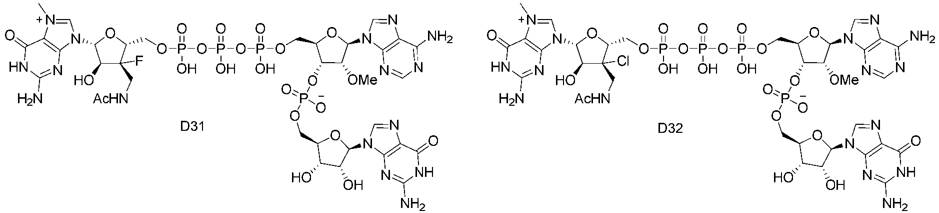

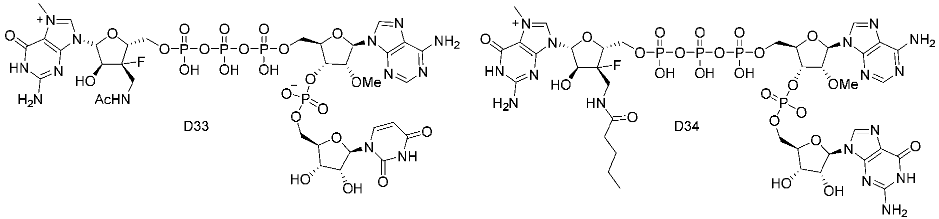

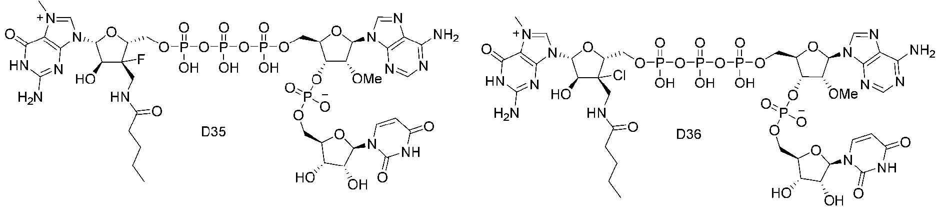

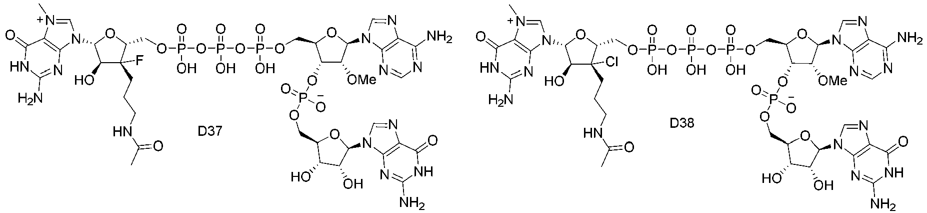

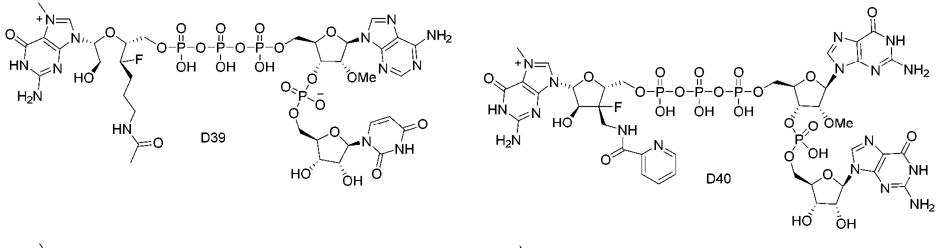

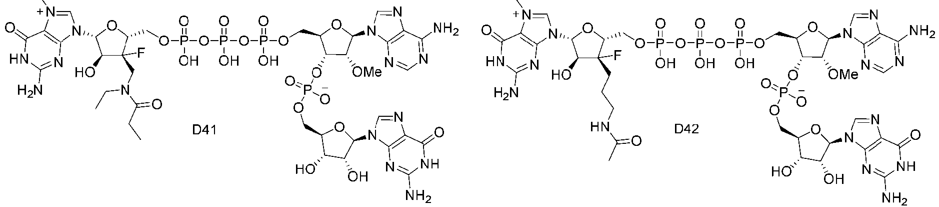

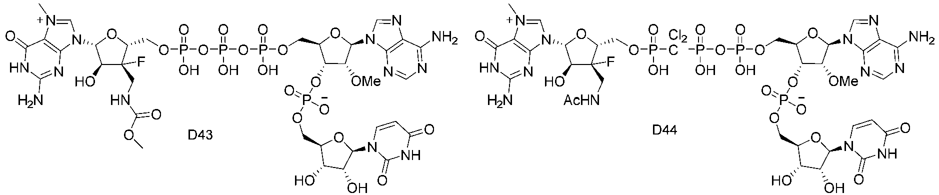

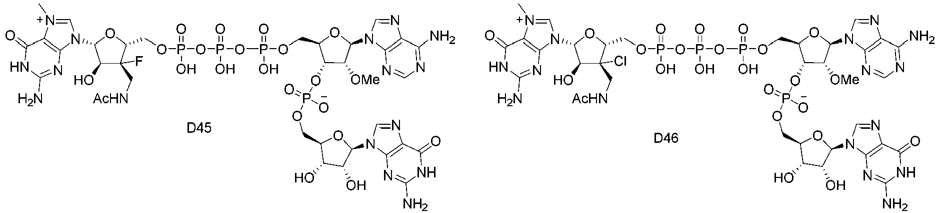

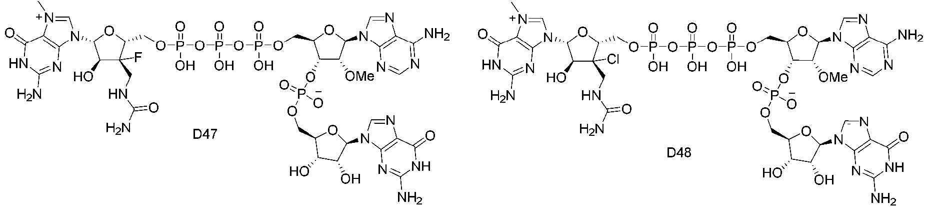

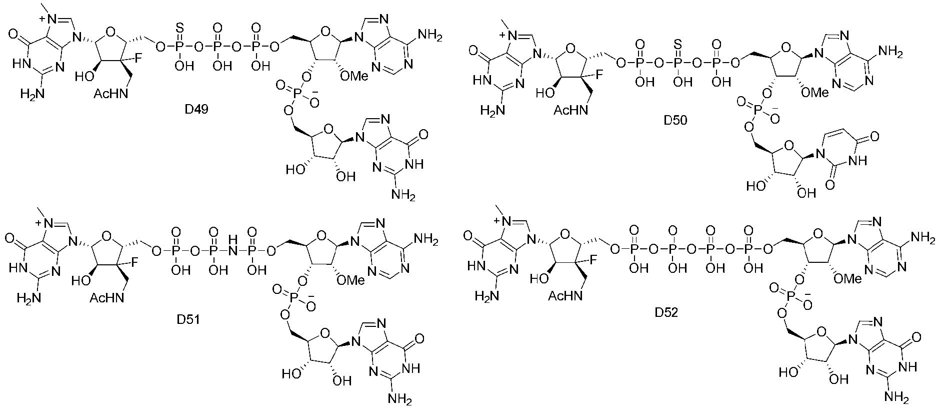

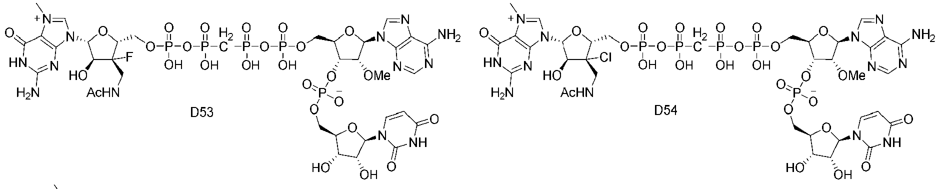

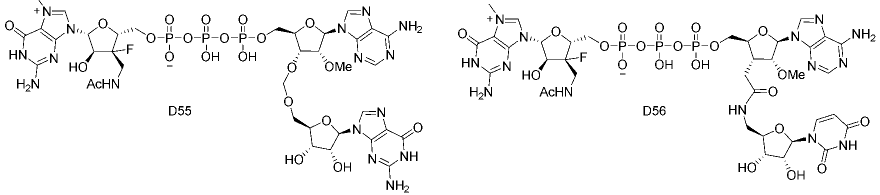

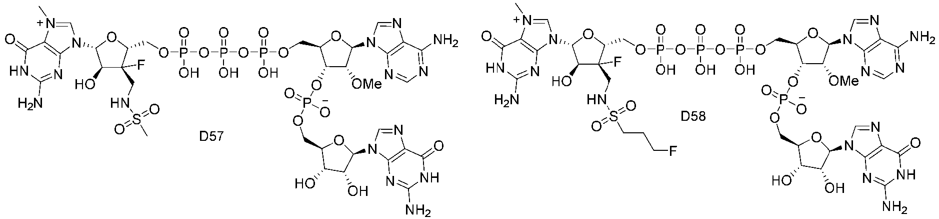

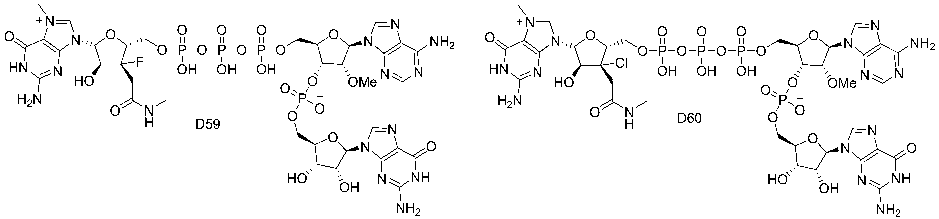

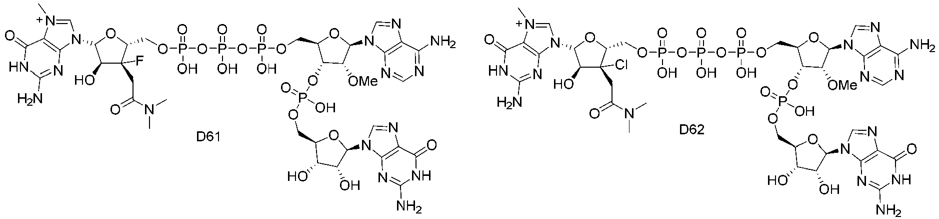

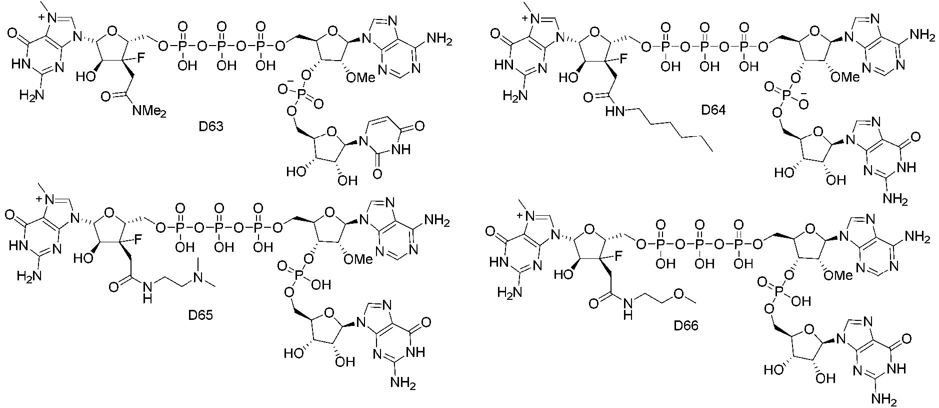

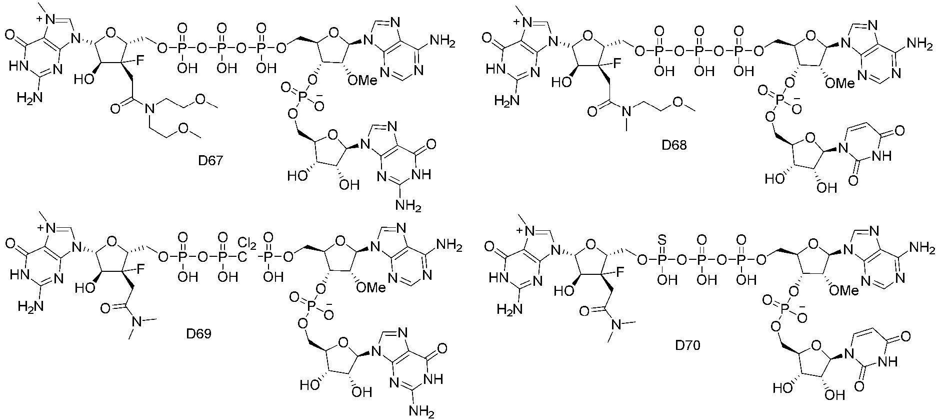

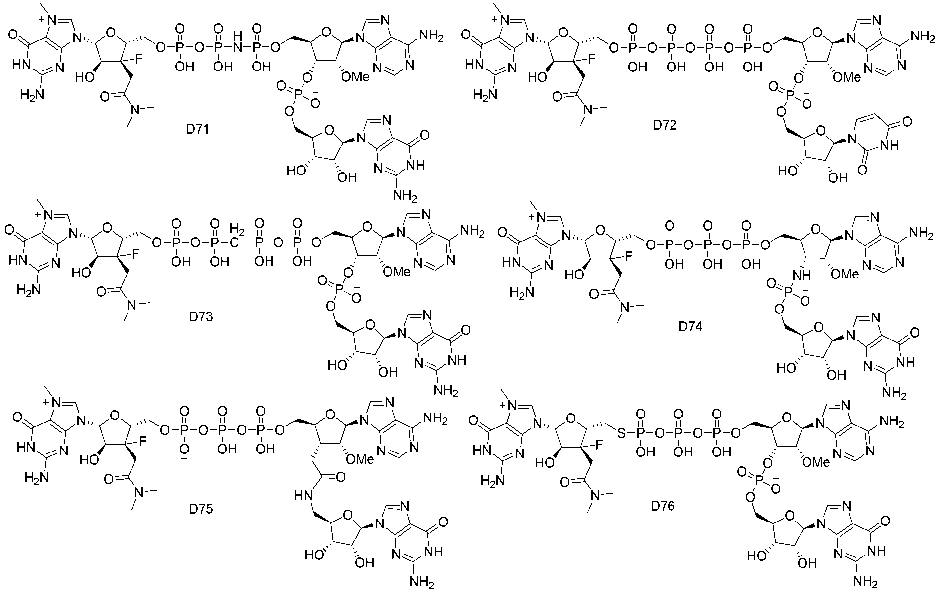

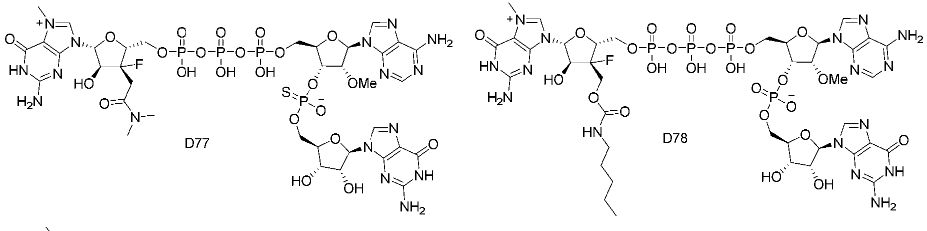

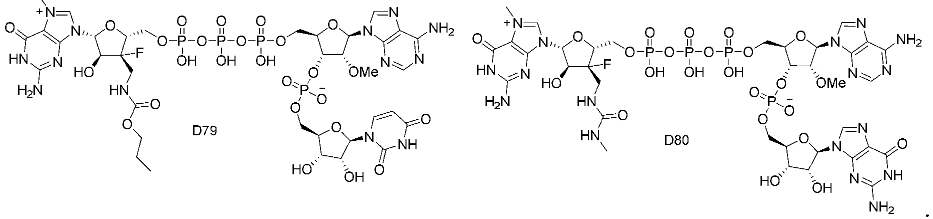

36. A compound for RNA capping, or a stereoisomer, pharmaceutically acceptable salt or solvate thereof, wherein the compound is represented by any one of the following structures:

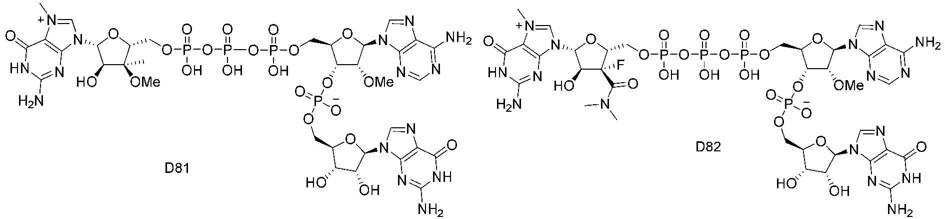

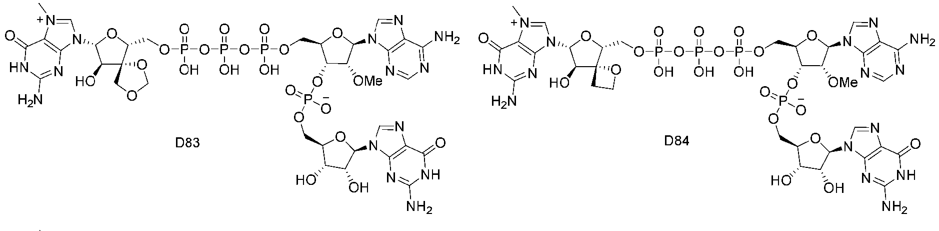

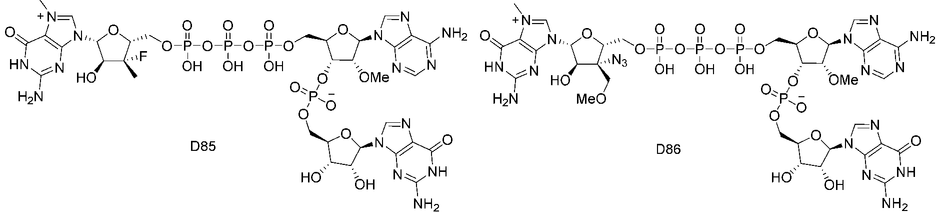

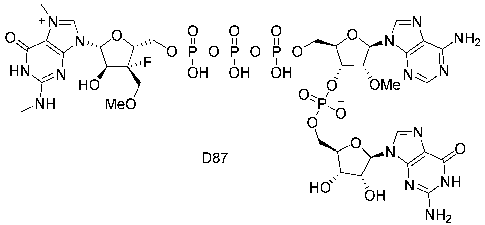

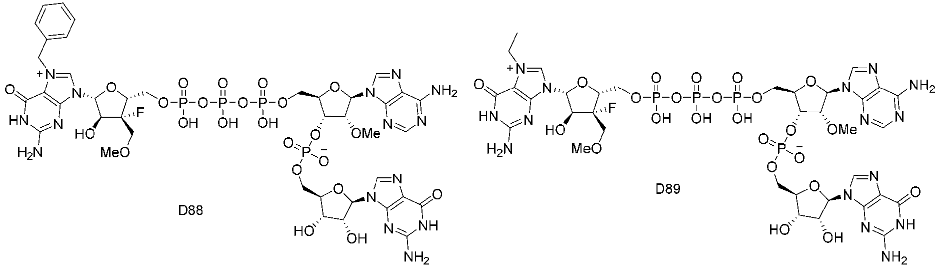

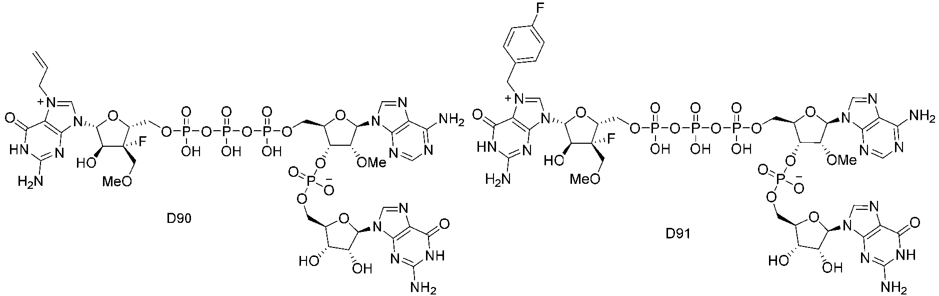

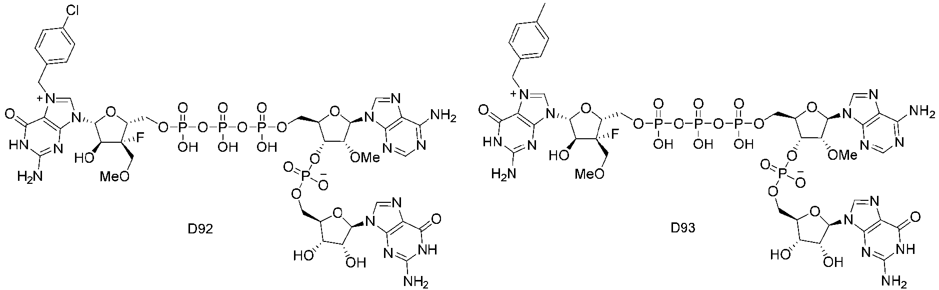

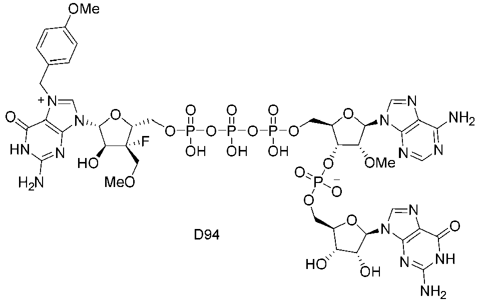

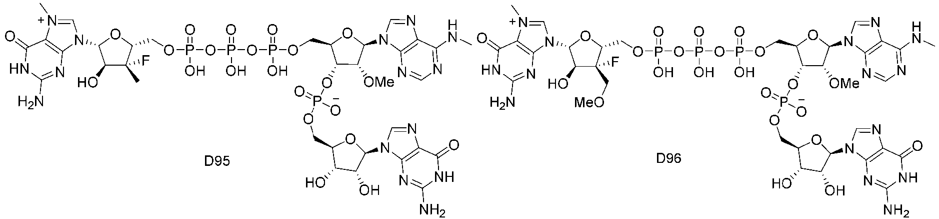

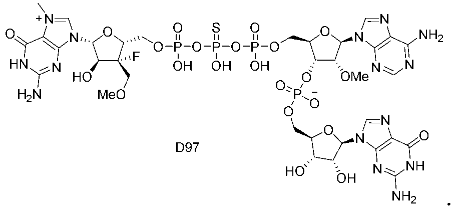

37. Use of the compound according to any one of claims 1-24 or 25-36 as an *in vitro* co-transcriptional RNA capping reagent.

38. An RNA molecule comprising the compound according to any one of claims 1-24 or 25-36 as a cap structure or a cap structure fragment.

39. A pharmaceutical composition comprising the RNA molecule according to claim 38 and a pharmaceutically acceptable carrier.

40. A method for synthesizing an RNA molecule, comprising the following step:
co-incubating the compound according to any one of claims 1-24 or 25-36 with a polynucleotide template for template transcription.

41. A capped RNA transcription reaction system, comprising: a polynucleotide template, the compound according to any one of claims 1-24 or 25-36, NTPs and an RNA polymerase.
